# EUROPEAN PATENT APPLICATION

(11) **EP 4 840 468 A1**
(43) Date of publication of application: **18.11.2026**
(21) Application number: 25738784.5
(22) Date of filing: 08.01.2025
(51) Int. Cl.: A61K 47/20, A61K 9/10, A61K 9/127, A61K 31/7088, A61K 45/00, A61K 48/00

(54) **FROZEN COMPOSITION CONTAINING NUCLEIC ACID-ENCAPSULATED LIPID NANOPARTICLES**

(30) Priority: 12.01.2024 JP 2024003329
(71) Applicant: NOF Corporation, Shibuya-ku Tokyo 150-6019 (JP); Tohoku University, Sendai-shi, Miyagi 980-8577 (JP)
(72) Inventor: AKITA, Hidetaka, Sendai-shi, Miyagi 980-8577 (JP); TANAKA, Hiroki, Sendai-shi, Miyagi 980-8577 (JP); NAKABAYASHI, Tomoya, Sendai-shi, Miyagi 980-8577 (JP); UENO, Sho, Kawasaki-shi, Kanagawa 210-0865 (JP); KITAZAWA, Yui, Kawasaki-shi, Kanagawa 210-0865 (JP); KOIKE, Ayano, Kawasaki-shi, Kanagawa 210-0865 (JP); NAKAI, Yuta, Kawasaki-shi, Kanagawa 210-0865 (JP); TANGE, Kota, Kawasaki-shi, Kanagawa 210-0865 (JP)
(74) Representative: Weickmann & Weickmann PartmbB
(86) International application number: PCT/JP2025/000384
(87) International publication number: WO 2025/150516

(57) **Abstract**

The present invention provides a frozen composition containing nucleic acid-encapsulating lipid nanoparticles, wherein the nucleic acid-encapsulating lipid nanoparticles contain an ionic lipid represented by the formula (1), and a molar ratio of amino groups of the ionic lipid represented by the formula (1)/phosphate groups of the nucleic acid is 7 or more and 250 or less (symbols in the formula (1) are as defined in the DESCRIPTION).

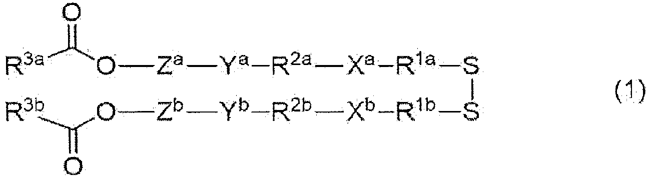

## Description

### [Technical Field]

The present invention relates to a frozen composition containing nucleic acid-encapsulating lipid nanoparticles.

### [Background Art]

For practicalization of nucleic acid therapy using oligonucleic acids such as siRNA, and gene therapy using mRNA, pDNA, or the like, an effective and safe nucleic acid delivery carrier is demanded (see Non Patent Literature 1). While virus vectors are nucleic acid delivery carriers with good expression efficiency, the development of non-viral nucleic acid delivery carriers that can be used more safely is ongoing.

Lipid nanoparticles are one type of nucleic acid delivery carrier. Important parameters to consider when using lipid nanoparticles as nucleic acid delivery carriers include the formation of lipid nanoparticles, storage stability of lipid nanoparticles, constituent components of lipid nanoparticles, physical properties of lipid nanoparticles, nucleic acid encapsulation efficiency, and toxicity of lipid nanoparticles.

Ionic lipids play a major role in the toxicity of nucleic acid-encapsulating lipid nanoparticles, and it is known that toxicity increases as the ionic lipid content (amino group content) becomes higher. The molar ratio of amino groups of ionic lipid/phosphate groups of nucleic acids (sometimes referred to as the "N/P ratio" in the present specification) is used as an indicator of the ionic lipid content in nucleic acid-encapsulating lipid nanoparticles. The N/P ratio is generally 3 to 6 (see Non Patent Literature 2). However, a higher ionic lipid content in nucleic acid-encapsulating lipid nanoparticles is desirable to enhance nucleic acid delivery efficiency (see Non Patent Literature 3).

### [Citation List]

### [Patent Literature]

[Patent Literature 1]
WO 2017/218704

### [Non Patent Literature]

[Non Patent Literature 1]
   Advanced Drug Delivery Reviews 154-155 (2020) 37-63
[Non Patent Literature 2]
   International Journal of Pharmaceutics 601 (2021) 120586
[Non Patent Literature 3]
   ACS Biomater Sci Eng. 2015 Sep 14; 1(9): 834-844

### [Summary of Invention]

### [Technical Problem]

From the viewpoint of toxicity, a low N/P ratio is desirable, but from the viewpoint of nucleic acid delivery efficiency, a high N/P ratio is desirable, and these are in a contradictory relationship. While various companies are working to resolve this dilemma, considering use as a pharmaceutical product, low toxicity is prioritized, and therefore, nucleic acid-encapsulating lipid nanoparticles with a low N/P ratio are being developed.

As mentioned above, nucleic acid-encapsulating lipid nanoparticles with a high N/P ratio become highly toxic due to the increased amount of amino groups. However, from the viewpoint of enhancing gene expression activity, a high N/P ratio is desirable. Therefore, producing nucleic acid-encapsulating lipid nanoparticles with a high N/P ratio and suppressed toxicity is the best way to solve the above-mentioned problem.

Furthermore, as one method for improving the stability of preparations of nucleic acid-encapsulating lipid nanoparticles, attempts have been made to freeze-dry a preparations of nucleic acid-encapsulating lipid nanoparticles and reconstitute the preparation by hydration at the time of use (see, for example, Patent Literature 1). When freeze-drying a preparation of nucleic acid-encapsulating lipid nanoparticles, it is required that the physical properties of the nucleic acid-encapsulating lipid nanoparticles remain unchanged and the gene delivery efficiency does not decrease even after freeze-drying and hydration process after storage.

Another form for enhancing the stability of preparations of nucleic acid-encapsulating lipid nanoparticle is to freeze and store nucleic acid-encapsulating lipid nanoparticles. When freezing a preparation of nucleic acid-encapsulating lipid nanoparticles, it is required that the physical properties of the nucleic acid-encapsulating lipid nanoparticles remain unchanged and the gene expression activity does not decrease even after a thawing process following freezing and storage. Furthermore, since the physical properties of nucleic acid-encapsulating lipid nanoparticles differ significantly depending on the ratio of ionic lipids constituting the nucleic acid-encapsulating lipid nanoparticles and other constituent components, it is necessary to consider the freezing conditions for each ionic lipid.

In view of the above-mentioned circumstances, the present invention aims to provide a frozen composition containing nucleic acid-encapsulating lipid nanoparticles that exhibits low toxicity and high gene expression activity after freezing and thawing, despite having a high N/P ratio.

### [Solution to Problem]

The toxicity of nucleic acid-encapsulating lipid nanoparticles increases as the number of amino groups increases. Therefore, the N/P ratio of typical nucleic acid-encapsulating lipid nanoparticles is generally low and around 5. However, a high N/P ratio is desirable for enhancing gene expression activity. In view of this situation, the present inventors have conducted intensive studies and found that, by using an ionic lipid represented by the following formula (1), a frozen composition containing nucleic acid-encapsulating lipid nanoparticles can be obtained that exhibits low toxicity and high gene expression activity after freezing and thawing, even though it has a high N/P ratio. The present invention based on this finding is as follows.
[1] A frozen composition comprising nucleic acid-encapsulating lipid nanoparticles, wherein
   the nucleic acid-encapsulating lipid nanoparticles comprise an ionic lipid represented by the formula (1): 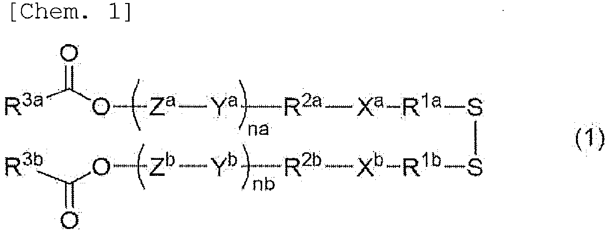 wherein, in the formula (1), R^{1a} and R^{1b} are each independently an alkylene group having 1 to 6 carbon atoms,
   X^{a} and X^{b} are each independently an acyclic alkyl tertiary amino group having 1 to 6 carbon atoms and 1 tertiary amino group, or a cyclic alkylene tertiary amino group having 2 to 5 carbon atoms and 1 to 2 tertiary amino groups,
   R^{2a} and R^{2b} are each independently an alkylene group having 1 to 8 carbon atoms or an oxydialkylene group having 2 to 8 carbon atoms,
   Y^{a} and Y^{b} are each independently an ester bond, an amide bond, a carbamate bond, an ether bond, or a urea bond,
   Z^{a} and Z^{b} are each independently a divalent group derived from an aromatic compound having 3 to 16 carbon atoms and at least one aromatic ring, and optionally having a hetero atom,
   na and nb are each independently 0 or 1, and
   R^{3a} and R^{3b} are each independently
      (i) a monovalent group having 10 to 50 carbon atoms, one carbonyl group, and at least one unsaturated bond selected from the group consisting of an olefinic carbon-carbon double bond and a carbon-carbon triple bond (excluding a monovalent group containing a residue of a liposoluble vitamin having a hydroxy group and a residue of a sterol derivative having a hydroxy group),
      (ii) a monovalent group having 10 to 50 carbon atoms and at least two carbonyl groups (excluding a monovalent group containing a residue of a liposoluble vitamin having a hydroxy group and a residue of a sterol derivative having a hydroxy group),
      (iii) a monovalent group represented by the formula (2):

         *-R⁴-X¹-R⁵ (2)

         wherein, in the formula (2), * is a bonding position,
         R⁴ is an alkylene group having 1 to 10 carbon atoms,
         X¹ is a carbamate bond, a carbonate bond, or an amide bond, and
         R⁵ is an alkyl group having 1 to 25 carbon atoms, and R⁵ is optionally substituted by a substituent selected from the group consisting of a halogen atom and a hydroxy group,
      (iv) a monovalent group represented by the formula (3):

         *-R⁶-CO-O-R⁷ (3)

         wherein, in the formula (3), * is a bonding position,
         R⁶ is an alkylene group having 1 to 10 carbon atoms, and
         R⁷ is an alkyl group having 1 to 25 carbon atoms and substituted by at least one halogen atom,
      (v) a monovalent group represented by the formula (4): 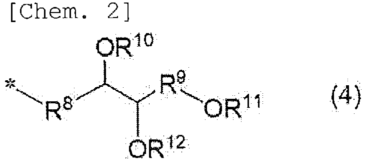 wherein, in the formula (4), * is a bonding position,
         R⁸ and R⁹ are each independently an alkylene group having 1 to 10 carbon atoms, an alkenediyl group having 2 to 10 carbon atoms, or an alkynediyl group having 2 to 10 carbon atoms,
         R¹⁰ to R¹² are each independently a hydrogen atom, a benzyl group, or a *-Si (R¹³) (R¹⁴) (R¹⁵) group (wherein * is a bonding position, and R¹³ to R¹⁵ are each independently an alkyl group having 1 to 4 carbon atoms or a phenyl group),
      (vi) a monovalent group represented by the formula (5): 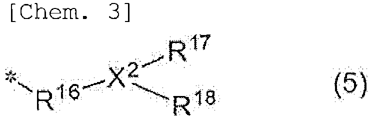
         wherein, in the formula (5), * is a bonding position,
            X² is a nitrogen atom or a trivalent group represented by the formula (6): 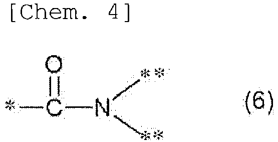
         wherein, in the formula (6), * is a bonding position with R¹⁶, and
            ** is a bonding position with R¹⁷ or R¹⁸,
            when X² is a nitrogen atom, R¹⁶ is an alkylene group having 1 to 10 carbon atoms, an alkenediyl group having 2 to 10 carbon atoms, or an alkynediyl group having 2 to 10 carbon atoms, and R¹⁶ is optionally substituted by a substituent selected from the group consisting of a halogen atom and a hydroxy group,
            when X² is a trivalent group represented by the formula (6), R¹⁶ is an alkylene group having 1 to 10 carbon atoms, and R¹⁶ is optionally substituted by a substituent selected from the group consisting of a halogen atom and a hydroxy group,
            when X² is a nitrogen atom, R¹⁷ and R¹⁸ are each independently an alkyl group having 1 to 10 carbon atoms, an alkenyl group having 2 to 10 carbon atoms, or an alkynyl group having 2 to 10 carbon atoms, and R¹⁷ and R¹⁸ are each independently optionally substituted by a substituent selected from the group consisting of a halogen atom and a hydroxy group, and
            when X² is a trivalent group represented by the formula (6), R¹⁷ and R¹⁸ are each independently an alkyl group having 1 to 10 carbon atoms, and R¹⁷ and R¹⁸ are each independently optionally substituted by a substituent selected from the group consisting of a halogen atom and a hydroxy group,
      (vii) a monovalent group represented by the formula (7): 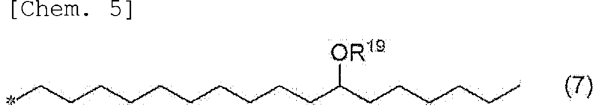 wherein, in the formula (7), * is a bonding position, and
         R¹⁹ is a hydrogen atom, a benzyl group, or a *-Si (R¹³) (R¹⁴) (R¹⁵) group (wherein * is a bonding position, and R¹³ to R¹⁵ are each independently an alkyl group having 1 to 4 carbon atoms or a phenyl group), or a *-CO-R²⁰ group (wherein * is a bonding position, and R²⁰ is an alkyl group having 1 to 9 carbon atoms),
      (viii) a monovalent group represented by the formula (8): 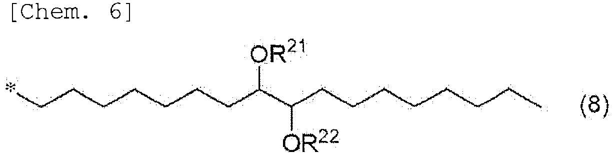 wherein, in the formula (8), * is a bonding position, and
         R²¹ and R²² are each independently a hydrogen atom, a benzyl group, or a *-Si (R¹³) (R¹⁴) (R¹⁵) group (wherein * is a bonding position, and R¹³ to R¹⁵ are each independently an alkyl group having 1 to 4 carbon atoms or a phenyl group),
      (ix) a monovalent group represented by the formula (9): 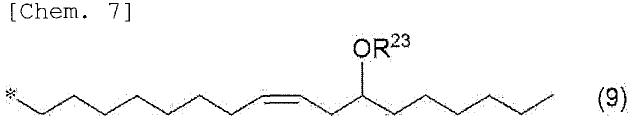 wherein, in the formula (9), * is a bonding position, and
         R²³ is a hydrogen atom, a benzyl group, or a *-Si (R¹³) (R¹⁴) (R¹⁵) group (wherein * is a bonding position, and R¹³ to R¹⁵ are each independently an alkyl group having 1 to 4 carbon atoms or a phenyl group),
      (x) a monovalent group represented by the formula (10): 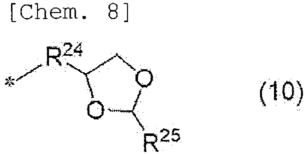 wherein, in the formula (10), * is a bonding position,
         R²⁴ is an alkylene group having 1 to 10 carbon atoms, an alkenediyl group having 2 to 10 carbon atoms, or an alkynediyl group having 2 to 10 carbon atoms, and
         R²⁵ is an alkyl group having 1 to 30 carbon atoms, an alkenyl group having 2 to 30 carbon atoms, or an alkynyl group having 2 to 30 carbon atoms,
      (xi) a monovalent group represented by the formula (11): 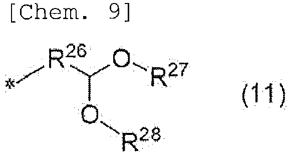 wherein, in the formula (11), * is a bonding position, and
         R²⁶ is an alkylene group having 1 to 10 carbon atoms, an alkenediyl group having 2 to 10 carbon atoms, or an alkynediyl group having 2 to 10 carbon atoms, and
         R²⁷ and R²⁸ are each independently an alkyl group having 1 to 10 carbon atoms, an alkenyl group having 2 to 10 carbon atoms, or an alkynyl group having 2 to 10 carbon atoms, or
      (xii) a monovalent group represented by the formula (12): 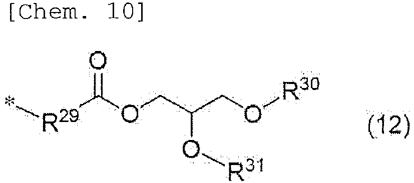 wherein, in the formula (12), * is a bonding position,
         R²⁹ is an alkylene group having 1 to 10 carbon atoms, and
         R³⁰ and R³¹ are each independently an alkyl group having 1 to 10 carbon atoms,
      (xiii) a monovalent group which is an alkyl group having 1 to 30 carbon atoms, an alkenyl group having 2 to 20 carbon atoms, or an alkynyl group having 2 to 20 carbon atoms, in which one ethylene group in the aforementioned alkyl group is optionally replaced by one ester bond, or
      (xiv) an R^{3c}-CO-(CH₂)ₚ- group (wherein R^{3c} is a residue of a liposoluble vitamin having a hydroxy group or a residue of a sterol derivative having a hydroxy group, and p is an integer of 1 to 8), and
   R^{3a} and R^{3b} may be the same or different, and
   a molar ratio of amino groups of the ionic lipid represented by the formula (1)/phosphate groups of the nucleic acid is 7 or more and 250 or less.
[2] The frozen composition of the aforementioned [1], wherein R^{3a} and R^{3b} are each independently
   (i) a monovalent group having 10 to 50 carbon atoms, one carbonyl group, and at least one unsaturated bond selected from the group consisting of an olefinic carbon-carbon double bond and a carbon-carbon triple bond (excluding a monovalent group containing a residue of a liposoluble vitamin having a hydroxy group and a residue of a sterol derivative having a hydroxy group),
   (ii) a monovalent group having 10 to 50 carbon atoms and at least two carbonyl groups (excluding a monovalent group containing a residue of a liposoluble vitamin having a hydroxy group and a residue of a sterol derivative having a hydroxy group),
   (iii) a monovalent group represented by the aforementioned formula (2),
   (vii) a monovalent group represented by the aforementioned formula (7),
   (ix) a monovalent group represented by the aforementioned formula (9),
   (xiii) a monovalent group which is an alkyl group having 1 to 30 carbon atoms, an alkenyl group having 2 to 20 carbon atoms, or an alkynyl group having 2 to 20 carbon atoms, in which one ethylene group in the aforementioned alkyl group is optionally replaced by one ester bond, or
   (xiv) an R^{3c}-CO-(CH₂)ₚ- group (wherein R^{3c} is a residue of a liposoluble vitamin having a hydroxy group or a residue of a sterol derivative having a hydroxy group, and p is an integer of 1 to 8).
[3] The frozen composition of the aforementioned [1] or [2], wherein the nucleic acid is RNA or DNA.
[4] The frozen composition of the aforementioned [1] or [2], wherein the nucleic acid is mRNA.
[5] The frozen composition of any one of the aforementioned [1] to [4], wherein nucleic acid-encapsulating lipid nanoparticles in a suspension obtained by thawing the frozen composition have a particle size of 10 nm or more and 200 nm or less.
[6] The frozen composition of any one of the aforementioned [1] to [5], wherein nucleic acid-encapsulating lipid nanoparticles in a suspension obtained by thawing the frozen composition have a PdI of 0.01 or more and 0.30 or less.
[7] The frozen composition of any one of the aforementioned [1] to [6], wherein nucleic acid-encapsulating lipid nanoparticles in a suspension obtained by thawing the frozen composition have a nucleic acid encapsulation rate of 75% or more and 100% or less.
[8] A method for producing a frozen composition comprising nucleic acid-encapsulating lipid nanoparticles, wherein
   the nucleic acid-encapsulating lipid nanoparticles comprise an ionic lipid represented by the aforementioned formula (1),
   a molar ratio of amino groups of the ionic lipid represented by the formula (1)/phosphate groups of the nucleic acid is 7 or more and 250 or less, and
   the method comprises
   the following step a), step b), step c), and step d),
   the following step a), step b'), and step d), or
   the following step a), step b'), step c), and step d):
      a) a step of mixing an alcohol solution containing the ionic lipid represented by the formula (1), a sterol, and a PEG lipid, and a nucleic acid solution containing an acidic buffer with a pH of 1.0 to 6.5 as a solvent to prepare a suspension containing nucleic acid-encapsulating lipid nanoparticles,
      b) a step of exchanging a dispersion medium of the suspension obtained in step a) with a buffer with a pH of 4.5 to 8.0, which is other than the acidic buffer used in step a), to obtain a suspension containing nucleic acid-encapsulating lipid nanoparticles,
      b') a step of exchanging a dispersion medium of the suspension obtained in step a) with a buffer with a pH of 4.5 to 8.0, which is other than the acidic buffer used in step a), and containing a cryoprotective agent to obtain a suspension containing nucleic acid-encapsulating lipid nanoparticles and the cryoprotective agent,
      c) a step of mixing the suspension obtained in step b) or step b') and a cryoprotective agent to obtain a suspension containing nucleic acid-encapsulating lipid nanoparticles and the cryoprotective agent,
      d) a step of freezing the suspension obtained in step b') or step c) at -10°C or less.
[9] The method of the aforementioned [8], wherein the method comprises step a), step b), step c), and step d), or step a), step b'), and step d), and wherein in step c), the suspension obtained in step b) and a cryoprotective agent is mixed to obtain a suspension containing nucleic acid-encapsulating lipid nanoparticles and the cryoprotective agent.
[10] The method of the aforementioned [8] or [9], wherein a ratio of (a particle size of nucleic acid-encapsulating lipid nanoparticles in a suspension obtained by thawing the frozen composition obtained in step d))/(a particle size of the nucleic acid-encapsulating lipid nanoparticles in the suspension obtained in step b') or step c)) is 0.8 or more and 1.5 or less.
[11] The method of any one of the aforementioned [8] to [10], wherein nucleic acid-encapsulating lipid nanoparticles in a suspension obtained by thawing the frozen composition obtained in step d) have a PdI of 0.01 or more and 0.25 or less.
[12] The method of any one of the aforementioned [8] to [11], wherein a ratio of (a nucleic acid encapsulation rate of nucleic acid-encapsulating lipid nanoparticles in a suspension obtained by thawing the frozen composition obtained in step d))/(a nucleic acid encapsulation rate of the nucleic acid-encapsulating lipid nanoparticles in the suspension obtained in step b') or step c)) is 0.8 or more and 1.0 or less.
[13] A method for producing a pharmaceutical product composition, comprising the method of any one of the aforementioned [8] to [12].
[14] A method for introducing a nucleic acid in a suspension into a cell, comprising a step of contacting in vitro the cell with a suspension obtained by thawing the frozen composition of any one of the aforementioned [1] to [7] or a frozen composition produced by the method of any one of the aforementioned [8] to [12], or a suspension obtained by exchanging a dispersion medium of the aforementioned suspension with a different dispersion medium.
[15] A method for introducing a nucleic acid in a suspension into a biological target cell of a living organism, comprising a step of administering, to the living organism, a suspension obtained by thawing the frozen composition of any one of the aforementioned [1] to [7] or a frozen composition produced by the method of any one of the aforementioned [8] to [12], or a suspension obtained by exchanging a dispersion medium of the aforementioned suspension with a different dispersion medium.

### [Advantageous Effects of Invention]

Even though the frozen composition containing nucleic acid-encapsulating lipid nanoparticles of the present invention exhibits a high N/P ratio compared to conventional technologies, the physical properties and gene expression activity of the nucleic acid-encapsulating lipid nanoparticles do not change greatly before and after freezing. Furthermore, even though the nucleic acid-encapsulating lipid nanoparticles contained in the frozen composition of the present invention have a high N/P ratio, they can simultaneously achieve high gene expression activity and low toxicity after freezing and thawing. Therefore, the frozen composition containing nucleic acid-encapsulating lipid nanoparticles of the present invention is advantageous for gene transfer into cells in vitro or in vivo, and is also useful as a pharmaceutical composition.

### [Brief Description of Drawings]

[Fig. 1]

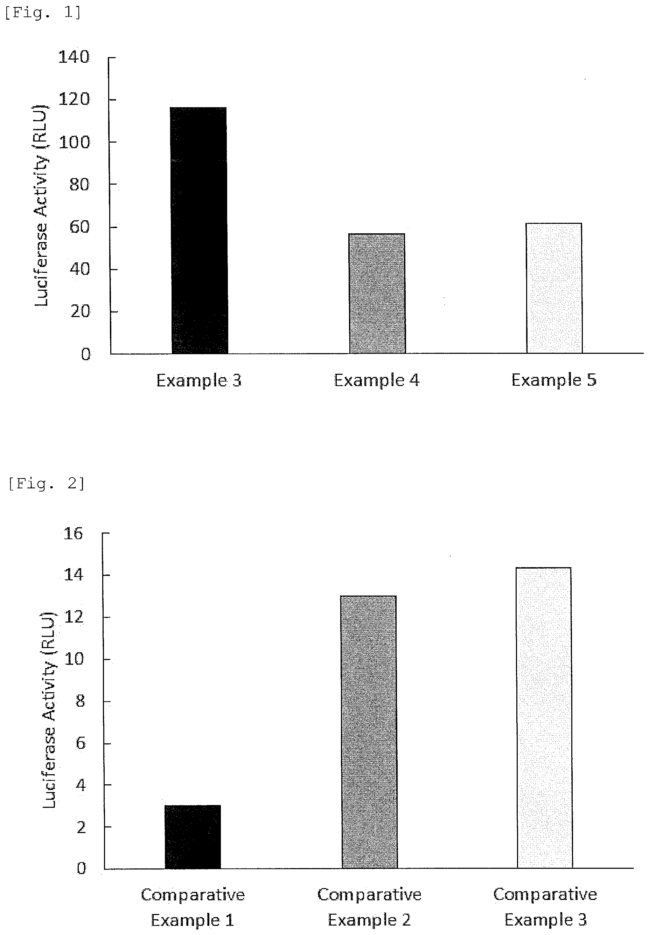

   Fig. 1 is a diagram showing the gene expression activity of nucleic acid-encapsulating lipid nanoparticles in suspensions obtained by thawing the frozen compositions of Example 3 (N/P ratio: 60), Example 4 (N/P ratio: 15), and Example 5 (N/P ratio: 8).
[Fig. 2]
   Fig. 2 is a diagram showing the gene expression activity of nucleic acid-encapsulating lipid nanoparticles in suspensions obtained by thawing the frozen compositions of Comparative Example 1 (N/P ratio: 62), Comparative Example 2 (N/P ratio: 31), and Comparative Example 3 (N/P ratio: 6).
[Fig. 3]

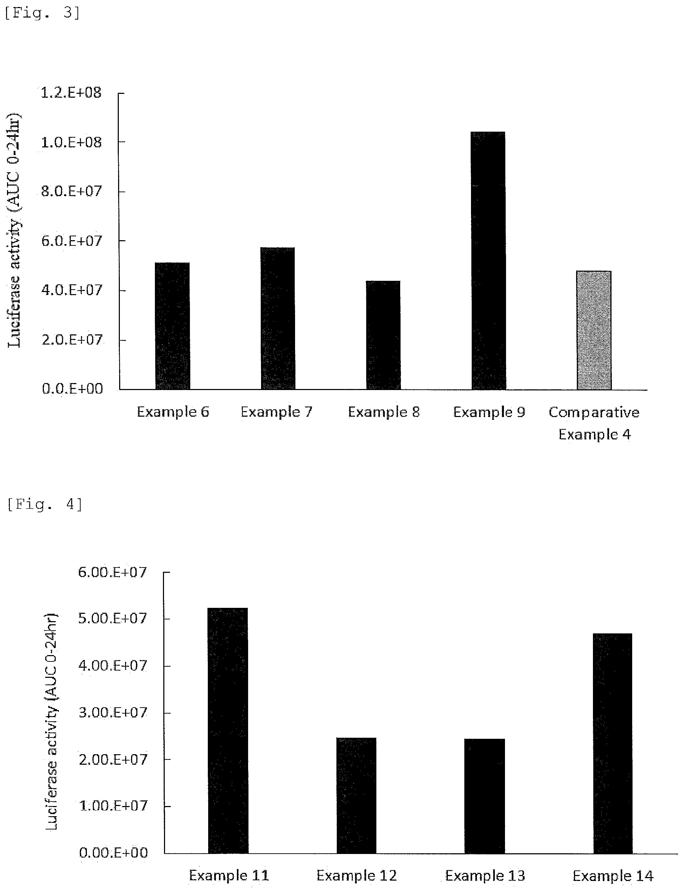

   Fig. 3 is a diagram showing the gene expression activity of nucleic acid-encapsulating lipid nanoparticles in suspensions obtained by thawing the frozen compositions of Example 6 (buffer in step a): acidic malic acid buffer, buffer in step b): MES buffer), Example 7 (buffer in step a): acidic citrate buffer, buffer in step b): MES buffer), Example 8 (buffer in step a): acidic citrate buffer, buffer in step b): Tris/maleate buffer), and Example 9 (buffer in step a): acidic citrate buffer, buffer in step b'): Tris/NaCl/sucrose buffer), and the gene expression activity of nucleic acid-encapsulating lipid nanoparticles in the suspension of Comparative Example 4 (buffer in step a): acidic citrate buffer, buffer in step b): Tris-buffered saline).
[Fig. 4]
   Fig. 4 is a diagram showing the gene expression activity of nucleic acid-encapsulating lipid nanoparticles in suspensions obtained by thawing the frozen compositions of Example 11 (ionic lipid: SS-OP in Table 2-1), Example 12 (ionic lipid: SS-EC in Table 2-3), Example 13 (ionic lipid: SS-OP in Table 2-1 and SS-EC in Table 2-3), and Example 14 (ionic lipid: compound 13 in Table 1-3).
[Fig. 5]

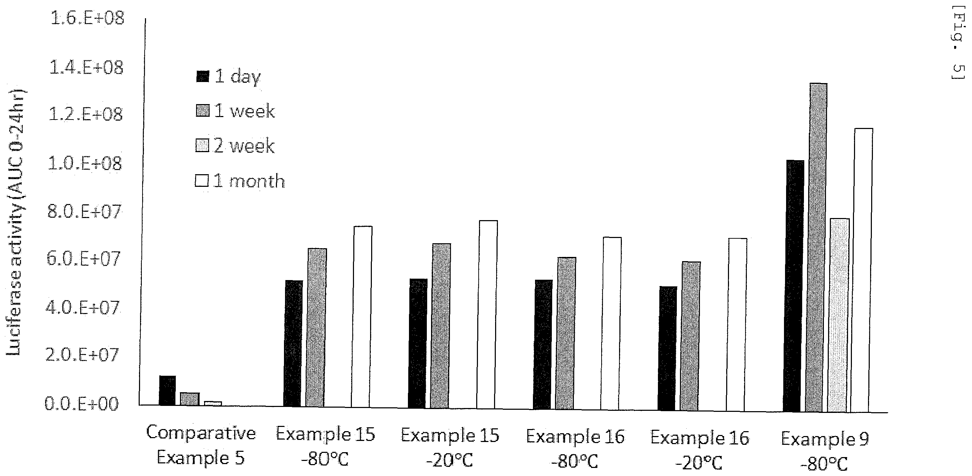

   Fig. 5 is a diagram showing the gene expression activity of nucleic acid-encapsulating lipid nanoparticles in suspensions obtained by storing for a given period of time and then thawing the frozen compositions of Example 9 (freezing temperature: -80°C, storage temperature: -20°C), Example 15 (freezing temperature: -20°C or -80°C, storage temperature: - 20°C), and Example 16 (freezing temperature: -20°C or -80°C, storage temperature: -20°C), and the gene expression activity of nucleic acid-encapsulating lipid nanoparticles in the suspension of Comparative Example 5 (storage temperature: 4°C).
[Fig. 6]

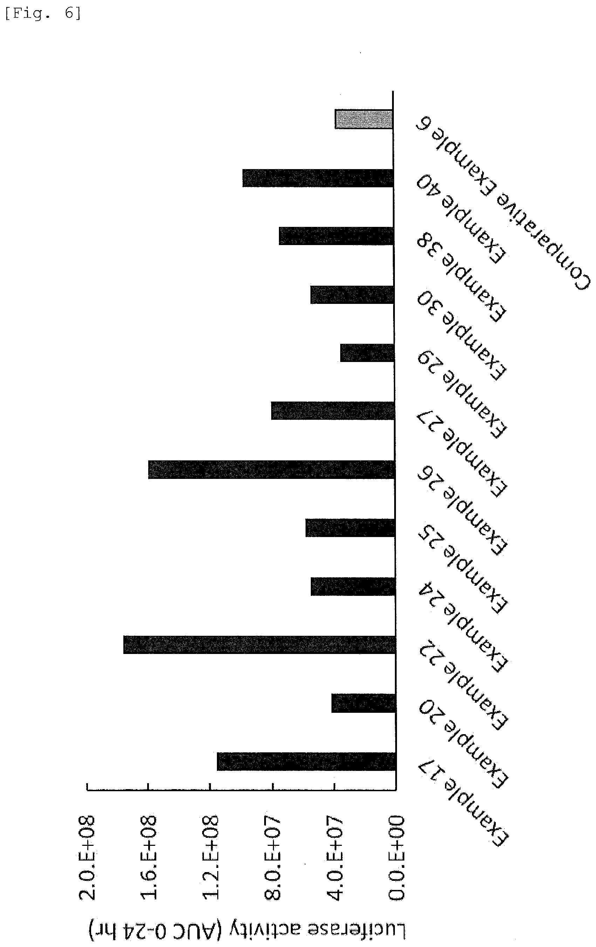

   Fig. 6 is a diagram showing the gene expression activity of nucleic acid-encapsulating lipid nanoparticles in suspensions obtained by thawing the frozen compositions of Example 17 (ionic lipid: compound 7 in Table 1-2), Example 20 (ionic lipid: compound 11 in Table 1-2), Example 22 (ionic lipid: compound 23 in Table 1-4), Example 24 (ionic lipid: compound 34 in Table 1-6), Example 25 (ionic lipid: compound 37 in Table 1-6), Example 26 (ionic lipid: compound 58 in Table 1-10), Example 27 (ionic lipid: compound 155 in Table 1-29), Example 29 (ionic lipid: compound 161 in Table 1-31), Example 30 (ionic lipid: compound 162 in Table 1-31), Example 38 (ionic lipid: compound 163 in Table 1-31), and Example 40 (ionic lipid: compound 196 in Table 1-38), and the gene expression activity of nucleic acid-encapsulating lipid nanoparticles in the suspension of Comparative Example 6 (ionic lipid: D-Lin-MC3-DMA (CAS number: 1224606-06-7)).
[Fig. 7]

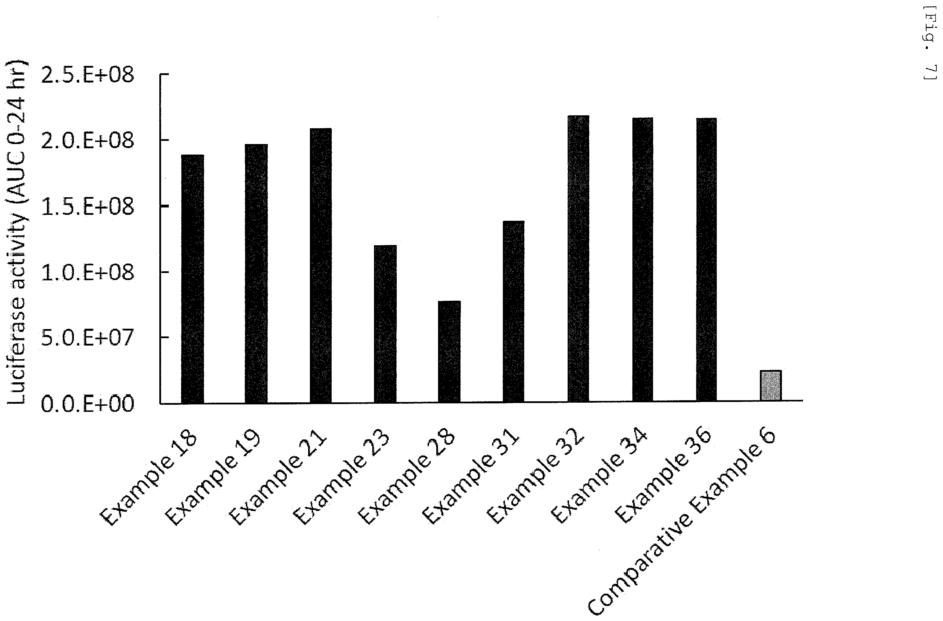

   Fig. 7 is a diagram showing the gene expression activity of nucleic acid-encapsulating lipid nanoparticles in suspensions obtained by thawing the frozen compositions of Example 18 (ionic lipid: compound 9 in Table 1-2), Example 19 (ionic lipid: compound 10 in Table 1-2), Example 21 (ionic lipid: compound 12 in Table 1-2), Example 23 (ionic lipid: compound 33 in Table 1-6), Example 28 (ionic lipid: compound 159 in Table 1-31), Example 31 (ionic lipid: compound 163 in Table 1-31), Example 32 (ionic lipid: compound 164 in Table 1-32), Example 34 (ionic lipid: compound 166 in Table 1-32), and Example 36 (ionic lipid: compound 168 in Table 1-32), and the gene expression activity of nucleic acid-encapsulating lipid nanoparticles in the suspension of Comparative Example 6 (ionic lipid: D-Lin-MC3-DMA (CAS number: 1224606-06-7)).
[Fig. 8]

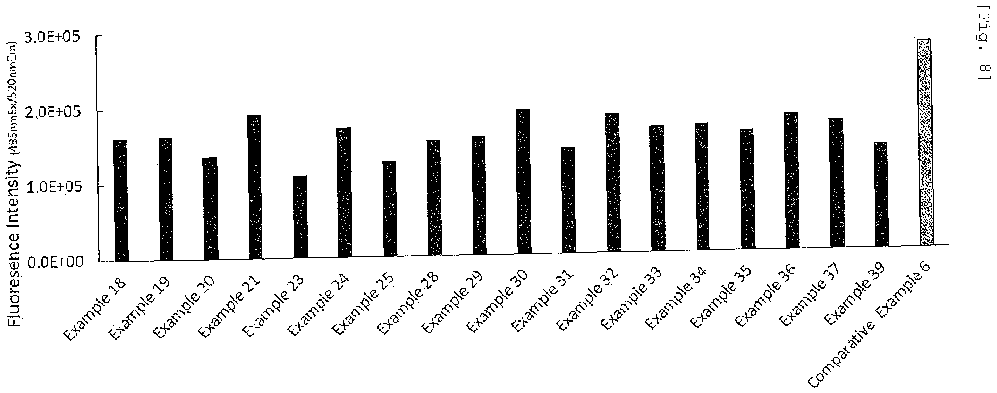

   Fig. 8 is a diagram showing the toxicity (dead cell fluorescence) of nucleic acid-encapsulating lipid nanoparticles in suspensions obtained by thawing the frozen compositions of Example 18 (ionic lipid: compound 9 in Table 1-2, N/P ratio: 16), Example 19 (ionic lipid: compound 10 in Table 1-2, N/P ratio: 16), Example 20 (ionic lipid: compound 11 in Table 1-2, N/P ratio: 64), Example 21 (ionic lipid: compound 12 in Table 1-2, N/P ratio: 16), Example 23 (ionic lipid: compound 33 in Table 1-6, N/P ratio: 16), Example 24 (ionic lipid: compound 34 in Table 1-6, N/P ratio: 64), Example 25 (ionic lipid: compound 37 in Table 1-7, N/P ratio: 64), Example 28 (ionic lipid: compound 159 in Table 1-31, N/P ratio: 16), Example 29 (ionic lipid: compound 161 in Table 1-31, N/P ratio: 16), Example 30 (ionic lipid: compound 162 in Table 1-31, N/P ratio: 64), Example 31 (ionic lipid: compound 163 in Table 1-31, N/P ratio: 16), Example 32 (ionic lipid: compound 164 in Table 1-32, N/P ratio: 16), Example 33 (ionic lipid: compound 165 in Table 1-32, N/P ratio: 16), Example 34 (ionic lipid: compound 166 in Table 1-32, N/P ratio: 16), Example 35 (ionic lipid: compound 167 in Table 1-32, N/P ratio: 16), Example 36 (ionic lipid: compound 168 in Table 1-32, N/P ratio: 16), Example 37 (ionic lipid: compound 169 in Table 1-32, N/P ratio: 16), and Example 39 (ionic lipid: compound 197 in Table 1-38, N/P ratio: 16), and the toxicity (dead cell fluorescence) of nucleic acid-encapsulating lipid nanoparticles in the suspension of Comparative Example 6 (ionic lipid: D-Lin-MC3-DMA (CAS number: 1224606-06-7), N/P ratio: 6).
[Fig. 9]

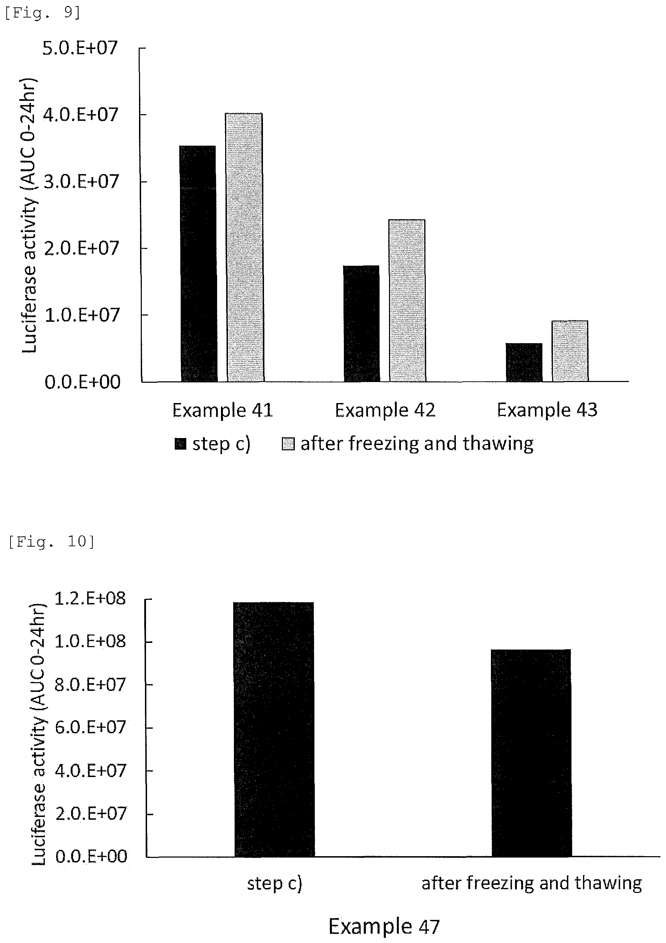

   Fig. 9 is a diagram showing the gene expression activity of the nucleic acid-encapsulating lipid nanoparticles in the suspensions obtained in steps c) of Example 41 (N/P ratio: 16), Example 42 (N/P ratio: 12), and Example 43 (N/P ratio: 8) ("step c)" in Fig. 9), and the gene expression activity of the nucleic acid-encapsulating lipid nanoparticles in the suspensions obtained by thawing the frozen compositions ("after freezing and thawing" in Fig. 9).
[Fig. 10]
   Fig. 10 is a diagram showing the gene expression activity of the nucleic acid-encapsulating lipid nanoparticles in the suspension obtained in step c) of Example 47 (phospholipid: DOPE) ("step c)" in Fig. 10), and the gene expression activity of the nucleic acid-encapsulating lipid nanoparticles in the suspension obtained by thawing the frozen composition ("after freezing and thawing" in Fig. 10).
[Fig. 11]

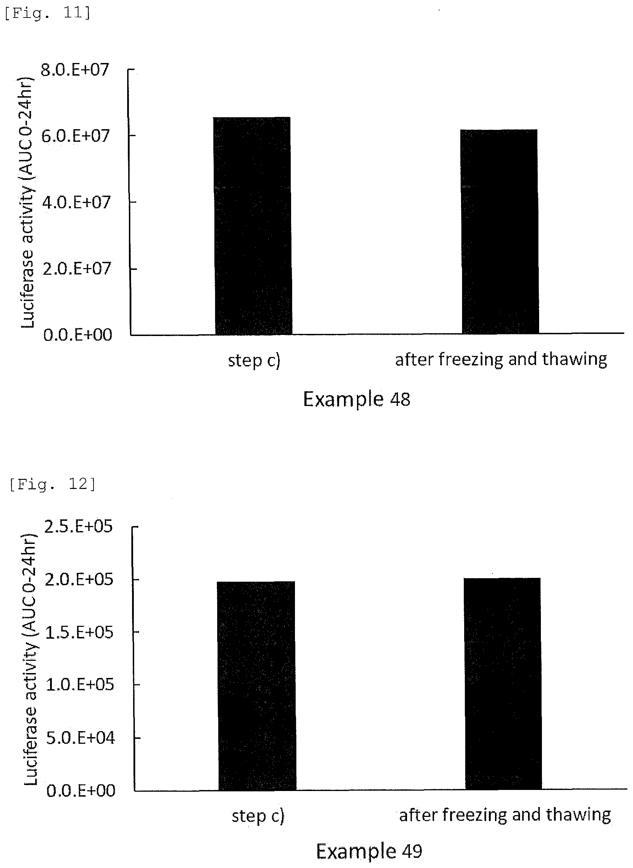

   Fig. 11 is a diagram showing the gene expression activity of the nucleic acid-encapsulating lipid nanoparticles in the suspension obtained in step c) of Example 48 (phospholipid: POPE) ("step c)" in Fig. 11), and the gene expression activity of the nucleic acid-encapsulating lipid nanoparticles in the suspension obtained by thawing the frozen composition ("after freezing and thawing" in Fig. 11).
[Fig. 12]
   Fig. 12 is a diagram showing the gene expression activity of the nucleic acid-encapsulating lipid nanoparticles in the suspension obtained in step c) of Example 49 (phospholipid: DSPC) ("step c)" in Fig. 12), and the gene expression activity of the nucleic acid-encapsulating lipid nanoparticles in the suspension obtained by thawing the frozen composition ("after freezing and thawing" in Fig. 12).
[Fig. 13]

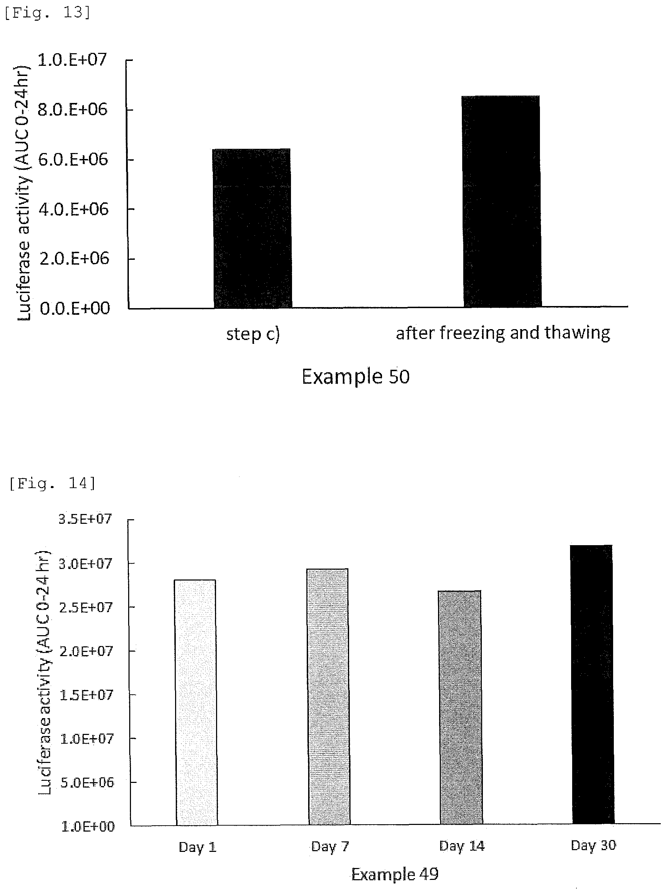

   Fig. 13 is a diagram showing the gene expression activity of the nucleic acid-encapsulating lipid nanoparticles in the suspension obtained in step c) of Example 50 (phospholipid: POPC) ("step c)" in Fig. 13), and the gene expression activity of the nucleic acid-encapsulating lipid nanoparticles in the suspension obtained by thawing the frozen composition ("after freezing and thawing" in Fig. 13).
[Fig. 14]
   Fig. 14 is a diagram showing the gene expression activity of the nucleic acid-encapsulating lipid nanoparticles in the suspensions obtained by storing for a given period of time and then thawing the frozen compositions of Example 49 (freezing temperature: -80°C, storage temperature: -80°C).
[Fig. 15]

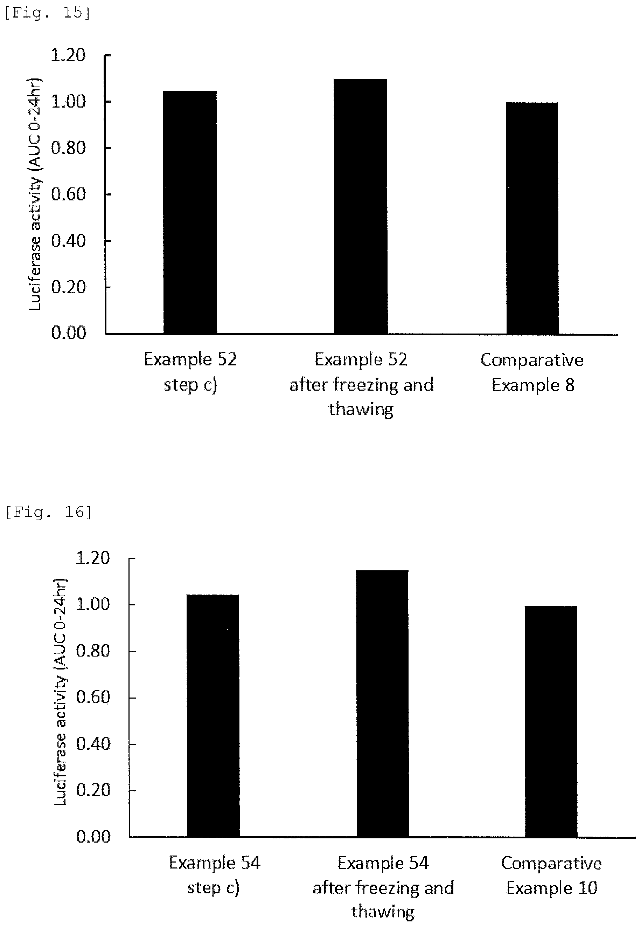

   Fig. 15 is a diagram showing the gene expression activity of the nucleic acid-encapsulating lipid nanoparticles in the suspension obtained in step c) of Example 52 (N/P ratio: 12) ("step c)" in Fig. 15), the gene expression activity of the nucleic acid-encapsulating lipid nanoparticles in the suspension obtained by thawing the frozen composition ("after freezing and thawing" in Fig. 15), and the gene expression activity of nucleic acid-encapsulating lipid nanoparticles in the suspension of Comparative Example 8 (N/P ratio: 12). Fig. 15 shows the relative values where the gene expression activity (luciferase activity) of Comparative Example 8 is 1.
[Fig. 16] Fig. 16 is a diagram showing the gene expression activity of the nucleic acid-encapsulating lipid nanoparticles in the suspension obtained in step c) of Example 54 (N/P ratio: 18) ("step c)" in Fig. 16), the gene expression activity of the nucleic acid-encapsulating lipid nanoparticles in the suspension obtained by thawing the frozen composition ("after freezing and thawing" in Fig. 16), and the gene expression activity of nucleic acid-encapsulating lipid nanoparticles in the suspension of Comparative Example 10 (N/P ratio: 18). Fig. 16 shows the relative values where the gene expression activity (luciferase activity) of Comparative Example 10 is 1.

### [Description of Embodiments]

While the embodiments of the present invention are described in the following, the present invention is not limited thereto. The descriptions in the present specification can be combined with each other unless it is clear that they cannot be combined.

The present invention provides a frozen composition containing nucleic acid-encapsulating lipid nanoparticles, wherein
the nucleic acid-encapsulating lipid nanoparticles contain an ionic lipid represented by the formula (1) (hereinafter sometimes to be abbreviated as "ionic lipid (1)"), and
a molar ratio of amino groups of the ionic lipid represented by the formula (1)/phosphate groups of the nucleic acid (hereinafter sometimes to be abbreviated as "N/P ratio") is 7 or more and 250 or less.

From the viewpoint of the nucleic acid encapsulation efficiency, the N/P ratio is preferably 10 or more, more preferably 13 or more. From the viewpoint of the toxicity, the N/P ratio is preferably 200 or less, more preferably 150 or less, further preferably 100 or less, particularly preferably 75 or less.

From the viewpoint of the nucleic acid encapsulation efficiency and toxicity, in one embodiment of the present invention, the N/P ratio is preferably 7 to 200, more preferably 7 to 150, further preferably 7 to 100, most preferably 7 to 75.

From the viewpoint of the nucleic acid encapsulation efficiency and toxicity, in another embodiment of the present invention, the N/P ratio is preferably 10 to 200, more preferably 13 to 150.

The N/P ratio can be calculated as a rough value from the following formula:
N/P ratio = amount of amino groups of ionic lipid (mol)/(mass of nucleic acid (g)/330). In the above-mentioned formula, "330" is the average molecular weight of deoxynucleoside.

The "lipid nanoparticle" means a particle having a membrane structure wherein the hydrophilic groups of amphiphilic lipid are arranged in the interface, facing the aqueous phase side. The "amphiphilic lipid" means a lipid having both a hydrophilic group exhibiting hydrophilicity and a hydrophobic group exhibiting hydrophobicity. Examples of the amphiphilic lipid include ionic lipid, phospholipid, PEG lipid, and the like.

In the present invention, the "nucleic acid-encapsulating lipid nanoparticle" means a lipid nanoparticle in which a nucleic acid is encapsulated inside the lipid nanoparticle.

The nucleic acid-encapsulating lipid nanoparticles in the frozen composition of the present invention contain ionic lipid (1) as a constituent component of the membrane. The nucleic acid-encapsulating lipid nanoparticles preferably further contain sterol, PEG lipid, and phospholipid.

In the present invention, the "total lipids" means the total amount of lipids. Examples of the lipid include ionic lipid, sterol, PEG lipid, and phospholipid.

### <ionic lipid (1)>

The ionic lipid (1) used in the present invention is a compound represented by the following formula (1). Only one kind of ionic lipid (1) may be used, or two or more kinds thereof may be used in combination.

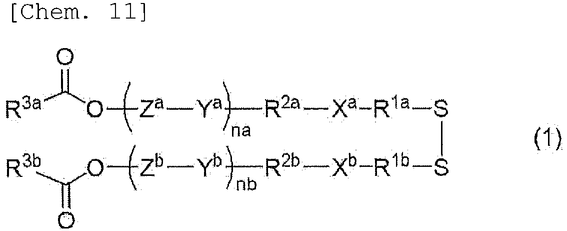

First, alkylene groups and the like included in the formula (1) are described.

In the present specification, the alkylene group may be linear or branched. Examples of the alkylene group include methylene group, ethylene group, trimethylene group (-(CH₂)₃-), propylene group (-CH(CH₃)CH₂-, -CH₂CH(CH₃)-), tetramethylene group (-(CH₂)₄-), butylene group (-CH(C₂H₅)CH₂-, -CH₂CH(C₂H₅)-), pentamethylene group (-(CH₂)₅-), hexamethylene group (-(CH₂)₆-), heptamethylene group (-(CH₂)₇-), octamethylene group (-(CH₂)₈-), nonamethylene group (-(CH₂)₉-), and decamethylene group (-(CH₂)₁₀-) (in the aforementioned formulas, "-" is a single bond).

In the present specification, the "alkenediyl group" refers to a divalent group having a structure obtained by removing two hydrogen atoms from an alkene. In the present specification, the alkenediyl group may be linear or branched. The number of olefinic carbon-carbon double bonds in the alkene or alkenediyl group may be one or two or more. Examples of the alkenediyl group include ethenediyl group, propenediyl group, butenediyl group, pentenediyl group, hexenediyl group, heptenediyl group, octenediyl group, nonenediyl group, and decenediyl group. In the present specification, "compound name + diyl group (e.g., ethenediyl group)" refers to a divalent group having a structure obtained by removing two hydrogen atoms from the aforementioned compound.

In the present specification, the "alkynediyl group" means a divalent group having a structure obtained by removing two hydrogen atoms from an alkyne. In the present specification, the alkynediyl group may be linear or branched. In the present specification, the number of carbon-carbon triple bonds in the alkyne or alkynediyl group may be one or two or more. Examples of the alkynediyl group include ethynediyl group, propynediyl group, butynediyl group, pentynediyl group, hexynediyl group, heptynediyl group, octynediyl group, noninediyl group, and decynediyl group.

In the present specification, the "oxydialkylene group" means a divalent group having a structure in which two alkylene groups are bonded via an oxy group (-O-) (in the aforementioned formulas, "-" is a single bond). The alkylene group in the "oxydialkylene group" is as described above.

In the present specification, the "ester bond" means -COO- or -O-CO- (in the aforementioned formulas, "-" is a single bond).

In the present specification, the "amide bond" means -CO-NH- or -NH-CO- (in the aforementioned formulas, "-" is a single bond) .

In the present specification, the "carbamate bond" means -O-CO-NH- or -NH-CO-O- (in the aforementioned formulas, "-" is a single bond).

In the present specification, the "ether bond" means -O-(in the aforementioned formulas, "-" is a single bond).

In the present specification, "urea bond" means -NH-CO-NH- (in the aforementioned formulas, "-" is a single bond).

In the present specification, "carbonate bond" means -O-CO-O- (in the aforementioned formulas, "-" is a single bond).

In the present specification, the "residue of a liposoluble vitamin having a hydroxy group" means a monovalent group having a structure obtained by removing a hydrogen atom from the hydroxy group of the aforementioned liposoluble vitamin. Examples of the liposoluble vitamin having a hydroxy group include retinol, ergosterol, 7-dehydrocholesterol, calciferol, cholecalciferol, dihydroergocalciferol, dihydrotachysterol, tocopherol, and tocotrienol.

In the present specification, the "sterol derivative having a hydroxy group residue" means a monovalent group having a structure obtained by removing a hydrogen atom from the hydroxy group of the aforementioned sterol derivative. Examples of the sterol derivative having a hydroxy group include cholesterol, cholestanol, stigmasterol, β-sitosterol, lanosterol, and ergosterol.

In the present specification, the alkyl group may be linear or branched. Examples of the alkyl group include methyl group, ethyl group, propyl group, isopropyl group, butyl group, isobutyl group, sec-butyl group, tert-butyl group, a pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, henicosyl group, docosyl group, tricosyl group, tetracosyl group, pentacosyl group, hexacosyl group, heptacosyl group, octacosyl group, nonacosyl group, triacontyl group, hentriacontyl group, dotriacontyl group, tritriacontyl group, tetratriacontyl group, pentatriacontyl group, hexatriacontyl group, tetracontyl group, hentetracontyl group, dotetracontyl group, tritetracontyl group, and tetratetracontyl group.

In the present specification, the alkenyl group may be linear or branched. In the present specification, the number of the olefinic carbon-carbon double bond in the alkenyl group may be only one, or two or more. Examples of the alkenyl group include ethenyl group, propenyl group, butenyl group, pentenyl group, hexenyl group, heptenyl group, octenyl group, nonenyl group, decenyl group, undecenyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, tricosenyl group, tetracosenyl group, pentacosenyl group, hexacosenyl group, heptacosenyl group, octacosenyl group, nonacosenyl group, triacontenyl group, hentriacontenyl group, and dotriacontenyl group.

In the present specification, the alkynyl group may be linear or branched. In the present specification, the number of the carbon-carbon triple bond of the alkynyl group may be only one, or two or more. Examples of the alkynyl group include ethynyl group, propynyl group, butynyl group, pentynyl group, hexynyl group, heptynyl group, octynyl group, nonynyl group, decynyl group, undecynyl group, dodecynyl group, tridecynyl group, tetradecynyl group, pentadecynyl group, hexadecynyl group, heptadecynyl group, octadecynyl group, nonadecynyl group, icosynyl group, henicosynyl group, docosynyl group, tricosynyl group, tetracosinyl group, pentacosinyl group, hexacosinyl group, heptacosinyl group, octacosinyl group, nonacosinyl group, triacontinyl group, hentriacontinyl group, and dotriacontinyl group.

In the present specification, examples of the halogen atom include fluorine atom, chlorine atom, bromine atom, and iodine atom.

In the present specification, the "hydrocarbon ring group having 3 to 12 carbon atoms" means a cyclic group wherein the ring thereof is composed of 3 to 12 carbon atoms. Examples of the hydrocarbon ring group having 3 to 12 carbon atoms include cycloalkyl group having 3 to 8 carbon atoms, phenyl group, naphthyl group, and adamantyl group. Examples of the cycloalkyl group having 3 to 8 carbon atoms include cyclopropyl group, cyclobutyl group, cyclopentyl group, cyclohexyl group, cycloheptyl group, and cyclooctyl group. The hydrocarbon ring group having 3 to 12 carbon atoms is preferably a non-aromatic hydrocarbon ring group having 3 to 12 carbon atoms, more preferably a cycloalkyl group having 3 to 8 carbon atoms or an adamantyl group, further preferably a cyclohexyl group or an adamantyl group.

In the present specification, the "3- to 14-membered heterocyclic group" means a heterocyclic group wherein the ring thereof is composed of 3 to 14 atoms. Examples of the 3- to 14-membered heterocyclic group include 5- to 14-membered aromatic heterocyclic group, and 3- to 14-membered non-aromatic heterocyclic group.

In the present specification, examples of the 5- to 14-membered aromatic heterocyclic group include the following:
(i) 5- to 6-membered monocyclic aromatic heterocyclic groups such as thienyl group, furyl group, pyrrolyl group, imidazolyl group, pyrazolyl group, thiazolyl group, isothiazolyl group, oxazolyl group, isoxazolyl group, pyridyl group, pyrazinyl group, pyrimidinyl group, pyridazinyl group, 1,2,4-oxadiazolyl group, 1,3,4-oxadiazolyl group, 1,2,4-thiadiazolyl group, 1,3,4-thiadiazolyl group, triazolyl group, tetrazolyl group, triazinyl group, and the like;
(ii) 8- to 14-membered fused polycyclic aromatic heterocyclic groups such as benzothiophenyl group, benzofuranyl group, benzoimidazolyl group, benzoxazolyl group, benzoisoxazolyl group, benzothiazolyl group, benzoisothiazolyl group, benzotriazolyl group, imidazopyridinyl group, thienopyridinyl group, furopyridinyl group, pyrrolopyridinyl group, pyrazolopyridinyl group, oxazolopyridinyl group, thiazolopyridinyl group, imidazopyrazinyl group, imidazopyrimidinyl group, thienopyrimidinyl group, furopyrimidinyl group, pyrrolopyrimidinyl group, pyrazolopyrimidinyl group, oxazolopyrimidinyl group, thiazolopyrimidinyl group, pyrazolotriazinyl group, naphtho[2,3-b]thienyl group, phenoxathiinyl group, indolyl group, isoindolyl group, 1H-indazolyl group, purinyl group, isoquinolyl group, quinolyl group, phthalazinyl group, naphthyridinyl group, quinoxalinyl group, quinazolinyl group, cinnolinyl group, carbazolyl group, β-carbolinyl group, phenanthridinyl group, acrydinyl group, phenazinyl group, phenothiazinyl group, phenoxazinyl group, and the like.

In the present specification, examples of the 3- to 14-membered non-aromatic heterocyclic group include the following:
(i) 3- to 8-membered monocyclic non-aromatic heterocyclic groups such as aziridinyl group, oxiranyl group, thiiranyl group, azetidinyl group, oxetanyl group, thietanyl group, tetrahydrothienyl group, tetrahydrofuranyl group, pyrrolinyl group, pyrrolidinyl group, imidazolinyl group, imidazolidinyl group, oxazolinyl group, oxazolidinyl group, pyrazolinyl group, pyrazolidinyl group, thiazolinyl group, thiazolidinyl group, tetrahydroisothiazolyl group, tetrahydroxazolyl group, tetrahydroisoxazolyl group, piperidinyl group, piperazinyl group, tetrahydropyridinyl group, dihydropyridinyl group, dihydrothiopyranyl group, tetrahydropyrimidinyl group, tetrahydropyridazinyl group, dihydropyranyl group, tetrahydropyranyl group, tetrahydrothiopyranyl group, morpholinyl group, thiomorpholinyl group, azepanyl group, diazepanyl group, azepinyl group, oxepanyl group, azocanyl group, diazocanyl group, dithioranyl group (e.g., 1,2-dithiolan-3-yl group), and the like;
(ii) 9- to 14-membered fused polycyclic non-aromatic heterocyclic groups such as dihydrobenzofuranyl group, dihydrobenzoimidazolyl group, dihydrobenzoxazolyl group, dihydrobenzothiazolyl group, dihydrobenzoisothiazolyl group, dihydronaphto[2,3-b]thienyl group, tetrahydroisoquinolyl group, tetrahydroquinolyl group, 4H-quinolizinyl group, indolinyl group, isoindolinyl group, tetrahydrothieno[2,3-c]pyridinyl group, tetrahydrobenzoazepinyl group, tetrahydroquinoxalinyl group, tetrahydrophenanthridinyl group, hexahydrophenothiazinyl group, hexahydrophenoxazinyl group, tetrahydrophthalazinyl group, tetrahydronaphthyridinyl group, tetrahydroquinazolinyl group, tetrahydrocinnolinyl group, tetrahydrocarbazolyl group, tetrahydro-β-carbolinyl group, tetrahydroacrydinyl group, tetrahydrophenazinyl group, tetrahydrothioxanthenyl group, octahydroisoquinolyl group, and the like.

R^{1a}, R^{1b} and the like in the formula (1) are described below. The explanations and preferred embodiments of the following R^{1a}, R^{1b} and the like can be combined with each other.

In the formula (1), R^{1a} and R^{1b} are each independently an alkylene group having 1 to 6 carbon atoms. R^{1a} and R^{1b} may be the same or different, and R^{1a} and R^{1b} are preferably the same groups. R^{1a} and R^{1b} are preferably each independently an alkylene group having 1 to 3 carbon atoms, more preferably each an alkylene group having 1 to 3 carbon atoms, further preferably an ethylene group. In the present specification, "R^{1a} and R^{1b} are each an alkylene group having 1 to 3 carbon atoms " means that R^{1a} and R^{1b} are the same and each is an alkylene group having 1 to 3 carbon atoms. Other expressions similar to "R^{1a} and R^{1b} are each an alkylene group having 1 to 3 carbon atoms" also have the same meaning as "R^{1a} and R^{1b} are each an alkylene group having 1 to 3 carbon atoms".

In the formula (1), X^{a} and X^{b} are each independently an acyclic alkyl tertiary amino group having 1 to 6 carbon atoms and 1 tertiary amino group, or a cyclic alkylene tertiary amino group having 2 to 5 carbon atoms and 1 to 2 tertiary amino groups.

In the present specification, the "acyclic alkyl tertiary amino group having 1 to 6 carbon atoms and 1 tertiary amino group" means a divalent group represented by the formula (25): 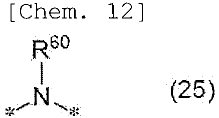 wherein, in the formula (25), * is a bonding position, and
R⁶⁰ is an alkyl group having 1 to 6 carbon atoms. In the present specification, "*", etc. show bonding positions, not carbon atoms, as described above. In the present specification, therefore, "-*" is a single bond.

R⁶⁰ in the formula (25) is preferably a methyl group, an ethyl group, a propyl group, or an isopropyl group, more preferably a methyl group.

In the present specification, the "cyclic alkylene tertiary amino group having 2 to 5 carbon atoms and 1 to 2 tertiary amino groups" means a divalent group in which an alkylene group having 2 to 5 carbon atoms and a tertiary amino group form a cyclic structure, and 1 or 2 tertiary amino groups are contained in the aforementioned cyclic structure. The aforementioned carbon number is preferably 4 or 5.

Examples of the a cyclic alkylene tertiary amino group having 2 to 5 carbon atoms and 1 to 2 tertiary amino groups include aziridinediyl group, azetidinediyl group, pyrrolidinediyl group, piperidinediyl group, imidazolidinediyl group, and piperazinediyl group.

The cyclic alkylene tertiary amino group having 2 to 5 carbon atoms and 1 tertiary amino group is preferably a divalent group represented by the formula (26): 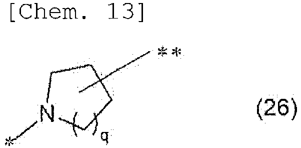 wherein, in the formula (26), * is a bonding position with R^{1a} or R^{1b} in the formula (1), ** is a bonding position with R^{2a} or R^{2b} in the formula (1), and q is 1 or 2.

Hereinafter the "divalent group represented by the formula (26)" is sometimes to be referred to as "group (26)". Groups represented by other formulas are also sometimes to be abbreviated in the same manner as the "divalent group represented by the formula (26)". When q is 1, group (26) is a pyrrolidinediyl group, and when q is 2, group (26) is a piperidinediyl group.

A cyclic alkylene tertiary amino group having 2 to 5 carbon atoms and 2 tertiary amino groups is preferably a divalent group represented by the formula (27): 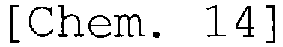 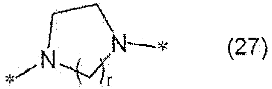 wherein, in the formula (27), * is a bonding position, and r is 1 or 2. When r is is 1, group (27) is an imidazolidinediyl group, and when r is 2, group (27) is a piperazinediyl group.

X^{a} and X^{b} may be the same or different, and X^{a} and X^{b} are preferably the same groups. In the formula (1), X^{a} and X^{b} are preferably each independently a cyclic alkylene tertiary amino group having 2 to 5 carbon atoms and 1 to 2 tertiary amino groups, more preferably each independently group (26) or group (27), further preferably each independently group (26), particularly preferably each group (26) wherein q is 2 (i.e., piperidinediyl group).

In the formula (1), R^{2a} and R^{2b} are each independently an alkylene group having 1 to 8 carbon atoms or an oxydialkylene group having 2 to 8 carbon atoms. The oxydialkylene group having 2 to 8 carbon atoms is preferably an oxydimethylene group, an oxydiethylene group, an oxydipropylene group, more preferably an oxydiethylene group.

In the formula (1), R^{2a} and R^{2b} are preferably each independently an alkylene group having 1 to 8 carbon atoms, more preferably each independently an alkylene group having 1 to 4 carbon atoms, further preferably each an ethylene group.

In the formula (1), Y^{a} and Y^{b} are each independently an ester bond, an amide bond, a carbamate bond, an ether bond, or a urea bond. Y^{a} and Y^{b} may be the same or different, preferably Y^{a} and Y^{b} are the same bonds. Y^{a} and Y^{b} are preferably each independently an ester bond or an amide bond, more preferably each an ester bond, further preferably each *-CO-O-** (wherein * is a bonding position with Z^{a} or Z^{b} in the formula (1), and ** is a bonding position with R^{2a} or R^{2b} in the formula (1)).

In the formula (1), Z^{a} and Z^{b} are each independently a divalent group derived from an aromatic compound having 3 to 16 carbon atoms and at least one aromatic ring, and optionally having a hetero atom. In the following, the aforementioned aromatic ring and the aforementioned aromatic compound are sometimes to be abbreviated as "aromatic ring of Z^{a} and Z^{b}".

The aromatic ring of Z^{a} and Z^{b} may be either an aromatic hydrocarbon ring or an aromatic heterocycle. Examples of the aromatic hydrocarbon ring include benzene ring, naphthalene ring, and anthracene ring. Examples of the aromatic heterocycle include imidazole ring, pyrazole ring, oxazole ring, isoxazole ring, thiazole ring, isothiazole ring, triazine ring, pyrrole ring, furanthiophene ring, pyrimidine ring, pyridazine ring, pyrazine ring, pyridine ring, purine ring, pteridine ring, benzimidazole ring, indole ring, benzofuran ring, quinazoline ring, phthalazine ring, quinoline ring, isoquinoline ring, coumarin ring, chromone ring, benzodiazepine ring, phenoxathiine ring, phenothiazine ring, and acridine ring. The aromatic ring of Z^{a} and Z^{b} is preferably an aromatic hydrocarbon ring, more preferably a benzene ring.

The aromatic ring of Z^{a} and Z^{b} may be substituted by a substituent. Examples of the substituent thereof include acyl group having 2 to 4 carbon atoms, alkoxycarbonyl group having 2 to 4 carbon atoms, alkylcarbamoyl group having 2 to 4 carbon atoms, acyloxy group having 2 to 4 carbon atoms, acylamino group having 2 to 4 carbon atoms, alkoxycarbonylamino group having 2 to 4 carbon atoms, halogen atom (i.e., fluorine atom, chlorine atom, bromine atom, iodine atom), alkylsulfanyl group having 1 to 4 carbon atoms, alkylsulfonyl group having 1 to 4 carbon atoms, arylsulfonyl group having 6 to 10 carbon atoms, nitro group, trifluoromethyl group, cyano group, alkyl group having 1 to 4 carbon atoms, ureido group, alkoxy group having 1 to 4 carbon atoms, aryl group having 6 to 10 carbons, and aryloxy group having 6 to 10 carbon atoms. Preferred examples of the aforementioned substituent include acetyl group, methoxycarbonyl group, methylcarbamoyl group, acetoxy group, acetamido group, methoxycarbonylamino group, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, and phenoxy group.

Z^{a} and Z^{b} are preferably each independently a divalent group represented by the formula (13): 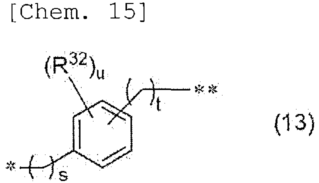 wherein, in the formula (13), * is a bonding position with O in the formula (1),
** is a bonding position with Y^{a} or Y^{b} in the formula (1),
s is an integer of 0 to 3,
t is an integer of 0 to 3,
u is an integer of 0 to 4, and
R³² in the number of u are each independently a substituent. The aforementioned "s and t" shows the number of methylene groups (CH₂), and "s or t is is 0" means that the corresponding methylene group is not present. The aforementioned "u" shows the number of R³², and "u is is 0" means that R³² is not present.

s is preferably 0 or 1, more preferably 0.

t is preferably an integer of 0 to 2, more preferably 1.

u is preferably an integer of 0 to 2, more preferably 0.

R³² is
preferably an acyl group having 2 to 4 carbon atoms, an alkoxycarbonyl group having 2 to 4 carbon atoms, a carbamoyl group having 2 to 4 carbon atoms, an acyloxy group having 2 to 4 carbon atoms, an acylamino group having 2 to 4 carbon atoms, an alkoxycarbonylamino group having 2 to 4 carbon atoms, a halogen atom (i.e., fluorine atom, chlorine atom, bromine atom, iodine atom), an alkylsulfanyl group having 1 to 4 carbon atoms, an alkylsulfonyl group having 1 to 4 carbon atoms, an arylsulfonyl group having 6 to 10 carbon atoms, a nitro group, a trifluoromethyl group, a cyano group, an alkyl group having 1 to 4 carbon atoms, a ureido group, an alkoxy group having 1 to 4 carbon atoms, an aryl group having 6 to 10 carbon atoms, or an aryloxy group having 6 to 10 carbon atoms,
more preferably an acetyl group, a methoxycarbonyl group, a methylcarbamoyl group, an acetoxy group, an acetamido group, a methoxycarbonylamino group, a halogen atom (i.e., fluorine atom, chlorine atom, bromine atom, iodine atom), a methylsulfanyl group, a phenylsulfonyl group, a nitro group, a trifluoromethyl group, a cyano group, an alkyl group having 1 to 4 carbon atoms (e.g., methyl group, ethyl group, propyl group, isopropyl group, tert-butyl group), a ureido group, an alkoxy group having 1 to 4 carbon atoms (e.g., methoxy group, ethoxy group, propoxy group, isopropoxy group, tert-butoxy group), a phenyl group, or a phenoxy group,
further preferably a halogen atom (i.e., fluorine atom, chlorine atom, bromine atom, iodine atom), an alkyl group having 1 to 4 carbon atoms (e.g., methyl group, ethyl group, propyl group, isopropyl group, tert-butyl group), or an alkoxy group having 1 to 4 carbon atoms (e.g., methoxy group, ethoxy group, propoxy group, isopropoxy group, tert-butoxy group),
particularly preferably a halogen atom (i.e., fluorine atom, chlorine atom, bromine atom, iodine atom) or an alkoxy group having 1 to 4 carbon atoms (e.g., methoxy group, ethoxy group).

When R³² is present in plurality, the R³² in plurality may be the same with or different from each other.

Z^{a} and Z^{b} may be the same or different, and Z^{a} and Z^{b} are preferably the same. Z^{a} and Z^{b} in the formula (1) are preferably each independently group (13), more preferably each independently group (13) in which s is 0 or 1, t is an integer of 0 to 2, and u is an integer of 0 to 2, more preferably each group (13) in which s is 0, t is 1, and u is 0.

In the formula (1), na and nb are each independently 0 or 1. That "na is 0" means that Y^{a} and Z^{a} do not exist, and "nb is 0" means that Y^{b} and Z^{b} do not exist.

na may be the same as or different from nb, and na is preferably the same as nb.

R^{3a} and R^{3b} in the formula (1) are each independently a monovalent group recited below:
(i) a monovalent group having 10 to 50 carbon atoms, one carbonyl group, and at least one unsaturated bond selected from the group consisting of an olefinic carbon-carbon double bond and a carbon-carbon triple bond (excluding a monovalent group containing a residue of a liposoluble vitamin having a hydroxy group and a residue of a sterol derivative having a hydroxy group) (hereinafter sometimes to be abbreviated as "group (i)"),
(ii) a monovalent group having 10 to 50 carbon atoms and at least two carbonyl groups (excluding a monovalent group containing a residue of a liposoluble vitamin having a hydroxy group and a residue of a sterol derivative having a hydroxy group) (hereinafter sometimes to be abbreviated as "group (ii)"),
(iii) a monovalent group represented by the formula (2):

   *-R⁴-X¹-R⁵ (2)

   wherein, in the formula (2), * is a bonding position,
   R⁴ is an alkylene group having 1 to 10 carbon atoms,
   X¹ is a carbamate bond, a carbonate bond, or an amide bond, and R⁵ is an alkyl group having 1 to 25 carbon atoms, and
   R⁵ is optionally substituted by a substituent selected from the group consisting of a halogen atom and a hydroxy group (hereinafter sometimes to be abbreviated as "group (iii)"),
(iv) a monovalent group represented by the formula (3):

   *-R⁶-CO-O-R⁷ (3)

   wherein, in the formula (3), * is a bonding position,
   R⁶ is an alkylene group having 1 to 10 carbon atoms, and
   R⁷ is an alkyl group having 1 to 25 carbon atoms and substituted by at least one halogen atom (hereinafter sometimes to be abbreviated as "group (iv)"),
(v) a monovalent group represented by the formula (4): 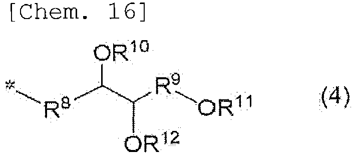 wherein, in the formula (4), * is a bonding position,
   R⁸ and R⁹ are each independently an alkylene group having 1 to 10 carbon atoms, an alkenediyl group having 2 to 10 carbon atoms, or an alkynediyl group having 2 to 10 carbon atoms,
   R¹⁰ to R¹² are each independently a hydrogen atom, a benzyl group, or a *-Si (R¹³) (R¹⁴) (R¹⁵) group (wherein * is a bonding position, and R¹³ to R¹⁵ are each independently an alkyl group having 1 to 4 carbon atoms or a phenyl group (hereinafter sometimes to be abbreviated as "group (v)"),
(vi) a monovalent group represented by the formula (5): 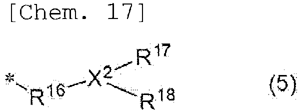
   wherein, in the formula (5), * is a bonding position, X² is a nitrogen atom or a trivalent group represented by the formula (6): 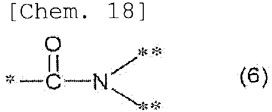
   wherein, in the formula (6), * is a bonding position with R¹⁶, and
      ** is a bonding position with R¹⁷ or R¹⁸,
      when X² is a nitrogen atom, R¹⁶ is an alkylene group having 1 to 10 carbon atoms, an alkenediyl group having 2 to 10 carbon atoms, or an alkynediyl group having 2 to 10 carbon atoms, and R¹⁶ is optionally substituted by a substituent selected from the group consisting of a halogen atom and a hydroxy group,
      when X² is a trivalent group represented by the formula (6), R¹⁶ is an alkylene group having 1 to 10 carbon atoms, and R¹⁶ is optionally substituted by a substituent selected from the group consisting of a halogen atom and a hydroxy group,
      when X² is a nitrogen atom, R¹⁷ and R¹⁸ are each independently an alkyl group having 1 to 10 carbon atoms, an alkenyl group having 2 to 10 carbon atoms, or an alkynyl group having 2 to 10 carbon atoms, and R¹⁷ and R¹⁸ are each independently optionally substituted by a substituent selected from the group consisting of a halogen atom and a hydroxy group, and
      when X² is a trivalent group represented by the formula (6), R¹⁷ and R¹⁸ are each independently an alkyl group having 1 to 10 carbon atoms, and R¹⁷ and R¹⁸ are each independently optionally substituted by a substituent selected from the group consisting of a halogen atom and a hydroxy group (hereinafter sometimes to be abbreviated as "group (vi)"),
(vii) a monovalent group represented by the formula (7): 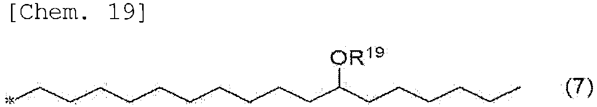 wherein, in the formula (7), * is a bonding position, and
   R¹⁹ is a hydrogen atom, a benzyl group, or a *-Si (R¹³) (R¹⁴) (R¹⁵) group (wherein * is a bonding position, and R¹³ to R¹⁵ are each independently an alkyl group having 1 to 4 carbon atoms or a phenyl group), or a *-CO-R²⁰ group (wherein * is a bonding position, and R²⁰ is an alkyl group having 1 to 9 carbon atoms) (hereinafter sometimes to be abbreviated as "group (vii)"),
(viii) a monovalent group represented by the formula (8): 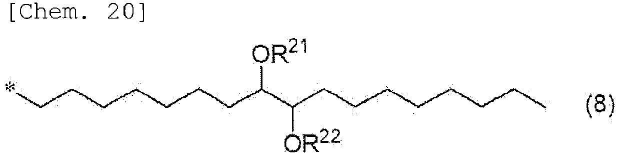 wherein, in the formula (8), * is a bonding position, and
   R²¹ and R²² are each independently a hydrogen atom, a benzyl group, or a *-Si (R¹³) (R¹⁴) (R¹⁵) group (wherein * is a bonding position, and R¹³ to R¹⁵ are each independently an alkyl group having 1 to 4 carbon atoms or a phenyl group) (hereinafter sometimes to be abbreviated as "group (viii)"),
(ix) a monovalent group represented by the formula (9): 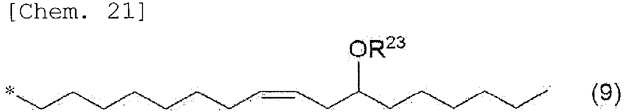 wherein, in the formula (9), * is a bonding position, and
   R²³ is a hydrogen atom, a benzyl group, or a *-Si (R¹³) (R¹⁴) (R¹⁵) group (wherein * is a bonding position, and R¹³ to R¹⁵ are each independently an alkyl group having 1 to 4 carbon atoms or a phenyl group) (hereinafter sometimes to be abbreviated as "group (ix)"),
(x) a monovalent group represented by the formula (10): 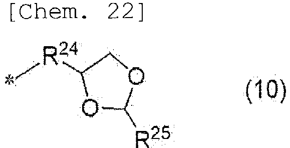 wherein, in the formula (10), * is a bonding position,
   R²⁴ is an alkylene group having 1 to 10 carbon atoms, an alkenediyl group having 2 to 10 carbon atoms, or an alkynediyl group having 2 to 10 carbon atoms, and
   R²⁵ is an alkyl group having 1 to 30 carbon atoms, an alkenyl group having 2 to 30 carbon atoms, or an alkynyl group having 2 to 30 carbon atoms (hereinafter sometimes to be abbreviated as "group (x)"),
(xi) a monovalent group represented by the formula (11): 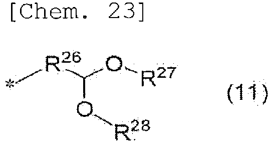 wherein, in the formula (11), * is a bonding position, and
   R²⁶ is an alkylene group having 1 to 10 carbon atoms, an alkenediyl group having 2 to 10 carbon atoms, or an alkynediyl group having 2 to 10 carbon atoms, and
   R²⁷ and R²⁸ are each independently an alkyl group having 1 to 10 carbon atoms, an alkenyl having 2 to 10 carbon atoms, or an alkynyl group having 2 to 10 carbon atoms (hereinafter sometimes to be abbreviated as "group (xi)"),
(xii) a monovalent group represented by the formula (12): 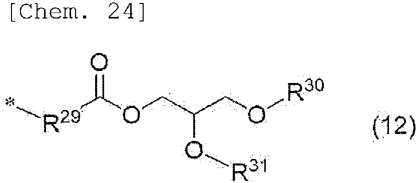 wherein, in the formula (12), * is a bonding position,
   R²⁹ is an alkylene group having 1 to 10 carbon atoms, and
   R³⁰ and R³¹ are each independently an alkyl group having 1 to 10 carbon atoms (hereinafter sometimes to be abbreviated as "group (xii)"),
(xiii) a monovalent group which is an alkyl group having 1 to 30 carbon atoms, an alkenyl group having 2 to 20 carbon atoms, or an alkynyl group having 2 to 20 carbon atoms, in which one ethylene group in the aforementioned alkyl group is optionally replaced by one ester bond (hereinafter sometimes to be abbreviated as "group (xiii)"), or
(xiv) a R^{3c}-CO-(CH₂)ₚ- group (wherein R^{3c} is a residue of a liposoluble vitamin having a hydroxy group or a residue of a sterol derivative having a hydroxy group, and p is an integer of 1 to 8) (hereinafter sometimes to be abbreviated as "group (xiv)").

In the formula (1), R^{3a} and R^{3b} may be the same or different.

In one embodiment of the present invention, R^{3a} and R^{3b} are
preferably each independently group (i), group (ii), group (iii), group (vii), group (ix), group (xiii), or group (xiv),
more preferably group (i) or group (xiii).

In another embodiment of the present invention, R^{3a} and R^{3b} are
preferably each independently group (ii), group (xiii), or group (xiv),
more preferably, each independently group (xiii).

In another embodiment of the present invention, R^{3a} and R^{3b} are preferably each independently group (i) or group (ii). In other words, in another embodiment of the present invention, R^{3a} is preferably group (i) or group (ii), and R^{3b} is preferably any of group (i) to group (xiv). In the aforementioned embodiment, R^{3b} is more preferably group (i), group (ii), group (xiii), or group (xiv). In the aforementioned embodiment, R^{3a} and R^{3b} may be the same or different.

In another embodiment of the present invention, R^{3a} is preferably any of group (iii) to group (xii), and R^{3b} is preferably any of group (iii) to group (xiv). In the aforementioned embodiment, R^{3b} is more preferably group (xiii) or group (xiv), further preferably group (xiii). In the aforementioned embodiment, R^{3a} and R^{3b} may be the same or different.

In another embodiment of the present invention, R^{3a} and R^{3b} are preferably each independently a monovalent group recited below:
(i-1) a monovalent group having 10 to 50 carbon atoms and represented by the formula (14):

   *-R³³-CO-X³-R³⁴ (14)

   wherein, in the formula (14), * is a bonding position,
   R³³ is an alkylene group having 2 to 10 carbon atoms, an alkenediyl group having 2 to 10 carbon atoms, or an alkynediyl group having 2 to 10 carbon atoms, and R³³ is optionally substituted by a substituent selected from the group consisting of a halogen atom, a hydroxy group, and a hydrocarbon ring group having 3 to 12 carbon atoms,
   R³⁴ is an alkyl group having 1 to 40 carbon atoms, an alkenyl group having 2 to 40 carbon atoms, or an alkynyl group having 2 to 40 carbon atoms, and R³⁴ is optionally substituted by a substituent selected from the group consisting of a halogen atom, a hydroxy group, and a hydrocarbon ring group having 3 to 12 carbon atoms,
   at least one of R³³ and R³⁴ has at least one unsaturated bond selected from the group consisting of an olefinic carbon-carbon double bond and a carbon-carbon triple bond, and
   X³ is an oxygen atom, NH, or a sulfur atom (hereinafter sometimes to be abbreviated as "group (i-1)"),
(i-2) a monovalent group having 50 or less carbon atoms and represented by the formula (15): 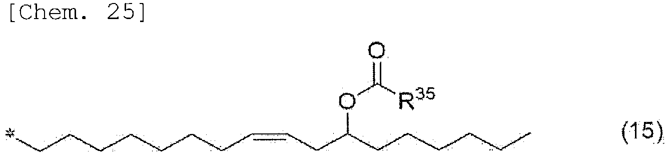 wherein, in the formula (15), * is a bonding position, and
   R³⁵ is an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, at least one ethylene group or at least one trimethylene group in R³⁵ is optionally replaced by at least one bond selected from the group consisting of an ester bond, an amide bond, a carbamate bond, and a carbonate bond, and R³⁵ is optionally substituted by a substituent selected from the group consisting of an alkoxy group having 1 to 4 carbon atoms, a 3- to 14-membered heterocyclic group, and a hydrocarbon ring group having 3 to 12 carbon atoms (preferably an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and R³⁵ is optionally substituted by a substituent selected from the group consisting of a 3- to 14-membered heterocyclic group and a hydrocarbon ring group having 3 to 12 carbon atoms) (hereinafter sometimes to be abbreviated as "group (i-2)"),
(ii-1) a monovalent group having 50 or less carbon atoms and represented by the formula (16):

   *-R³⁶-CO-X⁴-R³⁷ (16)

   wherein, in the formula (16), * is a bonding position,
   R³⁶ is an alkylene group having 2 to 9 carbon atoms, an alkenediyl group having 2 to 9 carbon atoms, or an alkynediyl group having 2 to 9 carbon atoms, and R³⁶ is optionally substituted by a substituent selected from the group consisting of a halogen atom, a hydroxy group, and a hydrocarbon ring group having 3 to 12 carbon atoms,
   R³⁷ is an alkyl group having 7 to 45 carbon atoms, an alkenyl group having 7 to 45 carbon atoms, or an alkynyl group having 7 to 45 carbon atoms, at least one ethylene group or at least one trimethylene group in R³⁷ is replaced by at least one bond selected from the group consisting of an ester bond, an amide bond, a carbamate bond, and a carbonate bond, and R³⁷ is optionally substituted by a substituent selected from the group consisting of a halogen atom, a hydroxy group, and a hydrocarbon ring group having 3 to 12 carbon atoms, and
   X⁴ is an oxygen atom, NH, or a sulfur atom (hereinafter sometimes to be abbreviated as "group (ii-1)"),
(ii-2) a monovalent group having 10 to 50 carbon atoms and represented by the formula (17):

   *-R³⁸-O-R³⁹ (17)

   wherein, in the formula (17), * is a bonding position,
   R³⁸ is an alkylene group having 2 to 10 carbon atoms, an alkenediyl group having 2 to 10 carbon atoms, or an alkynediyl group having 2 to 10 carbon atoms, at least one ethylene group or at least one trimethylene group in R³⁸ is optionally replaced by at least one bond selected from the group consisting of an ester bond, an amide bond, a carbamate bond, and a carbonate bond, and R³⁸ is optionally substituted by a substituent selected from the group consisting of a halogen atom, a hydroxy group, and a hydrocarbon ring group having 3 to 12 carbon atoms, and
   R³⁹ is an alkyl group having 3 to 30 carbon atoms, an alkenyl group having 4 to 30 carbon atoms, or an alkenyl group having 4 to 30 carbon atoms, at least two methylene groups in R³⁹ are replaced by at least two carbonyl groups, and at least one methylene group of R³⁹ is optionally replaced by at least one ether bond (hereinafter sometimes to be abbreviated as "group (ii-2)"),
(ii-3) a monovalent group having 50 or less carbon atoms and represented by the formula (18): 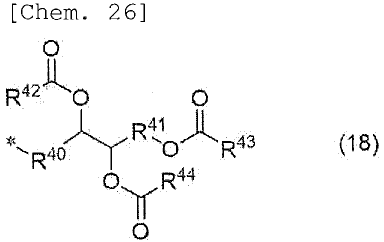 wherein, in the formula (18), * is a bonding position, and
   R⁴⁰ and R⁴¹ are each independently an alkylene group having 3 to 10 carbon atoms, and R⁴² to R⁴⁴ are each independently an alkyl group having 1 to 10 carbon atoms, an alkenyl group having 2 to 10 carbon atoms or an alkynyl group having 2 to 10 carbon atoms, at least one ethylene group or at least one trimethylene group in R⁴² is optionally replaced by at least one bond selected from the group consisting of an ester bond, an amide bond, a carbamate bond, and a carbonate bond, at least one ethylene group or at least one trimethylene group in R⁴³ is optionally replaced by at least one bond selected from the group consisting of an ester bond, an amide bond, a carbamate bond, and a carbonate bond, at least one ethylene group or at least one trimethylene group in R⁴⁴ is optionally replaced by at least one bond selected from the group consisting of an ester bond, an amide bond, a carbamate bond, and a carbonate bond, and R⁴² to R⁴⁴ are each independently optionally substituted by a substituent selected from the group consisting of a halogen atom, a hydroxy group, and a hydrocarbon ring group having 3 to 12 carbon atoms (hereinafter sometimes to be abbreviated as "group (ii-3)"),
(ii-4) a monovalent group having 50 or less carbon atoms and represented by the formula (19): 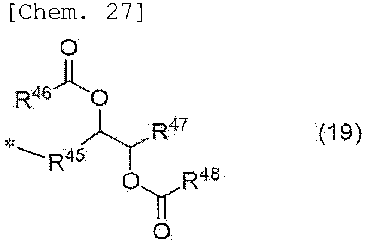
wherein, in the formula (19), * is a bonding position,
   R⁴⁵ is an alkylene group having 5 to 10 carbon atoms, and
   R⁴⁶ to R⁴⁸ are each independently an alkyl group having 1 to 10 carbon atoms, an alkenyl group having 2 to 10 carbon atoms, or an alkynyl group having 2 to 10 carbon atoms, at least one ethylene group or at least one trimethylene group in R⁴⁶ is optionally replaced by at least one bond selected from the group consisting of an ester bond, an amide bond, a carbamate bond, and a carbonate bond, at least one ethylene group or at least one trimethylene group in R⁴⁷ is optionally replaced by at least one bond selected from the group consisting of an ester bond, an amide bond, a carbamate bond, and a carbonate bond, at least one ethylene group or at least one trimethylene group in R⁴⁸ is optionally replaced by at least one bond selected from the group consisting of an ester bond, an amide bond, a carbamate bond, and a carbonate bond, and R⁴⁶ to R⁴⁸ are each independently optionally substituted by a substituent selected from the group consisting of a halogen atom, a hydroxy group, and a hydrocarbon ring group having 3 to 12 carbon atoms
      (hereinafter sometimes to be abbreviated as "group (ii-4)"), or any of group (iii) to group (xiv).

In another embodiment of the present invention, R^{3a} and R^{3b} are
preferably each independently group (i-2) (i.e., group (15)), group (ii-1) (i.e., group (16)), group (iii) (i.e., group (2)), group (ix) (i.e., group (9)), group (xiii), or group (xiv),
more preferably each independently group (i-2) (i.e., group (15)) or group (xiii).

In another embodiment of the present invention, R^{3a} and R^{3b} are
preferably each independently group (ii-1) (i.e., group (16)), group (ix) (i.e., group (9)), group (xiii), or group (xiv),
more preferably each independently group (xiii).

Group (i) and the like are described below in order.

Group (i) is preferably group (i-1) (i.e., group (14)) or group (i-2) (i.e., group (15)), more preferably group (i-2) (i.e., group (15)).

R³³ in the formula (14) is as mentioned above, and an alkylene group having 2 to 10 carbon atoms, an alkenediyl group having 2 to 10 carbon atoms, or an alkynediyl group having 2 to 10 carbon atoms, and R³³ is optionally substituted by a substituent selected from the group consisting of a halogen atom, a hydroxy group, and a hydrocarbon ring group having 3 to 12 carbon atoms.

In the present specification, "R³³ is optionally substituted by a substituent selected from the group consisting of a halogen atom, a hydroxy group, and a hydrocarbon ring group having 3 to 12 carbon atoms" means that the aforementioned alkylene group, the aforementioned alkenediyl group and the aforementioned alkynediyl group for R³³ are each independently optionally substituted by a substituent selected from the group consisting of a halogen atom, a hydroxy group, and a hydrocarbon ring group having 3 to 12 carbon atoms. Other expressions similar to "R³³ is a optionally substituted by a substituent selected from the group consisting of a halogen atom, a hydroxy group, and a hydrocarbon ring group having 3 to 12 carbon atoms" also have the same meaning as "R³³ is a optionally substituted by a substituent selected from the group consisting of a halogen atom, a hydroxy group, and a hydrocarbon ring group having 3 to 12 carbon atoms".

R³³ in the formula (14) is preferably an alkylene group having 2 to 10 carbon atoms, an alkenediyl group having 2 to 10 carbon atoms, or an alkynediyl group having 2 to 10 carbon atoms, more preferably an alkylene group having 2 to 8 carbon atoms or an alkenediyl group having 2 to 8 carbon atoms. In the present specification, unless otherwise specified regarding the substituent, "alkylene group", "alkenediyl group", "alkynediyl group", "alkyl group", "alkenyl group", and "alkynyl group" show unsubstituted groups.

R³⁴ in the formula (14) is preferably an alkyl group having 1 to 40 carbon atoms, an alkenyl group having 2 to 40 carbon atoms, or an alkynyl group having 2 to 40 carbon atoms, more preferably an alkyl group having 1 to 20 carbon atoms, an alkenyl group having 2 to 20 carbon atoms, or an alkynyl group having 2 to 20 carbon atoms.

At least one of R³³ and R³⁴ in the formula (14) has, as mentioned above, at least one unsaturated bond selected from the group consisting of an olefinic carbon-carbon double bond and a carbon-carbon triple bond.

X³ in the formula (14) is preferably an oxygen atom.

R³⁵ in the formula (15) is preferably an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and R³⁵ is optionally substituted by a substituent selected from the group consisting of a 3- to 14-membered heterocyclic group and a hydrocarbon ring group having 3 to 12 carbon atoms.

R³⁵ in the formula (15) is more preferably an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and the aforementioned alkyl group is optionally substituted by a hydrocarbon ring group having 3 to 12 carbon atoms.

In one embodiment of the present invention, R³⁵ is further preferably an alkyl group having 1 to 20 carbon atoms, an alkenyl group having 2 to 20 carbon atoms, an alkynyl group having 2 to 20 carbon atoms, or a cyclohexyl group, and the aforementioned alkyl group is optionally substituted by an adamantyl group.

In one embodiment of the present invention, R³⁵ in the formula (15) is further preferably an alkyl group having 1 to 20 carbon atoms, an alkenyl group having 2 to 20 carbon atoms, or an alkynyl group having 2 to 20 carbon atoms, and the aforementioned alkyl group is optionally substituted by a cyclohexyl group.

R³⁵ in the formula (15) is particularly preferably an alkyl group having 1 to 20 carbon atoms, an alkenyl group having 2 to 20 carbon atoms, or an alkynyl group having 2 to 20 carbon atoms.

Group (ii) is preferably group (ii-1) (i.e., group (16)), group (ii-2) (i.e., group (17)), group (ii-3) (i.e., group (18)), or group (ii-4) (i.e., group (19)), more preferably group (ii-1) (i.e., group (16)).

X⁴ in the formula (16) is preferably an oxygen atom or NH, more preferably an oxygen atom.

In one embodiment of the present invention, R³⁶ in the formula (16) is preferably an alkylene group having 2 to 9 carbon atoms, an alkenediyl group having 2 to 9 carbon atoms, or an alkynediyl group having 2 to 9 carbon atoms, more preferably an alkylene group having 2 to 9 carbon atoms or an alkenediyl group having 2 to 9 carbon atoms, further preferably an alkylene group having 2 or 3 carbon atoms or an alkenediyl group having 2 or 3 carbon atoms.

In one embodiment of the present invention, R³⁶ in the formula (16) is preferably an alkylene group having 2 to 9 carbon atoms, an alkenediyl group having 2 to 9 carbon atoms, or an alkynediyl group having 2 to 9 carbon atoms, more preferably an alkylene group having 2 to 9 carbon atoms, further preferably an alkylene group having 2 or 3 carbon atoms.

R³⁷ in the formula (16) is, as mentioned above, an alkyl group having 7 to 45 carbon atoms, an alkenyl group having 7 to 45 carbon atoms, or an alkynyl group having 7 to 45 carbon atoms, at least one ethylene group or at least one trimethylene group in R³⁷ is replaced by at least one bond selected from the group consisting of an ester bond, an amide bond, a carbamate bond, and a carbonate bond, and R³⁷ is optionally substituted by a substituent selected from the group consisting of a halogen atom, a hydroxy group, and a hydrocarbon ring group having 3 to 12 carbon atoms.

In the present specification, "at least one ethylene group or at least one trimethylene group in R³⁷ is replaced by at least one bond selected from the group consisting of an ester bond, an amide bond, a carbamate bond, and a carbonate bond" means that at least one ethylene group or at least one trimethylene group in the aforementioned alkyl group for R³⁷ is replaced by at least one bond selected from the group consisting of an ester bond, an amide bond, a carbamate bond, and a carbonate bond, or at least one ethylene group or at least one trimethylene group in the aforementioned alkenyl for R³⁷ is replaced by at least one bond selected from the group consisting of an ester bond, an amide bond, a carbamate bond, and a carbonate bond, or at least one ethylene group or at least one trimethylene group in the aforementioned alkynyl group for R³⁷ is replaced by at least one bond selected from the group consisting of an ester bond, an amide bond, a carbamate bond, and a carbonate bond. Other expressions similar to "at least one ethylene group or at least one trimethylene group in R³⁷ is replaced by at least one bond selected from the group consisting of an ester bond, an amide bond, a carbamate bond, and a carbonate bond" also have the same meaning as "at least one ethylene group or at least one trimethylene group in R³⁷ is replaced by at least one bond selected from the group consisting of an ester bond, an amide bond, a carbamate bond, and a carbonate bond".

R³⁷ in the formula (16) is preferably
(ii-1-1) a monovalent group represented by the formula (20): 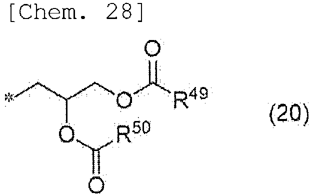 wherein, in the formula (20), * is a bonding position, and
   R⁴⁹ and R⁵⁰ are each independently an alkyl group having 1 to 17 carbon atoms, an alkenyl group having 2 to 17 carbon atoms, or an alkynyl group having 2 to 17 carbon atoms, at least one ethylene group or at least one trimethylene group in R⁴⁹ is optionally replaced by at least one bond selected from the group consisting of an ester bond, an amide bond, a carbamate bond, and a carbonate bond, at least one ethylene group or at least one trimethylene group in R⁵⁰ is optionally replaced by at least one bond selected from the group consisting of an ester bond, an amide bond, a carbamate bond, and a carbonate bond, and R⁴⁹ and R⁵⁰ are each independently optionally substituted by a substituent selected from the group consisting of a halogen atom, a hydroxy group, and a hydrocarbon ring group having 3 to 12 carbon atoms,
(ii-1-2) a monovalent group represented by the formula (21): 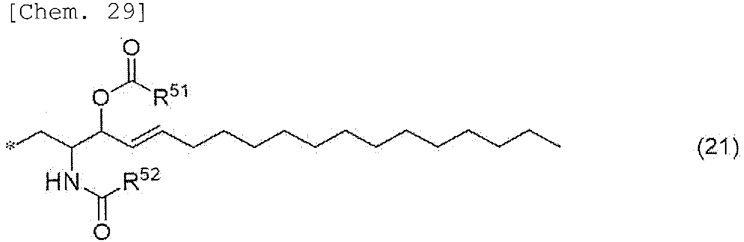 wherein, in the formula (21), * is a bonding position, and
   R⁵¹ and R⁵² are each independently an alkyl group having 1 to 10 carbon atoms, an alkenyl group having 2 to 10 carbon atoms, or an alkynyl group having 2 to 10 carbon atoms, at least one ethylene group or at least one trimethylene group in R⁵¹ is optionally replaced by at least one bond selected from the group consisting of an ester bond, an amide bond, a carbamate bond, and a carbonate bond, at least one ethylene group or at least one trimethylene group in R⁵² is optionally replaced by at least one bond selected from the group consisting of an ester bond, an amide bond, a carbamate bond, and a carbonate bond, and R⁵¹ and R⁵² are each independently optionally substituted by a substituent selected from the group consisting of a halogen atom, a hydroxy group, and a hydrocarbon ring group having 3 to 12 carbon atoms, or
(ii-1-3) a monovalent group represented by the formula (22): 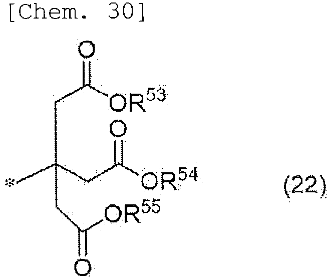 wherein, in the formula (22), * is a bonding position, and
   R⁵³ to R⁵⁵ are each independently an alkyl group having 1 to 10 carbon atoms, an alkenyl group having 2 to 10 carbon atoms, or an alkynyl group having 2 to 10 carbon atoms, at least one ethylene group or at least one trimethylene group in R⁵³ is optionally replaced by at least one bond selected from the group consisting of an ester bond, an amide bond, a carbamate bond, and a carbonate bond, at least one ethylene group or at least one trimethylene group in R⁵⁴ is optionally replaced by at least one bond selected from the group consisting of an ester bond, an amide bond, a carbamate bond, and a carbonate bond, at least one ethylene group or at least one trimethylene group in R⁵⁵ is optionally replaced by at least one bond selected from the group consisting of an ester bond, an amide bond, a carbamate bond, and a carbonate bond, and R⁵³ to R⁵⁵ are each independently optionally substituted by a substituent selected from the group consisting of a halogen atom, a hydroxy group, and a hydrocarbon ring group having 3 to 12 carbon atoms.

In other words, group (16) is preferably a monovalent group having 50 or less carbon atoms and represented by the following formula (16-20), a monovalent group having 50 or less carbon atoms and represented by the following formula (16-21), or a monovalent group having 50 or less carbon atoms and represented by the following formula (16-22), more preferably a monovalent group having 50 or less carbon atoms and represented by the following formula (16-20) or a monovalent group having 50 or less carbon atoms and represented by the following formula (16-22), further preferably a monovalent group having 50 or less carbon atoms and represented by the following formula (16-20) (the definitions of the symbols in the following formulas are as mentioned above).

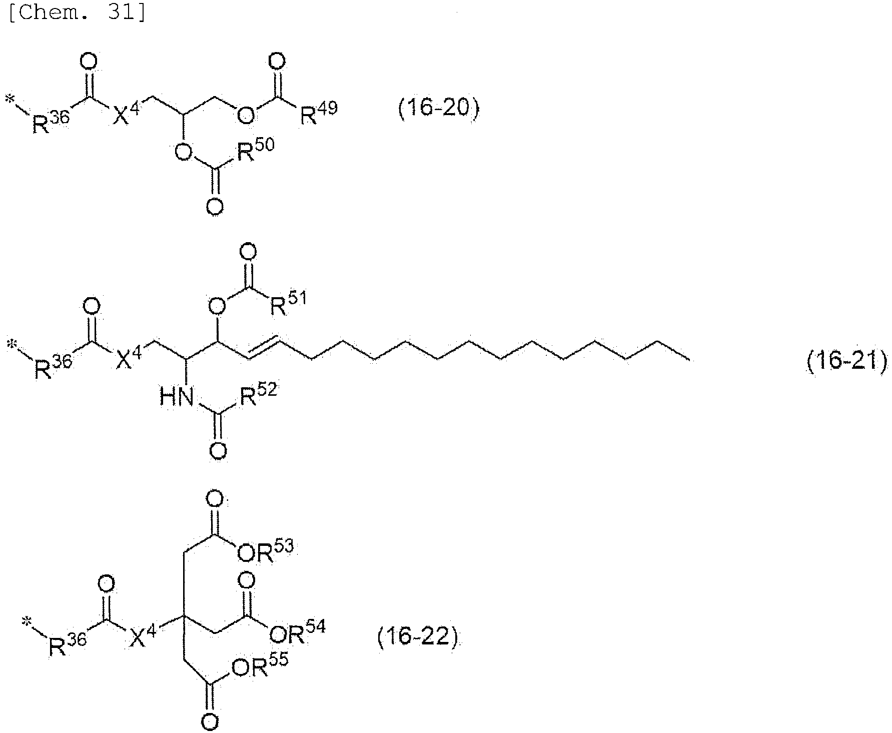

The explanation of R³⁶ and X⁴ in the formula (16-20) to the formula (16-22) is as mentioned above.

R⁴⁹ and R⁵⁰ in the formula (20) and the formula (16-20) are preferably each independently an alkyl group having 1 to 17 carbon atoms, an alkenyl group having 2 to 17 carbon atoms, or an alkynyl group having 2 to 17 carbon atoms, more preferably each independently an alkyl group having 1 to 17 carbon atoms or an alkenyl group having 2 to 17 carbon atoms, further preferably each independently an alkyl having 1 to 17 carbon atoms.

R⁵¹ and R⁵² in the formula (21) and the formula (16-21) are preferably each independently an alkyl group having 1 to 10 carbon atoms, an alkenyl group having 2 to 10 carbon atoms, or an alkynyl group having 2 to 10 carbon atoms, more preferably each independently an alkyl group having 1 to 10 carbon atoms.

R⁵³ to R⁵⁵ in the formula (22) and the formula (16-22) are preferably each independently an alkyl group having 1 to 10 carbon atoms, an alkenyl group having 2 to 10 carbon atoms, or an alkynyl group having 2 to 10 carbon atoms, more preferably an alkyl group having 1 to 10 carbon atoms.

R³⁸ in the formula (17) is preferably an alkylene group having 2 to 10 carbon atoms, an alkenediyl group having 2 to 10 carbon atoms, or an alkynediyl group having 2 to 10 carbon atoms, more preferably an alkylene group having 2 to 10 carbon atoms, further preferably an alkylene group having 2 or 3 carbon atoms.

R³⁹ in the formula (17) is preferably
(ii-2-1) a monovalent group represented by the formula (23): 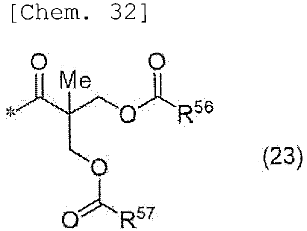 wherein, in the formula (23), * is a bonding position,
   Me is a methyl group, and
   R⁵⁶ and R⁵⁷ are each independently an alkyl group having 1 to 10 carbon atoms, an alkenyl group having 2 to 10 carbon atoms, or an alkynyl group having 2 to 10 carbon atoms, at least one ethylene group or at least one trimethylene group in R⁵⁶ is optionally replaced by at least one bond selected from the group consisting of an ester bond, an amide bond, a carbamate bond, and a carbonate bond, at least one ethylene group or at least one trimethylene group in R⁵⁷ is optionally replaced by at least one bond selected from the group consisting of an ester bond, an amide bond, a carbamate bond, and a carbonate bond, and R⁵⁶ and R⁵⁷ are each independently optionally substituted by a substituent selected from the group consisting of a halogen atom, a hydroxy group, and a hydrocarbon ring group having 3 to 12 carbon atoms, or
(ii-2-2) a monovalent group represented by the formula (24): 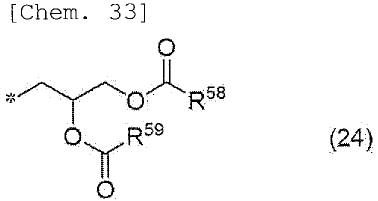 wherein, in the formula (24), * is a bonding position, and
   R⁵⁸ and R⁵⁹ are each independently an alkyl group having 1 to 17 carbon atoms, an alkenyl group having 2 to 17 carbon atoms, or an alkynyl group having 2 to 17 carbon atoms, at least one ethylene group or at least one trimethylene group in R⁵⁸ is optionally replaced by at least one bond selected from the group consisting of an ester bond, an amide bond, a carbamate bond, and a carbonate bond, at least one ethylene group or at least one trimethylene group in R⁵⁹ is optionally replaced by at least one bond selected from the group consisting of an ester bond, an amide bond, a carbamate bond, and a carbonate bond, and R⁵⁸ and R⁵⁹ are each independently optionally substituted by a substituent selected from the group consisting of a halogen atom, a hydroxy group, and a hydrocarbon ring group having 3 to 12 carbon atoms.

In other words, group (17) is preferably a monovalent group having 50 or less carbon atoms and represented by the following formula (17-23), or a monovalent group having 1 to 50 carbon atoms and represented by the following formula (17-24) (the definitions of the symbols in the following formulas are as mentioned above).

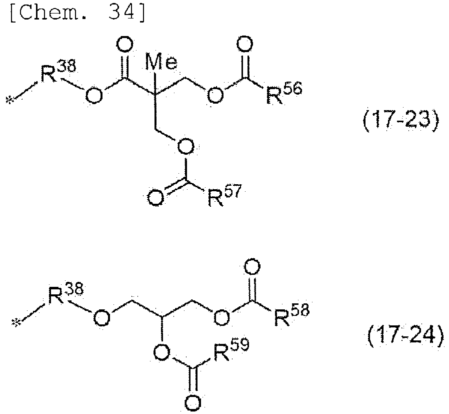

R³⁸ in the formula (17-23) and the formula (17-24) is as described above.

Me in the formula (17-23) is, as mentioned above, a methyl group.

R⁵⁶ and R⁵⁷ in the formula (23) and the formula (17-23) are preferably each independently an alkyl group having 1 to 10 carbon atoms, an alkenyl group having 2 to 10 carbon atoms, or an alkynyl group having 2 to 10 carbon atoms, more preferably each independently an alkyl group having 1 to 10 carbon atoms.

R⁵⁸ and R⁵⁹ in the formula (24) and the formula (17-24) are preferably each independently an alkyl group having 1 to 17 carbon atoms, an alkenyl group having 2 to 17 carbon atoms, or an alkynyl group having 2 to 17 carbon atoms, more preferably each independently an alkyl group having 1 to 17 carbon atoms, further preferably each independently an alkyl group having 1 to 10 carbon atoms.

R⁴⁰ and R⁴¹ in the formula (18) are, as mentioned above, each independently an alkylene group having 3 to 10 carbon atoms.

R⁴² to R⁴⁴ in the formula (18) are preferably each independently an alkyl group having 1 to 10 carbon atoms, an alkenyl group having 2 to 10 carbon atoms, or an alkynyl group having 2 to 10 carbon atoms, more preferably an alkyl group having 1 to 10 carbon atoms.

R⁴⁵ in the formula (19) is, as mentioned above, an alkylene group having 5 to 10 carbon atoms.

R⁴⁶ to R⁴⁸ in the formula (19) are preferably each independently an alkyl group having 1 to 10 carbon atoms, an alkenyl group having 2 to 10 carbon atoms, or an alkynyl group having 2 to 10 carbon atoms, more preferably each independently an alkyl group having 1 to 10 carbon atoms.

R⁴ in the formula (2) is preferably an alkylene group having 1 to 8 carbon atoms, more preferably an alkylene group having 1 to 6 carbon atoms, further preferably an alkylene group having 1 to 4 carbon atoms.

X¹ in the formula (2) is
preferably *-NH-CO-O-** (wherein * is a bonding position with R⁴ in the formula (2), and ** is a bonding position with R⁵ in the formula (2)), or *-O-CO-O-* (wherein * is a bonding position),
more preferably *-NH-CO-O-** (wherein * is a bonding position with R⁴ in the formula (2), and ** is a bonding position with R⁵ in the formula (2)).

R⁵ in the formula (2) is preferably an alkyl group having 1 to 25 carbon atoms.

R⁶ in the formula (3) is, as mentioned above, an alkylene group having 1 to 10 carbon atoms.

R⁷ in the formula (3) is preferably an alkyl group having 1 to 25 carbon atoms substituted by at least one fluorine atom.

R⁸ and R⁹ in the formula (4) are preferably each independently an alkylene group having 1 to 10 carbon atoms.

R¹⁹ to R¹² in the formula (4) are preferably each independently a hydrogen atom, a benzyl group, or a *-Si(R¹³) (R¹⁴) (R¹⁵) group (wherein * is a bonding position, R¹³ is a tert-butyl group, and R¹⁴ and R¹⁵ are each a methyl group or a phenyl group), more preferably each independently a hydrogen atom or a *-Si (R¹³) (R¹⁴) (R¹⁵) group (wherein * is a bonding position, R¹³ is a tert-butyl group, and R¹⁴ and R¹⁵ are each a methyl group or a phenyl group), further preferably each independently a hydrogen atom or a *-Si (R¹³) (R¹⁴) (R¹⁵) group (wherein * is a bonding position, R¹³ is a tert-butyl group, and R¹⁴ and R¹⁵ are each a methyl group).

X² in the formula (5) is, as mentioned above, a nitrogen atom or group (6).

When X² in the formula (5) is a nitrogen atom, R¹⁶ in the formula (5) is preferably an alkylene group having 1 to 10 carbon atoms, an alkenediyl group having 2 to 10 carbon atoms, or an alkynediyl group having 2 to 10 carbon atoms, more preferably an alkylene group having 1 to 10 carbon atoms.

When X² in the formula (5) is group (6), R¹⁶ in the formula (5) is preferably an alkylene group having 1 to 10 carbon atoms.

When X² in the formula (5) is a nitrogen atom or group (6), R¹⁷ and R¹⁸ in the formula (5) are preferably each independently an alkyl group having 1 to 10 carbon atoms, and R¹⁷ and R¹⁸ are each independently optionally substituted by a hydroxy group.

R¹⁹ in the formula (7) is preferably a hydrogen atom, a benzyl group, or a *-Si (R¹³) (R¹⁴) (R¹⁵) group (wherein * is a bonding position, R¹³ is a tert-butyl group, and R¹⁴ and R¹⁵ are each a methyl group or a phenyl group), or a *-CO-R²⁰ group (wherein * is a bonding position, and R²⁰ is an alkyl group having 1 to 9 carbon atoms), more preferably a hydrogen atom, a *-Si (R¹³) (R¹⁴) (R¹⁵) group (wherein * is a bonding position, R¹³ is a tert-butyl group, and R¹⁴ and R¹⁵ are each a methyl group or a phenyl group), or a *-CO-R²⁰ group (wherein * is a bonding position, and R²⁰ is an alkyl group having 1 to 9 carbon atoms), further preferably a hydrogen atom, a *-Si (R¹³) (R¹⁴) (R¹⁵) group (wherein * is a bonding position, R¹³ is a tert-butyl group, and R¹⁴ and R¹⁵ are each a methyl group), or a *-CO-R²⁰ group (wherein * is a bonding position, and R²⁰ is an alkyl group having 1 to 9 carbon atoms).

R²¹ and R²² in the formula (8) are preferably each independently a hydrogen atom, a benzyl group, or a *-Si(R¹³) (R¹⁴) (R¹⁵) group (wherein * is a bonding position, R¹³ is a tert-butyl group, and R¹⁴ and R¹⁵ are each a methyl group or a phenyl group), more preferably each independently a hydrogen atom or a *-Si (R¹³) (R¹⁴) (R¹⁵) group (wherein * is a bonding position, R¹³ is a tert-butyl group, and R¹⁴ and R¹⁵ are each a methyl group or a phenyl group), further preferably each independently a hydrogen atom or a *-Si (R¹³) (R¹⁴) (R¹⁵) group (wherein * is a bonding position, R¹³ is a tert-butyl group, and R¹⁴ and R¹⁵ are each a methyl group).

R²³ in the formula (9) is
preferably a hydrogen atom, a benzyl group, or a *-Si(R¹³) (R¹⁴) (R¹⁵) group (wherein * is a bonding position, R¹³ is a tert-butyl group, and R¹⁴ and R¹⁵ are each a methyl group or a phenyl group),
more preferably a hydrogen atom or *-Si (R¹³) (R¹⁴) (R¹⁵) group (wherein * is a bonding position, R¹³ is a tert-butyl group, and R¹⁴ and R¹⁵ are each a methyl group or a phenyl group),
further preferably a hydrogen atom or a *-Si (R¹³) (R¹⁴) (R¹⁵) group (wherein * is a bonding position, R¹³ is a tert-butyl group, and R¹⁴ and R¹⁵ are each a methyl group).

R²⁴ in the formula (10) is preferably an alkylene group having 1 to 10 carbon atoms.

R²⁵ in the formula (10) is preferably an alkyl group having 1 to 30 carbon atoms.

R²⁶ in the formula (11) is preferably an alkylene group having 1 to 10 carbon atoms.

R²⁷ and R²⁸ in the formula (11) are preferably each independently an alkyl group having 2 to 10 carbon atoms or an alkenyl group having 2 to 10 carbon atoms, more preferably each independently an alkenyl group having 2 to 10 carbon atoms.

R²⁹ in the formula (12) is, as mentioned above, an alkylene group having 1 to 10 carbon atoms.

R³⁰ and R³¹ in the formula (12) are each independently an alkyl group having 1 to 10 carbon atoms.

Group (xiii), which is one of the options for R^{3a} and R^{3b} in formula (1), is preferably a monovalent group which is an alkyl group having 1 to 30 carbon atoms or an alkenyl group having 2 to 20 carbon atoms, wherein one ethylene group in the aforementioned alkyl group is optionally replaced by one ester bond. In other words, group (xiii) is preferably an alkyl group having 1 to 30 carbon atoms in which one ethylene group is optionally replaced by one ester bond, or an alkenyl group having 2 to 20 carbon atoms. Group (xiii) is more preferably an alkenyl group having 2 to 20 carbon atoms.

Group (xiii) is further preferably a monovalent group derived from oleic acid. In the present specification, the "monovalent group derived from oleic acid" means a monovalent group having a structure obtained by removing a carboxy group from oleic acid (i.e., (Z)-8-heptadecenyl group).

Group (xiv), which is one of the options for R^{3a} and R^{3b} in formula (1), is as mentioned above, R^{3c}-CO-(CH₂)ₚ- group (wherein R^{3c} is a residue of a liposoluble vitamin having a hydroxy group or a residue of a sterol derivative having a hydroxy group, and p is an integer of 1 to 8). The p is preferably 2 or 3. The liposoluble vitamin having a hydroxy group is preferably a tocopherol. The sterol derivative having a hydroxy group is preferably a cholesterol or a cholestanol, more preferably cholesterol. R^{3c} is preferably a residue of a liposoluble vitamin having a hydroxy group, more preferably a residue of tocopherol.

In another embodiment of the present invention, R^{3a} and R^{3b} are
preferably each independently group (i-2) (i.e., group (15)), group (16-20), group (16-22), group (iii) (i.e., group (2)), group (ix) (i.e., group (9)), group (xiii), or group (xiv),
more preferably each independently group (i-2) (i.e., group (15)) or group (xiii).

In another embodiment of the present invention, R^{3a} and R^{3b} are
preferably each independently group (16-20), group (xiii), or group (xiv),
more preferably each independently group (xiii).

In another embodiment of the present invention, R^{3a} and R^{3b} are preferably each independently
a monovalent group having 50 or less carbon atoms and represented by the formula (15): 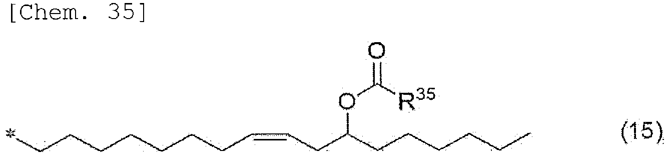 wherein, in the formula (15), * is a bonding position, and
R³⁵ is an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and R³⁵ is optionally substituted by a substituent selected from the group consisting of a 3- to 14-membered heterocyclic group and a hydrocarbon ring group having 3 to 12 carbon atoms,
a monovalent group having 50 or less carbon atoms and represented by the formula (16-20): 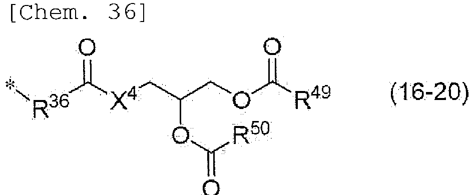 wherein, in the formula (16-20), * is a bonding position,
R³⁶ is an alkylene group having 2 to 9 carbon atoms, an alkenediyl group having 2 to 9 carbon atoms, or an alkynediyl group having 2 to 9 carbon atoms,
X⁴ is an oxygen atom, NH, or a sulfur atom, and
R⁴⁹ and R⁵⁰ are each independently an alkyl group having 1 to 17 carbon atoms, an alkenyl group having 2 to 17 carbon atoms, or an alkynyl group having 2 to 17 carbon atoms,
a monovalent group represented by the formula (2):

   *-R⁴-X¹-R⁵ (2)

   wherein, in the formula (2), * is a bonding position,
R⁴ is an alkylene group having 1 to 8 carbon atoms,
X¹ is *-NH-CO-O-** (wherein * is a bonding position with R⁴ in the formula (2), and ** is a bonding position with R⁵ in the formula (2)), or *-O-CO-O-* (wherein * is a bonding position), and
R⁵ is an alkyl group having 1 to 25 carbon atoms,
a monovalent group represented by the formula (9): 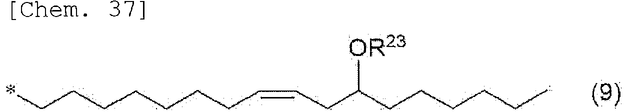 wherein, in the formula (9), * is a bonding position, and
R²³ is a hydrogen atom, a benzyl group, or a *-Si(R¹³) (R¹⁴) (R¹⁵) group (wherein * is a bonding position, R¹³ is a tert-butyl group, and R¹⁴ and R¹⁵ are each a methyl group or a phenyl group),
a monovalent group which is an alkyl group having 1 to 30 carbon atoms, an alkenyl group having 2 to 20 carbon atoms, or an alkynyl group having 2 to 20 carbon atoms, in which one ethylene group in the aforementioned alkyl group is optionally replaced by one ester bond, or
an R^{3c}-Co-(CH₂)ₚ- group (wherein R^{3c} is a residue of a liposoluble vitamin having a hydroxy group or a residue of a sterol derivative having a hydroxy group, and p is an integer of 1 to 8).

In another embodiment of the present invention, R^{3a} and R^{3b} are preferably each independently
a monovalent group having 50 or less carbon atoms and represented by the formula (15): 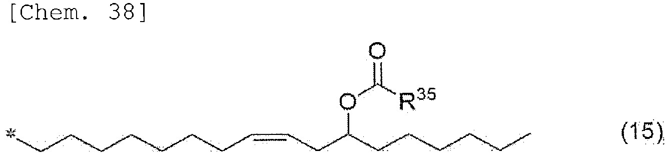 wherein, in the formula (15), * is a bonding position, and
R³⁵ is an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and the aforementioned alkyl group is optionally substituted by a hydrocarbon ring group having 3 to 12 carbon atoms,
a monovalent group having 50 or less carbon atoms and represented by the formula (16-20): 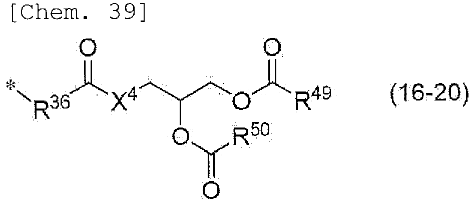 wherein, in the formula (16-20), * is a bonding position,
R³⁶ is an alkylene group having 2 to 9 carbon atoms or an alkenediyl group having 2 to 9 carbon atoms,
X⁴ is an oxygen atom or NH, and
R⁴⁹ and R⁵⁰ are each independently an alkyl group having 1 to 17 carbon atoms or an alkenyl group having 2 to 17 carbon atoms,
a monovalent group represented by the formula (2):

   *-R⁴-X⁴-R⁵ (2)

   wherein, in the formula (2), * is a bonding position,
R⁴ is an alkylene group having 1 to 6 carbon atoms,
X¹ is *-NH-CO-O-** (wherein * is a bonding position with R⁴ in the formula (2), and ** is a bonding position with R⁵ in the formula (2), and
R⁵ is an alkyl group having 1 to 25 carbon atoms,
a monovalent group represented by the formula (9): 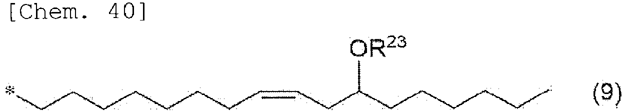 wherein, in the formula (9), * is a bonding position, and
R²³ is a hydrogen atom or a *-Si (R¹³) (R¹⁴) (R¹⁵) group (wherein * is a bonding position, R¹³ is a tert-butyl group, and R¹⁴ and R¹⁵ are each a methyl group or a phenyl group),
a monovalent group which is an alkyl group having 1 to 30 carbon atoms or an alkenyl group having 2 to 20 carbon atoms, in which one ethylene group in the aforementioned alkyl group is optionally replaced by one ester bond, or
an R^{3c}-CO- (CH₂)ₚ- group (wherein R^{3c} is a residue of a liposoluble vitamin having a hydroxy group, and p is 2 or 3).

In another embodiment of the present invention, R^{3a} and R^{3b} are preferably each independently
a monovalent group having 50 or less carbon atoms and represented by the formula (15): 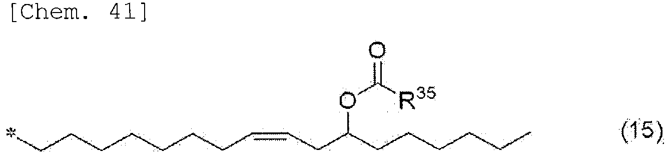 wherein, in the formula (15), * is a bonding position, and
R³⁵ is an alkyl group having 1 to 20 carbon atoms, an alkenyl group having 2 to 20 carbon atoms, or an alkynyl group having 2 to 20 carbon atoms, and the aforementioned alkyl group is optionally substituted by a cyclohexyl group,
a monovalent group having 50 or less carbon atoms and represented by the formula (16-20): 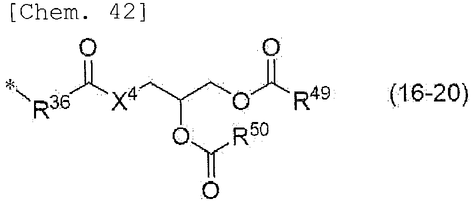 wherein, in the formula (16-20), * is a bonding position,
R³⁶ is an alkylene group having 2 or 3 carbon atoms or an alkenediyl group having 2 or 3 carbon atoms,
X⁴ is an oxygen atom, and
R⁴⁹ and R⁵⁰ are each independently an alkyl having 1 to 17 carbon atoms,
a monovalent group represented by the formula (2):

   *-R⁴-X¹-R⁵ (2)

   wherein, in the formula (2), * is a bonding position,
R⁴ is an alkylene group having 1 to 4 carbon atoms,
X¹ is *-NH-CO-O-** (wherein * is a bonding position with R⁴ in the formula (2), and ** is a bonding position with R⁵ in the formula (2)), and
R⁵ is an alkyl group having 1 to 25 carbon atoms, or
a monovalent group represented by the formula (9): 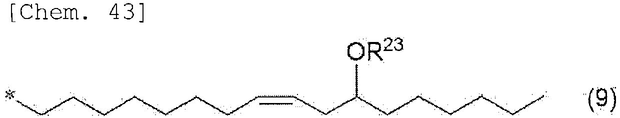 wherein, in the formula (9), * is a bonding position, and
R²³ is a hydrogen atom or a *-Si (R¹³) (R¹⁴) (R¹⁵) group (wherein * is a bonding position, R¹³ is a tert-butyl group, and R¹⁴ and R¹⁵ are each a methyl group),
an alkenyl group having 2 to 20 carbon atoms, or
an R^{3c}-CO- (CH₂)ₚ- group (wherein R^{3c} is a residue of a liposoluble vitamin having a hydroxy group, and p is 2 or 3).

In another embodiment of the present invention, R^{3a} and R^{3b} are preferably each independently
a monovalent group having 50 or less carbon atoms and represented by the formula (16-20): 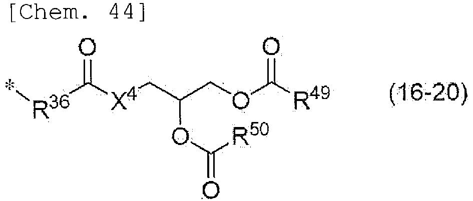 wherein, in the formula (16-20), * is a bonding position,
R³⁶ is an alkylene group having 2 to 9 carbon atoms, an alkenediyl group having 2 to 9 carbon atoms, or an alkynediyl group having 2 to 9 carbon atoms,
X⁴ is an oxygen atom, NH, or a sulfur atom, and
R⁴⁹ and R⁵⁰ are each independently an alkyl group having 1 to 17 carbon atoms, an alkenyl group having 2 to 17 carbon atoms, or an alkynyl group having 2 to 17 carbon atoms,
a monovalent group which is an alkyl group having 1 to 30 carbon atoms, an alkenyl group having 2 to 20 carbon atoms, or an alkynyl group having 2 to 20 carbon atoms, in which one ethylene group in the aforementioned alkyl group is optionally replaced by one ester bond, or
an R^{3c}-CO- (CH₂)ₚ- group (wherein R^{3c} is a residue of a liposoluble vitamin having a hydroxy group or a residue of a sterol derivative having a hydroxy group, and p is an integer of 1 to 8.

In another embodiment of the present invention, R^{3a} and R^{3b} are preferably each independently
a monovalent group having 50 or less carbon atoms and represented by the formula (16-20): 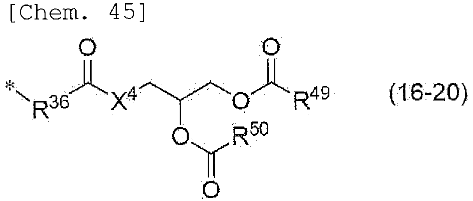 wherein, in the formula (16-20), * is a bonding position,
R³⁶ is an alkylene group having 2 to 9 carbon atoms or an alkenediyl group having 2 to 9 carbon atoms,
X⁴ is an oxygen atom or NH, and
R⁴⁹ and R⁵⁰ are each independently an alkyl group having 1 to 17 carbon atoms or an alkenyl group having 2 to 17 carbon atoms,
a monovalent group which is an alkyl group having 1 to 30 carbon atoms or an alkenyl group having 2 to 20 carbon atoms, in which one ethylene group in the aforementioned alkyl group is optionally replaced by one ester bond, or
an R^{3c}-CO- (CH₂)ₚ- group (wherein R^{3c} is a residue of a liposoluble vitamin having a hydroxy group, and p is 2 or 3).

In another embodiment of the present invention, R^{3a} and R^{3b} are preferably each independently
a monovalent group having 50 or less carbon atoms and represented by the formula (16-20): 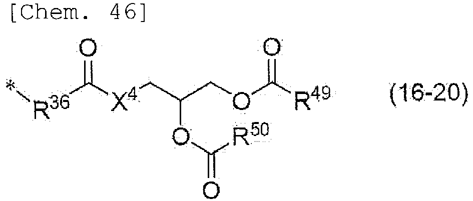 wherein, in the formula (16-20), * is a bonding position,
R³⁶ is an alkylene group having 2 or 3 carbon atoms or an alkenediyl group having 2 or 3 carbon atoms,
X⁴ is an oxygen atom, and
R⁴⁹ and R⁵⁰ are each independently an alkyl having 1 to 17 carbon atoms,
an alkenyl group having 2 to 20 carbon atoms, or
an R^{3c}-CO- (CH₂)ₚ- group (wherein R^{3c} is a residue of a liposoluble vitamin having a hydroxy group, and p is 2 or 3).

In another embodiment of the present invention, R^{3a} and R^{3b} are
preferably each independently a monovalent group which is an alkyl group having 1 to 30 carbon atoms, an alkenyl group having 2 to 20 carbon atoms, or an alkynyl group having 2 to 20 carbon atoms, in which one ethylene group in the aforementioned alkyl group is optionally replaced by one ester bond,
more preferably each independently a monovalent group which is an alkyl group having 1 to 30 carbon atoms or an alkenyl group having 2 to 20 carbon atoms, in which one ethylene group in the aforementioned alkyl group is optionally replaced by one ester bond,
further preferably each independently an alkenyl group having 2 to 20 carbon atoms.

R^{3a} may be the same as or different from R^{3b}, and R^{3a} is preferably the same group as R^{3b}.

In one embodiment of the present invention, R^{1a} is the same as R^{1b}, X^{a} is the same as X^{b}, R^{2a} is the same as R^{2b}, Y^{a} is the same as Y^{b}, Z^{a} is the same as Z^{b}, and R^{3a} is the same as R^{3b}.

Preferred examples of the ionic lipid represented by the formula (1) (sometimes to be abbreviated as "ionic lipid (1)" in the present specification) include the following ionic lipids.

### [Ionic lipid (1-1a)]

Ionic lipid (1) wherein
R^{1a} and R^{1b} are each independently an alkylene group having 1 to 6 carbon atoms (e.g., methylene group, ethylene group);
X^{a} and X^{b} are each independently an acyclic alkyl tertiary amino group having 1 to 6 carbon atoms and 1 tertiary amino group (e.g., -N(CH₃)-), or a cyclic alkylene tertiary amino group having 2 to 5 carbon atoms and 1 to 2 tertiary amino groups (e.g., piperidinediyl group);
R^{2a} and R^{2b} are each independently an alkylene group having 1 to 8 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 to 16 carbon atoms and at least one aromatic ring, and optionally having a hetero atom (e.g., -C₆H₄-CH₂-, -CH₂-C₆H₄-CH₂-) ;
na and nb are each independently 0 or 1; and
R^{3a} and R^{3b} are each independently
a monovalent group having 50 or less carbon atoms and represented by the formula (15): 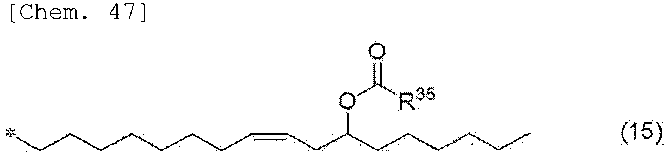 wherein, in the formula (15), * is a bonding position, and
R³⁵ is an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and R³⁵ is optionally substituted by a substituent selected from the group consisting of a 3- to 14-membered heterocyclic group and a hydrocarbon ring group having 3 to 12 carbon atoms,
a monovalent group having 50 or less carbon atoms and represented by the formula (16-20): 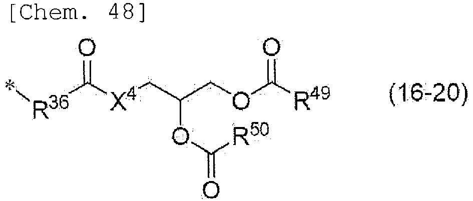 wherein, in the formula (16-20), * is a bonding position,
R³⁶ is an alkylene group having 2 to 9 carbon atoms, an alkenediyl group having 2 to 9 carbon atoms, or an alkynediyl group having 2 to 9 carbon atoms,
X⁴ is an oxygen atom, NH, or a sulfur atom, and
R⁴⁹ and R⁵⁰ are each independently an alkyl group having 1 to 17 carbon atoms, an alkenyl group having 2 to 17 carbon atoms, or an alkynyl group having 2 to 17 carbon atoms,
a monovalent group having 50 or less carbon atoms and represented by the formula (16-22): 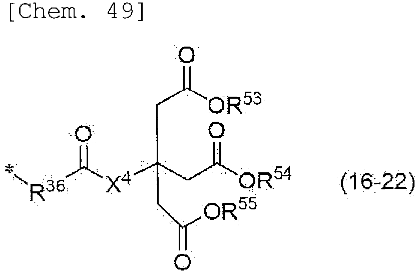 wherein, in the formula (16-22), * is a bonding position,
R³⁶ is an alkylene group having 2 to 9 carbon atoms, an alkenediyl group having 2 to 9 carbon atoms, or an alkynediyl group having 2 to 9 carbon atoms,
X⁴ is an oxygen atom, NH, or a sulfur atom, and
R⁵³ to R⁵⁵ are each independently an alkyl group having 1 to 10 carbon atoms, an alkenyl group having 2 to 10 carbon atoms, or an alkynyl group having 2 to 10 carbon atoms,
a monovalent group represented by the formula (2):

   *-R⁴-X¹-R⁵ (2)

   wherein, in the formula (2), * is a bonding position,
R⁴ is an alkylene group having 1 to 8 carbon atoms,
X¹ is *-NH-CO-O-** (wherein * is a bonding position with R⁴ in the formula (2), and ** is a bonding position with R⁵ in the formula (2)), or *-O-CO-O-* (wherein * is a bonding position), and
R⁵ is an alkyl group having 1 to 25 carbon atoms,
a monovalent group represented by the formula (9): 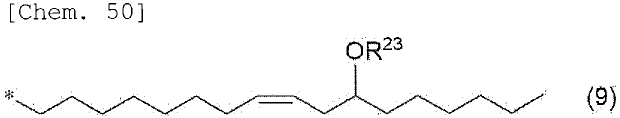 wherein, in the formula (9), * is a bonding position, and
R²³ is a hydrogen atom, a benzyl group, or a *-Si(R¹³) (R¹⁴) (R¹⁵) group (wherein * is a bonding position, R¹³ is a tert-butyl group, and R¹⁴ and R¹⁵ are each a methyl group or a phenyl group),
a monovalent group which is an alkyl group having 1 to 30 carbon atoms, an alkenyl group having 2 to 20 carbon atoms, or an alkynyl group having 2 to 20 carbon atoms, in which one ethylene group in the aforementioned alkyl group is optionally replaced by one ester bond, or
an R^{3c}-CO-(CH₂)ₚ- group (wherein R^{3c} is a residue of a liposoluble vitamin having a hydroxy group or a residue of a sterol derivative having a hydroxy group, and p is an integer of 1 to 8).

### [Ionic lipid (1-2a)]

Ionic lipid (1) wherein
R^{1a} and R^{1b} are each independently an alkylene group having 1 to 4 carbon atoms (e.g., methylene group, ethylene group);
X^{a} and X^{b} are each independently an acyclic alkyl tertiary amino group having 1 to 3 carbon atoms and 1 tertiary amino group (e.g., -N(CH₃)-), or a cyclic alkylene tertiary amino group having 2 to 5 carbon atoms and 1 to 2 tertiary amino groups (e.g., piperidinediyl group);
R^{2a} and R^{2b} are each independently an alkylene group having 1 to 6 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 6 to 12 carbon atoms and one aromatic ring, and optionally having a hetero atom (e.g., - C₆H₄-CH₂-, -CH₂-C₆H₄-CH₂-) ;
na and nb are each independently 0 or 1; and
R^{3a} and R^{3b} are each independently
a monovalent group having 50 or less carbon atoms and represented by the formula (15): 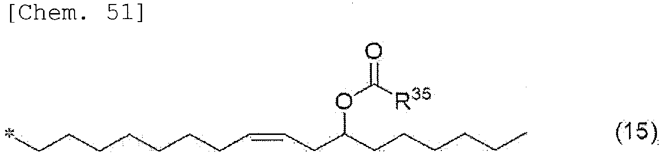 wherein, in the formula (15), * is a bonding position, and
R³⁵ is an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and the aforementioned alkyl group is optionally substituted by a hydrocarbon ring group having 3 to 12 carbon atoms,
a monovalent group having 50 or less carbon atoms and represented by the formula (16-20): 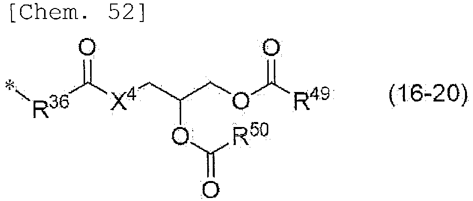 wherein, in the formula (16-20), * is a bonding position,
R³⁶ is an alkylene group having 2 to 9 carbon atoms or an alkenediyl group having 2 to 9 carbon atoms (preferably an alkylene group having 2 to 9 carbon atoms),
X⁴ is an oxygen atom or NH, and
R⁴⁹ and R⁵⁰ are each independently an alkyl group having 1 to 17 carbon atoms or an alkenyl group having 2 to 17 carbon atoms,
a monovalent group having 50 or less carbon atoms and represented by the formula (16-22): 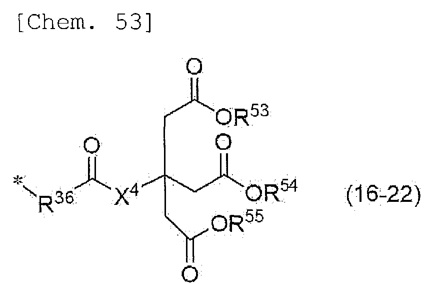 wherein, in the formula (16-22), * is a bonding position,
R³⁶ is an alkylene group having 2 to 9 carbon atoms or an alkenediyl group having 2 to 9 carbon atoms (preferably an alkylene group having 2 to 9 carbon atoms),
X⁴ is an oxygen atom or NH, and
R⁵³ to R⁵⁵ are each independently an alkyl group having 1 to 10 carbon atoms,
a monovalent group represented by the formula (2):

   *-R⁴-X¹-R⁵ (2)

   wherein, in the formula (2), * is a bonding position,
R⁴ is an alkylene group having 1 to 6 carbon atoms,
X¹ is *-NH-CO-O-** (wherein * is a bonding position with R⁴ in the formula (2), and ** is a bonding position with R⁵ in the formula (2)), and
R⁵ is an alkyl group having 1 to 25 carbon atoms,
a monovalent group represented by the formula (9): 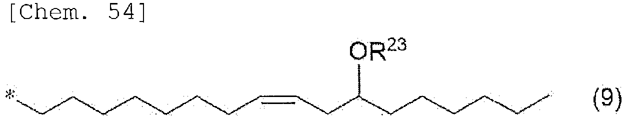 wherein, in the formula (9), * is a bonding position, and
R²³ is a hydrogen atom or a *-Si (R¹³) (R¹⁴) (R¹⁵) group (wherein * is a bonding position, R¹³ is a tert-butyl group, and R¹⁴ and R¹⁵ are each a methyl group or a phenyl group),
a monovalent group which is an alkyl group having 1 to 30 carbon atoms or an alkenyl group having 2 to 20 carbon atoms, in which one ethylene group in the aforementioned alkyl group is optionally replaced by one ester bond, or
an R^{3c}-CO-(CH₂)ₚ- group (wherein R^{3c} is a residue of a liposoluble vitamin having a hydroxy group, and p is 2 or 3).

### [Ionic lipid (1-3a)]

Ionic lipid (1) wherein
R^{1a} and R^{1b} are each independently an alkylene group having 1 to 2 carbon atoms (e.g., methylene group, ethylene group);
X^{a} and X^{b} are each independently a divalent group represented by the formula (25): 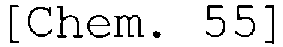 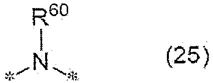 wherein, in the formula (25), * is a bonding position, and
R⁶⁰ is a methyl group, an ethyl group, a propyl group, or an isopropyl group, or
   a divalent group represented by the formula (26): 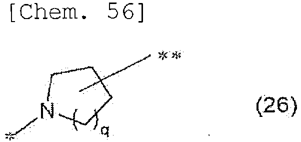
   wherein, in the formula (26), * is a bonding position with R^{1a} or R^{1b} in the formula (1),
** is a bonding position with R^{2a} or R^{2b} in the formula (1), and
q is 1 or 2;
R^{2a} and R^{2b} are each independently an alkylene group having 1 to 4 carbon atoms (e.g., methylene group, ethylene group, trimethylene group);
Y^{a} and Y^{b} are each independently an ester bond or an amide bond;
Z^{a} and Z^{b} are each independently a divalent group represented by the formula (13): 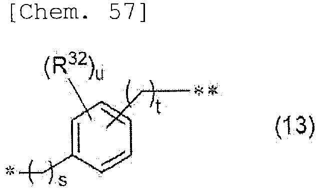 wherein, in the formula (13), * is a bonding position with O in the formula (1),
** is a bonding position with Y^{a} or Y^{b} in the formula (1),
s is 0 or 1,
t is an integer of 0 to 2,
u is an integer of 0 to 2 (preferably 0), and
R³² in the number of u are each independently a substituent;
na and nb are each independently 0 or 1; and
R^{3a} and R^{3b} are each independently
a monovalent group having 50 or less carbon atoms and represented by the formula (15): 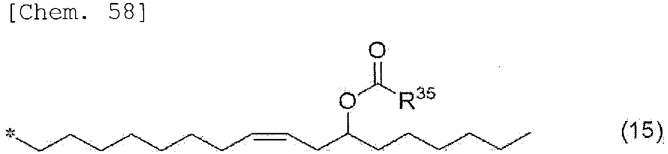 wherein, in the formula (15), * is a bonding position, and
R³⁵ is an alkyl group having 1 to 20 carbon atoms, an alkenyl group having 2 to 20 carbon atoms, or an alkynyl group having 2 to 20 carbon atoms, and the aforementioned alkyl group is optionally substituted by a cyclohexyl group,
a monovalent group having 50 or less carbon atoms and represented by the formula (16-20): 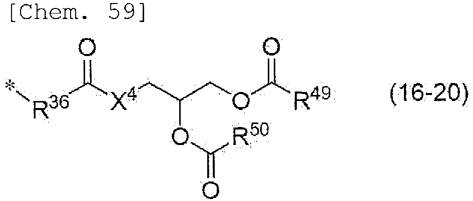 wherein, in the formula (16-20), * is a bonding position,
R³⁶ is an alkylene group having 2 or 3 carbon atoms or an alkenediyl group having 2 or 3 carbon atoms (preferably an alkylene group having 2 or 3 carbon atoms),
X⁴ is an oxygen atom, and
R⁴⁹ and R⁵⁰ are each independently an alkyl having 1 to 17 carbon atoms,
a monovalent group having 50 or less carbon atoms and represented by the formula (16-22): 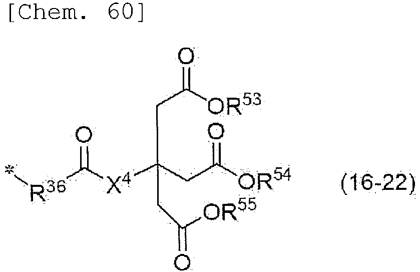 wherein, in the formula (16-22), * is a bonding position,
R³⁶ is an alkylene group having 2 or 3 carbon atoms or an alkenediyl group having 2 or 3 carbon atoms (preferably an alkylene group having 2 or 3 carbon atoms),
X⁴ is an oxygen atom, and
R⁵³ to R⁵⁵ are each independently an alkyl group having 1 to 10 carbon atoms,
a monovalent group represented by the formula (2):

   *-R⁴-X¹-R⁵ (2)

   wherein, in the formula (2), * is a bonding position,
R⁴ is an alkylene group having 1 to 4 carbon atoms,
X¹ is *-NH-CO-O-** (wherein * is a bonding position with R⁴ in the formula (2), and ** is a bonding position with R⁵ in the formula (2)), and
R⁵ is an alkyl group having 1 to 25 carbon atoms,
a monovalent group represented by the formula (9): 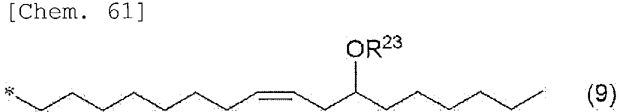 wherein, in the formula (9), * is a bonding position, and
R²³ is a hydrogen atom or a *-Si (R¹³) (R¹⁴) (R¹⁵) group (wherein * is a bonding position, R¹³ is a tert-butyl group, and R¹⁴ and R¹⁵ are each a methyl group or a phenyl group),
an alkenyl group having 2 to 20 carbon atoms, or
an R^{3c}-CO- (CH₂)ₚ- group (wherein R^{3c} is a residue of a liposoluble vitamin having a hydroxy group or a hydroxy group, and p is 2).

### [Ionic lipid (1-1b)]

Ionic lipid (1) wherein
R¹³ and R^{1b} are each independently an alkylene group having 1 to 6 carbon atoms (e.g., methylene group, ethylene group);
X^{a} and X^{b} are each independently an acyclic alkyl tertiary amino group having 1 to 6 carbon atoms and 1 tertiary amino group (e.g., -N(CH₃)-), or a cyclic alkylene tertiary amino group having 2 to 5 carbon atoms and 1 to 2 tertiary amino groups (e.g., piperidinediyl group);
R^{2a} and R^{2b} are each independently an alkylene group having 1 to 8 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 to 16 carbon atoms and at least one aromatic ring, and optionally having a hetero atom (e.g., -C₆H₄-CH₂-, -CH₂-C₆H₄-CH₂-) ;
na and nb are each independently 0 or 1; and
R^{3a} and R^{3b} are each independently
a monovalent group having 50 or less carbon atoms and represented by the formula (16-20): 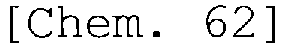 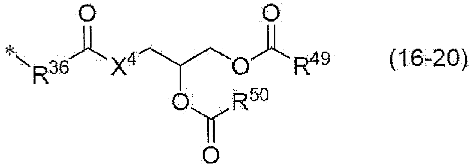 wherein, in the formula (16-20), * is a bonding position,
R³⁶ is an alkylene group having 2 to 9 carbon atoms, an alkenediyl group having 2 to 9 carbon atoms, or an alkynediyl group having 2 to 9 carbon atoms,
X⁴ is an oxygen atom, NH, or a sulfur atom, and
R⁴⁹ and R⁵⁰ are each independently an alkyl group having 1 to 17 carbon atoms, an alkenyl group having 2 to 17 carbon atoms, or an alkynyl group having 2 to 17 carbon atoms,
a monovalent group which is an alkyl group having 1 to 30 carbon atoms, an alkenyl group having 2 to 20 carbon atoms, or an alkynyl group having 2 to 20 carbon atoms, in which one ethylene group in the aforementioned alkyl group is optionally replaced by one ester bond, or
an R^{3c}-CO-(CH₂)ₚ- group (wherein R^{3c} is a residue of a liposoluble vitamin having a hydroxy group or a residue of a sterol derivative having a hydroxy group, and p is an integer of 1 to 8).

### [Ionic lipid (1-2b)]

Ionic lipid (1) wherein
R^{1a} and R^{1b} are each independently an alkylene group having 1 to 4 carbon atoms (e.g., methylene group, ethylene group);
X^{a} and X^{b} are each independently an acyclic alkyl tertiary amino group having 1 to 3 carbon atoms and 1 tertiary amino group (e.g., -N(CH₃)-), or a cyclic alkylene tertiary amino group having 2 to 5 carbon atoms and 1 to 2 tertiary amino groups (e.g., piperidinediyl group);
R^{2a} and R^{2b} are each independently an alkylene group having 1 to 6 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 6 to 12 carbon atoms and one aromatic ring, and optionally having a hetero atom (e.g., - C₆H₄-CH₂-, -CH₂-C₆H₄-CH₂-) ;
na and nb are each independently 0 or 1; and
R^{3a} and R^{3b} are each independently
a monovalent group having 50 or less carbon atoms and represented by the formula (16-20): 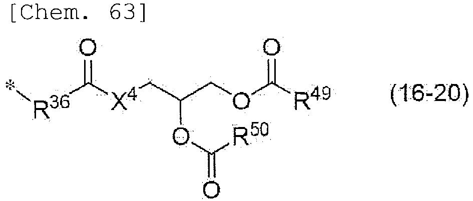 wherein, in the formula (16-20), * is a bonding position,
R³⁶ is an alkylene group having 2 to 9 carbon atoms or an alkenediyl group having 2 to 9 carbon atoms (preferably an alkylene group having 2 to 9 carbon atoms),
X⁴ is an oxygen atom or NH, and
R⁴⁹ and R⁵⁰ are each independently an alkyl group having 1 to 17 carbon atoms or an alkenyl group having 2 to 17 carbon atoms,
a monovalent group which is an alkyl group having 1 to 30 carbon atoms or an alkenyl group having 2 to 20 carbon atoms, in which one ethylene group in the aforementioned alkyl group is optionally replaced by one ester bond, or
an R^{3c}-CO-(CH₂)ₚ- group (wherein R^{3c} is a residue of a liposoluble vitamin having a hydroxy group, and p is 2 or 3).

### [Ionic lipid (1-3b)]

Ionic lipid (1) wherein
R^{1a} and R^{1b} are each independently an alkylene group having 1 to 2 carbon atoms (e.g., methylene group, ethylene group);
X^{a} and X^{b} are each independently a divalent group represented by the formula (25): 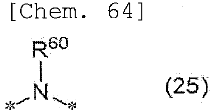 wherein, in the formula (25), * is a bonding position, and
R⁶⁰ is a methyl group, an ethyl group, a propyl group, or an isopropyl group, or
   a divalent group represented by the formula (26): 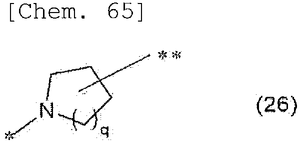
   wherein, in the formula (26), * is a bonding position with R^{1a} or R^{1b} in the formula (1),
** is a bonding position with R^{2a} or R^{2b} in the formula (1), and
q is 1 or 2;
R^{2a} and R^{2b} are each independently an alkylene group having 1 to 4 carbon atoms (e.g., methylene group, ethylene group, trimethylene group);
Y^{a} and Y^{b} are each independently an ester bond or an amide bond;
Z^{a} and Z^{b} are each independently a divalent group represented by the formula (13): 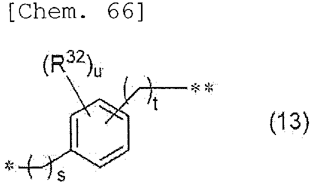 wherein, in the formula (13), * is a bonding position with O in the formula (1),
** is a bonding position with Y^{a} or Y^{b} in the formula (1),
s is 0 or 1,
t is an integer of 0 to 2,
u is an integer of 0 to 2 (preferably 0), and
R³² in the number of u are each independently a substituent;
na and nb are each independently 0 or 1; and
R^{3a} and R^{3b} are each independently
a monovalent group having 50 or less carbon atoms and represented by the formula (16-20): 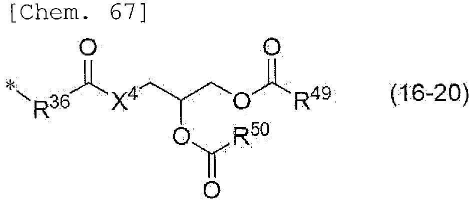 wherein, in the formula (16-20), * is a bonding position,
R³⁶ is an alkylene group having 2 or 3 carbon atoms or an alkenediyl group having 2 or 3 carbon atoms (preferably an alkylene group having 2 or 3 carbon atoms),
X⁴ is an oxygen atom, and
R⁴⁹ and R⁵⁰ are each independently an alkyl having 1 to 17 carbon atoms,
an alkenyl group having 2 to 20 carbon atoms, or
an R^{3c}-CO-(CH₂)ₚ- group (wherein R^{3c} is a residue of a liposoluble vitamin having a hydroxy group or a hydroxy group, and p is 2).

### [Ionic lipid (1-1c)]

Ionic lipid (1) wherein
R^{1a} and R^{1b} are each independently an alkylene group having 1 to 6 carbon atoms (e.g., methylene group, ethylene group) ;
X^{a} and X^{b} are each independently an acyclic alkyl tertiary amino group having 1 to 6 carbon atoms and 1 tertiary amino group (e.g., -N(CH₃)-), or a cyclic alkylene tertiary amino group having 2 to 5 carbon atoms and 1 to 2 tertiary amino groups (e.g., piperidinediyl group);
R^{2a} and R^{2b} are each independently an alkylene group having 1 to 8 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 to 16 carbon atoms and at least one aromatic ring, and optionally having a hetero atom (e.g., -C₆H₄-CH₂-, -CH₂-C₆H₄-CH₂-) ;
na and nb are each independently 0 or 1; and
R^{3a} and R^{3b} are each independently
a monovalent group having 50 or less carbon atoms and represented by the formula (15): 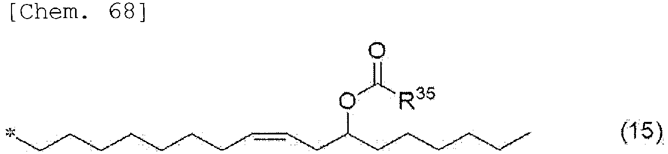 wherein, in the formula (15), * is a bonding position, and
R³⁵ is an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, R³⁵ is optionally substituted by a substituent selected from the group consisting of a 3- to 14-membered heterocyclic group and a hydrocarbon ring group having 3 to 12 carbon atoms, or
a monovalent group which is an alkyl group having 1 to 30 carbon atoms, an alkenyl group having 2 to 20 carbon atoms, or an alkynyl group having 2 to 20 carbon atoms, in which one ethylene group in the aforementioned alkyl group is optionally replaced by one ester bond.

### [Ionic lipid (1-2c)]

Ionic lipid (1) wherein
R¹³ and R^{1b} are each independently an alkylene group having 1 to 4 carbon atoms (e.g., methylene group, ethylene group);
X^{a} and X^{b} are each independently an acyclic alkyl tertiary amino group having 1 to 3 carbon atoms and 1 tertiary amino group (e.g., -N(CH₃)-), or a cyclic alkylene tertiary amino group having 2 to 5 carbon atoms and 1 to 2 tertiary amino groups (e.g., piperidinediyl group);
R^{2a} and R^{2b} are each independently an alkylene group having 1 to 6 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 6 to 12 carbon atoms and one aromatic ring, and optionally having a hetero atom (e.g., - C₆H₉-CH₂-, -CH₂-C₆H₄-CH₂-) ;
na and nb are each independently 0 or 1; and
R^{3a} and R^{3b} are each independently
a monovalent group having 50 or less carbon atoms and represented by the formula (15): 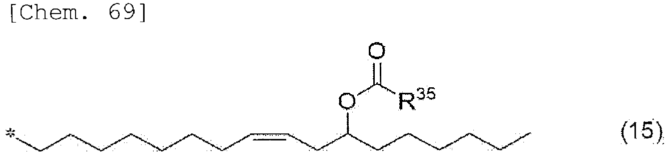 wherein, in the formula (15), * is a bonding position, and
R³⁵ is an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, the aforementioned alkyl group is optionally substituted by a hydrocarbon ring group having 3 to 12 carbon atoms, or
a monovalent group which is an alkyl group having 1 to 30 carbon atoms or an alkenyl group having 2 to 20 carbon atoms, in which one ethylene group in the aforementioned alkyl group is optionally replaced by one ester bond.

### [Ionic lipid (1-3c)]

Ionic lipid (1) wherein
R^{1a} and R^{1b} are each independently an alkylene group having 1 to 2 carbon atoms (e.g., methylene group, ethylene group);
X^{a} and X^{b} are each independently a divalent group represented by the formula (25): 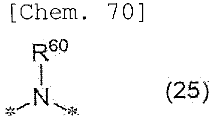 wherein, in the formula (25), * is a bonding position, and
R⁶⁰ is a methyl group, an ethyl group, a propyl group, or an isopropyl group, or
   a divalent group represented by the formula (26): 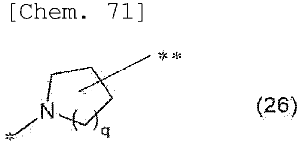
   wherein, in the formula (26), * is a bonding position with R^{1a} or R^{1b} in the formula (1),
** is a bonding position with R^{2a} or R^{2b} in the formula (1), and
q is 1 or 2;
R^{2a} and R^{2b} are each independently an alkylene group having 1 to 4 carbon atoms (e.g., methylene group, ethylene group, trimethylene group);
Y^{a} and Y^{b} are each independently an ester bond or an amide bond;
Z^{a} and Z^{b} are each independently a divalent group represented by the formula (13): 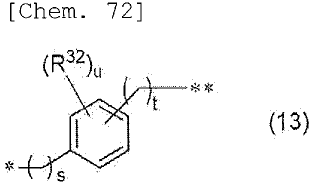 wherein, in the formula (13), * is a bonding position with O in the formula (1),
** is a bonding position with Y^{a} or Y^{b} in the formula (1),
s is 0 or 1,
t is an integer of 0 to 2,
u is an integer of 0 to 2 (preferably 0), and
R³² in the number of u are each independently a substituent;
na and nb are each independently 0 or 1; and
R^{3a} and R^{3b} are each independently
a monovalent group having 50 or less carbon atoms and represented by the formula (15): 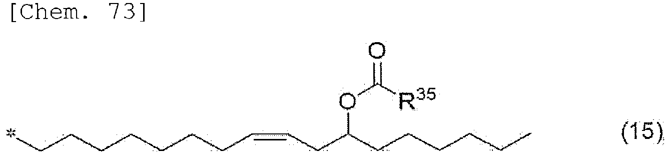 wherein, in the formula (15), * is a bonding position, and
R³⁵ is an alkyl group having 1 to 20 carbon atoms, an alkenyl group having 2 to 20 carbon atoms, or an alkynyl group having 2 to 20 carbon atoms, and the aforementioned alkyl group is optionally substituted by a cyclohexyl group, or
an alkenyl group having 2 to 20 carbon atoms.

### [Ionic lipid (1-1d)]

Ionic lipid (1) wherein
R^{1a} and R^{1b} are each independently an alkylene group having 1 to 6 carbon atoms (e.g., methylene group, ethylene group);
X^{a} and X^{b} are each independently an acyclic alkyl tertiary amino group having 1 to 6 carbon atoms and 1 tertiary amino group (e.g., -N(CH₃)-), or a cyclic alkylene tertiary amino group having 2 to 5 carbon atoms and 1 to 2 tertiary amino groups (e.g., piperidinediyl group);
R^{2a} and R^{2b} are each independently an alkylene group having 1 to 8 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 to 16 carbon atoms and at least one aromatic ring, and optionally having a hetero atom (e.g., -C₆H₄-CH₂-, -CH₂-C₆H₄-CH₂-) ;
na and nb are each independently 0 or 1; and
R^{3a} and R^{3b} are each independently a monovalent group which is an alkyl group having 1 to 30 carbon atoms, an alkenyl group having 2 to 20 carbon atoms, or an alkynyl group having 2 to 20 carbon atoms, in which one ethylene group in the aforementioned alkyl group is optionally replaced by one ester bond.

### [Ionic lipid (1-2d)]

Ionic lipid (1) wherein
R^{1a} and R^{1b} are each independently an alkylene group having 1 to 4 carbon atoms (e.g., methylene group, ethylene group) ;
X^{a} and X^{b} are each independently an acyclic alkyl tertiary amino group having 1 to 3 carbon atoms and 1 tertiary amino group (e.g., -N(CH₃)-), or a cyclic alkylene tertiary amino group having 2 to 5 carbon atoms and 1 to 2 tertiary amino groups (e.g., piperidinediyl group);
R^{2a} and R^{2b} are each independently an alkylene group having 1 to 6 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 6 to 12 carbon atoms and one aromatic ring, and optionally having a hetero atom (e.g., - C₆H₄-CH₂-, -CH₂-C₆H₄-CH₂-) ;
na and nb are each independently 0 or 1; and
R^{3a} and R^{3b} are each independently a monovalent group which is an alkyl group having 1 to 30 carbon atoms or an alkenyl group having 2 to 20 carbon atoms, in which one ethylene group in the aforementioned alkyl group is optionally replaced by one ester bond.

### [Ionic lipid (1-3d)]

Ionic lipid (1) wherein
R¹³ and R^{1b} are each independently an alkylene group having 1 to 2 carbon atoms (e.g., methylene group, ethylene group);
X^{a} and X^{b} are each independently a divalent group represented by the formula (25): 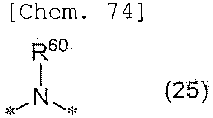 wherein, in the formula (25), * is a bonding position, and
R⁶⁰ is a methyl group, an ethyl group, a propyl group, or an isopropyl group, or
   a divalent group represented by the formula (26): 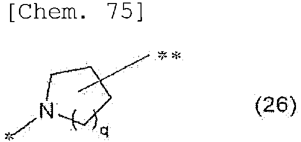
   wherein, in the formula (26), * is a bonding position with R¹³ or R^{1b} in the formula (1),
** is a bonding position with R^{2a} or R^{2b} in the formula (1), and
q is 1 or 2;
R^{2a} and R^{2b} are each independently an alkylene group having 1 to 4 carbon atoms (e.g., methylene group, ethylene group, trimethylene group);
Y^{a} and Y^{b} are each independently an ester bond or an amide bond;
Z^{a} and Z^{b} are each independently a divalent group represented by the formula (13): 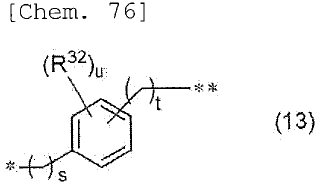 wherein, in the formula (13), * is a bonding position with O in the formula (1),
** is a bonding position with Y^{a} or Y^{b} in the formula (1),
s is 0 or 1,
t is an integer of 0 to 2,
u is an integer of 0 to 2 (preferably 0), and
R³² in the number of u are each independently a substituent;
na and nb are each independently 0 or 1; and
R^{3a} and R^{3b} are each independently an alkenyl group having 2 to 20 carbon atoms.

Specific examples of ionic lipid (1) include compounds 1 to 198 described in the following Table 1-1 to Table 1-38 (hereinafter these are collectively referred to as "Table 1"). In Table 1, "TBS" means tert-butyldimethylsilyl and "Me" means methyl.

**[Table 1-1]**

| name | structure |
|---|---|
| compound 1 | 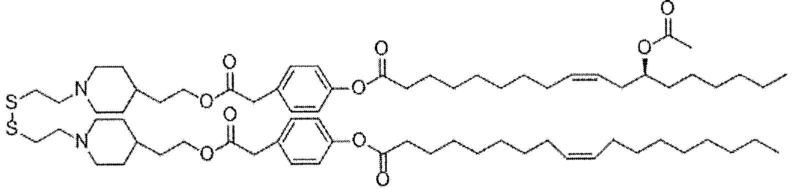 |
| compound 2 | 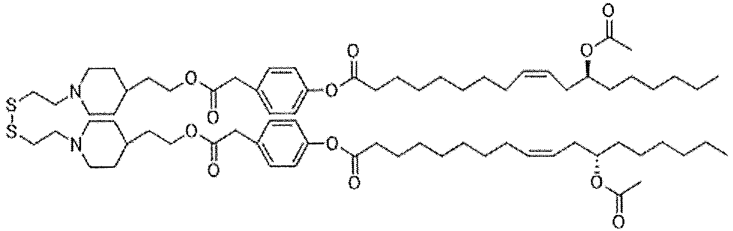 |
| compound 3 | 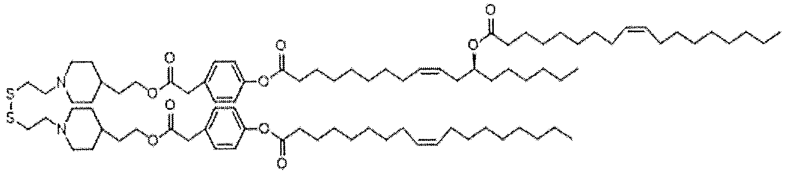 |
| compound 4 | 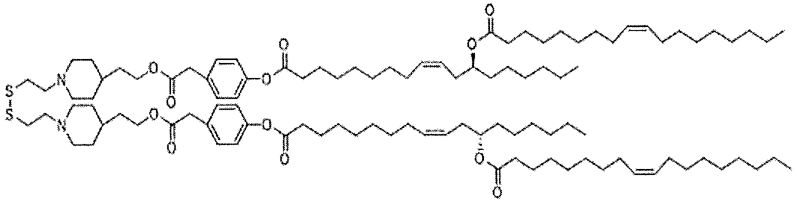 |
| compound 5 | 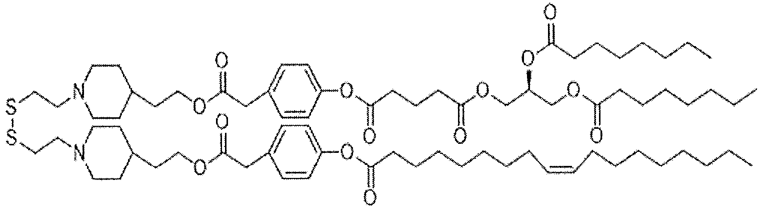 |
| compound 6 | 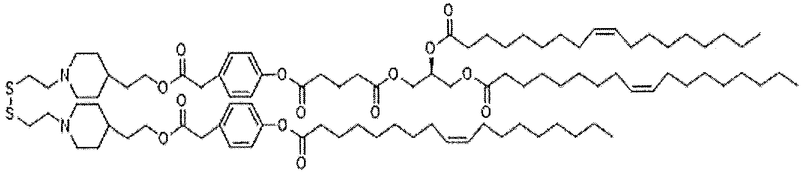 |

**[Table 1-2]**

| name | structure |
|---|---|
| compound 7 | 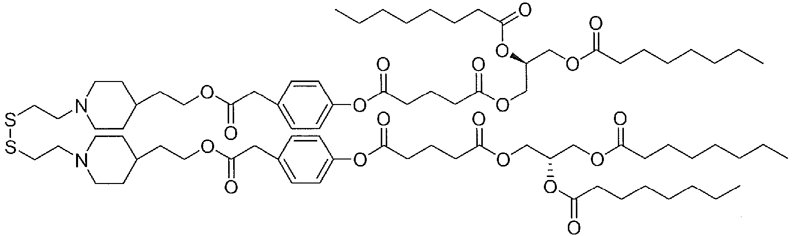 |
| compound 8 | 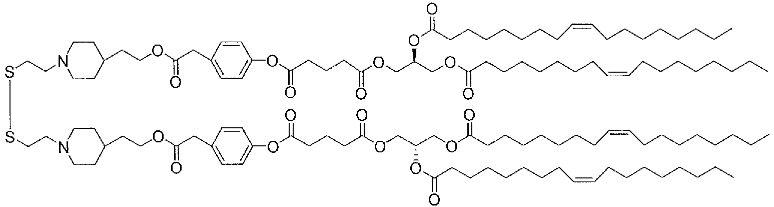 |
| compound 9 | 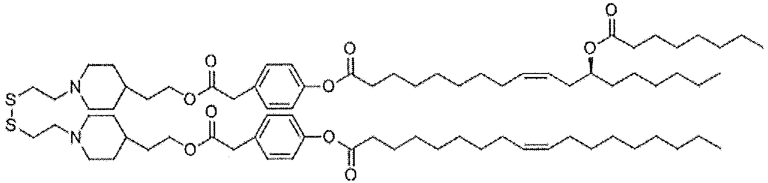 |
| compound 10 | 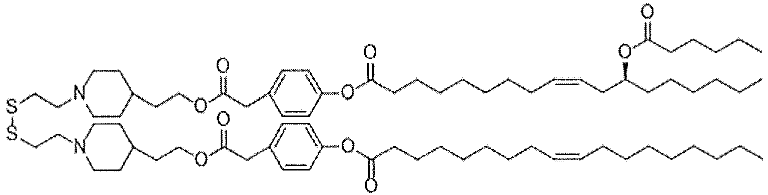 |
| compound 11 | 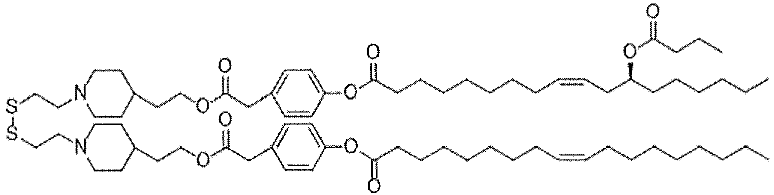 |
| compound 12 | 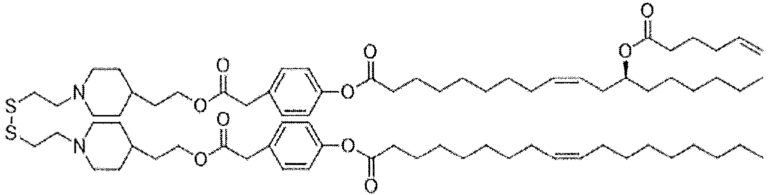 |

**[Table 1-3]**

| name | structure |
|---|---|
| compound 13 | 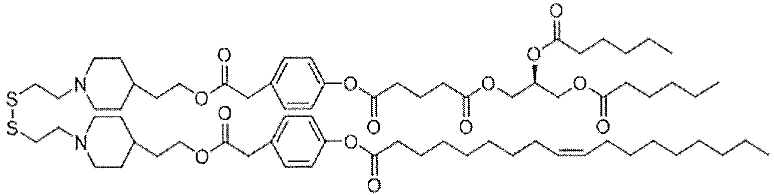 |
| compound 14 | 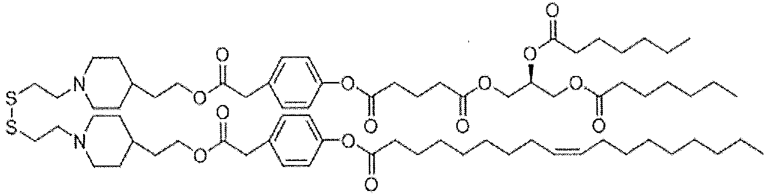 |
| compound 15 | 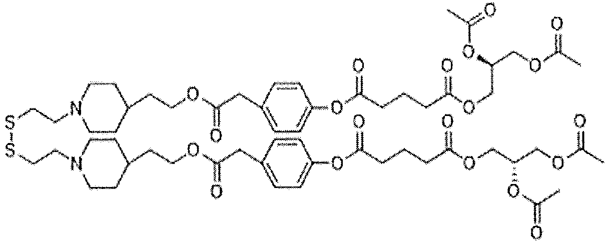 |
| compound 16 | 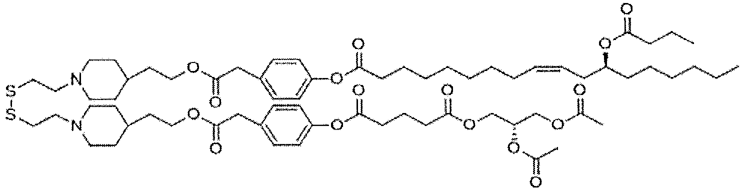 |
| compound 17 | 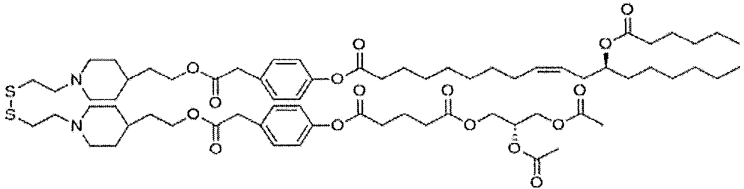 |
| compound 18 | 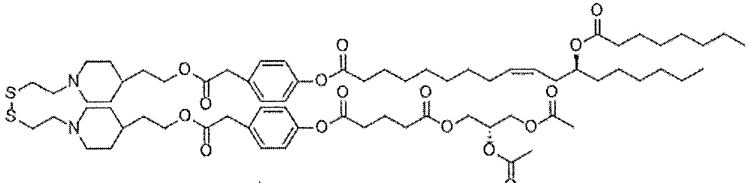 |

**[Table 1-4]**

| name | structure |
|---|---|
| compound 19 | 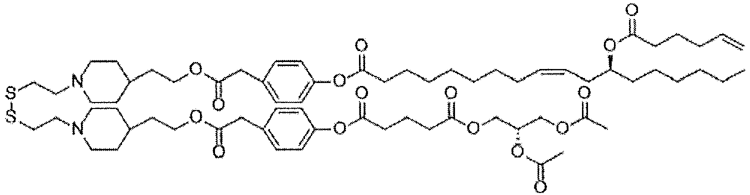 |
| compound 20 | 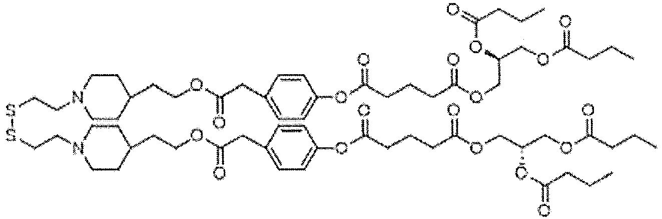 |
| compound 21 | 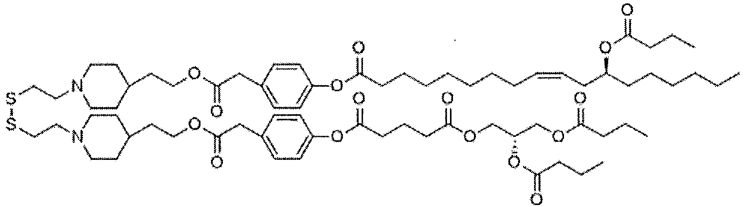 |
| compound 22 | 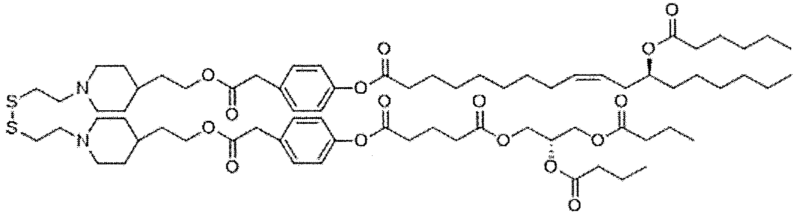 |
| compound 23 | 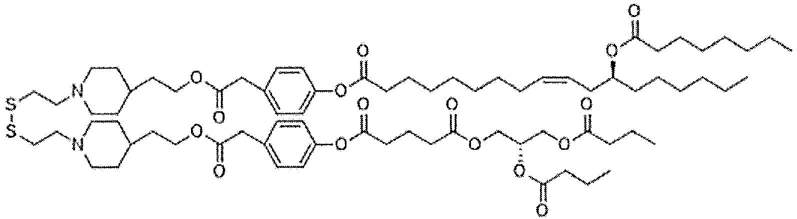 |
| compound 24 | 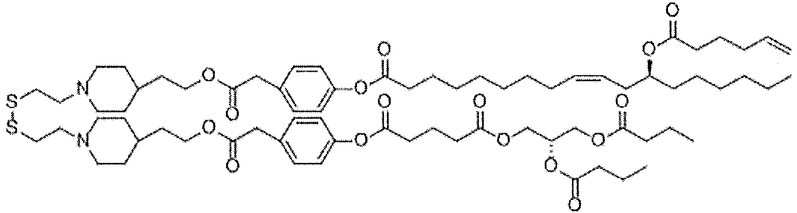 |

**[Table 1-5]**

| name | structure |
|---|---|
| compound 25 | 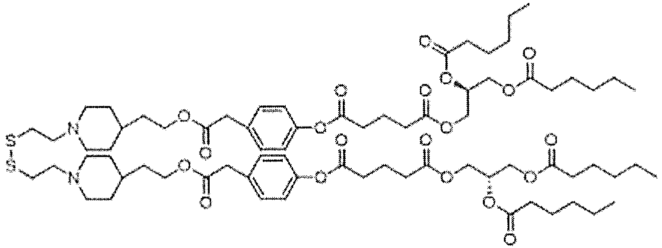 |
| compound 26 | 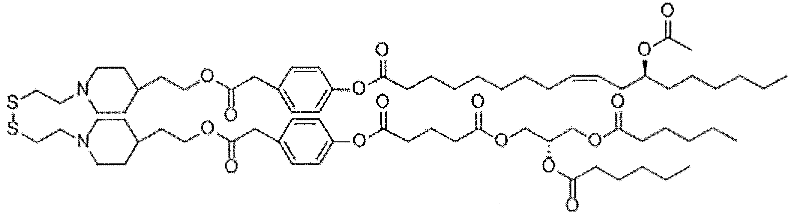 |
| compound 27 | 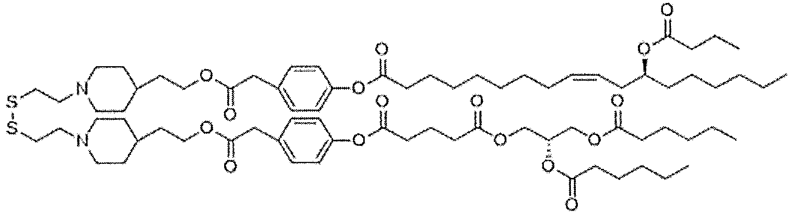 |
| compound 28 | 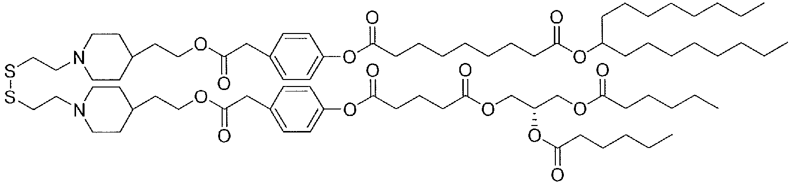 |
| compound 29 | 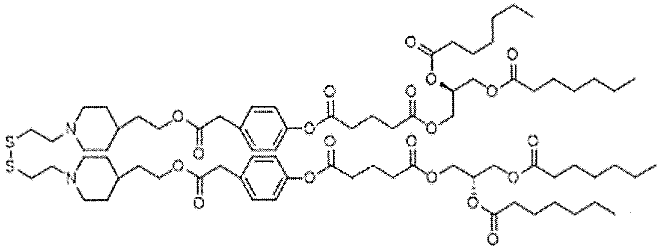 |
| compound 30 | 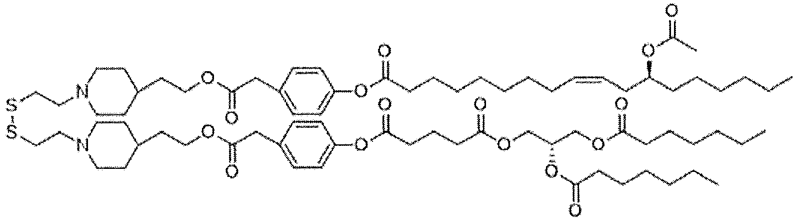 |

**[Table 1-6]**

| name | structure |
|---|---|
| compound 31 | 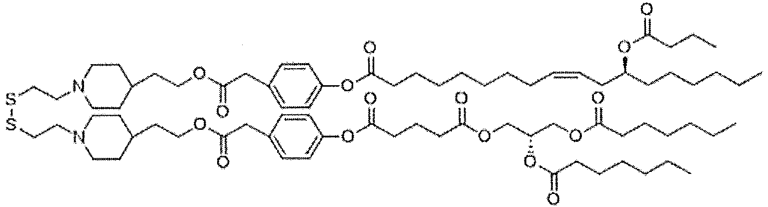 |
| compound 32 | 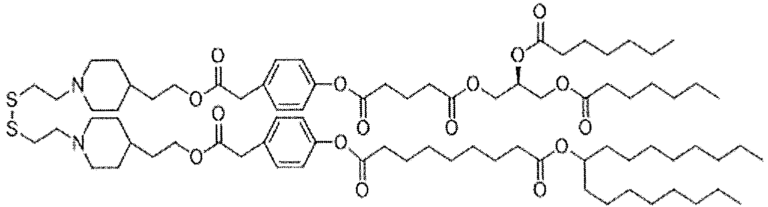 |
| compound 33 | 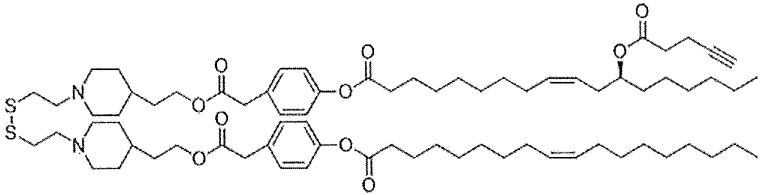 |
| compound 34 | 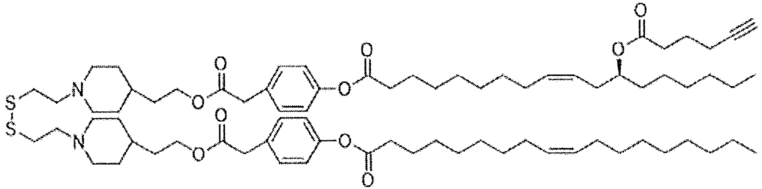 |
| compound 35 | 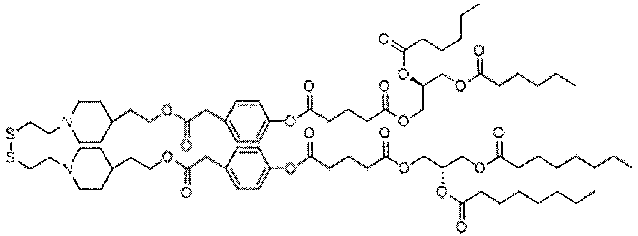 |
| compound 36 | 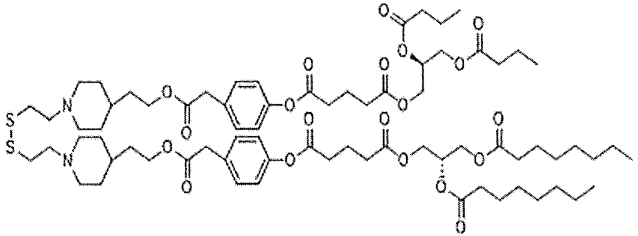 |

**[Table 1-7]**

| name | structure |
|---|---|
| compound 37 | 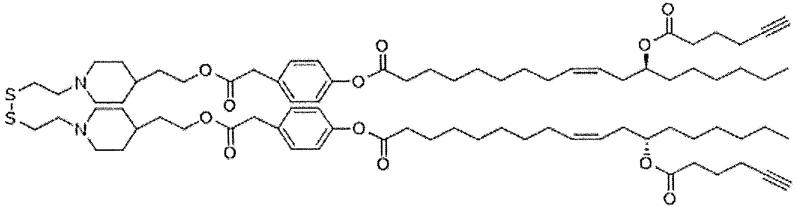 |
| compound 38 | 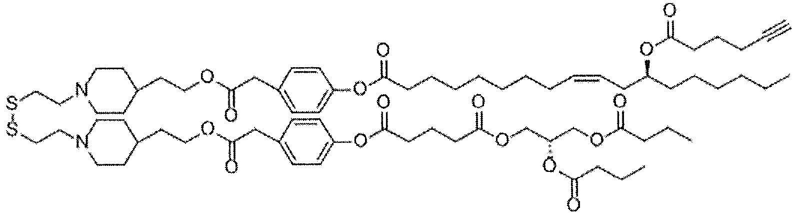 |
| compound 39 | 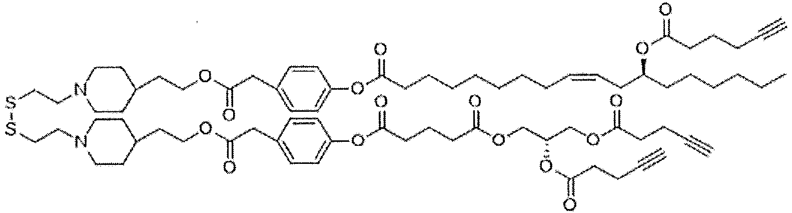 |
| compound 40 | 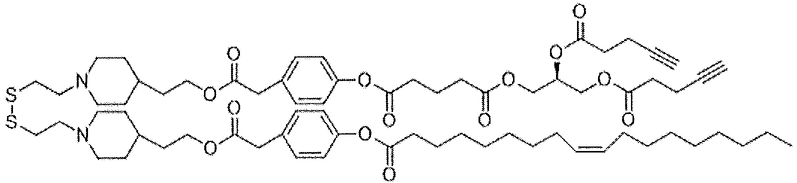 |
| compound 41 | 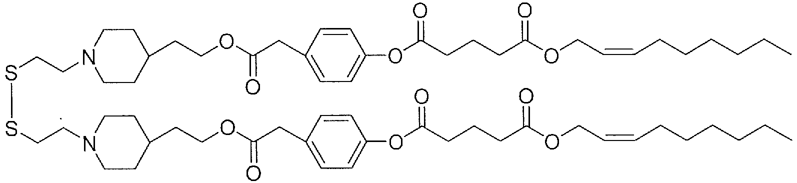 |
| compound 42 | 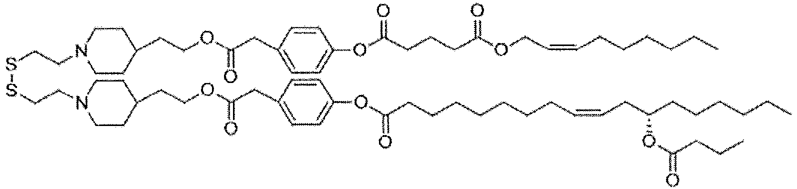 |

**[Table 1-8]**

| name | structure |
|---|---|
| compound 43 | 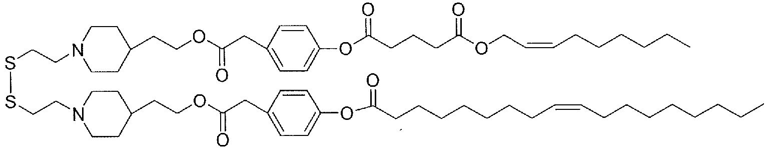 |
| compound 44 | 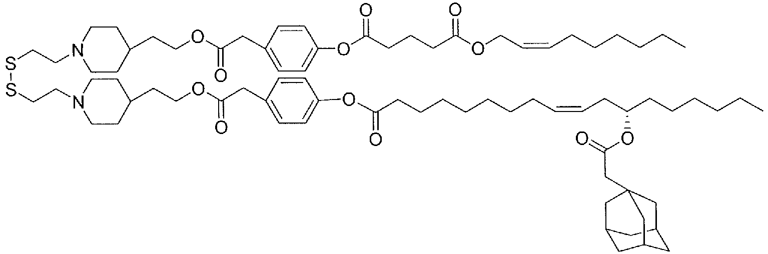 |
| compound 45 | 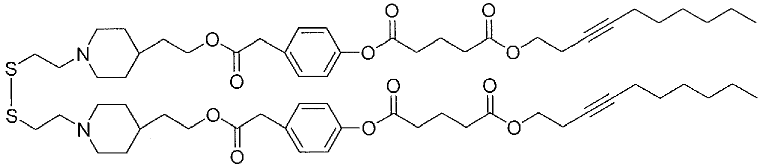 |
| compound 46 | 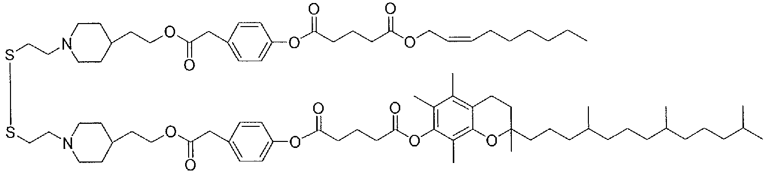 |
| compound 47 | 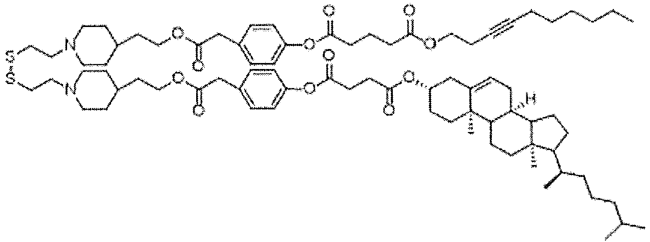 |
| compound 48 | 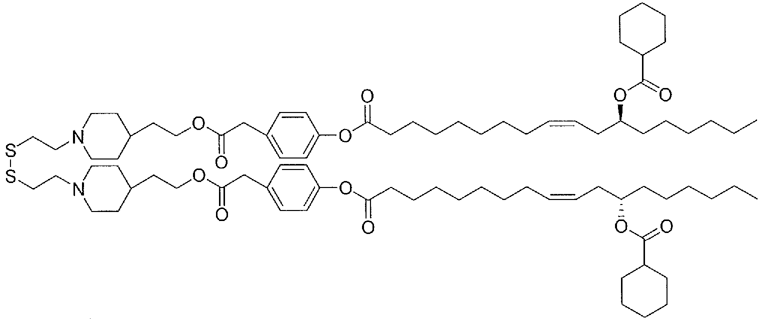 |

**[Table 1-9]**

| name | structure |
|---|---|
| compound 49 | 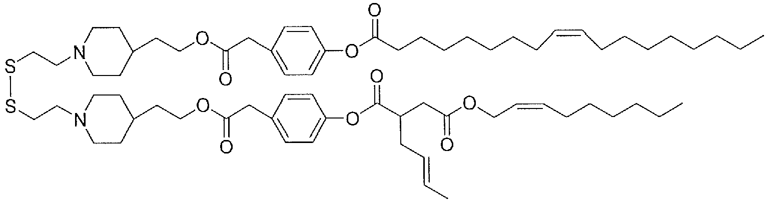 |
| compound 50 | 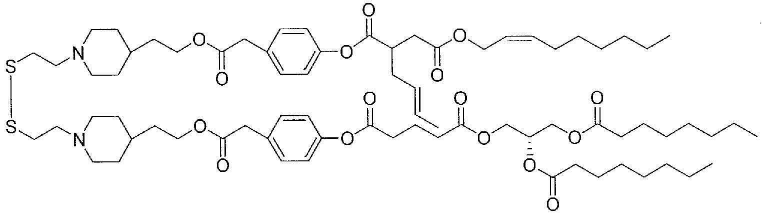 |
| compound 51 | 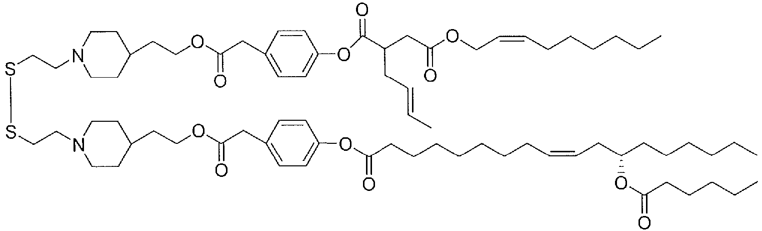 |
| compound 52 | 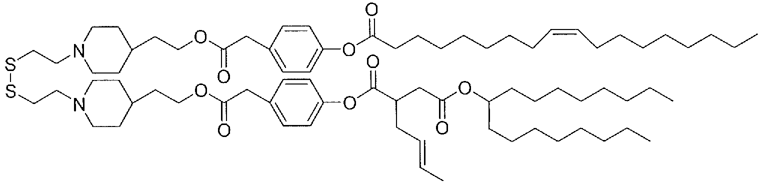 |
| compound 53 | 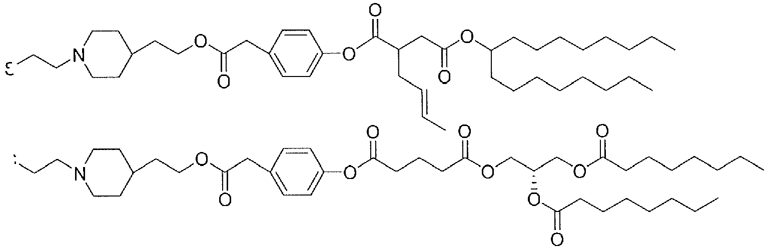 |
| compound 54 | 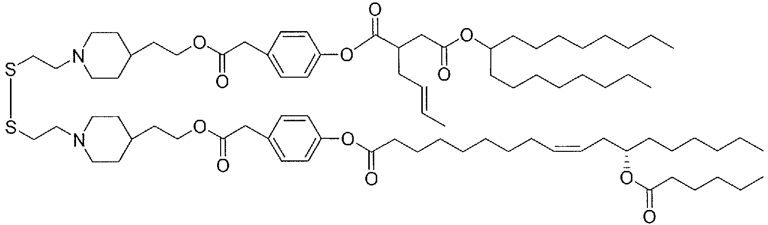 |

**[Table 1-10]**

| name | structure |
|---|---|
| compound 55 | 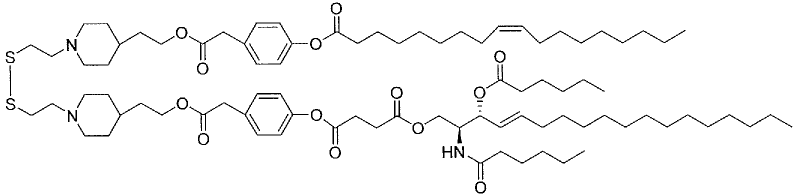 |
| compound 56 | 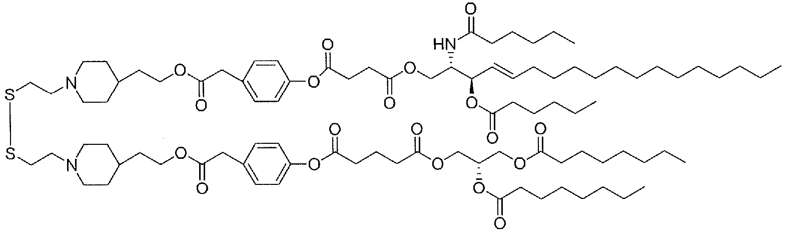 |
| compound 57 | 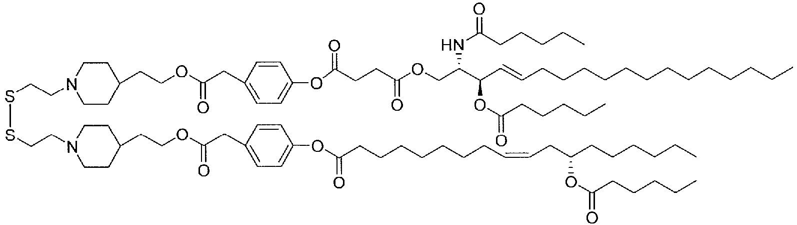 |
| compound 58 | 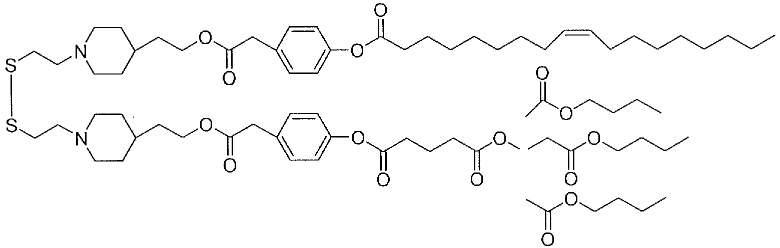 |
| compound 59 | 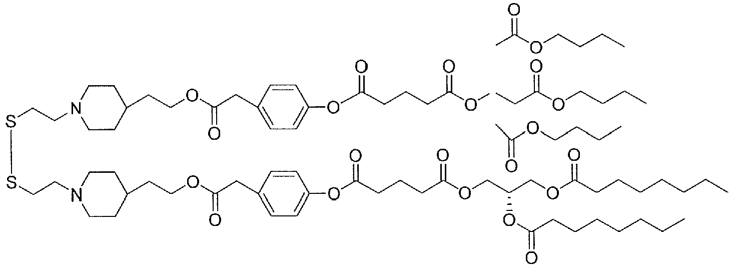 |

**[Table 1-11]**

| name | structure |
|---|---|
| compound 60 | 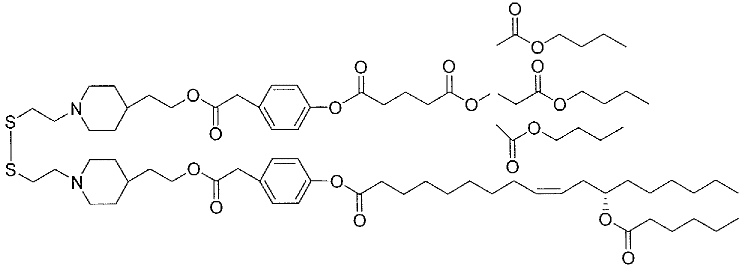 |
| compound 61 | 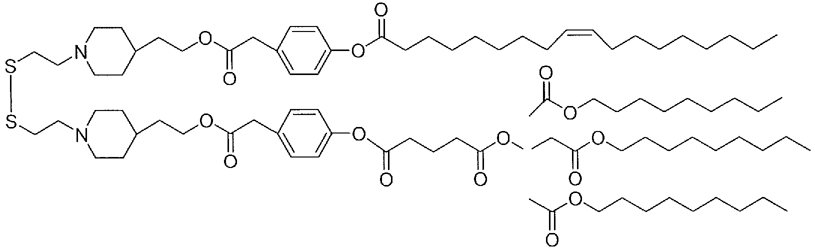 |
| compound 62 | 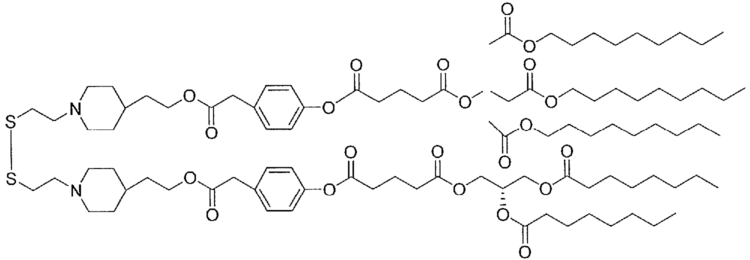 |
| compound 63 | 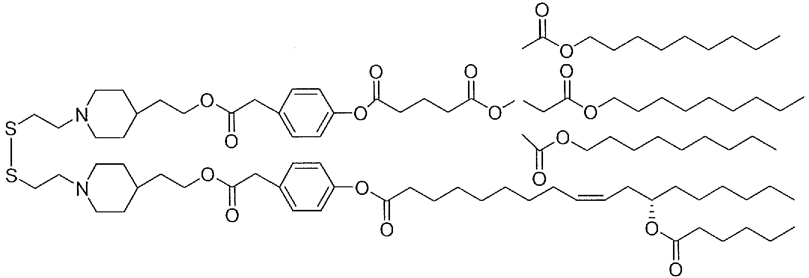 |
| compound 64 | 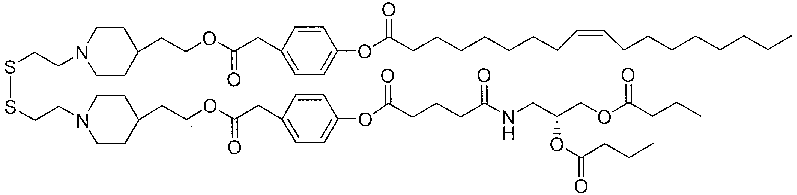 |

**[Table 1-12]**

| name | structure |
|---|---|
| compound 65 | 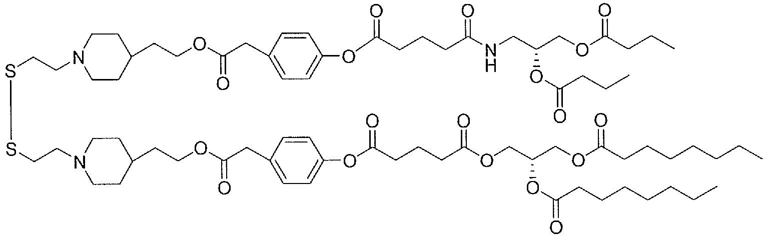 |
| compound 66 | 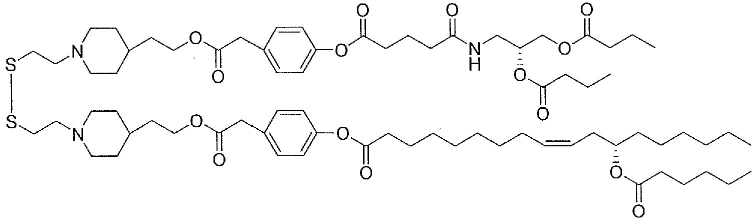 |
| compound 67 | 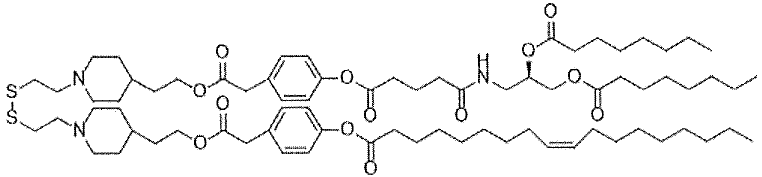 |
| compound 68 | 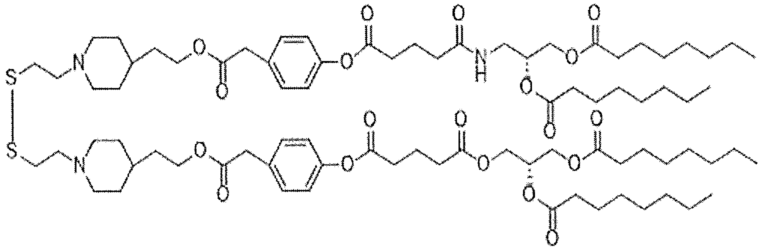 |
| compound 69 | 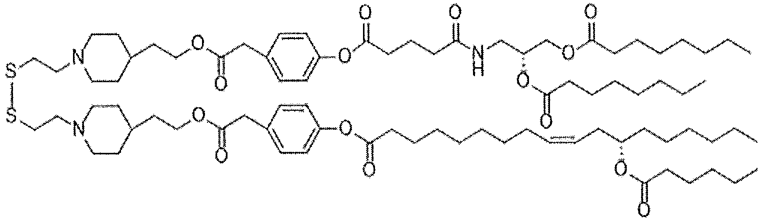 |
| compound 70 | 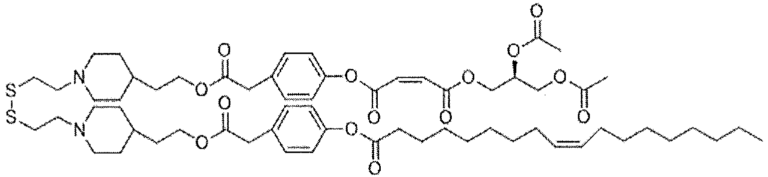 |

**[Table 1-13]**

| name | structure |
|---|---|
| compound 71 | 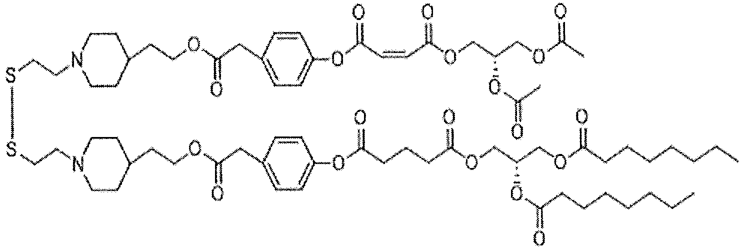 |
| compound 72 | 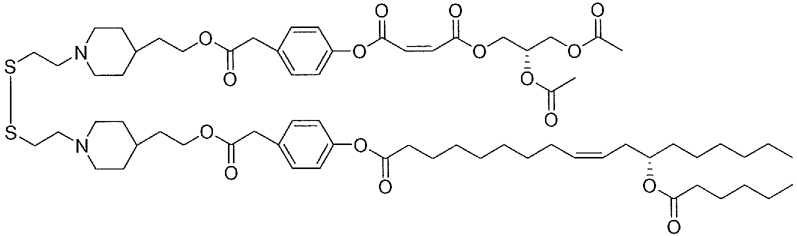 |
| compound 73 | 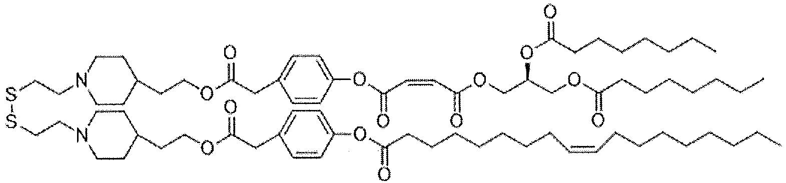 |
| compound 74 | 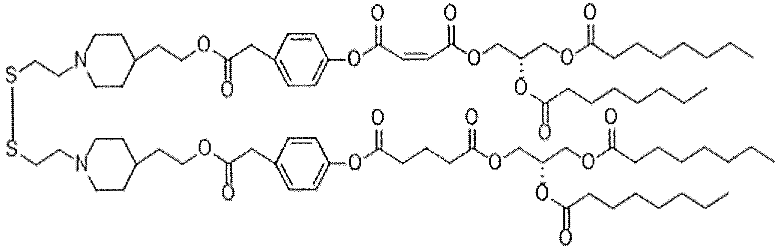 |
| compound 75 | 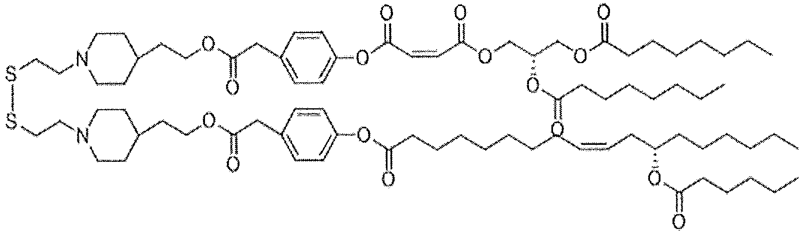 |
| compound 76 | 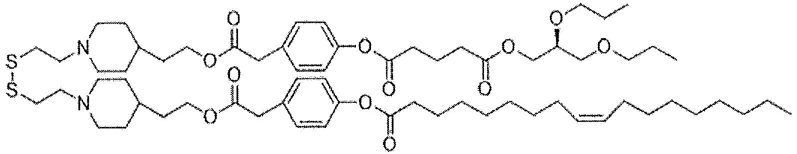 |

**[Table 1-14]**

| name | structure |
|---|---|
| compound 77 | 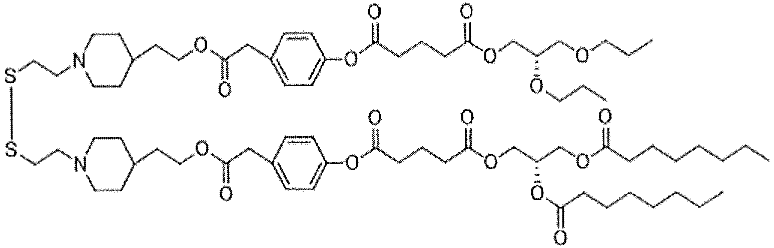 |
| compound 78 | 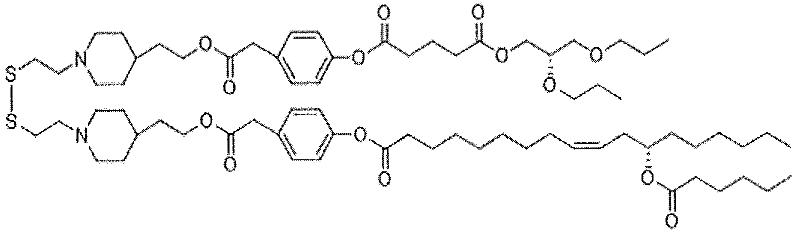 |
| compound 79 | 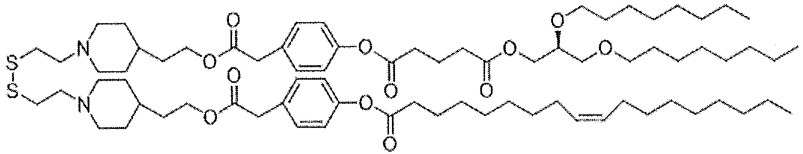 |
| compound 80 | 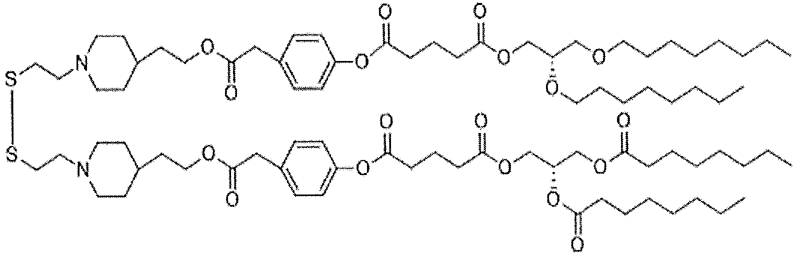 |
| compound 81 | 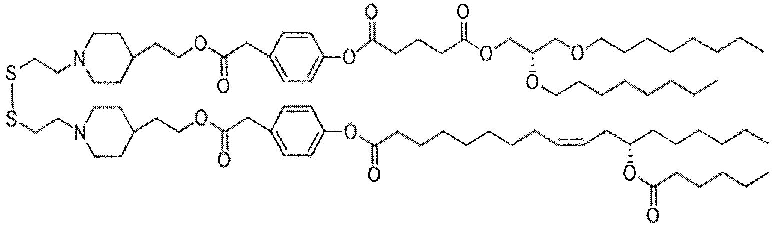 |
| compound 82 | 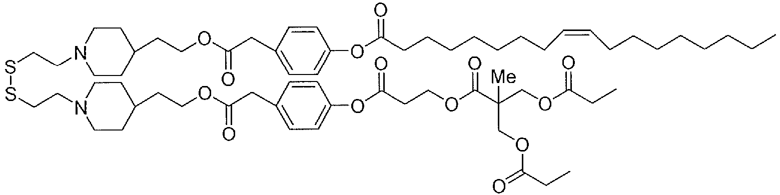 |

**[Table 1-15].**

| name | structure |
|---|---|
| compound 83 | 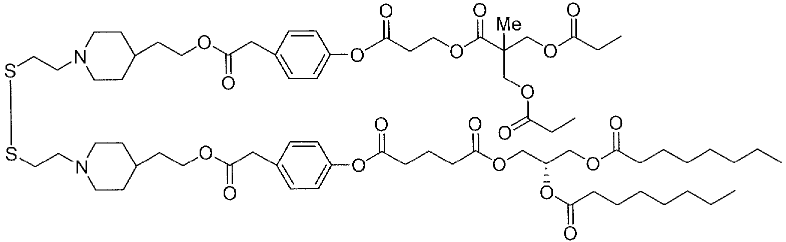 |
| compound 84 | 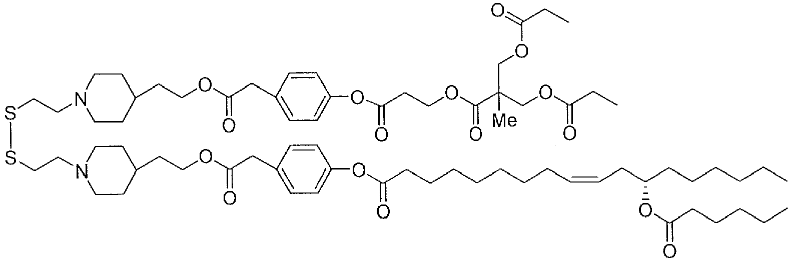 |
| compound 85 | 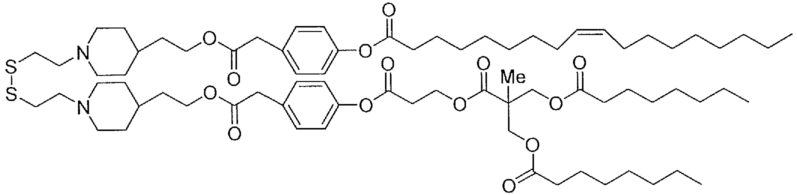 |
| compound 86 | 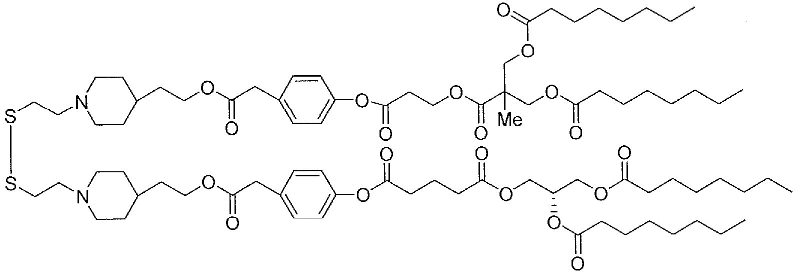 |
| compound 87 | 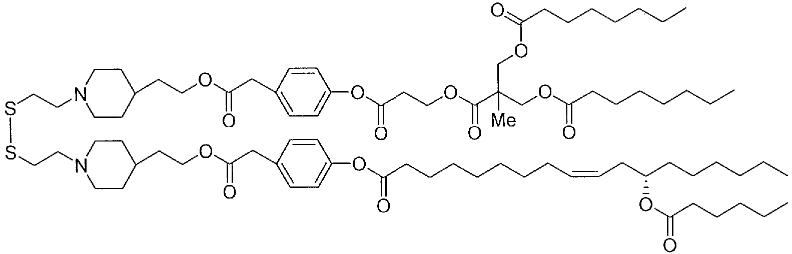 |

**[Table 1-16]**

| name | structure |
|---|---|
| compound 88 | 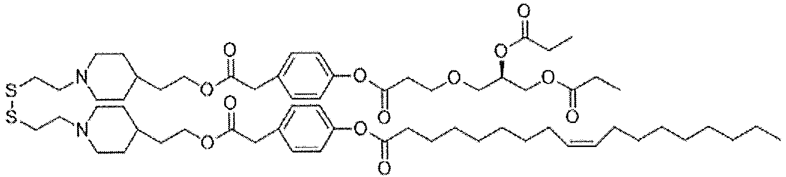 |
| compound 89 | 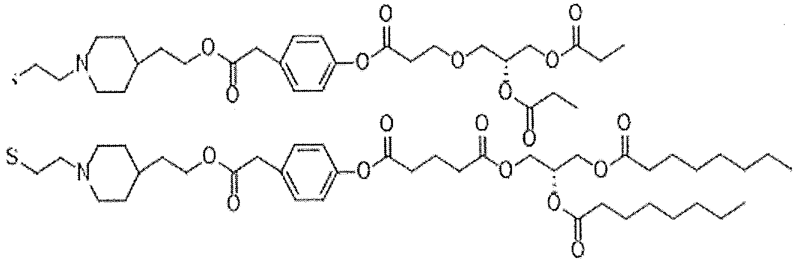 |
| compound 90 | 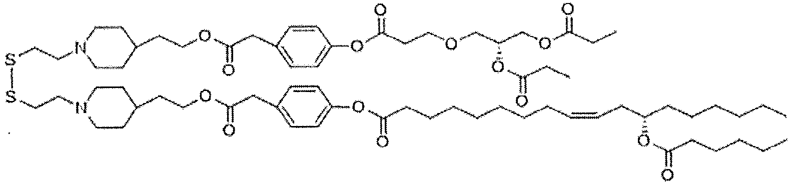 |
| compound 91 | 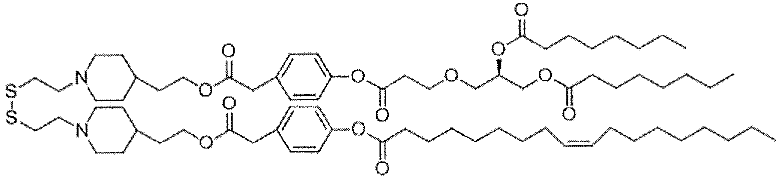 |
| compound 92 | 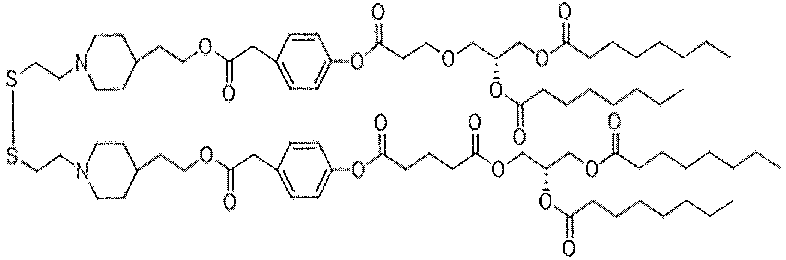 |
| compound 93 | 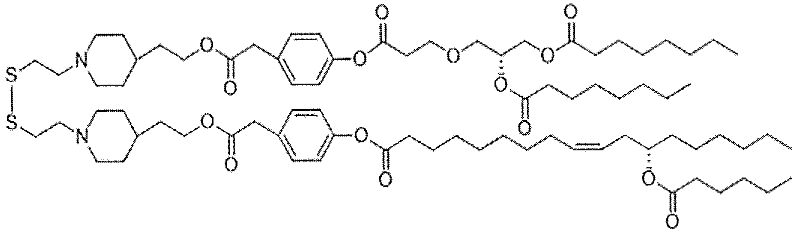 |

**Table 1-17]**

| name | structure |
|---|---|
| compound 94 | 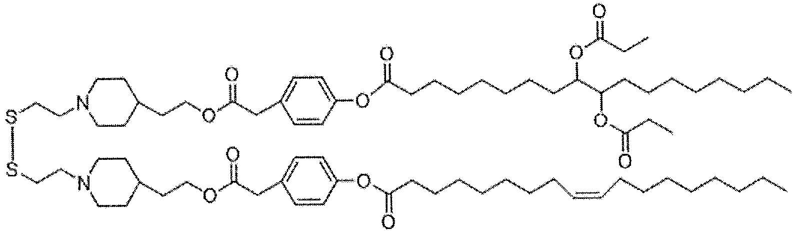 |
| compound 95 | 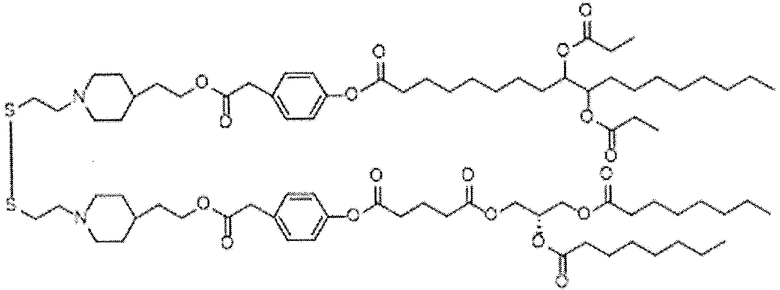 |
| compound 96 | 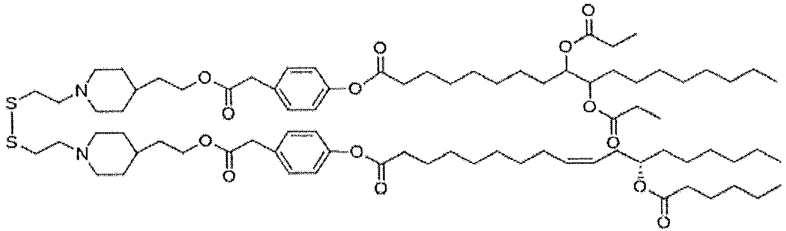 |
| compound 97 | 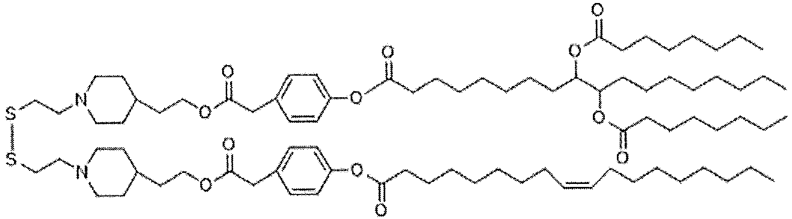 |
| compound 98 | 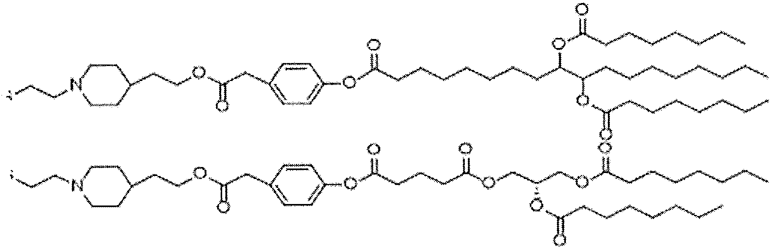 |

**[Table 1-18]**

| name | structure |
|---|---|
| compound 99 | 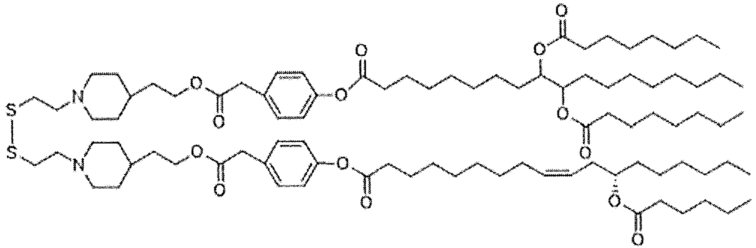 |
| compound 100 | 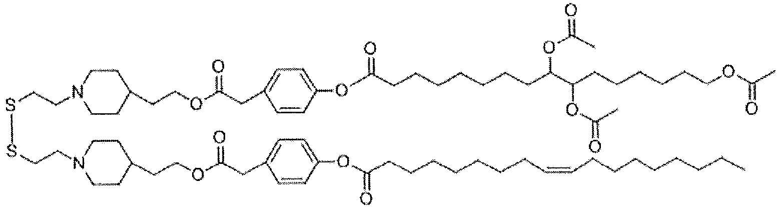 |
| compound 101 | 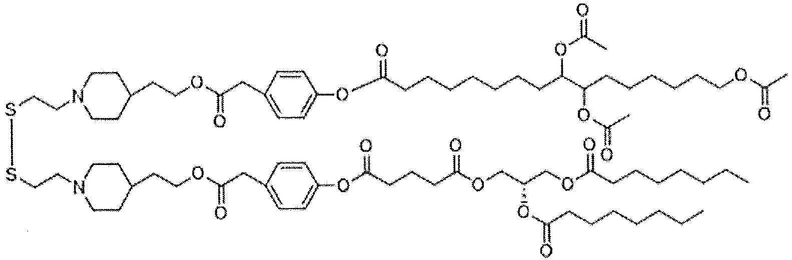 |
| compound 102 | 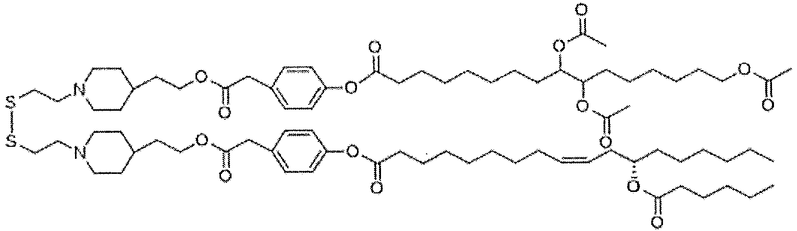 |
| compound 103 | 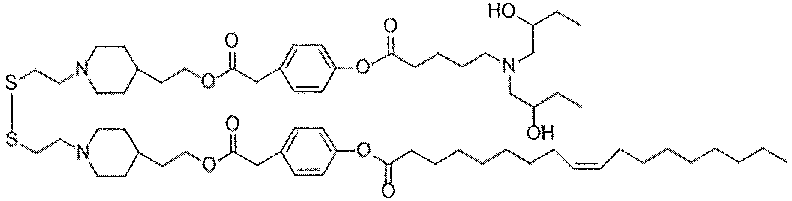 |

**[Table 1-19]**

| name | structure |
|---|---|
| compound 104 | 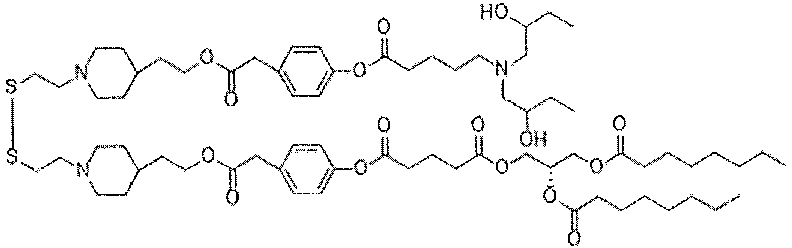 |
| compound 105 | 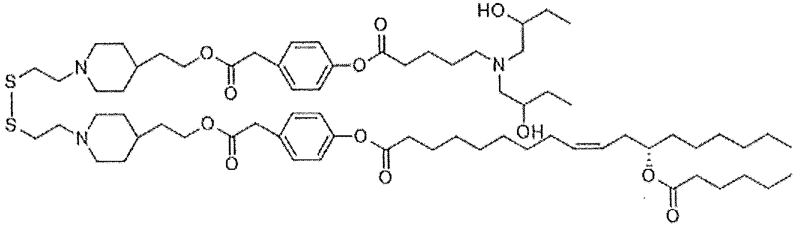 |
| compound 106 | 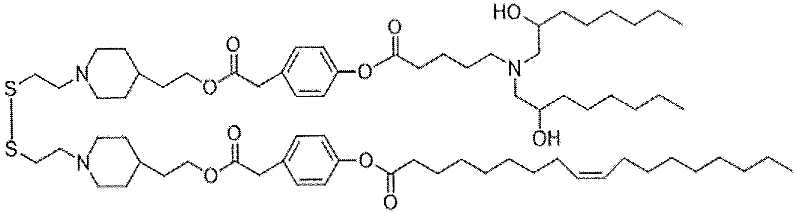 |
| compound 107 | 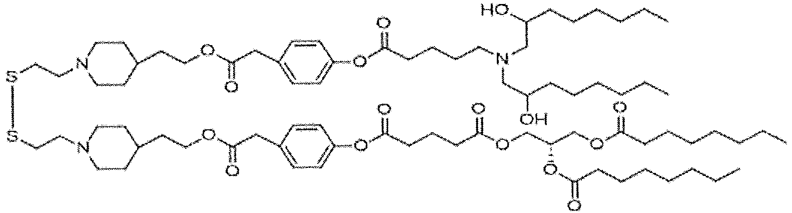 |
| compound 108 | 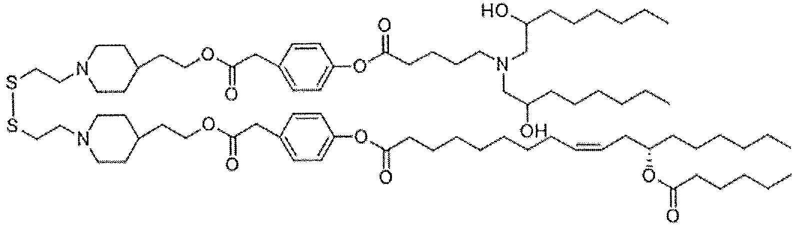 |
| compound 109 | 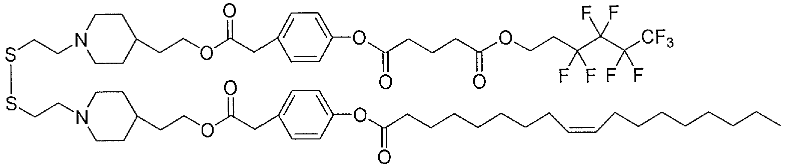 |

**[Table 1-20]**

| name | structure |
|---|---|
| compound 110 | 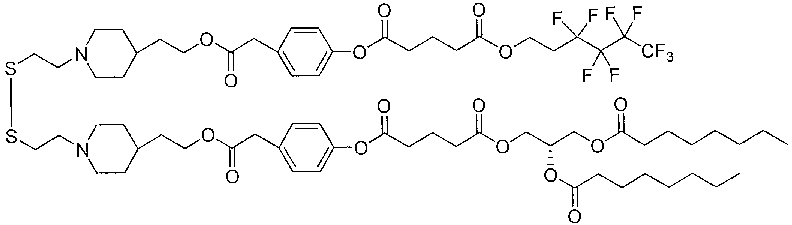 |
| compound 111 | 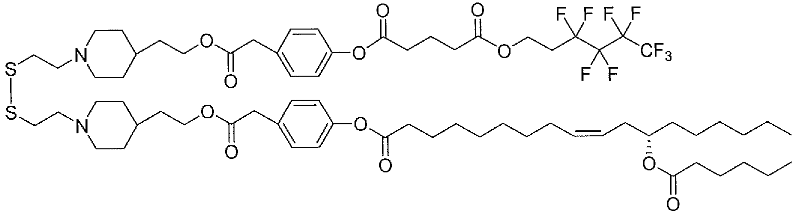 |
| compound 112 | 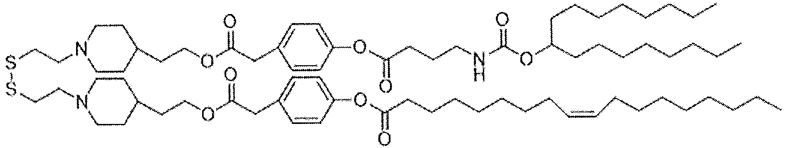 |
| compound 113 | 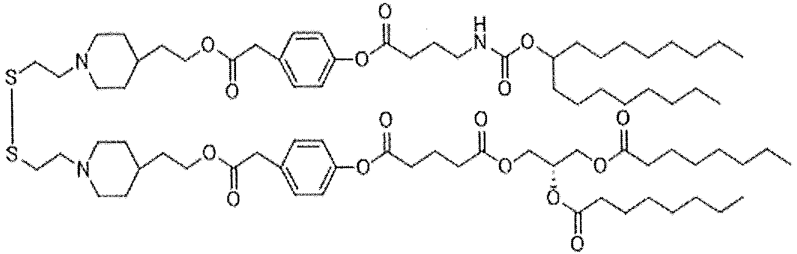 |
| compound 114 | 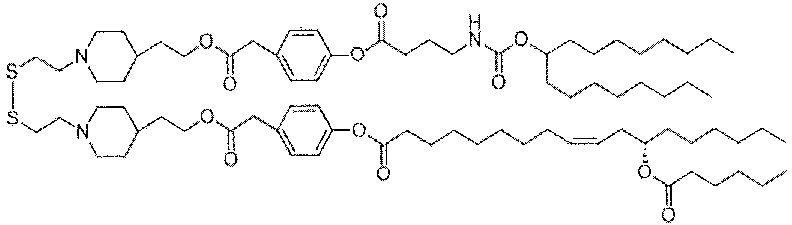 |
| compound 115 | 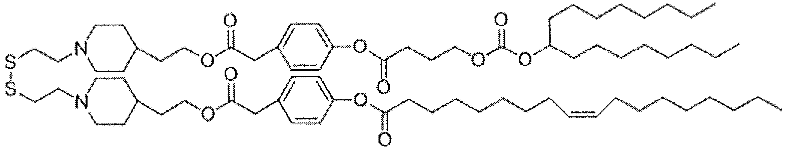 |

**[Table 1-21]**

| name | structure |
|---|---|
| compound 116 | 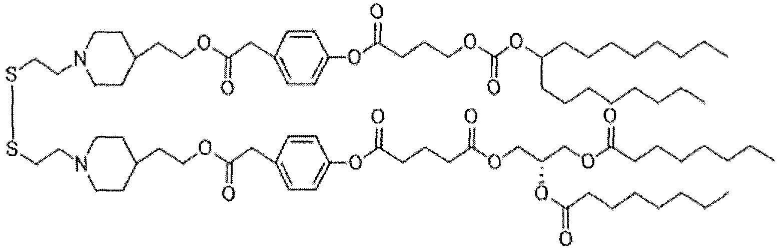 |
| compound 117 | 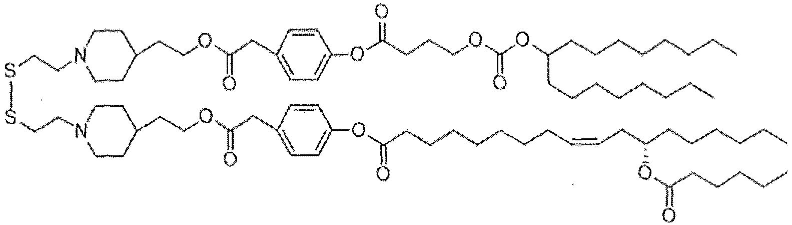 |
| compound 118a | 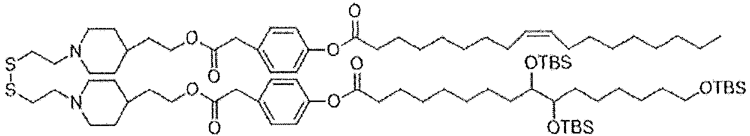 |
| compound 118 | 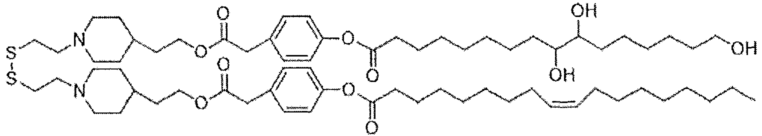 |
| compound 119a | 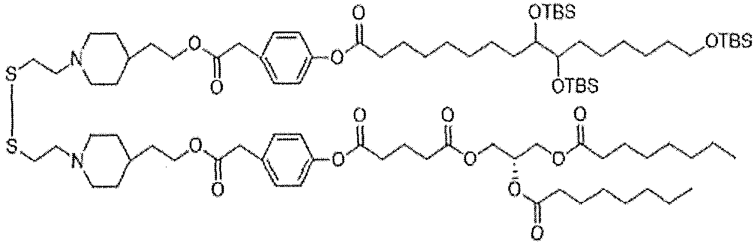 |
| compound 119 | 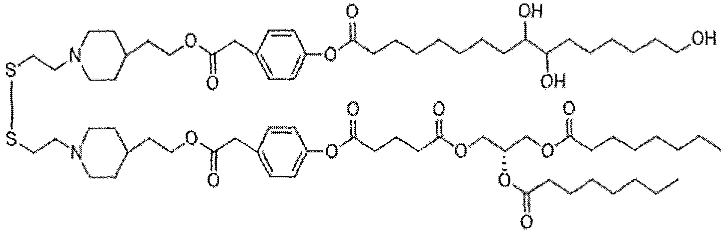 |

**[Table 1-22]**

| name | structure |
|---|---|
| compound 120a | 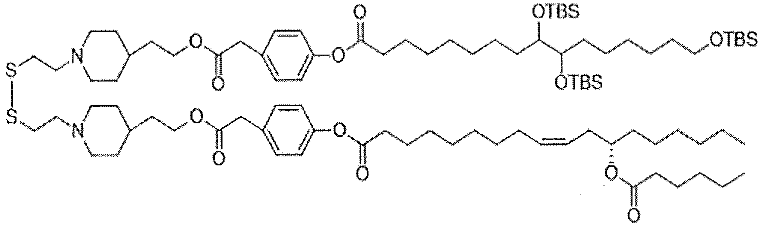 |
| compound 120 | 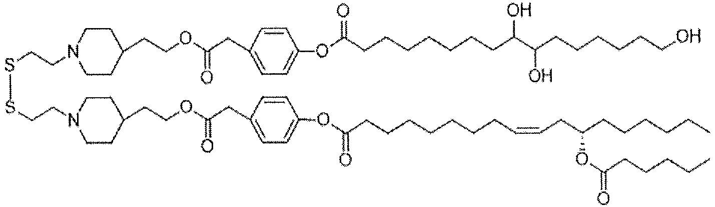 |
| compound 121 | 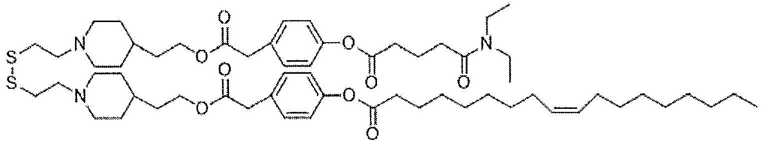 |
| compound 122 | 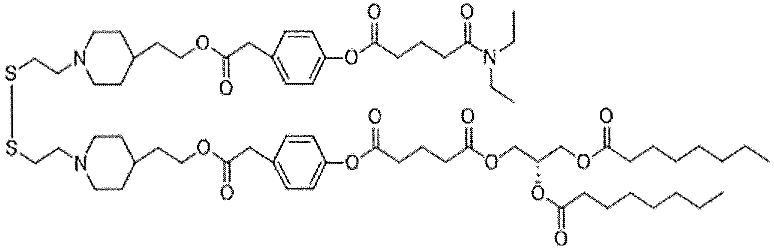 |
| compound 123 | 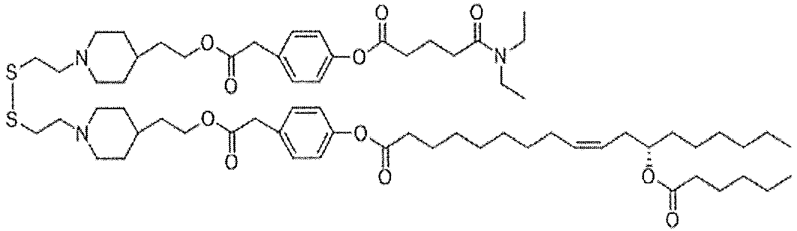 |
| compound 124 | 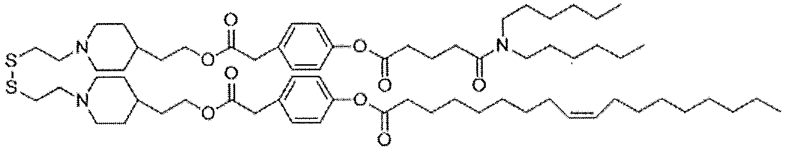 |

**[Table 1-23]**

| name | structure |
|---|---|
| compound 125 | 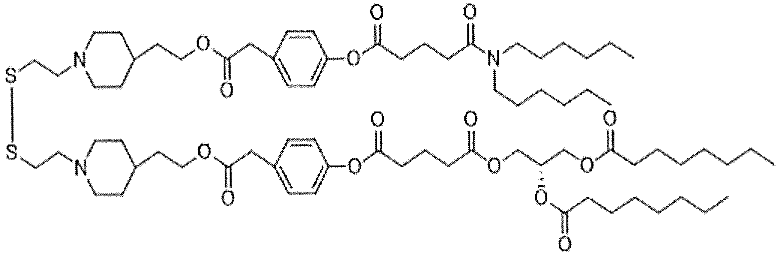 |
| compound 126 | 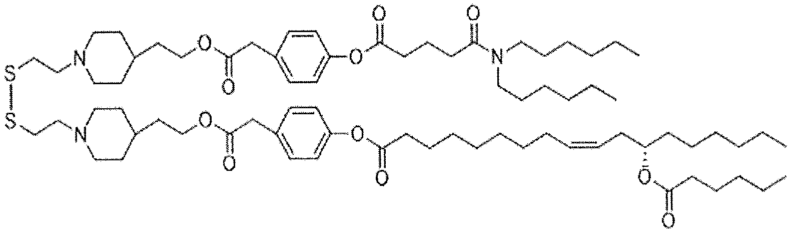 |
| compound 127a | 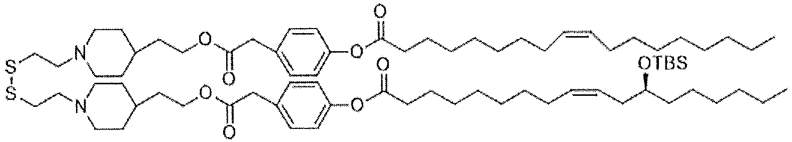 |
| compound 127 | 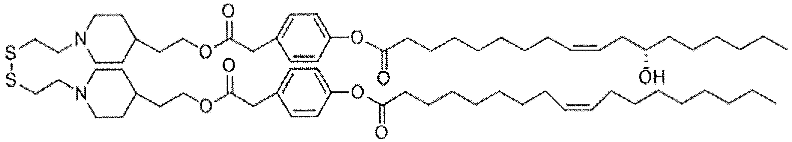 |
| compound 128a | 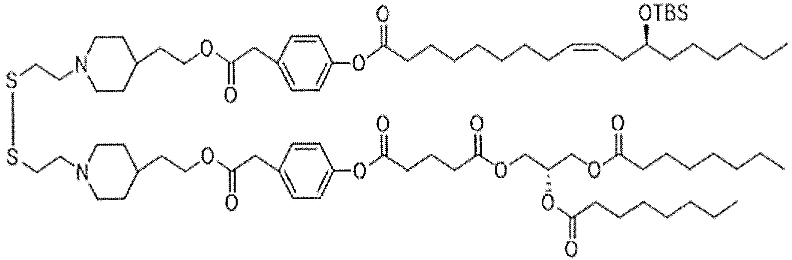 |
| compound 128 | 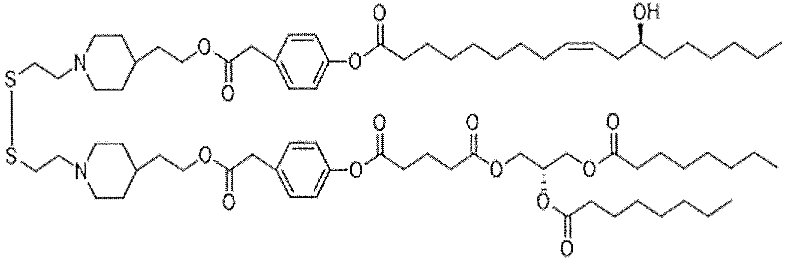 |

**[Table 1-24]**

| name | structure |
|---|---|
| compound 129a | 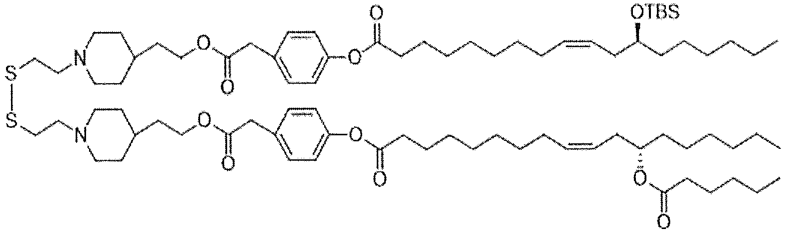 |
| compound 129 | 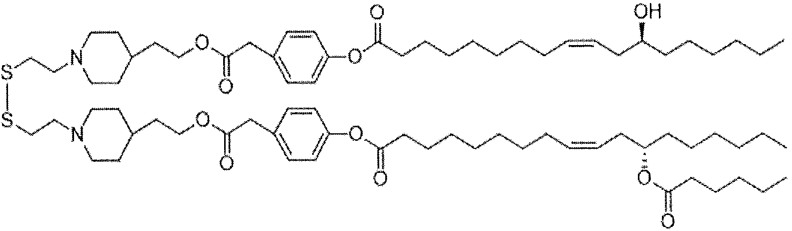 |
| compound 130 | 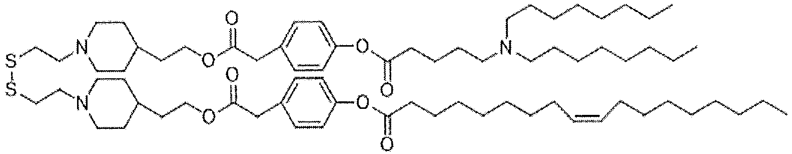 |
| compound 131 | 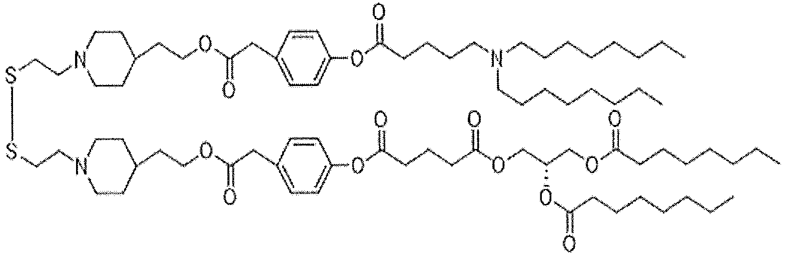 |
| compound 132 | 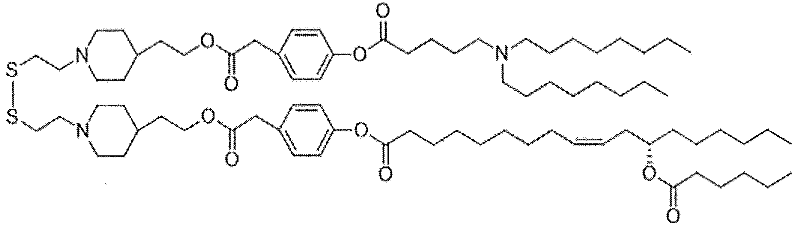 |
| compound 133a | 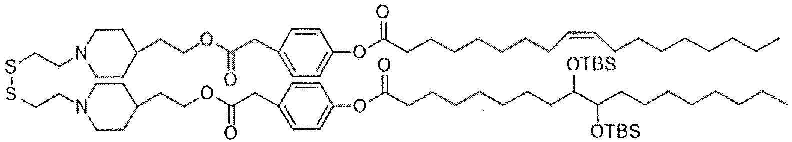 |

**[Table 1-25]**

| name | structure |
|---|---|
| compound 133 | 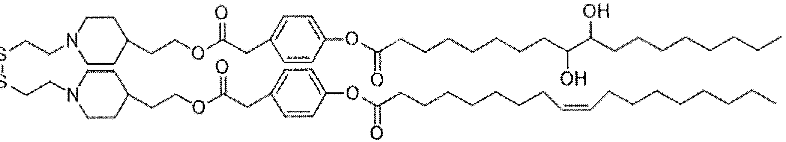 |
| compound 134a | 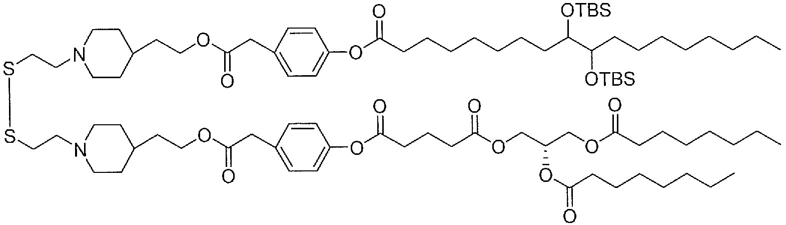 |
| compound 134 | 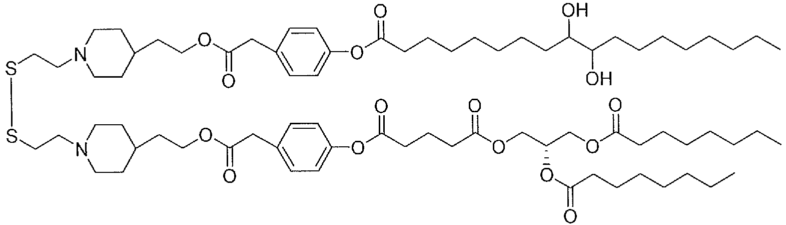 |
| compound 135a | 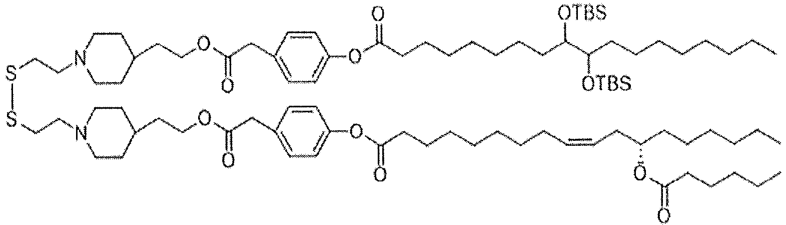 |
| compound 135 | 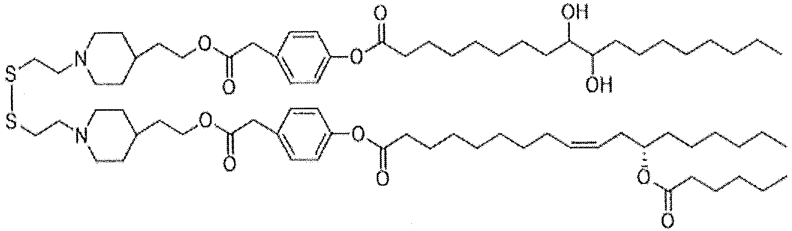 |
| compound 136 | 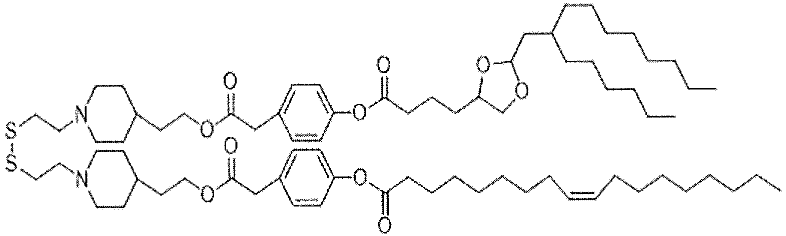 |

**[Table 1-26]**

| name | structure |
|---|---|
| compound 137 | 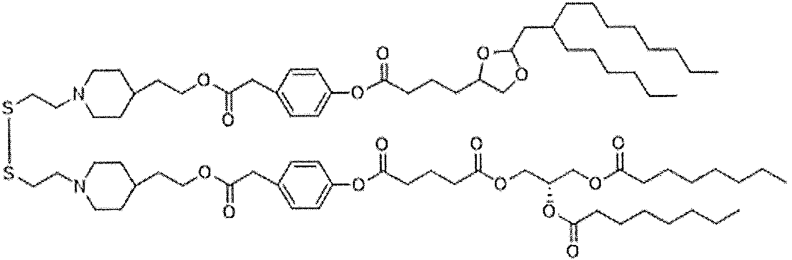 |
| compound 138 | 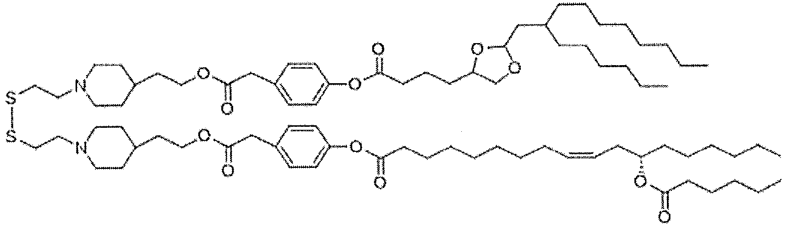 |
| compound 139a | 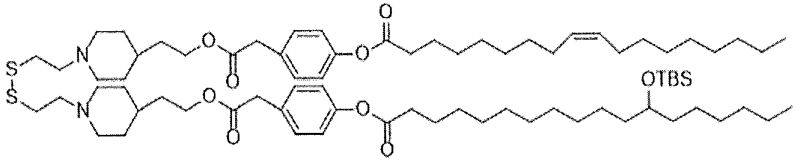 |
| compound 139 | 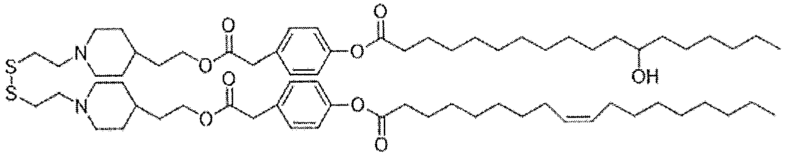 |
| compound 140a | 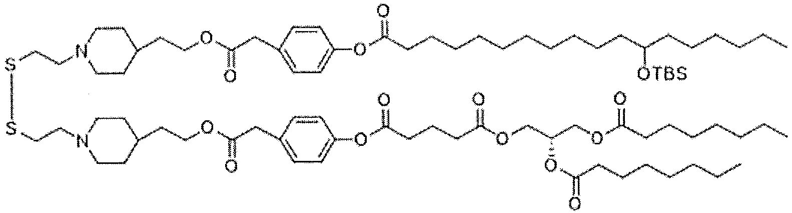 |
| compound 140 | 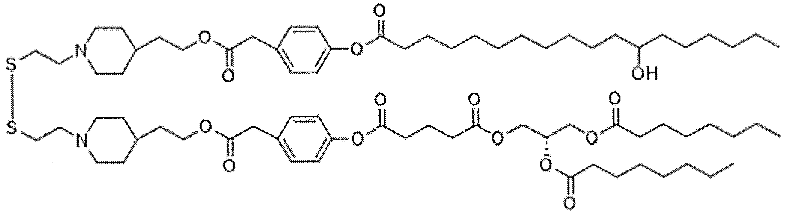 |

**[Table 1-27]**

| name | structure |
|---|---|
| compound 141a | 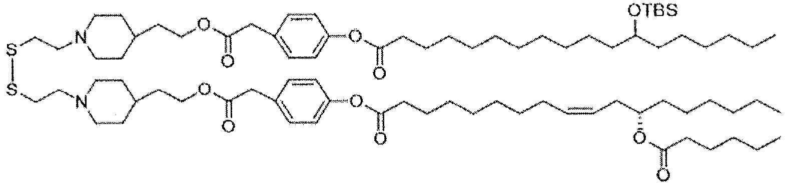 |
| compound 141 | 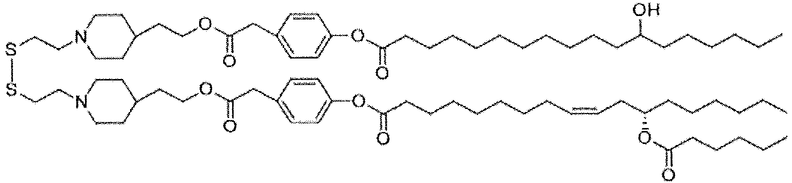 |
| compound 142 | 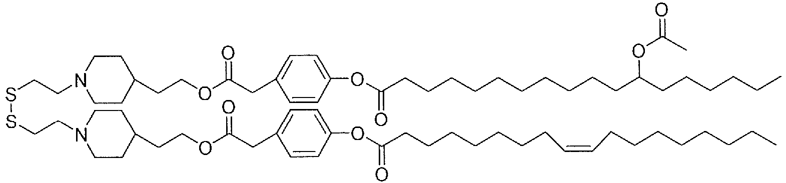 |
| compound 143 | 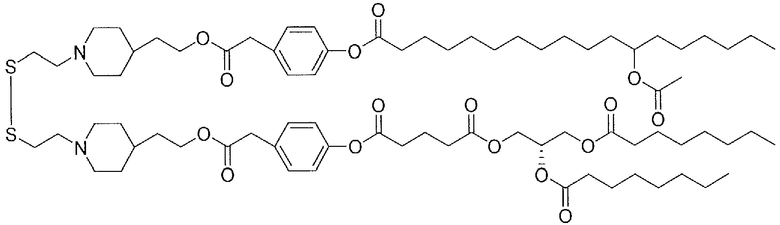 |
| compound 144 | 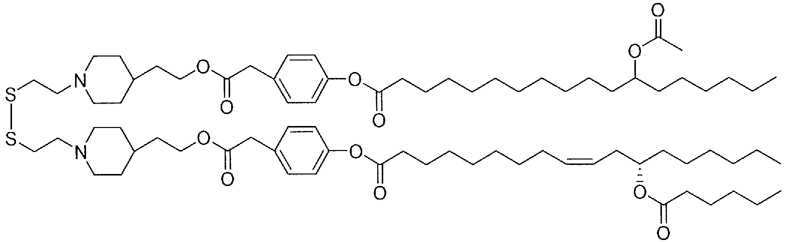 |
| compound 145 | 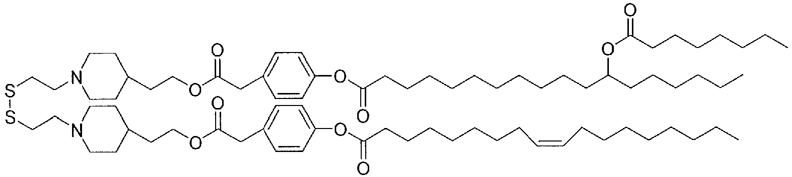 |

**[Table 1-28]**

| name | structure |
|---|---|
| compound 146 | 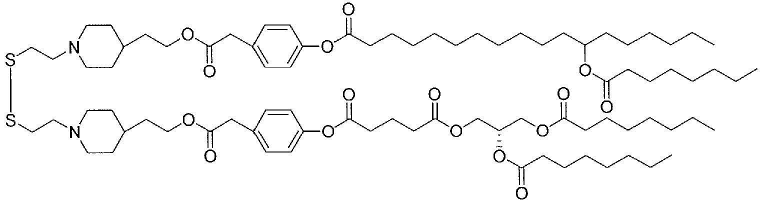 |
| compound 147 | 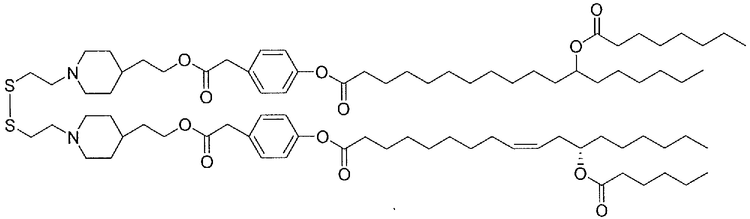 |
| compound 148 | 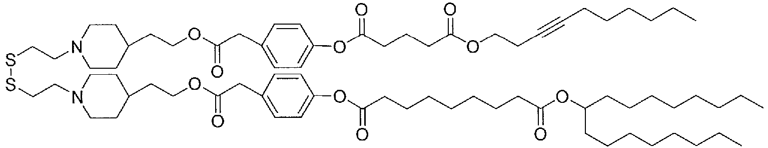 |
| compound 149 | 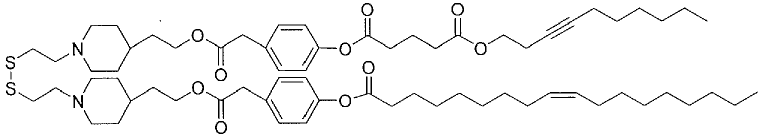 |
| compound 150 | 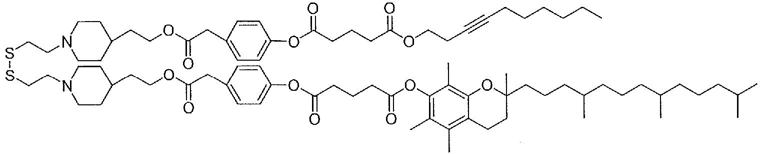 |
| compound 151 | 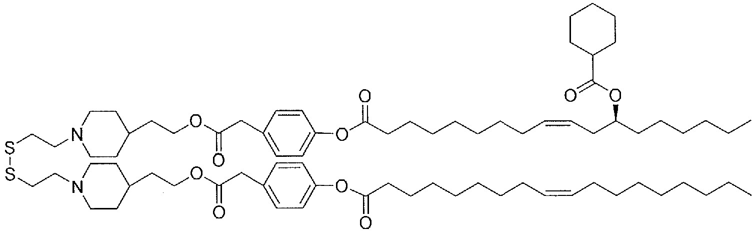 |
| compound 152 | 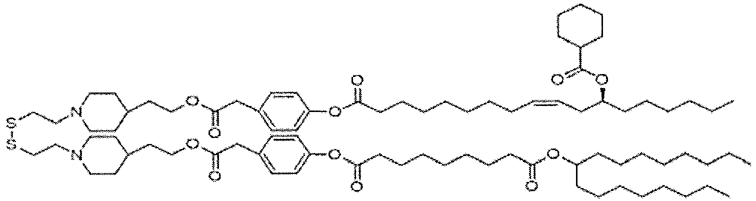 |

**[Table 1-29]**

| name | structure |
|---|---|
| compound 153 | 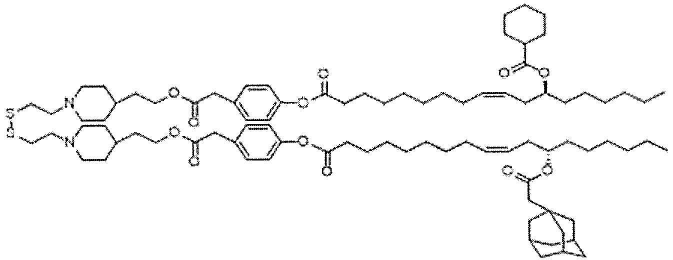 |
| compound 154 | 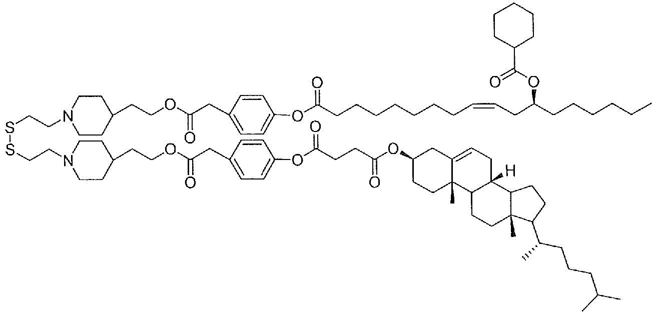 |
| compound 155 | 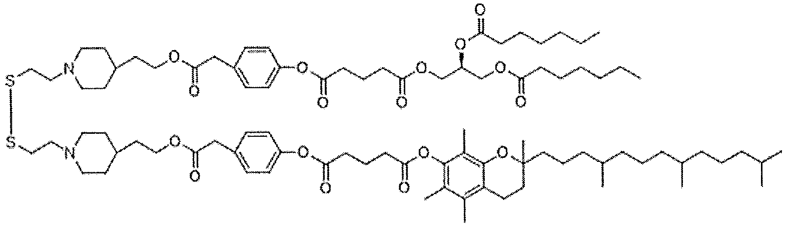 |
| compound 156 | 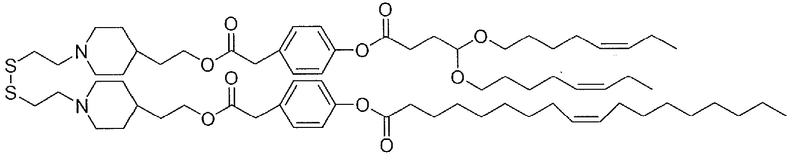 |
| compound 157 | 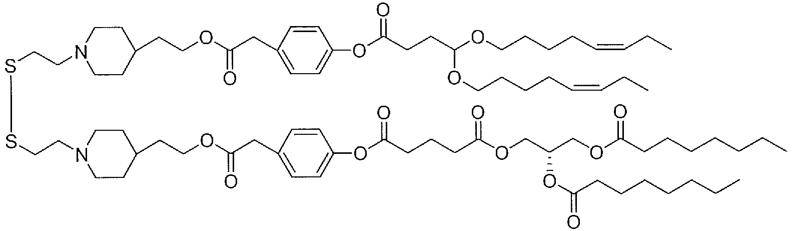 |

**[Table 1-30]**

| name | structure |
|---|---|
| compound 158 | 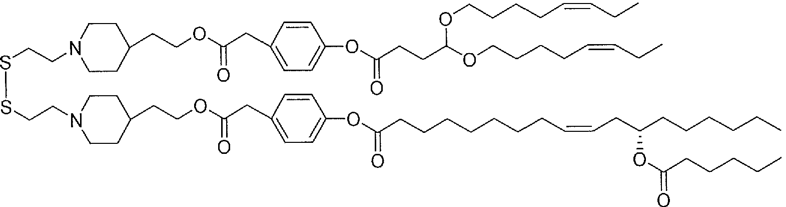 |

**[Table 1-31]**

| name | structure |
|---|---|
| compound 159 | 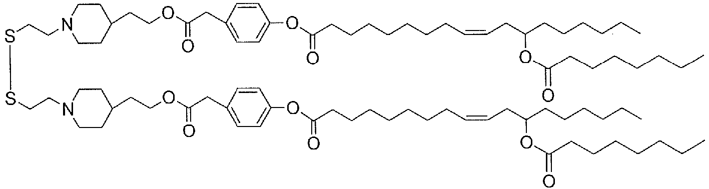 |
| compound 160 | 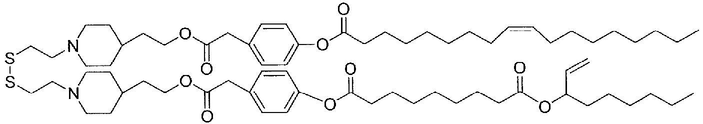 |
| compound 161 | 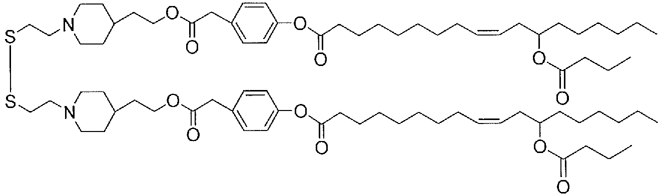 |
| compound 162 | 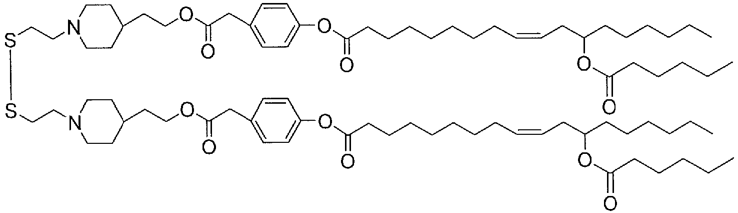 |
| compound 163 | 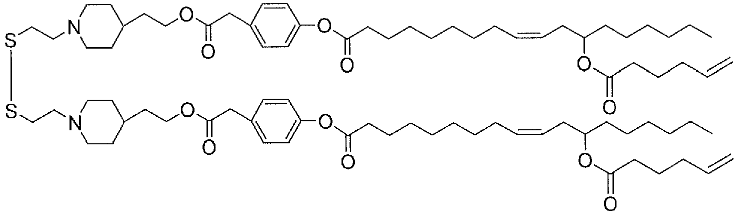 |

**[Table 1-32]**

| name | structure |
|---|---|
| compound 164 | 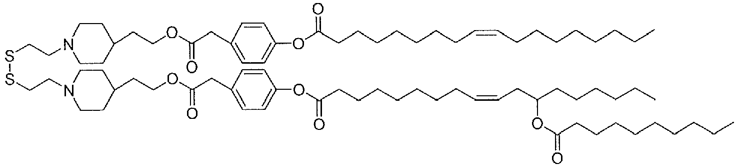 |
| compound 165 | 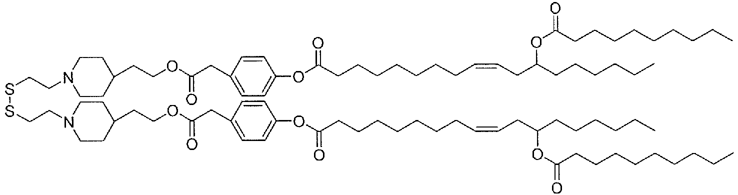 |
| compound 166 | 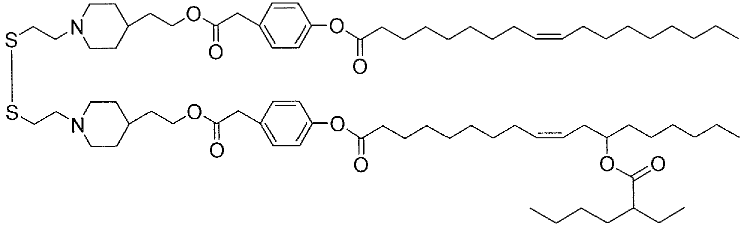 |
| compound 167 | 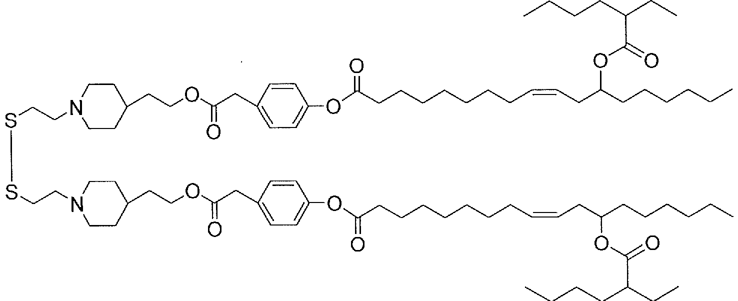 |
| compound 168 | 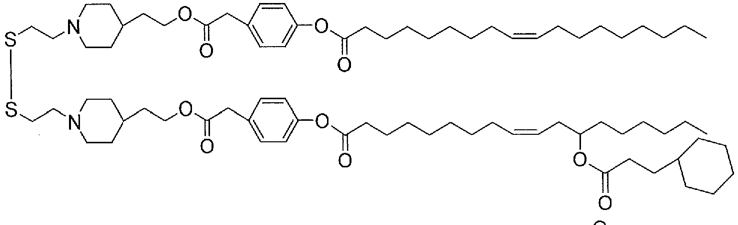 |
| compound 169 | 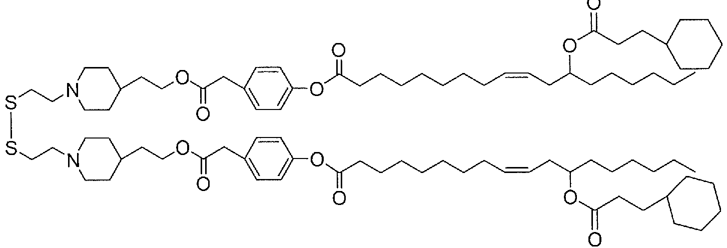 |

**[Table 1-33]**

| name | structure |
|---|---|
| compound 170 | 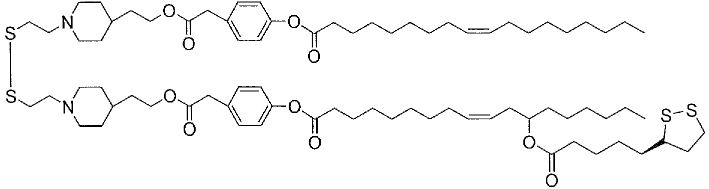 |
| compound 171 | 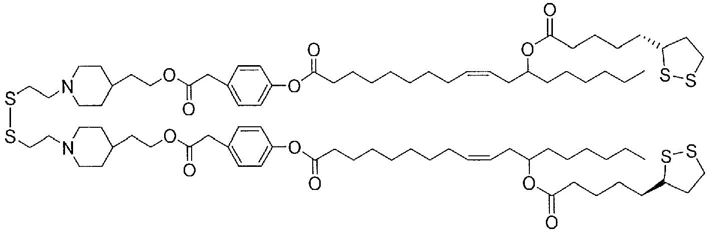 |
| compound 172 | 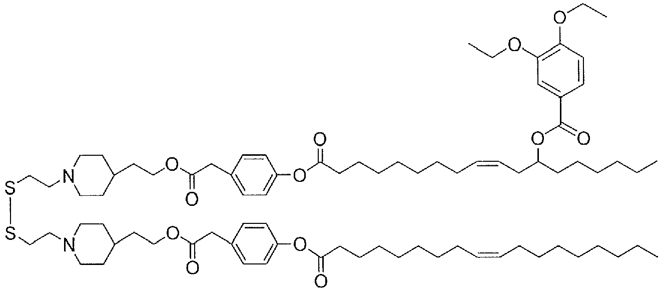 |
| compound 173 | 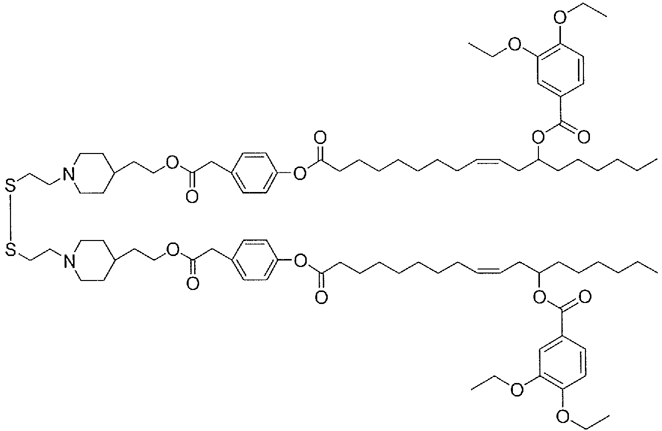 |
| compound 174 | 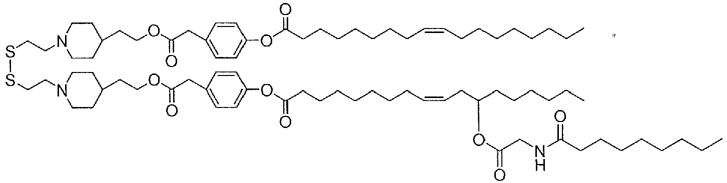 |

**[Table 1-34]**

| name | structure |
|---|---|
| compound 175 | 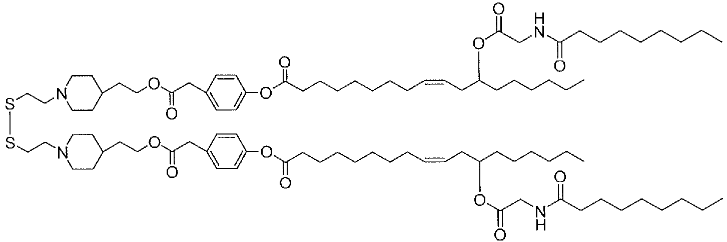 |
| compound 176 | 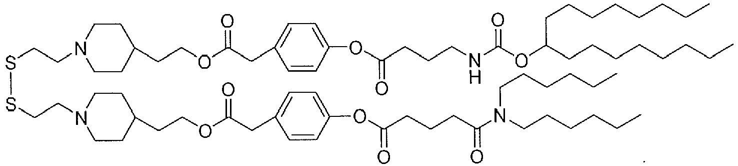 |
| compound 177 | 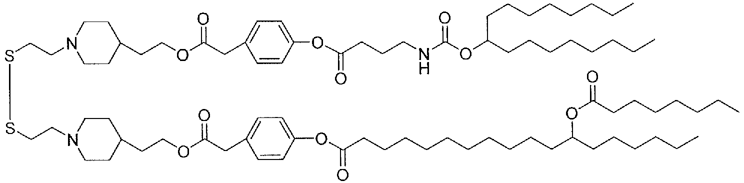 |
| compound 178 | 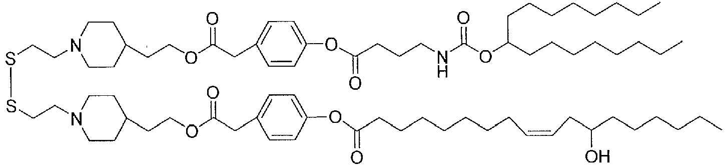 |
| compound 179 | 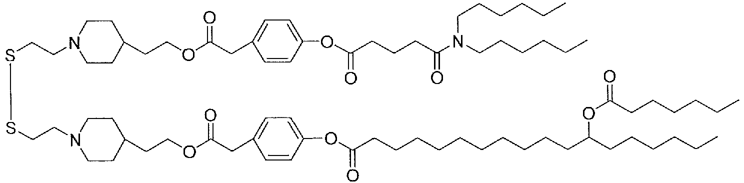 |
| compound 180 | 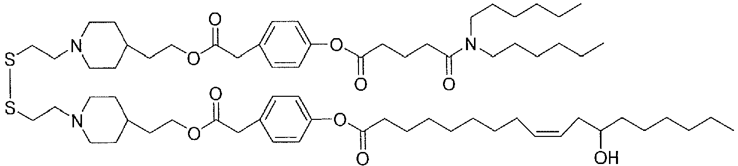 |
| compound 181 | 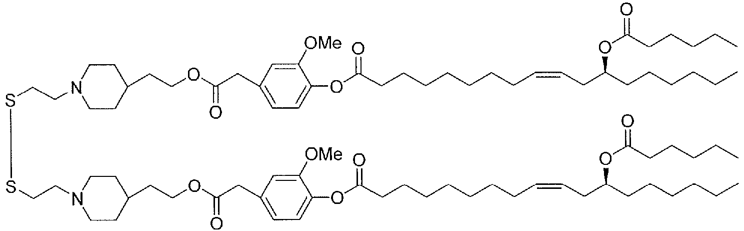 |

**[Table 1-35]**

| name | structure |
|---|---|
| compound 182 | 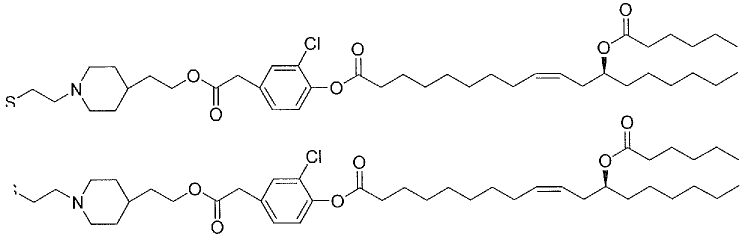 |
| compound 183 | 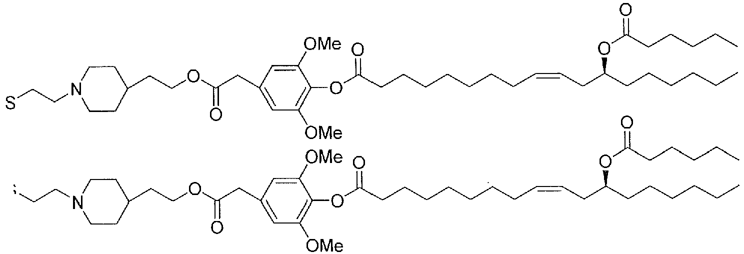 |
| compound 184 | 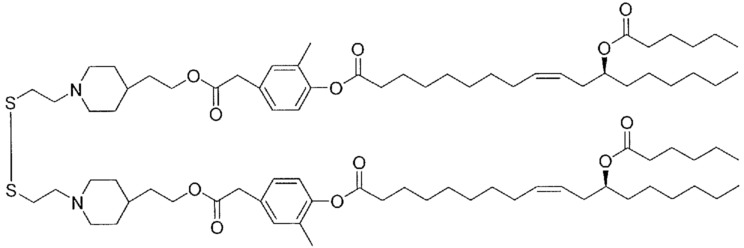 |
| compound 185 | 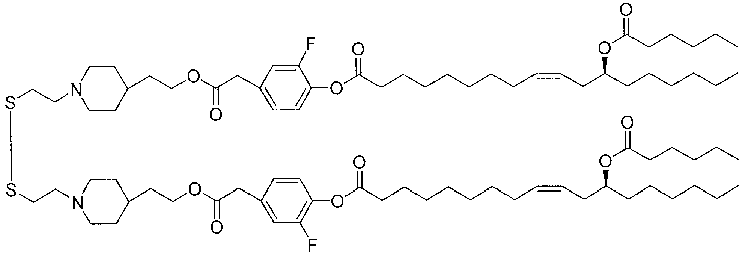 |
| compound 186 | 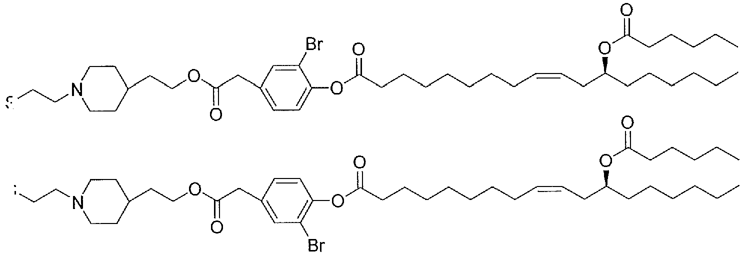 |

**[Table 1-36]**

| name | structure |
|---|---|
| compound 187 | 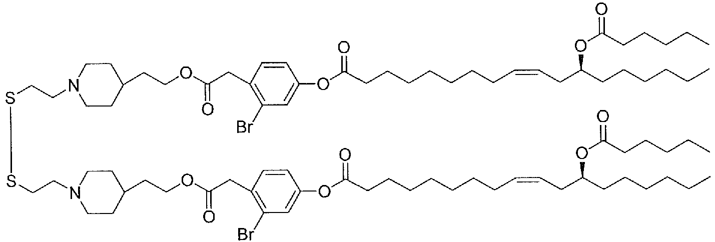 |
| compound 188 | 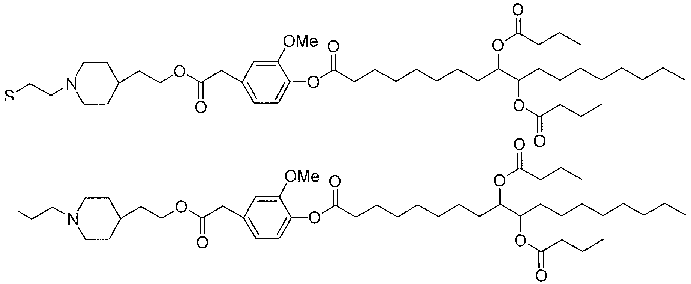 |
| compound 189 | 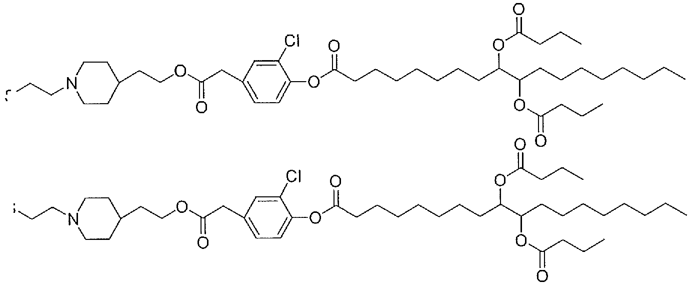 |
| compound 190 | 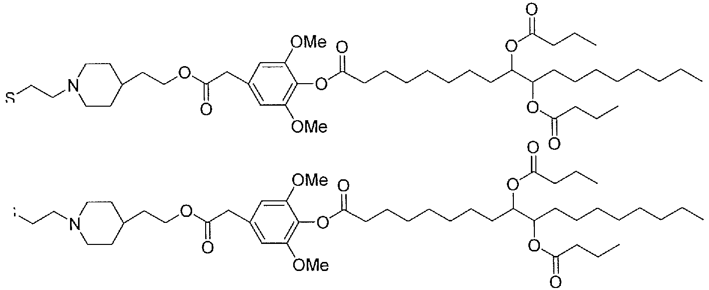 |
| compound 191 | 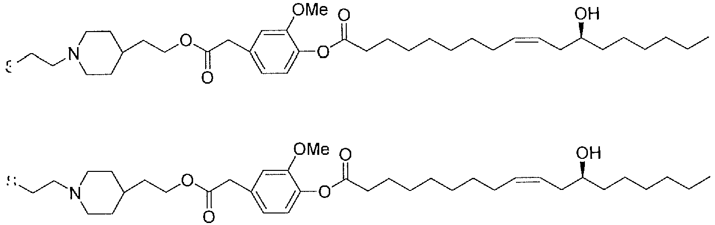 |

**[Table 1-37]**

| name | structure |
|---|---|
| compound 192 | 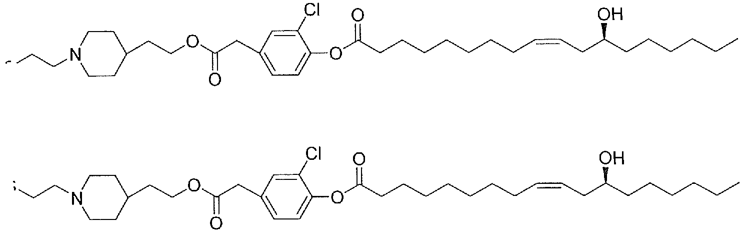 |
| compound 193 | 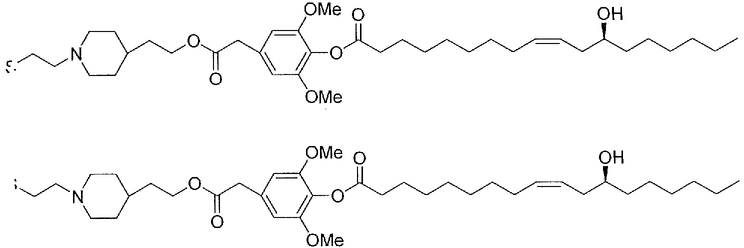 |
| compound 194 | 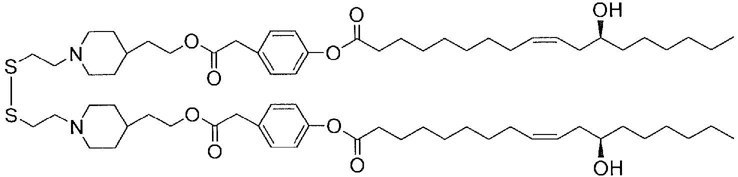 |
| compound 195 | 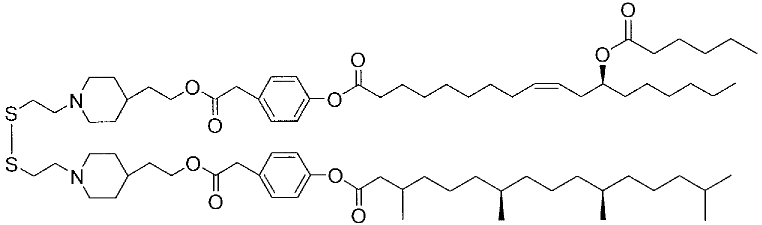 |

**[Table 1-38]**

| | |
|---|---|
| compound 196 | 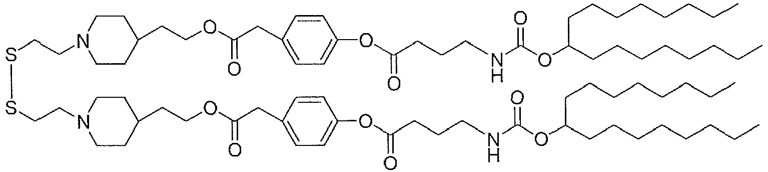 |
| compound 197 | 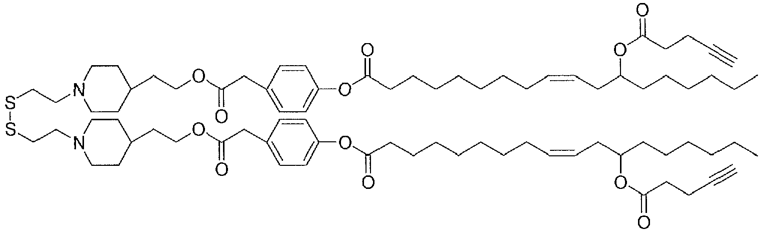 |
| compound 198 | 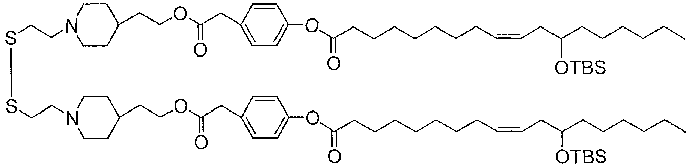 |

Specific examples of ionic lipid (1) include O-Ph-P3C1, O-Ph-P4C1, SS-OP, O-Bn-P4C2, SS-EP, L-Ph-P4C2, HD-Ph-P4C2, O-Ph-amide-P4C2, O-Ph-C3M, B-2, B-2-5, SS-EC, L-P4C2, and SS-OC described in the following Table 2-1 to Table 2-3 (hereinafter these are collectively referred to as "Table 2").

**[Table 2-1]**

| name | structure |
|---|---|
| O-Ph-P3Cl | 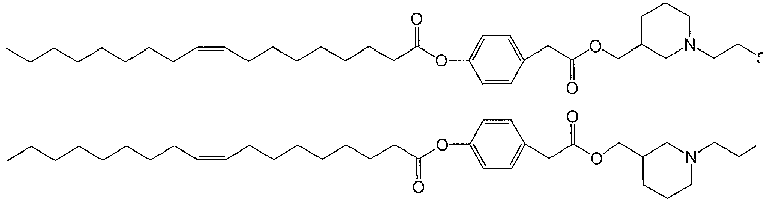 |
| O-Ph-P4C1 | 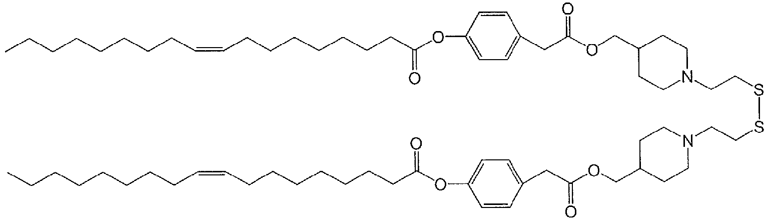 |
| SS-OP | 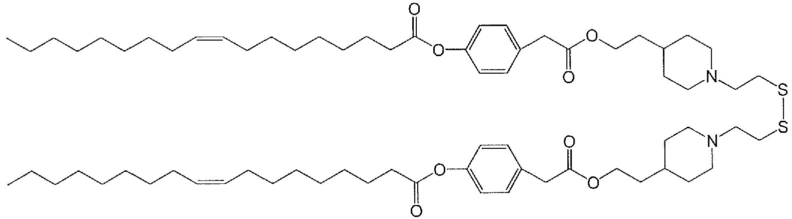 |
| O-Bn-P4C2 | 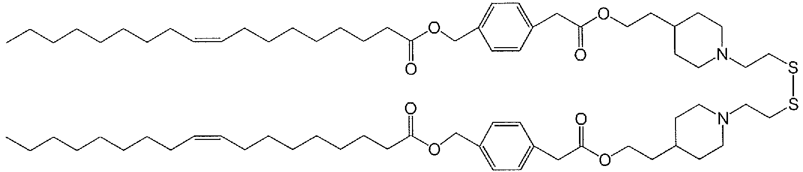 |
| SS-EP | 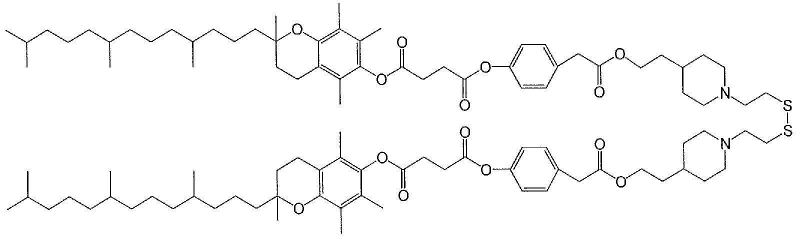 |

**[Table 2-2]**

| name | structure |
|---|---|
| L-Ph-P4C2 | 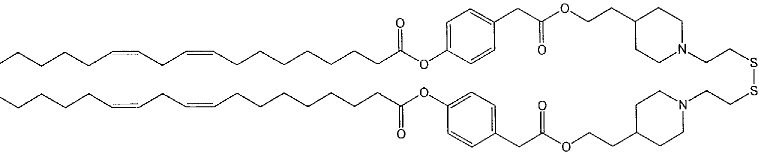 |
| HD-Ph-P4C2 | 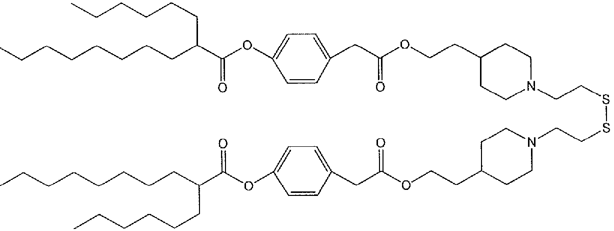 |
| O-Ph-amide-P4C2 | 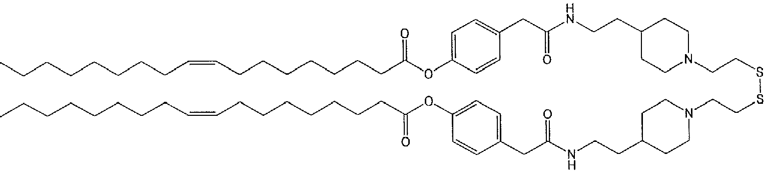 |
| O-Ph-C3M | 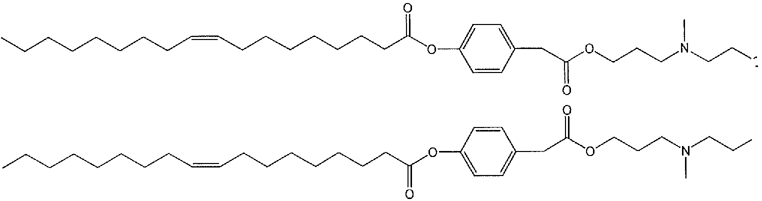 |

**[Table 2-3]**

| name | structure |
|---|---|
| B-2 | 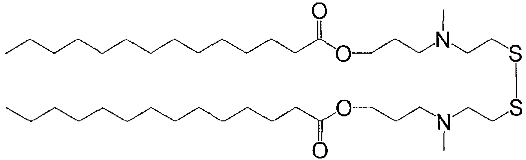 |
| B-2-5 | 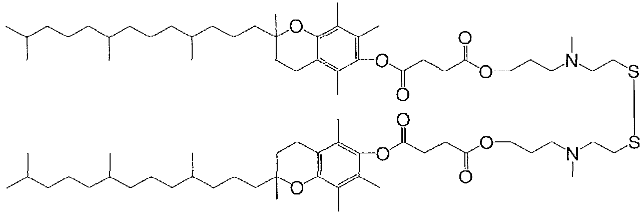 |
| SS-EC | 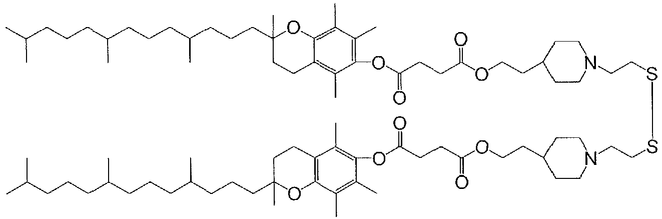 |
| L-P4C2 | 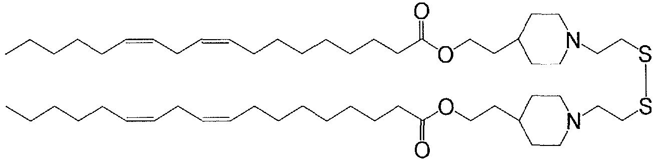 |
| SS-OC | 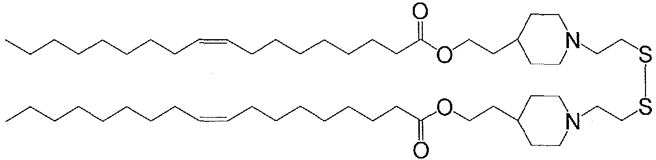 |

In one embodiment of the present invention, ionic lipid (1) is
preferably at least one selected from the group consisting of the compounds shown in Table 1 and Table 2,
more preferably at least one selected from the group consisting of SS-OP, SS-EC, and compound 13,
further preferably SS-OP; combination of SS-OP and SS-EC; SS-EC; or compound 13,
particularly preferably SS-OP; or combination of SS-OP and SS-EC,
most preferably SS-OP.

In another embodiment of the present invention, ionic lipid (1) is
preferably at least one selected from the group consisting of SS-OP, and SS-EC, compound 7, compound 9 to compound 13, compound 23, compound 33, compound 34, compound 37, compound 58, compound 155, compound 159, compound 161 to compound 169, and compound 196 to compound 198,
more preferably at least one selected from the group consisting of SS-OP, compound 9 to compound 12, compound 33, compound 34, compound 37, compound 159, compound 161 to compound 169, and compound 197.

In another embodiment of the present invention, ionic lipid (1) is
preferably at least one selected from the group consisting of SS-OP, SS-EC, compound 9, compound 10, compound 11, compound 12, compound 33, compound 34, compound 37, compound 159, compound 161, compound 162, compound 163, compound 164, compound 165, compound 166, compound 167, compound 168, compound 169, and compound 197,
more preferably SS-OP; combination of SS-OP and SS-EC; SS-EC; compound 11; compound 33; compound 37; compound 163 or compound 197,
further preferably SS-OP; combination of SS-OP and SS-EC; or compound 11,
particularly preferably SS-OP or compound 11.

Ionic lipids (1) shown in Table 2 can be produced by a known method (e.g., methods described in WO 2019/188867 A1 (US 2021/0023008 A1), US 9708628 B2, WO 2021/195529 A2, WO 2024/203577 A1). Furthermore, compound 13 used in the Examples was produced by the following synthesis method. Other ionic lipids (1) that are listed in Table 1 and other than compound 13 can also be produced in the same manner as the synthesis method for compound 13.

The meanings of the abbreviations used in the synthesis method for compound 13 are as follows.
DMAP: 4-dimethylaminopyridine
EDC hydrochloride: 1-ethyl-3-(3-dimethylaminopropyl)carbodiimide hydrochloride
TBAF: tetra-n-butylammonium fluoride
TBDPS-Cl: tert-butyldiphenylsilyl chloride
THF: tetrahydrofuran

### <synthesis method of compound 13>

### <Synthesis of intermediate 11-2>

Intermediate 11-2 was synthesized by the following synthesis route.

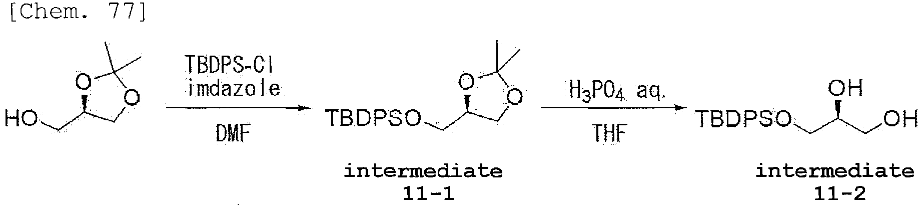

### <Synthesis of intermediate 11-1>

(R)-(-)-2,2-dimethyl-1,3-dioxolan-4-methanol (25.0 g, 189 mmol) and imidazole (19.3 g, 284 mmol) were dissolved in dimethylformamide (251 g) at room temperature. To the obtained mixture was added TBDPS-Cl (57.3 g, 208 mmol) and they were reacted at room temperature for 1 hr. Thereafter, to the reaction solution was added chloroform, and the mixture was washed with 5 wt% sodium hydrogenphosphate aqueous solution, and ion-exchanged water, respectively, and dehydrated by adding sodium sulfate. Sodium sulfate was removed by filtration, and the filtrate was concentrated in an evaporator to give 56.0 g of intermediate 11-1.

### <Synthesis of intermediate 11-2>

Intermediate 11-1 (55.9 g, 151 mmol) and 0.5 M phosphate buffer (pH 1.0, 503 g) were dissolved in THF (530 g) at room temperature, and the mixture was stirred at 50°C for 12 hr. 1.0 M NaOH aqueous solution was added to adjust to pH 7.0. To the obtained mixture was added chloroform, and the mixture was washed with 20 wt% saline and dehydrated by adding sodium sulfate. Sodium sulfate was removed by filtration, and the filtrate was concentrated in an evaporator to give 44.9 g of intermediate 11-2.

### <Synthesis of intermediate 13-1>

Intermediate 11-2 (6.90 g, 20.9 mmol), hexanoic acid (5.38 g, 46.0 mmol), and DMAP (0.511 g, 4.18 mmol) were dissolved in chloroform (69.5 g) at room temperature. To the obtained mixture was added EDC hydrochloride (12.0 g, 62.7 mmol) and the mixture was reacted at room temperature for 1 hr. Thereafter, the reaction solution was washed with 0.5 M phosphate buffer (pH 4.0), 7 wt% sodium bicarbonate water, and 20 wt% saline, and dehydrated by adding sodium sulfate. Sodium sulfate was removed by filtration, and the filtrate was concentrated in an evaporator to give 9.405 g of intermediate 13-1.

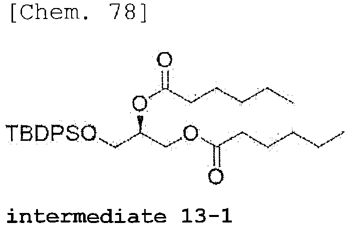

### <Synthesis of intermediate 13-2>

Intermediate 13-1 (9.41 g, 17.9 mmol) and acetic acid (4.29 g, 71.6 mmol) were dissolved in THF (28.2 g) at room temperature. To the obtained mixture was added THF solution (65.9 g, 71.6 mmol) of 1 M TBAF and the mixture was reacted at room temperature for 18 hr. Thereafter, to the reaction solution was added ethyl acetate (94.3 g). Thereafter, the reaction solution was washed with 0.5 M phosphate buffer (pH 4.0), and 20 wt% saline, and dehydrated by adding sodium sulfate. Sodium sulfate was removed by filtration, and the filtrate was concentrated in an evaporator to give 3.10 g of intermediate 13-2.

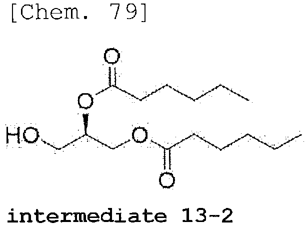

### <Synthesis of intermediate 13>

Intermediate 13-2 (3.10 g, 10.7 mmol), glutaric anhydride (2.45 g, 21.5 mmol), triethylamine (3.25 g, 32.1 mmol), and DMAP (0.261 g, 2.14 mmol) were dissolved in chloroform (51.6 g) at room temperature, and the mixture was reacted at room temperature for 1 hr. Thereafter, the reaction solution was washed with 0.5 M phosphate buffer (pH 4.0), 7 wt% sodium bicarbonate water, and 20 wt% saline, and dehydrated by adding sodium sulfate. Sodium sulfate was removed by filtration, and the filtrate was concentrated in an evaporator to give 3.21 g of intermediate 13.

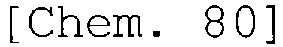

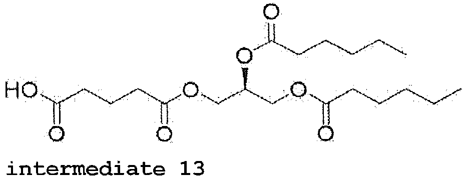

### <Synthesis of intermediate 1>

Intermediate 1 was synthesized by the following synthesis pathway.

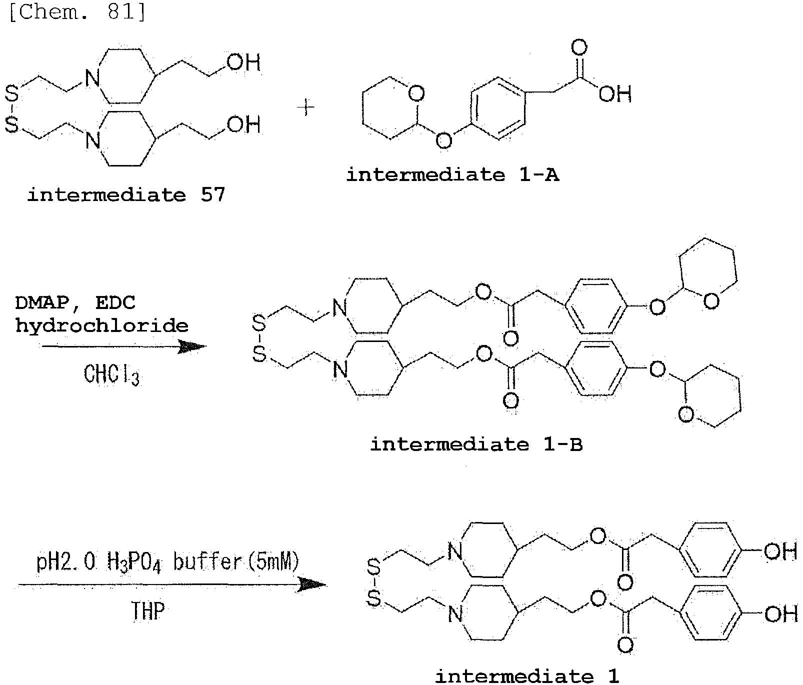

### <Synthesis of intermediate 1-A>

4-Hydroxyphenylacetic acid (30.0 g, 197 mmol), and pyridinium p-toluenesulfonate (5.00 g, 19.9 mmol) were dissolved in dichloromethane (120 mL) at room temperature. To the obtained mixture was added dropwise a mixed solution of 3,4-dihydro-2H-pyran (83.0 g, 987 mmol) and dichloromethane (31.1 mL) at 20°C or below and the mixture was reacted at room temperature for 2 hr. Thereafter, the reaction mixture was neutralized with DMAP (12.0 g, 98.2 mmol). To the obtained mixture were added 2-propanol (301 mL), and 100 g/L NaOH aqueous solution (160 g), and the mixture was reacted at room temperature for 1 hr. The mixed solution was concentrated in an evaporator, the concentrate was washed with chloroform and neutralized with 6 M hydrochloric acid. The obtained mixture was extracted with chloroform, and the organic layer was dehydrated by adding sodium sulfate. Sodium sulfate was removed by filtration, and the filtrate was concentrated in an evaporator to give 47.0 g of intermediate 1-A.

### <Synthesis of intermediate 1-B>

Intermediate 57 (36.0 g, 95.6 mmol) synthesized according to the method described in WO 2016/121942, intermediate 1-A (49.7 g, 210 mmol), and DMAP (4.68 mg, 38.3 mmol) were dissolved in chloroform (240 mL) at room temperature. To the obtained mixture was added EDC hydrochloride (55.1 g, 287 mmol), and the mixture was reacted at room temperature for 2 hr. Thereafter, the reaction solution was washed with 5 wt% sodium dihydrogen phosphate aqueous solution, 9 wt% sodium hydrogen carbonate aqueous solution, and 20 wt% saline, and dehydrated by adding sodium sulfate. Sodium sulfate was removed by filtration, and the filtrate was concentrated in an evaporator to give 77.9 g of intermediate 1-B.

### <Synthesis of intermediate 1>

Intermediate 1-B (77.7 g, 95.6 mmol) was dissolved in THF (325 mL) at room temperature. To the obtained mixture were added 2-propanol (319 mL) and p-toluenesulfonic acid monohydrate (38.2 g, 201 mmol), and the mixture was reacted at 25°C or below for 1 hr. Thereafter, to the reaction mixture was neutralized with DMAP (25.0 g, 205 mmol). DMAP was removed by filtration, and the filtrate was concentrated in an evaporator. The obtained residue was dissolved in chloroform (627 mL), washed with 0.5 M phosphate buffer (pH=6.5) and 0.5 M glycine buffer (pH=9.5), and dehydrated by adding sodium sulfate. Sodium sulfate was removed by filtration, and toluene (1.56 L) was added to allow for crystallization. The obtained crude product was washed with hexane and vacuum dried to give 40.3 g of intermediate 1.

### <¹H-NMR of intermediate 1 (600 MHz, DMSO-d₆)>

δ: 1.07-1.11(m, 4H), 1.16-1.25(m, 2H), 1.42-1.59(m, 8H), 1.79-1.88(m, 4H), 2.25-2.59(m, 4H), 2.76-2.89(m, 8H), 3.49(s, 4H), 4.02-4.09(m, 4H), 6.67-6.72(m, 4H), 7.02-7.07(m, 4H)

### <Synthesis of intermediate 1-1>

Oleic acid (5.11 g, 18.1 mmol), intermediate 1 (16.6 g, 25.8 mmol), and DMAP (630 mg, 5.16 mmol) were dissolved in chloroform (166 mL) at room temperature. To the obtained mixture was added EDC hydrochloride (5.94 g, 31.0 mmol) and the mixture was reacted at room temperature for 2 hr. Thereafter, the reaction solution was washed with 5 wt% sodium hydrogen phosphate aqueous solution, 0.5 M phosphate buffer (pH=2.0), and 20 wt% saline, and dehydrated by adding sodium sulfate. Sodium sulfate was removed by filtration, and the filtrate was concentrated in an evaporator. The obtained residue was purified by silica gel column chromatography (chloroform/ethanol=88/12) to give 8.21 g of intermediate 1-1.

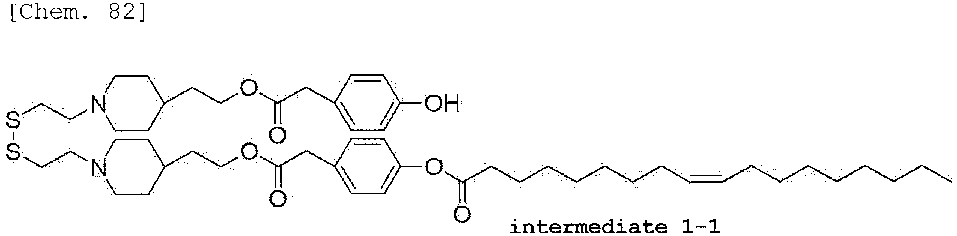

### <Synthesis of compound 13 using intermediate 1-1>

Intermediate 1-1 (214.1 mg, 0.235 mmol) was dissolved in chloroform (2.23 g), intermediate 13 (88.4 mg, 0.220 mmol), DMAP (5.4 mg, 0.044 mmol) and EDC hydrochloride (65.8 mg, 0.34 mmol) were added and the mixture was reacted at room temperature for 5.5 hr. Thereafter, the reaction solution was washed with 5 wt% sodium hydrogenphosphate aqueous solution and 20 wt% saline, and dehydrated by adding sodium sulfate. Chloroform was then added, sodium sulfate was removed by filtration, and the filtrate was concentrated in an evaporator to give 279 mg of compound 13.

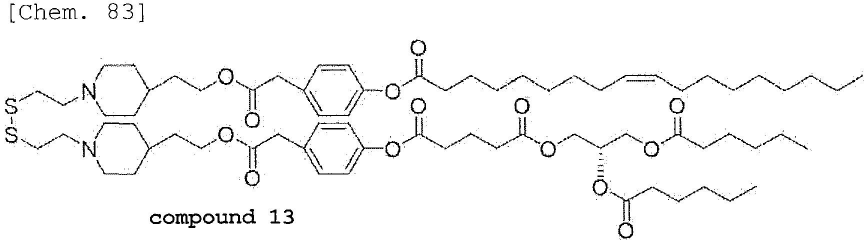

The content of ionic lipid (1) in the frozen composition is preferably 20 to 75 mol%, more preferably 30 to 70 mol%, further preferably 35 to 60 mol%, with respect to the total lipids, from the viewpoint of efficiency of nucleic acid encapsulation, efficiency of intracellular release of nucleic acid, and stability of the lipid nanoparticles. In the present specification, the "content of B with respect to A (mol%)" means "100×amount of B (mol)/amount of A (mol)".

The content of ionic lipid (1) in the frozen composition is preferably 0.012 to 8.1 wt%, more preferably 0.018 to 7.56 wt%, further preferably 0.021 to 7.02 wt%, with respect to the entire frozen composition, from the viewpoint of efficiency of nucleic acid encapsulation, efficiency of intracellular release of nucleic acid, and stability of the lipid nanoparticles. In the present specification, the "content of B with respect to A (wt%)" means "100×amount of B (g)/amount of A (g)".

### <Nucleic acid>

The nucleic acid encapsulated in the lipid nanoparticles may be either RNA or DNA, for example, those described below. Furthermore, either RNA or DNA may be an antisense oligonucleotide. The nucleic acid is preferably mRNA. Once the N/P ratio and the content of ionic lipid (1) are specified, the content of nucleic acid in the frozen composition can be specified accordingly.

### <Phospholipid>

Phospholipids can be used as a lipid membrane constituting component of lipid nanoparticles. Only one kind of the phospholipid may be used, or two or more kinds thereof may be used in combination.

Examples of the phospholipid include 1,2-diacyl-sn-glycero-3-phosphocholine (PC), 1,2-diacyl-sn-glycero-3-phosphoethanolamine (PE), 1,2-diacyl-sn-glycero-3-phosphoserine (PS), 1,2-diacyl-sn-glycero-3-phosphoglycerol (PG), 1,2-diacyl-sn-glycero-3-phosphatidic acid (PA), and lyso forms of these.

Specific examples of 1,2-diacyl-sn-glycero-3-phosphocholine (PC) include 1,2-didecanoyl-sn-glycero-3-phosphocholine (DDPC), 1,2-dilauroyl-sn-glycero-3-phosphocholine (DLPC), 1,2-dimyristoyl-sn-glycero-3-phosphocholine (DMPC), 1,2-dipalmitoyl-sn-glycero-3-phosphocholine (DPPC), 1,2-distearoyl-sn-glycero-3-phosphocholine (DSPC), 1,2-dioleoyl-sn-glycero-3-phosphocholine (DOPC), 1,2-dilinoleoyl-sn-glycero-3-phosphocholine (DLoPC), 1,2-dierucoyl-sn-glycero-3-phosphocholine (DEPC), 1-myristoyl-2-palmitoyl-sn-glycero-3-phosphocholine (MPPC), 1-myristoyl-2-stearoyl-sn-glycero-3-phosphocholine (MSPC), 1-palmitoyl-2-myristoyl-sn-glycero-3-phosphocholine (PMPC), 1-palmitoyl-2-stearoyl-sn-glycero-3-phosphocholine (PSPC), 1-palmitoyl-2-oleoyl-sn-glycero-3-phosphocholine (POPC), and 1-stearoyl-2-oleoyl-sn-glycero-3-phosphocholine (SOPC).

The above-mentioned specific examples of 1,2-diacyl-sn-glycero-3-phosphocholine (PC) wherein the phosphocholine moiety is substituted by phosphoethanolamine, phosphoserine, phosphoglycerol, or phosphatidic acid, such as 1,2-diacyl-sn-glycero-3-phosphoethanolamine (PE), 1,2-diacyl-sn-glycero-3-phosphoserine (PS), 1,2-diacyl-sn-glycero-3-phosphoglycerol (PG), and 1,2-diacyl-sn-glycero-3-phosphatidic acid (PA), can also be used.

Phospholipid is
preferably at least one selected from the group consisting of PC, PE, PS, and PG,
more preferably at least one selected from the group consisting of DOPC, DSPC, DEPC, POPC, DOPE (1,2-dioleoyl-sn-glycero-3-phosphoethanolamine), POPE (1-palmitoyl-2-oleoyl-sn-glycero-3-phosphoethanolamine), DOPS (1,2-dioleoyl-sn-glycero-3-phosphoserine), DLoPS (1,2-dilinoleoyl-sn-glycero-phosphoserine), and DOPG (1,2-dioleoyl-sn-glycero-3-phosphoglycerol).

In one embodiment of the present invention, phospholipid is
further preferably at least one selected from the group consisting of DOPC, DSPC, DOPG, DEPC, POPE, and DOPE,
particularly preferably at least one selected from the group consisting of DOPC, DOPG, DEPC, POPE, and DOPE,
most preferably DOPC, DOPE, or POPE.

In another embodiment of the present invention, phospholipid is
further preferably at least one selected from the group consisting of DOPC, DSPC, DEPC, and DOPE,
particularly preferably at least one selected from the group consisting of DOPC and DOPE,
most preferably DOPC or DOPE.

The content of the phospholipid in the frozen composition is preferably 0 to 35 mol%, more preferably 2.5 to 35 mol%, further preferably 5 to 32.5 mol%, with respect to the total lipids, from the viewpoint of efficiency of nucleic acid encapsulation, efficiency of intracellular release of nucleic acid, and stability of the lipid nanoparticles.

### <Sterol>

Sterol can be used as a component that regulates fluidity of the lipid membrane of lipid nanoparticles. Only one kind of the sterol may be used, or two or more kinds thereof may be used in combination.

Examples of the sterol include cholesterol, lanosterol, phytosterol, zymosterol, zymostenol, desmosterol, stigmastanol, dihydrolanosterol, and 7-dehydrocholesterol. Sterol is preferably at least one selected from the group consisting of cholesterol, lanosterol, and phytosterol, more preferably cholesterol.

When sterol is used, the content thereof in the frozen composition is preferably 5 to 60 mol%, more preferably 7 to 55 mol%, further preferably 10 to 50 mol%, with respect to the total lipids, from the viewpoint of efficiency of nucleic acid encapsulation, efficiency of intracellular release of nucleic acid, and stability of the lipid nanoparticles.

### <PEG lipid>

PEG lipids are used as stabilizers that coat the surface of lipid nanoparticles with hydrophilic polyethylene glycol (PEG) and suppress the aggregation of particles, or used to suppress the interaction between biological components and particles when administered to a living body. Only one kind of the PEG lipid may be used or two or more kinds thereof may be used in combination.

The PEG domain can have any molecular weight. In some embodiments, the PEG domain has a number average molecular weight of 200 to 10,000. The PEG domain may be linear or branched.

Examples of the PEG lipid include, phospholipid-PEG, ceramide-PEG, diacylglycerol-PEG, and cholesterol-PEG.

PEG lipid is
preferably diacylglycerol-PEG with a number average molecular weight of PEG of 1,000 to 10,000,
more preferably at least one selected from the group consisting of dimyristoylglycerol-PEG with a number average molecular weight of PEG of 1,000 to 10,000, and distearoylglycerol-PEG with a number average molecular weight of PEG of 1,000 to 10,000,
further preferably dimyristoylglycerol-PEG with a number average molecular weight of PEG of 1,000 to 10,000.

When a PEG lipid is used, the content thereof in the frozen composition is preferably 0.1 to 6.0 mol%, more preferably 0.5 to 5.0 mol%, further preferably 0.7 to 3.0 mol%, with respect to the total (mol) of different lipids, from the viewpoint of efficiency of nucleic acid encapsulation, efficiency of intracellular release of nucleic acid, and stability of the lipid nanoparticles. Here, the "different lipids" refers to lipids other than the PEG lipid. For example, when ionic lipid (1) and a PEG lipid are used, "the total of different lipids" means the ionic lipid (1). Furthermore, for example, when ionic lipid (1), a phospholipid, a sterol, and a PEG lipid are used, "the total of different lipids" means the total of ionic the lipid (1), the phospholipid, and the sterol.

### <Other component>

The frozen composition of the present invention may contain other components besides nucleic acids and lipids, as long as they do not inhibit the effects of the present invention. Only one kind of such other components may be used or two or more kinds thereof may be used in combination.

### <Water (ice)>

The frozen composition of the present invention preferably contains water (ice). When the frozen composition of the present invention contains water (ice), the content of water (ice) is preferably 75 to 99 wt%, more preferably 80 to 99 wt%, further preferably 85 to 99 wt%, with respect to the entire frozen composition.

### <Cryoprotective agent>

As other components, one or more kinds of cryoprotective agent may be used. Examples of the cryoprotective agent include saccharides such as monosaccharide, sugar alcohol, disaccharide, oligosaccharide, polysaccharide, and the like, or PVP, PVA, Poloxamer, Brij, TERGITOL, Thesit, or PEG. Saccharides is preferably disaccharide, further preferably sucrose.

When a cryoprotective agent is used, the content thereof in the frozen composition is preferably 40.0 to 99.9 wt%, more preferably 50.0 to 99.9 wt%, further preferably 60.0 to 99.9 wt%, particularly preferably 70.0 to 99.9 wt%, most preferably 90.0 to 99.9 wt%, with respect to the entire frozen composition less water (ice), from the viewpoints of the formation of the nucleic acid-encapsulating lipid nanoparticles and stability thereof, and cryoprotective ability.

When a cryoprotective agent is used, the content thereof in the frozen composition is preferably 2 to 50 wt%, more preferably 4 to 32 wt%, further preferably 6 to 16 wt%, with respect to the entire frozen composition, from the viewpoints of the formation of the nucleic acid-encapsulating lipid nanoparticles and stability thereof, and cryoprotective ability.

The particle size of nucleic acid-encapsulating lipid nanoparticles in a suspension obtained by thawing the frozen composition of the present invention is not particularly limited, and is preferably 10 nm or more and 200 nm or less, more preferably 30 nm or more and 150 nm or less. The thawing of the frozen composition for measuring the aforementioned particle size is performed by allowing 20 mL or less of the frozen composition to stand in an atmospheric pressure environment at 4 to 25°C for 30 min to 2 hr. The aforementioned particle size can be measured and calculated by using a particle size distribution measuring device such as Zetasizer Nano (Malvern) or the like. The particle size of the lipid nanoparticle encapsulating a nucleic acid can be appropriately adjusted by the production method thereof. In the present invention, the "particle size" means the Z-average size (average particle size) measured and calculated by dynamic light scattering method.

The surface charge (zeta potential) of nucleic acid-encapsulating lipid nanoparticles in a suspension obtained by thawing the frozen composition of the present invention is not particularly limited and preferably -15 mV to +15 mV, further preferably -10 mV to +10 mV. The surface potential (zeta potential) can be measured using a zeta potential measuring device such as Zetasizer Nano or the like. The surface potential (zeta potential) of the nucleic acid-encapsulating lipid nanoparticles can be adjusted by the composition of the constituent components of the lipid nanoparticles.

The PdI of nucleic acid-encapsulating lipid nanoparticles in a suspension obtained by thawing the frozen composition of the present invention is not particularly limited, and is preferably 0.01 or more and 0.30 or less, more preferably 0.01 or more and 0.25 or less. The thawing of the frozen composition for measuring the aforementioned PdI is performed by allowing 20 mL or less of the frozen composition to stand in an atmospheric pressure environment at 4 to 25°C for 30 min to 2 hr. The aforementioned PdI can be measured and calculated by using a particle size distribution measuring device such as Zetasizer Nano (Malvern) or the like.

The nucleic acid encapsulation rate of the nucleic acid-encapsulating lipid nanoparticles in a suspension obtained by thawing the frozen composition of the present invention is not particularly limited, and is preferably 75% or more and 100% or less, more preferably 80% or more and 100% or less, further preferably 85% or more and 100% or less. The thawing of the frozen composition for measuring the aforementioned encapsulation rate is performed by allowing 20 mL or less of the frozen composition to stand in an atmospheric pressure environment at 4 to 25°C for 30 min to 2 hr. The aforementioned encapsulation rate can be measured and calculated by using Ribogreen (registered trademark) assay.

### <Production method of frozen composition>

The present invention also provides a method for producing a frozen composition of nucleic acid-encapsulating lipid nanoparticles.
In the production method of the present invention,
the nucleic acid-encapsulating lipid nanoparticles contain ionic lipid (1),
has a molar ratio of amino groups of ionic lipid (1)/phosphate groups of the nucleic acid is 7 or more and 250 or less, and
the production method of the present invention includes the following step a), step b), step c), and step d),
the following step a), step b'), and step d), or
the following step a), step b'), step c), and step d):
   a) a step of mixing an alcohol solution containing an ionic lipid (1), a sterol, and a PEG lipid, and a nucleic acid solution containing an acidic buffer with a pH of 1.0 to 6.5 as a solvent to prepare a suspension containing nucleic acid-encapsulating lipid nanoparticles,
   b) a step of exchanging a dispersion medium of the suspension obtained in step a) with a buffer with a pH of 4.5 to 8.0, which is other than the acidic buffer used in step a), to obtain a suspension containing nucleic acid-encapsulating lipid nanoparticles,
   b') a step of exchanging the dispersion medium of the suspension obtained in step a) with a buffer with a pH of 4.5 to 8.0, which is other than the acidic buffer used in step a), and containing a cryoprotective agent to obtain a suspension containing nucleic acid-encapsulating lipid nanoparticles and the cryoprotective agent,
   c) a step of mixing the suspension obtained in step b) or step b') and a cryoprotective agent to obtain a suspension containing nucleic acid-encapsulating lipid nanoparticles and the cryoprotective agent,
   d) a step of freezing the suspension obtained in step b') or step c) at -10°C or less.

The production method of the present invention preferably includes step a), step b), step c), and step d), or step a), step b'), and step d), wherein in step c), the suspension obtained in step b) and a cryoprotective agent is mixed to obtain a suspension containing nucleic acid-encapsulating lipid nanoparticles and the cryoprotective agent.

The descriptions of the ionic lipid (1) and N/P ratio in the production method of the present invention are the same as the aforementioned descriptions for the frozen composition of the present invention. Each step in the production method of the present invention is described below in order.

### <Step a)>

In step a), an alcohol solution containing ionic lipid (1), a sterol, and a PEG lipid (hereinafter sometimes to be abbreviated as "alcohol solution"), and a nucleic acid solution (hereinafter sometimes to be abbreviated as "nucleic acid solution") containing an acidic buffer with a pH of 1.0 to 6.5 as a solvent (hereinafter sometimes to be abbreviated as "acidic buffer") are mixed to prepare a suspension containing nucleic acid-encapsulating lipid nanoparticles. In order to prepare nucleic acid-encapsulating lipid nanoparticles, any operation to induce organization can be used. The alcohol solution preferably contains phospholipids.

The descriptions of the phospholipid, sterol, PEG lipid, and nucleic acid used in step a) are the same as the aforementioned descriptions for the frozen composition of the present invention.

Examples of the alcohol used in step a) include ethanol, tert-butanol, and the like.

The concentration of the total lipids (i.e., total of ionic lipid (1), phospholipid, sterol and PEG lipid) in the alcohol solution used in step a) is preferably 0.1 to 30 mM, more preferably 0.5 to 25 mM, further preferably 1 to 20 mM, from the viewpoints of lipid solubility and nucleic acid encapsulation.

The concentration of ionic lipid (1) in the alcohol solution used in step a) is preferably 0.02 to 22.5 mM, more preferably 0.15 to 17.5 mM, further preferably 0.35 to 15 mM, from the viewpoint of lipid solubility.

The concentration of sterol in the alcohol solution used in step a) is preferably 0.005 to 18 mM, more preferably 0.035 to 13.75 mM, further preferably 0.1 to 12.5 mM, from the viewpoint of lipid solubility.

The concentration of phospholipid in the alcohol solution used in step a) is preferably 0 to 12 mM, more preferably 0.0125 to 8.75 mM, further preferably 0.05 to 8.125 mM, from the viewpoint of lipid solubility.

The concentration of PEG lipid in the alcohol solution used in step a) is preferably 0.0001 to 1.8 mM, more preferably 0.0025 to 1.25 mM, further preferably 0.007 to 0.75 mM, from the viewpoint of lipid solubility.

In step a), an acidic buffer with pH 1.0 to 6.5 is used. The aforementioned pH is preferably pH 3.0 to 6.5, more preferably pH 3.0 to 5.0.

Examples of acidic buffer include tartaric acid/NaOH buffer, KH phthalate/HCl buffer, glycine/HCl buffer, trans-aconitic acid/NaOH buffer, formic acid/Na formate buffer, 3,3-dimethylglutaric acid/NaOH buffer, 3,3-dimethylglutaric acid/NaOH/0.1 M NaCl buffer, phenylacetic acid/Na phenylacetate buffer, acetic acid/Na acetate buffer, succinic acid/NaOH buffer, KH phthalate/NaOH buffer, Na cacodylate/HCl buffer, NaH maleate/NaOH buffer, maleic acid/Tris/NaOH buffer, phosphate buffer, KH₂PO₄/NaOH buffer, 2-morpholinoethanesulfonic acid (MES) buffer, malic acid buffer, phthalate buffer, maleate buffer, Tris/maleate buffer, succinate buffer, tartrate buffer, citrate buffer, bis-Tris buffer, glycyl glycine buffer, and the like.

The acidic buffer is preferably malic acid buffer, citrate buffer, acetate buffer, or MES buffer (more preferably malic acid buffer or citrate buffer) having a pH of 1.0 to 6.5 (more preferably 3.0 to 6.5, further preferably 3.0 to 5.0).

The concentration of buffer components in the acidic buffer used in step a) is preferably 10 to 100 mM, more preferably 10 to 80 mM, from the viewpoint of stability of the nucleic acid. For example, when the acidic buffer is malic acid buffer, the "concentration of buffer components" here means the total concentration of malic acid and a salt thereof. In addition, for example, when the acidic buffer is maleic acid/Tris/NaOH buffer, the "concentration of buffer components" here means the total concentration of maleic acid and a salt thereof, and Tris and a salt thereof. The "concentration of buffer components" of other acidic buffers also means the same as the "concentration of buffer components" of the malic acid buffer and the "concentration of buffer components" of the maleic acid/Tris/NaOH buffer.

The concentration of nucleic acid in the nucleic acid solution used in step a) is preferably 0.1 to 720 µg/mL, more preferably 0.5 to 350 µg/mL, further preferably 1 to 250 ug/mL, from the viewpoint of solubility of the nucleic acid.

When mixing an nucleic acid solution and an alcohol solution of lipid, the volume ratio of these (nucleic acid solution:lipid alcohol solution) is preferably 1:1 to 20:1, more preferably 1.5:1 to 15:1, further preferably 2:1 to 8:1, from the viewpoints of particle size of the nucleic acid-encapsulating lipid nanoparticles and nucleic acid encapsulation rate.

Examples of the "operation to induce organization" for preparing nucleic acid-encapsulating lipid nanoparticles include methods known per se such as an alcohol dilution method using a micro flow path or vortex, a simple hydration method, sonication, heating, vortex, an ether injecting method, a French press method, a cholic acid method, a Ca²⁺ fusion method, a freeze-thaw method, a reversed-phase evaporation method and the like, preferably an alcohol dilution method using a micro flow path or vortex, further preferably an alcohol dilution method using a micro flow path. Preparation of particles by the alcohol dilution method using a micro flow path can be performed using, for example, NanoAssemblr (manufactured by Precision NanoSystems).

### <Step b)>

In step b), the dispersion medium of the suspension obtained in step a) is exchanged with a buffer with a pH of 4.5 to 8.0, which is other than the acidic buffer used in step a) (hereinafter sometimes to be abbreviated as "different buffer"), to obtain a suspension containing nucleic acid-encapsulating lipid nanoparticles. The pH of different buffer is preferably 5.0 to 7.6, more preferably 5.5 to 6.9.

Specific examples of different buffer include tartaric acid/NaOH buffer, KH phthalate/HCl buffer, glycine/HCl buffer, trans-aconitic acid/NaOH buffer, formic acid/Na formate buffer, 3,3-dimethylglutaric acid/NaOH buffer, 3,3-dimethylglutaric acid/NaOH/0.1 M NaCl buffer, phenylacetic acid/Na phenylacetate buffer, acetic acid/Na acetate buffer, succinic acid/NaOH buffer, KH phthalate/NaOH buffer, Na cacodylate/HCl buffer, NaH maleate/NaOH buffer, maleic acid/Tris/NaOH buffer, phosphate buffer, KH₂PO₄/NaOH buffer, MES buffer, malic acid buffer, phthalate buffer, maleate buffer, Tris/maleate buffer, succinate buffer, tartrate buffer, citrate buffer, bis-Tris buffer, glycyl glycine buffer, Tris/NaCl buffer, Tris/HCl buffer, Tris-buffered saline, and the like.

Different buffer is preferably malic acid buffer, citrate buffer, MES buffer, maleic acid/Tris/NaOH buffer, Tris/maleate buffer, or Tris-buffered saline (more preferably Tris/maleate buffer or Tris-buffered saline) having a pH of 4.5 to 8.0 (more preferably 5.0 to 7.6, further preferably 5.5 to 6.9).

The concentration of buffer components in the different buffer is preferably 10 to 100 mM, more preferably 20 to 50 mM, from the viewpoints of nucleic acid encapsulation rate and formation of nucleic acid-encapsulating lipid nanoparticles. For example, when the different buffer is citrate buffer, the "concentration of buffer components" here means the total concentration of citric acid and a salt thereof. In addition, for example, when the different buffer is maleic acid/Tris/NaOH buffer, the "concentration of buffer components" here means the total concentration of maleic acid and a salt thereof, and Tris and a salt thereof. The "concentration of buffer components" in the different buffers other than citrate buffer and maleic acid/Tris/NaOH buffer also means the same as the "concentration of buffer components" of the citrate buffer and the "concentration of buffer components" of the maleic acid/Tris/NaOH buffer.

The concentration of nucleic acid in the suspension obtained in step b) is not particularly limited as long as nucleic acid-encapsulating lipid nanoparticles can be maintained after exchange of a different buffer, and is preferably 0.25 to 3300 µg/mL, more preferably 2.5 to 1500 µg/mL, further preferably 3 to 800 µg/mL, from the viewpoint of particle size of the nucleic acid-encapsulating lipid nanoparticles. In step b), a different buffer may be used in an amount that affords the above-mentioned nucleic acid concentration.

The exchange of the dispersion medium of the suspension obtained in step a) with a different buffer can be performed, for example, by concentrating the suspension obtained in step a) and diluting same with the different buffer. The aforementioned concentration can be performed, for example, by ultrafiltration and the like. The ultrafiltration can be performed, for example, by applying centrifugal force using Amicon Ultra 15 (centrifugal filter unit manufactured by Merck), or by tangential flow filtration. Here, the "tangential flow filtration " is a method of filtering while suppressing the phenomenon of accumulation, on the membrane surface, of a suspended substance in the membrane feed water, by creating a flow parallel to the membrane surface, and can be performed, for example, using "KrosFlo (registered trademark) KR2i TFF System" manufactured by REPLIGEN. The aforementioned dilution can be performed by adding a buffer or by dialysis. The order of the aforementioned concentration and dilution is not particularly limited, and concentration may be performed first, followed by dilution, or dilution may be performed first, followed by concentration, and then dilution. Multiple concentrations and multiple dilutions may also be performed. Another method for exchanging the dispersion medium without accompanying concentration or dilution is, for example, diafiltration. Here, the "diafiltration" is a method in which a different buffer moves to the suspension side at the same pace as filtration, constantly exchanging the buffer from the suspension. It can be performed using, for example, "KrosFlo (registered trademark) KR2i TFF System" manufactured by REPLIGEN.

### <Step b')>

In step b'), the dispersion medium of the suspension obtained in step a) is exchanged with a buffer with a pH of 4.5 to 8.0 and containing a cryoprotective agent, which is other than the acidic buffer used in step a) (hereinafter sometimes to be abbreviated as "different buffer containing cryoprotective agent"), to obtain a suspension containing nucleic acid-encapsulating lipid nanoparticles and a cryoprotective agent.

The description of the cryoprotective agent used in step b') is the same as the aforementioned description in the frozen composition of the present invention.

The different buffer containing cryoprotective agent in step b') is, for example, the different buffer in step b) to which sucrose has been added. Specific examples thereof include tartaric acid/NaOH/sucrose buffer, KH phthalate/HCl/sucrose buffer, glycine/HCl/sucrose buffer, trans-aconitic acid/NaOH/sucrose buffer, formic acid/Na formate/sucrose buffer, 3,3-dimethylglutaric acid/NaOH/sucrose buffer, 3,3-dimethylglutaric acid/NaOH/0.1 M NaCl/sucrose buffer, phenylacetic acid/Na phenylacetate/sucrose buffer, acetic acid/Na acetate/sucrose buffer, succinic acid/NaOH/sucrose buffer, KH phthalate/NaOH/sucrose buffer, Na cacodylate/HCl/sucrose buffer, NaH maleate/NaOH/sucrose buffer, maleic acid/Tris/NaOH/sucrose buffer, phosphorate/sucrose buffer, KH₂PO₄/NaOH/sucrose buffer, MES/sucrose buffer, malic acid/sucrose buffer, phthalate/sucrose buffer, maleate/sucrose buffer, Tris/maleate/sucrose buffer, succinate/sucrose buffer, tartarate/sucrose buffer, citrate/sucrose buffer, bis-Tris/sucrose buffer, glycyl glycine/sucrose buffer, Tris/NaCl/sucrose buffer, Tris/HCl/sucrose buffer, Tris-buffered saline/sucrose buffer, and the like. As used herein, the "tartaric acid/NaOH/sucrose buffer" means a "buffer obtained by adding sucrose to tartaric acid/NaOH buffer". The aforementioned other specific examples are the same as the "tartaric acid/NaOH/sucrose buffer".

The different buffer containing cryoprotective agent is preferably malic acid/sucrose buffer, citrate/sucrose buffer, MES/sucrose buffer, maleic acid/Tris/NaOH/sucrose buffer, Tris/maleate/sucrose buffer, or Tris/NaCl/sucrose buffer (more preferably MES/sucrose buffer, Tris/NaCl/sucrose buffer, or Tris/maleate/sucrose buffer) having a pH of 4.5 to 8.0 (more preferably 5.0 to 7.6, further preferably 5.5 to 6.9).

The concentration of buffer components in the different buffer containing cryoprotective agent is preferably 10 to 100 mM, more preferably 20 to 50 mM, from the viewpoints of nucleic acid encapsulation rate and formation of nucleic acid-encapsulating lipid nanoparticles. The description of the concentration of the buffer components in the different buffer containing cryoprotective agent in step b') is the same as the description of the concentration of the buffer components in the different buffer in step b).

The concentration of the cryoprotective agent in the different buffer containing cryoprotective agent is preferably 20 to 320 mg/mL, more preferably 80 to 160 mg/mL, from the viewpoint of protection of nucleic acid-encapsulating lipid nanoparticles from the stress of freezing-thawing.

The concentration of the cryoprotective agent in the suspension obtained in step b') is preferably 20 to 320 mg/mL, more preferably 40 to 240 mg/mL, further preferably 80 to 160 mg/mL, from the viewpoint of protection of nucleic acid-encapsulating lipid nanoparticles from the stress of freezing-thawing.

The concentration of nucleic acid in the suspension obtained in step b') is not particularly limited as long as nucleic acid-encapsulating lipid nanoparticles can be maintained after exchange of a different buffer containing cryoprotective agent, and is preferably 0.25 to 3300 µg/mL, more preferably 2.5 to 1500 µg/mL, further preferably 3 to 800 µg/mL, from the viewpoint of particle size of the nucleic acid-encapsulating lipid nanoparticles. In step b'), a different buffer containing a cryoprotective agent may be used in an amount that affords the above-mentioned nucleic acid concentration.

The exchange of the dispersion medium of the suspension obtained in step a) with a different buffer containing cryoprotective agent can be performed, for example, by concentrating the suspension obtained in step a) and diluting same with the different buffer containing cryoprotective agent. The aforementioned concentration and dilution can be performed, for example, by ultrafiltration, dialysis, tangential flow filtration, and the like. The order of the aforementioned concentration and dilution is not particularly limited, and concentration may be performed first, followed by dilution, or dilution may be performed first, followed by concentration, and then dilution. Multiple concentrations and multiple dilutions may also be performed.

### <Step c)>

In step c), the suspension obtained in step b) or step b') is mixed with a cryoprotective agent to obtain a suspension containing nucleic acid-encapsulating lipid nanoparticles and the cryoprotectant.

The description of the cryoprotective agent used in step c) is the same as the aforementioned description in the frozen composition of the present invention.

In step c), a solution of a cryoprotective agent may also be used as a cryoprotective agent. Examples of the solvent of the solution include water, ethanol, dimethyl sulfoxide, and the like. The solvent is preferably water, and the solution of a cryoprotective agent is preferably an aqueous solution of a cryoprotective agent. When a solution of a cryoprotective agent is used in step c), the concentration of the cryoprotective agent in the solution is, in one embodiment of the present invention, preferably 40 to 1040 mg/mL, more preferably 40 to 640 mg/mL, further preferably 60 to 640 mg/mL, particularly preferably 60 to 480 mg/mL, most preferably 80 to 480 mg/mL, from the viewpoint of protection of nucleic acid-encapsulating lipid nanoparticles from the stress of freezing-thawing.

The concentration of the cryoprotective agent in the suspension obtained in step c) is preferably 40 to 320 mg/mL, more preferably 60 to 240 mg/mL, further preferably 80 to 480 mg/mL, from the viewpoint of protection of nucleic acid-encapsulating lipid nanoparticles from the stress of freezing-thawing.

### <Step d)>

In step d), the suspension obtained in step b') or step c) is frozen at -10°C or below. The freezing temperature is preferably -80°C to -10°C, more preferably -25°C to -10°C.

The freezing in step d) can be performed in any suitable container known in the technique field of medicaments, such as polypropylene (PP) tube, glass container (e.g., glass vial), two-chamber container, or the like. A glass container is preferred as the container.

The suspension obtained in step b') or step c) is added to a container (e.g., PP tube or glass container). The volume of the suspension to be added to the container is preferably 0.01 to 20 mL, more preferably 0.1 to 15 mL, further preferably 1 to 10 mL.

In the production method of the present invention, a ratio of (the particle size of nucleic acid-encapsulating lipid nanoparticles in a suspension obtained by thawing the frozen composition obtained in step d))/(the particle size of the nucleic acid-encapsulating lipid nanoparticles in the suspension obtained in step b') or step c)) is preferably 0.8 or more and 1.5 or less, more preferably 0.8 or more and 1.2 or less. The thawing of the frozen composition for measuring the aforementioned particle size is performed by allowing 20 mL or less of the frozen composition to stand in an atmospheric pressure environment at 4 to 25°C for 30 min to 2 hr. The aforementioned particle size can be measured and calculated by using a particle size distribution measuring device such as Zetasizer Nano (Malvern) or the like. In the present invention, the "particle size" means the Z-average size (average particle size) measured and calculated by dynamic light scattering method.

In the production method of the present invention, the particle size of nucleic acid-encapsulating lipid nanoparticles in a suspension obtained by thawing the frozen composition obtained in step d) is not particularly limited, and is preferably 10 nm or more and 200 nm or less, more preferably 30 nm or more and 150 nm or less.

In the production method of the present invention, the PdI of nucleic acid-encapsulating lipid nanoparticles in a suspension obtained by thawing the frozen composition obtained in step d) is preferably 0.01 or more and 0.30 or less, more preferably 0.01 or more and 0.25 or less. The thawing of the frozen composition for measuring the aforementioned PdI is performed by allowing 20 mL or less of the frozen composition to stand in an atmospheric pressure environment at 4 to 25°C for 30 min to 2 hr. The PdI can be measured and calculated by using a particle size distribution measuring device such as Zetasizer Nano (Malvern) or the like.

In the production method of the present invention, a ratio of the nucleic acid encapsulation rate of nucleic acid-encapsulating lipid nanoparticles in a suspension obtained by thawing the frozen composition, obtained in step d))/(the nucleic acid encapsulation rate of the nucleic acid-encapsulating lipid nanoparticles in the suspension obtained in step b') or step c)) is preferably 0.7 or more and 1.0 or less, more preferably 0.85 or more and 1.0 or less. The thawing of the frozen composition for measuring the aforementioned encapsulation rate is performed by allowing 20 mL or less of the frozen composition to stand in an atmospheric pressure environment at 4 to 25°C for 30 min to 2 hr. The aforementioned encapsulation rate can be measured and calculated by using Ribogreen (registered trademark) assay.

In the production method of the present invention, the nucleic acid encapsulation rate of nucleic acid-encapsulating lipid nanoparticles in a suspension obtained by thawing the frozen composition obtained in step d) is not particularly limited, and is preferably 75% or more and 100% or less, more preferably 80% or more and 100% or less, further preferably 85% or more and 100% or less.

<Storage of frozen materials containing nucleic acid-encapsulating lipid nanoparticles>

The frozen composition of the present invention, and a frozen composition obtained by the production method of the present invention are preferably stored at a temperature of - 10°C or less, more preferably -80°C to -10°C, further preferably -25°C to -15°C.

### <Production method of pharmaceutical product composition>

The present invention also provides a method for producing pharmaceutical product compositions, including the production method of the frozen composition of the present invention.

### <Method for introducing nucleic acid into cell>

The present invention also provides
(I) a method for introducing a nucleic acid in a suspension into a cell, comprising a step of contacting in vitro the cell with a suspension obtained by thawing the frozen composition of the present invention or a frozen composition produced by the production method of the present invention, or a suspension obtained by exchanging a dispersion medium of the aforementioned suspension with a different dispersion medium, and
(II) a method for introducing a nucleic acid in a suspension into a biological target cell of a living organism, comprising a step of administering, to the living organism, a suspension obtained by thawing the frozen composition of the present invention or a frozen composition produced by the production method of the present invention, or a suspension obtained by exchanging a dispersion medium of the aforementioned suspension with dispersion medium.

The aforementioned method (I) and the aforementioned method (II) are sometimes to be collectively abbreviated as "the introduction method of the present invention".

The "suspension obtained by thawing the frozen composition of the present invention or a frozen composition produced by the production method of the present invention, and a suspension obtained by exchanging a dispersion medium of the aforementioned suspension with a different dispersion medium" which are used in the introduction method of the present invention are sometimes to be abbreviated as "the suspension of the present invention" in the following.

The dispersion medium of the suspension obtained by thawing the frozen composition of the present invention or a frozen composition produced by the production method of the present invention can be exchanged with a different dispersion medium by concentration, dilution with a different dispersion medium, and the like. The aforementioned concentration and dilution can be performed, for example, by ultrafiltration, dialysis, tangential flow filtration, and the like. The order of the aforementioned concentration and dilution is not particularly limited, and concentration may be performed first, followed by dilution, or dilution may be performed first, followed by concentration, and then dilution. Multiple concentrations and multiple dilutions may also be performed. Examples of the different dispersion medium include the buffers described in step a) and step b).

The suspension of the present invention (i.e., a suspension obtained by thawing the frozen composition of the present invention or a frozen composition produced by the production method of the present invention, or a suspension obtained by exchanging a dispersion medium of the aforementioned suspension with a different dispersion medium) may also be used in the introduction method of the present invention, after concentration or dilution thereof.

Any nucleic acid can be introduced into cells by the introduction method of the present invention. The type of nucleic acid includes, but is not limited to, DNA, RNA, RNA chimeric nucleic acid, DNA/RNA hybrid, and the like. While any of single-stranded to triple-stranded nucleic acids can be used, single-stranded or double-strand one is preferred. The nucleic acid may also be other type of nucleotide that is N-glycoside of a purine or pyrimidine base, other oligomer having a non-nucleotide backbone (e.g., commercially available peptide nucleic acid (PNA) and the like), other oligomer containing a special bond (said oligomer containing a nucleotide having a configuration permitting base pairing or base attachment, which are found in DNA and RNA), or the like. Furthermore, the nucleic acid may also be, for example, a nucleic acid added with known modification, a nucleic acid with a label known in the pertinent field, a nucleic acid with a cap, a methylated nucleic acid, a nucleic acid in which one or more natural nucleotides substituted by an analog, a nucleic acid with an intramolecularly modified nucleotide, a nucleic acid with a non-charge bond (e.g., methyl sulfonate, phosphotriester, phosphoramidate, carbamate, etc.), a nucleic acid with a charged bond or sulfur-containing bond (e.g., phosphorothioate, phosphorodithioate, etc.), a nucleic acid with a side chain group such as protein (nuclease, nuclease inhibitor, toxin, antibody, signal peptide, poly-L-lysine, etc.), sugar (e.g., monosaccharide and the like), or the like, a nucleic acid containing an intercurrent compound (e.g., acridine, psoralen, etc.), a nucleic acid containing a chelate compound (e.g., metal, radioactive metal, boron, oxidative metal, etc.), a nucleic acid containing an alkylating agent, a nucleic acid with a modified bond (e.g., α anomer-type nucleic acid and the like), or the like.

The type of the DNA that can be used in the present invention is not particularly limited, and can be selected as appropriate according to the purpose of use. Examples thereof include plasmid DNA, cDNA, antisense DNA, chromosomal DNA, PAC, BAC, CpG oligosaccharide, and the like. Preferred are plasmid DNA, cDNA and antisense DNA, and more preferred is plasmid DNA. A circular DNA such as plasmid DNA and the like can be digested as appropriate with a restriction enzyme and the like, and also used as a linear DNA.

The type of the RNA that can be used in the present invention is not particularly limited, and can be selected as appropriate according to the purpose of use. Examples thereof include siRNA, miRNA, shRNA, antisense RNA, messenger RNA (mRNA), single-stranded RNA genome, double-stranded RNA genome, RNA replicon, transfer RNA, ribosomal RNA, and the like, with preference given to siRNA, miRNA, shRNA, mRNA, antisense RNA, and RNA replicon.

The nucleic acid used in the present invention is preferably purified by a method generally used by those of ordinary skill in the art.

The suspension of the present invention, which is used in the introduction method of the present invention, can be administered in vivo for the purpose of, for example, prevention and/or treatment of diseases. Therefore, the nucleic acid to be used in the present invention is preferably one having a preventive and/or therapeutic activity against a given disease (prophylactic/therapeutic nucleic acid). Examples of such nucleic acid include nucleic acids used for so-called gene therapy, and the like.

The suspension of the present invention, which is used in the introduction method of the present invention, can be used as a drug delivery system for selectively delivering a nucleic acid and the like into a particular cell, and is useful for, for example, DNA vaccines by introduction of antigen gene into dendritic cells, gene therapy drugs for tumor, nucleic acid pharmaceutical products that suppress expression of target genes by utilizing RNA interference, and the like.

The step of contacting the suspension of the present invention with a cell in vitro is specifically described below.

Cells are suspended in a suitable medium several days before contact with the suspension of the present invention, and cultured under appropriate conditions. At the time of contact with the suspension of the present invention, the cells may or may not be in a proliferative phase.

The culture medium at the time of the contact may be a serum-containing medium or a serum-free medium. The serum concentration of the medium is preferably 30 wt% or less, more preferably 20 wt% or less. When the medium contains excess proteins such as serum and the like, the contact between the suspension of the present invention and the cell may be inhibited.

The cell density at the time of the contact is not particularly limited, and can be appropriately determined in consideration of the kind of the cell and the like. It is generally within the range of 1×10⁴ to 1×10⁷ cells/mL.

The suspension of the present invention is added to cells. The amount of the suspension of the present invention to be added is not particularly limited, and can be appropriately determined in consideration of the cell number and the like. The concentration of total lipids when contacting cells with nucleic acid-encapsulating lipid nanoparticles and the like is not particularly limited as long as the desired introduction of the nucleic acid into the cells can be achieved. The concentration of the total lipids is generally generally 1 to 300 nmol/mL, preferably 10 to 200 nmol/mL, and the concentration of the nucleic acid is generally 0.01 to 100 µg/mL, preferably 0.05 to 10 µg/mL.

After the suspension of the present invention is added to cells, the cells are cultured. The temperature, humidity, CO₂ concentration, and the like during culturing are appropriately determined in consideration of the kind of the cell. When the cell is derived from a mammal, generally, the temperature is about 37°C, the humidity is about 95%, and the CO₂ concentration is about 5%. While the culture time can also be appropriately determined in consideration of the conditions such as the kind of the cell and the like, it is generally a range of 0.1 to 96 hr, preferably a range of 0.2 to 72 hr, more preferably a range of 0.5 to 48 hr. When the above-mentioned culture time is too short, the nucleic acid is not sufficiently introduced into the cells, and when the culture time is too long, the cells may become weak.

By the above-mentioned culture, a nucleic acid is introduced into cells. The culture is further continued preferably by exchanging the medium with a fresh medium, or adding a fresh medium to the medium. When the cell is a mammal-derived cell, the fresh medium preferably contains a serum or nutrition factor.

As mentioned above, a nucleic acid can be introduced into cells not only in vitro but also in vivo by using the suspension of the present invention. That is, by administering the suspension of the present invention to a subject, the nucleic acid-encapsulating lipid nanoparticles contained therein reach and contact the target cells, and the nucleic acid encapsulated in the nucleic acid-encapsulating lipid nanoparticle is introduced into the cells. The subject to which the suspension of the present invention can be administered is not particularly limited and examples thereof include vertebrates such as mammals (e.g., human, monkey, mouse, rat, hamster, bovine, etc.), birds (e.g., chicken, ostrich, etc.), amphibia (e.g., frog and the like), fishes (e.g., zebrafish, rice-fish, etc.), and the like, invertebrates such as insects (e.g., silk moth, moth, Drosophila, etc.) and the like, plants, and the like. The subject of administration of the suspension of the present invention is preferably human or other mammal.

The kind of the target cell is not particularly limited, and a nucleic acid can be introduced into cells in various tissues (e.g., liver, kidney, pancreas, lung, spleen, heart, blood, muscle, bone, brain, stomach, small intestine, large intestine, skin, adipose tissue, lymph node, tumor, etc.) by using the suspension of the present invention.

The administration method of the suspension of the present invention to a subject (e.g., vertebrate, invertebrate, and the like) is not particularly limited as long as the lipid nanoparticles contained in the suspension of the present invention can reach and contact with the target cells, and an administration method known per se (e.g., oral administration, parenteral administration (e.g., intravenous administration, intramuscular administration, topical administration, transdermal administration, subcutaneous administration, intraperitoneal administration, spray, etc.), etc.) can be appropriately selected in consideration of the kind and the site of the target cell, and the like. The dose of the suspension of the present invention is not particularly limited as long as the introduction of the compound into the cells can be achieved, and can be appropriately selected in consideration of the kind of the subject of administration, administration method, the kind of the compound to be introduced, the kind and the site of the target cell and the like.

### [Example]

Examples of the present invention are further described in detail in the following, but the present invention is not limited to the Examples.

In the following Examples, ionic lipid (1) is indicated by the names listed in Table 1 and Table 2. In addition, ionic lipid "D-Lin-MC3-DMA" (CAS number: 1224606-06-7) used in Comparative Examples is abbreviated as "MC3" in the following. This MC3 is an ionic lipid that does not have S-S bonds and is different from ionic lipid (1).

The abbreviations used in the following Examples and the like each mean the following.
Chol: cholesterol
DMG-PEG2000: 1,2-dimyristoyl-rac-glycerol, methoxypolyethylene glycol (number average molecular weight (Mn) of PEG: 2000)
DEPC: 1,2-dierucoyl-sn-glycero-3-phosphocholine
DOPC: 1,2-dioleoyl-sn-glycero-3-phosphocholine
DOPE: 1,2-dioleoyl-sn-glycero-3-phosphoethanolamine
DOPG: 1,2-dioleoyl-sn-glycero-3-phosphoglycerol
DSPC: 1,2-distearoyl-sn-glycero-3-phosphocholine
POPC: 1-palmitoyl-2-oleoyl-sn-glycero-3-phosphocholine
POPE: 1-palmitoyl-2-oleoyl-sn-glycero-3-phosphoethanolamine
MES: 2-morpholinoethanesulfonic acid
PBS: phosphate-buffered saline
DDW: deionized distilled water
tBuOH: tert-butanol
EtOH: ethanol

### Example 1 (Preparation of frozen composition containing nucleic acid-encapsulating lipid nanoparticles)

SS-OP was used as the ionic lipid (1), and DOPC, Chol, and DMG-PEG2000 were used as different lipids. The molar ratio of SS-OP:DOPC:Chol:DMG-PEG2000 used was 48.5:7.5:42.5:1.5. The lipids were mixed such that the total lipids in a lipid ethanol solution were 8.0 mM.

2075 µL of a nucleic acid solution (nucleic acid concentration: 3.3 µg/mL) obtained by diluting mRNA (CleanCap (registered trademark) FLuc mRNA(L-7602), hereinafter referred to as "FLuc mRNA") with an acidic citrate buffer (buffer component concentration: 20 mM, pH: 5.0), and 725 µL of the lipid ethanol solution were each measured into syringes. Using NanoAssemblr (registered trademark) ultra-fast nano pharmaceutical production device (manufactured by Precision NanoSystems), a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 14000 µL of a MES buffer (buffer component concentration: 20 mM, pH: 6.0), and the obtained suspension was transferred to an Amicon Ultra 15 (centrifugal filter unit manufactured by Merck). Ultrafiltration was performed under centrifugation conditions (25°C, 1000 g, 3 min) to concentrate same to about 2000 µL. The obtained concentrate was diluted to 15 mL with a MES buffer (buffer component concentration: 20 mM, pH: 6.0), and ultrafiltration was performed again under centrifugation conditions (25°C, 1000 g, 3 min) to concentrate same to about 500 µL. The obtained concentrate was transferred to an Amicon Ultra 4 (centrifugal filter unit manufactured by Merck), diluted to 4 mL with a MES buffer (buffer component concentration: 20 mM, pH: 6.0), concentrated again to about 100 µL by ultrafiltration under centrifugation conditions (25°C, 1000 g, 3 min), and diluted with a MES buffer (buffer component concentration: 20 mM, pH: 6.0) to obtain a suspension containing nucleic acid-encapsulating lipid nanoparticles (100 µL, nucleic acid concentration: 50 µg/mL) (step b)).

To the obtained suspension was added a sucrose aqueous solution to achieve a final sucrose concentration of 160 mg/mL (step c)). The obtained suspension was placed in a screw tube, and frozen at -80°C to obtain a frozen composition (ionic lipid (1) content: 0.9 wt%, nucleic acid content: 5.0 µg, N/P ratio: 241, cryoprotective agent (sucrose) content: 14 wt%, water (ice) content: 85 wt%) (step d)).

After freezing, the frozen composition was stored at - 20°C, and before use, thawed by standing same in an atmospheric pressure environment in a refrigerator at 4°C for 30 min to obtain a suspension.

### Example 2 (Preparation of frozen composition containing nucleic acid-encapsulating lipid nanoparticles)

SS-OP was used as the ionic lipid (1), and DOPC, Chol, and DMG-PEG2000 were used as different lipids. The molar ratio of SS-OP:DOPC:Chol:DMG-PEG2000 used was 48.5:7.5:42.5:1.5. The lipids were mixed such that the total lipids in a lipid ethanol solution were 8.0 mM.

2075 µL of a nucleic acid solution (nucleic acid concentration: 6.7 µg/mL) obtained by diluting FLuc mRNA with an acidic citrate buffer (buffer component concentration: 20 mM, pH: 5.0), and 725 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 14000 µL of a MES buffer (buffer component concentration: 20 mM, pH: 6.0), and the obtained suspension was transferred to an Amicon Ultra 15. Ultrafiltration was performed under centrifugation conditions (25°C, 1000 g, 3 min) to concentrate same to about 2000 µL. The obtained concentrate was diluted to 15 mL with a MES buffer (buffer component concentration: 20 mM, pH: 6.0), and ultrafiltration was performed again under centrifugation conditions (25°C, 1000 g, 3 min) to concentrate same to about 500 µL. The obtained concentrate was transferred to an Amicon Ultra 4, diluted to 4 mL with a MES buffer (buffer component concentration: 20 mM, pH: 6.0), concentrated again to about 100 µL by ultrafiltration under centrifugation conditions (25°C, 1000 g, 3 min), and diluted with a MES buffer (buffer component concentration: 20 mM, pH: 6.0) to obtain a suspension containing nucleic acid-encapsulating lipid nanoparticles (100 µL, nucleic acid concentration: 100 µg/mL) (step b)).

To the obtained suspension was added a sucrose aqueous solution to achieve a final sucrose concentration of 160 mg/mL (step c)). The obtained suspension was placed in a screw tube, and frozen at -80°C to obtain a frozen composition (ionic lipid (1) content: 0.9 wt%, nucleic acid content: 10 µg, N/P ratio: 120, cryoprotective agent (sucrose) content: 14 wt%, water (ice) content: 85 wt%) (step d)).

After freezing, the frozen composition was stored at - 20°C, and before use, thawed by standing same in an atmospheric pressure environment in a refrigerator at 4°C for 30 min to obtain a suspension.

### Example 3 (Preparation of frozen composition containing nucleic acid-encapsulating lipid nanoparticles)

SS-OP was used as the ionic lipid (1), and DOPC, Chol, and DMG-PEG2000 were used as different lipids. The molar ratio of SS-OP:DOPC:Chol:DMG-PEG2000 used was 48.5:7.5:42.5:1.5. The lipids were mixed such that the total lipids in a lipid ethanol solution were 8.0 mM.

2075 µL of a nucleic acid solution (nucleic acid concentration: 13.3 µg/mL) obtained by diluting FLuc mRNA with an acidic citrate buffer (buffer component concentration: 20 mM, pH: 5.0), and 725 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 14000 µL of a MES buffer (buffer component concentration: 20 mM, pH: 6.0), and the obtained suspension was transferred to an Amicon Ultra 15. Ultrafiltration was performed under centrifugation conditions (25°C, 1000 g, 3 min) to concentrate same to about 2000 µL. The obtained concentrate was diluted to 15 mL with a MES buffer (buffer component concentration: 20 mM, pH: 6.0), and ultrafiltration was performed again under centrifugation conditions (25°C, 1000 g, 3 min) to concentrate same to about 500 µL. The obtained concentrate was transferred to an Amicon Ultra 4, diluted to 4 mL with a MES buffer (buffer component concentration: 20 mM, pH: 6.0), concentrated again to about 100 µL by ultrafiltration under centrifugation conditions (25°C, 1000 g, 3 min), and diluted with a MES buffer (buffer component concentration: 20 mM, pH: 6.0) to obtain a suspension containing nucleic acid-encapsulating lipid nanoparticles (100 µL, nucleic acid concentration: 200 µg/mL) (step b)).

To the obtained suspension was added a sucrose aqueous solution to achieve a final sucrose concentration of 160 mg/mL (step c)). The obtained suspension was placed in a screw tube, and frozen at -80°C to obtain a frozen composition (ionic lipid (1) content: 0.9 wt%, nucleic acid content: 20 µg, N/P ratio: 60, cryoprotective agent (sucrose) content: 14 wt%, water (ice) content: 85 wt%) (step d)).

After freezing, the frozen composition was stored at - 20°C, and before use, thawed by standing same in an atmospheric pressure environment in a refrigerator at 4°C for 30 min to obtain a suspension.

### Example 4 (Preparation of frozen composition containing nucleic acid-encapsulating lipid nanoparticles)

SS-OP was used as the ionic lipid (1), and DOPC, Chol, and DMG-PEG2000 were used as different lipids. The molar ratio of SS-OP:DOPC:Chol:DMG-PEG2000 used was 48.5:7.5:42.5:1.5. The lipids were mixed such that the total lipids in a lipid ethanol solution were 4.0 mM.

1325 µL of a nucleic acid solution (nucleic acid concentration: 26.7 µg/mL) obtained by diluting FLuc mRNA with an acidic citrate buffer (buffer component concentration: 20 mM, pH: 5.0), and 475 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 7000 µL of a MES buffer (buffer component concentration: 20 mM, pH: 6.0), and the obtained suspension was transferred to an Amicon Ultra 15, and ultrafiltrated under centrifugation conditions (25°C, 1000 g, 3 min), and concentrated to about 1000 µL. The obtained concentrate was diluted to 15 mL with a MES buffer (buffer component concentration: 20 mM, pH: 6.0), and ultrafiltration was performed again under centrifugation conditions (25°C, 1000 g, 3 min) to concentrate same to about 500 µL. The obtained concentrate was transferred to an Amicon Ultra 4, diluted to 4 mL with a MES buffer (buffer component concentration: 20 mM, pH: 6.0), concentrated again to about 100 µL by ultrafiltration under centrifugation conditions (25°C, 1000 g, 3 min), and diluted with a MES buffer (buffer component concentration: 20 mM, pH: 6.0) to obtain a suspension containing nucleic acid-encapsulating lipid nanoparticles (100 µL, nucleic acid concentration: 200 µg/mL) (step b)).

To the obtained suspension was added a sucrose aqueous solution to achieve a final sucrose concentration of 160 mg/mL (step c)). The obtained suspension was placed in a screw tube, and frozen at -80°C to obtain a frozen composition (ionic lipid (1) content: 0.2 wt%, nucleic acid content: 20 µg, N/P ratio: 15, cryoprotective agent (sucrose) content: 14 wt%, water (ice) content: 86 wt%) (step d)).

After freezing, the frozen composition was stored at - 20°C, and before use, thawed by standing same in an atmospheric pressure environment in a refrigerator at 4°C for 30 min to obtain a suspension.

### Example 5 (Preparation of frozen composition containing nucleic acid-encapsulating lipid nanoparticles)

SS-OP was used as the ionic lipid (1), and DOPC, Chol, and DMG-PEG2000 were used as different lipids. The molar ratio of SS-OP:DOPC:Chol:DMG-PEG2000 used was 48.5:7.5:42.5:1.5. The lipids were mixed such that the total lipids in a lipid ethanol solution were 4.0 mM.

1325 µL of a nucleic acid solution (nucleic acid concentration: 53.3 µg/mL) obtained by diluting FLuc mRNA with an acidic citrate buffer (buffer component concentration: 20 mM, pH: 5.0), and 475 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 7000 µL of a MES buffer (buffer component concentration: 20 mM, pH: 6.0), and the obtained suspension was transferred to an Amicon Ultra 15, and ultrafiltrated under centrifugation conditions (25°C, 1000 g, 3 min), and concentrated to about 1000 µL. The obtained concentrate was diluted to 15 mL with a MES buffer (buffer component concentration: 20 mM, pH: 6.0), and ultrafiltration was performed again under centrifugation conditions (25°C, 1000 g, 3 min) to concentrate same to about 500 µL. The obtained concentrate was transferred to an Amicon Ultra 4, diluted to 4 mL with a MES buffer (buffer component concentration: 20 mM, pH: 6.0), concentrated again to about 100 µL by ultrafiltration under centrifugation conditions (25°C, 1000 g, 3 min), and diluted with a MES buffer (buffer component concentration: 20 mM, pH: 6.0) to obtain a suspension containing nucleic acid-encapsulating lipid nanoparticles (100 µL, nucleic acid concentration: 400 µg/mL) (step b)).

To the obtained suspension was added a sucrose aqueous solution to achieve a final sucrose concentration of 160 mg/mL (step c)). The obtained suspension was placed in a screw tube, and frozen at -80°C to obtain a frozen composition (ionic lipid (1) content: 0.2 wt%, nucleic acid content: 40 µg, N/P ratio: 8, cryoprotective agent (sucrose) content: 14 wt%, water (ice) content: 85 wt%) (step d)).

After freezing, the frozen composition was stored at - 20°C, and before use, thawed by standing same in an atmospheric pressure environment in a refrigerator at 4°C for 30 min to obtain a suspension.

### Comparative Example 1 (Preparation of frozen composition containing nucleic acid-encapsulating lipid nanoparticles)

MC3 was used as the ionic lipid, and DSPC, Chol, and DMG-PEG2000 were further used as different lipids. The molar ratio of MC3:DSPC:Chol:DMG-PEG2000 used was 50:10:38.5:1.5. The lipids were mixed such that the total lipids in a lipid ethanol solution were 8.0 mM.

2075 µL of a nucleic acid solution (nucleic acid concentration: 13.3 µg/mL) obtained by diluting FLuc mRNA with an acidic citrate buffer (buffer component concentration: 20 mM, pH: 5.0), and 725 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 14000 µL of a MES buffer (buffer component concentration: 20 mM, pH: 6.0), and the obtained suspension was transferred to an Amicon Ultra 15. Ultrafiltration was performed under centrifugation conditions (25°C, 1000 g, 3 min) to concentrate same to about 2000 µL. The obtained concentrate was diluted to 15 mL with a MES buffer (buffer component concentration: 20 mM, pH: 6.0), and ultrafiltration was performed again under centrifugation conditions (25°C, 1000 g, 3 min) to concentrate same to about 500 µL. The obtained concentrate was transferred to an Amicon Ultra 4, diluted to 4 mL with a MES buffer (buffer component concentration: 20 mM, pH: 6.0), concentrated again to about 100 µL by ultrafiltration under centrifugation conditions (25°C, 1000 g, 3 min), and diluted with a MES buffer (buffer component concentration: 20 mM, pH: 6.0) to obtain a suspension containing nucleic acid-encapsulating lipid nanoparticles (100 µL, nucleic acid concentration: 200 µg/mL) (step b)).

To the obtained suspension was added a sucrose aqueous solution to achieve a final sucrose concentration of 160 mg/mL (step c)). The obtained suspension was placed in a screw tube, and frozen at -80°C to obtain a frozen composition (ionic lipid content: 1.0 wt%, nucleic acid content: 20 µg, N/P ratio: 62, cryoprotective agent (sucrose) content: 14 wt%, water (ice) content: 84 wt%) (step d)).

After freezing, the frozen composition was stored at - 20°C, and before use, thawed by standing same in an atmospheric pressure environment in a refrigerator at 4°C for 30 min to obtain a suspension.

### Comparative Example 2 (Preparation of frozen composition containing nucleic acid-encapsulating lipid nanoparticles)

MC3 was used as the ionic lipid, and DSPC, Chol, and DMG-PEG2000 were further used as different lipids. The molar ratio of MC3:DSPC:Chol:DMG-PEG2000 used was 50:10:38.5:1.5. The lipids were mixed such that the total lipids in a lipid ethanol solution were 4.0 mM.

2075 µL of a nucleic acid solution (nucleic acid concentration: 6.7 µg/mL) obtained by diluting FLuc mRNA with an acidic citrate buffer (buffer component concentration: 20 mM, pH: 5.0), and 725 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 14000 µL of a MES buffer (buffer component concentration: 20 mM, pH: 6.0), and the obtained suspension was transferred to an Amicon Ultra 15. Ultrafiltration was performed under centrifugation conditions (25°C, 1000 g, 3 min) to concentrate same to about 2000 µL. The obtained concentrate was diluted to 15 mL with a MES buffer (buffer component concentration: 20 mM, pH: 6.0), and ultrafiltration was performed again under centrifugation conditions (25°C, 1000 g, 3 min) to concentrate same to about 500 µL. The obtained concentrate was transferred to an Amicon Ultra 4, diluted to 4 mL with a MES buffer (buffer component concentration: 20 mM, pH: 6.0), concentrated again to about 100 µL by ultrafiltration under centrifugation conditions (25°C, 1000 g, 3 min), and diluted with a MES buffer (buffer component concentration: 20 mM, pH: 6.0) to obtain a suspension containing nucleic acid-encapsulating lipid nanoparticles (100 µL, nucleic acid concentration: 100 µg/mL) (step b)).

To the obtained suspension was added a sucrose aqueous solution to achieve a final sucrose concentration of 160 mg/mL (step c)). The obtained suspension was placed in a screw tube, and frozen at -80°C to obtain a frozen composition (ionic lipid content: 0.3 wt%, nucleic acid content: 10 µg, N/P ratio: 31, cryoprotective agent (sucrose) content: 14 wt%, water (ice) content: 85 wt%) (step d)).

After freezing, the frozen composition was stored at - 20°C, and before use, thawed by standing same in an atmospheric pressure environment in a refrigerator at 4°C for 30 min to obtain a suspension.

### Comparative Example 3 (Preparation of frozen composition containing nucleic acid-encapsulating lipid nanoparticles)

MC3 was used as the ionic lipid, and DSPC, Chol, and DMG-PEG2000 were further used as different lipids. The molar ratio of MC3:DSPC:Chol:DMG-PEG2000 used was 50:10:38.5:1.5. The lipids were mixed such that the total lipids in a lipid ethanol solution were 4.0 mM.

1775 µL of a nucleic acid solution (nucleic acid concentration: 33.3 µg/mL) obtained by diluting FLuc mRNA with an acidic citrate buffer (buffer component concentration: 20 mM, pH: 5.0), and 625 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 11200 µL of a MES buffer (buffer component concentration: 20 mM, pH: 6.0), and the obtained suspension was transferred to an Amicon Ultra 15. Ultrafiltration was performed under centrifugation conditions (25°C, 1000 g, 3 min) to concentrate same to about 2000 µL. The obtained concentrate was diluted to 15 mL with a MES buffer (buffer component concentration: 20 mM, pH: 6.0), and ultrafiltration was performed again under centrifugation conditions (25°C, 1000 g, 3 min) to concentrate same to about 500 µL. The obtained concentrate was transferred to an Amicon Ultra 4, diluted to 4 mL with a MES buffer (buffer component concentration: 20 mM, pH: 6.0), concentrated again to about 100 µL by ultrafiltration under centrifugation conditions (25°C, 1000 g, 3 min), and diluted with a MES buffer (buffer component concentration: 20 mM, pH: 6.0) to obtain a suspension containing nucleic acid-encapsulating lipid nanoparticles (100 µL, nucleic acid concentration: 400 µg/mL) (step b)).

To the obtained suspension was added a sucrose aqueous solution to achieve a final sucrose concentration of 160 mg/mL (step c)). The obtained suspension was placed in a screw tube, and frozen at -80°C to obtain a frozen composition (ionic lipid content: 0.2 wt%, nucleic acid content: 40 µg, N/P ratio: 6, cryoprotective agent (sucrose) content: 14 wt%, water (ice) content: 86 wt%) (step d)).

After freezing, the frozen composition was stored at - 20°C, and before use, thawed by standing same in an atmospheric pressure environment in a refrigerator at 4°C for 30 min to obtain a suspension.

The particle size, Polydispersity Index (PdI), and mRNA encapsulation rate of the nucleic acid-encapsulating lipid nanoparticles in the suspensions obtained by thawing the frozen compositions of Examples 1 to 5 and Comparative Examples 1 to 3 were analyzed. Also, the encapsulation rate of mRNA in nucleic acid-encapsulating lipid nanoparticles in the suspensions obtained in Examples 1 to 5 and Comparative Examples 1 to 3, step c) was analyzed. The particle size and PdI were measured by a dynamic light scattering method using ZetaSizer (registered trademark). The mRNA encapsulation rate (referred to as "encapsulation rate" in the table below) was measured by the Ribogreen (registered trademark) assay. The results are shown in Table 3. In the following Table, the encapsulation rate of mRNA in nucleic acid-encapsulating lipid nanoparticles in the suspension obtained by thawing the frozen composition is shown in the row of "after freezing and thawing" and the encapsulation rate of mRNA in nucleic acid-encapsulating lipid nanoparticles in the suspension obtained in step c) is shown in the row of "step c)" The particle size is expressed in Z-Average size (referred to as "Z-ave" in the Table below).

**[Table 3]**

| | encapsulation rate (%) | | Z-ave (nm) | PdI |
|---|---|---|---|---|
| | step c) | after freezing and thawing | | |
| Example 1 | 98 | 97 | 113.4 | 0.122 |
| Example 2 | 98 | 97 | 113.9 | 0.129 |
| Example 3 | 97 | 96 | 122.8 | 0.151 |
| Example 4 | 93 | 86 | 149.6 | 0.195 |
| Example 5 | 90 | 84 | 146.3 | 0.217 |
| Comparative Example 1 | 97 | 98 | 86.03 | 0.079 |
| Comparative Example 2 | 96 | 97 | 97.15 | 0.058 |
| Comparative Example 3 | 91 | 93 | 99.47 | 0.089 |

The frozen composition of Example 3, Example 5, Comparative Example 1, or Comparative Example 3 was thawed and further diluted with a Tris-buffered saline (pH: 7.6) to obtain a suspension containing nucleic acid-encapsulating lipid nanoparticles (nucleic acid concentration: 2 µg/mL). The nucleic acid-encapsulating lipid nanoparticles in the obtained suspension were applied to HeLa cells, and the cytotoxicity thereof was evaluated. Specifically, HeLa cells were seeded in D-MEM medium (high glucose) (containing L-glutamine, phenol red) (10% by volume FBS, 1% by volume penicillin-streptomycin solution added) at 5×10³ cells/100 µL into the wells of a 96-well flat-bottom clear black plate. A suspension containing nucleic acid-encapsulating lipid nanoparticles obtained by thawing the frozen composition was added to the aforementioned well such that the amount of nucleic acid in the medium was 7.8 to 125 ng. The 50% survival dose after 24 hours of incubation was evaluated using Cell Counting Kit-8 (DOJINDO LABORATORIES). The results are shown in Table 4-1 and Table 4-2.

**[Table 4-1]**

| high N/P ratio | | | |
|---|---|---|---|
| | N/P ratio | ionic lipid | 50% survival dose (ng) |
| Example 3 | 60 | SS-OP | 89.3 |
| Comparative Example 1 | 62 | MC3 | 49.0 |

**[Table 4-2]**

| low N/P ratio | | | |
|---|---|---|---|
| | N/P ratio | ionic lipid | 50% survival dose (ng) |
| Example 5 | 8 | SS-OP | ≥125 |
| Comparative Example 3 | 6 | MC3 | ≥125 |

As shown in Table 4-1, the N/P ratios of the nucleic acid-encapsulating lipid nanoparticles in Example 3 and Comparative Example 1, which have a high N/P ratio, were similar (around 60). However, the nucleic acid-encapsulating lipid nanoparticles in Example 1 showed a higher 50% survival dose compared to those in Comparative Example 1. In addition, as shown in Table 4-2, the N/P ratios of the nucleic acid-encapsulating lipid nanoparticles in Example 5 and Comparative Example 3, which have a low N/P ratio, were similar (6 to 8). Both the nucleic acid-encapsulating lipid nanoparticles in Example 5 and Comparative Example 3 showed a 50% survival dose of 125 ng or more.

The frozen compositions of Examples 3 to 5 or Comparative Examples 1 to 3 were thawed and further diluted with a Tris-buffered saline (pH: 7.6) to obtain suspensions containing nucleic acid-encapsulating lipid nanoparticles (nucleic acid concentration: 2 µg/mL). The nucleic acid-encapsulating lipid nanoparticles in the obtained suspension were applied to HeLa cells, and the gene expression activity thereof was evaluated. Specifically, HeLa cells were seeded in D-MEM medium (high glucose) (containing L-glutamine, phenol red) (10% by volume FBS, 1% by volume penicillin-streptomycin solution added) at 5×10³ cells/100 µL into the wells of a 96-well flat-bottom clear black plate. The obtained suspension containing nucleic acid-encapsulating lipid nanoparticles was added to the aforementioned well such that the nucleic acid concentration in the medium was 0.031 µg/10 µL. Luciferin was added to the aforementioned well such that the concentration in the medium was 0.1 mM, and the amount of luciferase protein introduced was evaluated 24 hr later using the amount of luminescence. HeLa cells cultured in the medium containing nucleic acid-encapsulating lipid nanoparticles and luciferin were measured for luminescence at 560 nm using a plate reader. The results are shown in Fig. 1 and Fig. 2.

As shown in Fig. 1, the gene expression activity of Example 3, with an N/P ratio of 60, was 1.9 times that of Example 5, with an N/P ratio of 8. On the other hand, as shown in Fig. 2, the gene expression activity of Comparative Example 1, with an N/P ratio of 62, was 0.2 times that of Comparative Example 3, with an N/P ratio of 6. In addition, the gene expression activity of the nucleic acid-encapsulating lipid nanoparticles of Example 3 was 38.8 times that of the nucleic acid-encapsulating lipid nanoparticles Comparative Example 1.

From Table 4, Fig. 1 and Fig. 2, it is shown that the nucleic acid-encapsulating lipid nanoparticles in the frozen composition of the present invention possess both low toxicity and high gene expression activity.

### Comparative Example 4 (Preparation of suspension containing nucleic acid-encapsulating lipid nanoparticles)

SS-OP was used as the ionic lipid (1), and DOPC, Chol, and DMG-PEG2000 were used as different lipids. The molar ratio of SS-OP:DOPC:Chol used was 52.5:7.5:40, and 1.5 mol% of DMG-PEG2000 was used with respect to the total of SS-OP, DOPC and Chol. The lipids were mixed such that the total lipids in a lipid ethanol solution were 3.0 mM.

975 µL of a nucleic acid solution (nucleic acid concentration: 5.0 µg/mL) obtained by diluting FLuc mRNA with an acidic citrate buffer (buffer component concentration: 20 mM, pH: 5.0), and 433 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 1590 µL of a Tris-buffered saline (buffer component concentration: 50 mM, pH: 7.6), and the obtained suspension was transferred to an Amicon Ultra 4, and ultrafiltrated under centrifugation conditions (25°C, 1000 g, 3 min), and concentrated to about 250 µL. The following operation was performed twice, and concentrated to 200 µL to obtain a suspension containing nucleic acid-encapsulating lipid nanoparticles (nucleic acid concentration: 10 µg/mL, N/P ratio: 60) (step b)).

### Example 6 (Preparation of frozen composition containing nucleic acid-encapsulating lipid nanoparticles)

SS-OP was used as the ionic lipid (1), and DOPC, Chol, and DMG-PEG2000 were used as different lipids. The molar ratio of SS-OP:DOPC:Chol used was 52.5:7.5:40, and 1.5 mol% of DMG-PEG2000 was used with respect to the total of SS-OP, DOPC and Chol. The lipids were mixed such that the total lipids in a lipid ethanol solution were 8.0 mM.

2225 µL of a nucleic acid solution (nucleic acid concentration: 13.3 µg/mL) obtained by diluting FLuc mRNA with an acidic malic acid buffer (buffer component concentration: 20 mM, pH: 3.0), and 775 µL of the lipid ethanol solution were each measured into syringes. Using NanoAssemblr (registered trademark) ultra-fast nano pharmaceutical production device, nucleic acid-encapsulating lipid nanoparticles were prepared under the conditions of a nucleic acid solution:lipid ethanol solution flow rate ratio of 3:1 and a total flow rate of 4 mL/min (step a)).

To the obtained suspension was added 12000 µL of a MES buffer (buffer component concentration: 20 mM, pH: 6.0), and the obtained suspension was transferred to an Amicon Ultra 15. Ultrafiltration was performed under centrifugation conditions (25°C, 1000 g, 3 min) to concentrate same to about 2000 µL. The obtained concentrate was diluted to 15 mL with a MES buffer (buffer component concentration: 20 mM, pH: 6.0), and ultrafiltration was performed again under centrifugation conditions (25°C, 1000 g, 3 min) to concentrate same to about 500 µL. The obtained concentrate was diluted to 15 mL with a MES buffer (buffer component concentration: 20 mM, pH: 6.0), ultrafiltrated again under centrifugation conditions (25°C, 1000 g, 3 min) to concentrate to about 100 µL, and diluted with a MES buffer (buffer component concentration: 20 mM, pH: 6.0) to obtain a suspension containing nucleic acid-encapsulating lipid nanoparticles (100 µL, nucleic acid concentration: 200 µg/mL) (step b)).

To the obtained suspension was added a sucrose aqueous solution to achieve a final sucrose concentration of 160 mg/mL (step c)). The obtained suspension was placed in an Eppendorf tube by 25 µL, and frozen at -80°C to obtain a frozen composition (ionic lipid (1) content: 0.9 wt%, nucleic acid content: 2.5 µg, N/P ratio: 60, cryoprotective agent (sucrose) content: 14 wt%, water (ice) content: 85 wt%) (step d)).

After freezing, the frozen composition was stored at - 20°C, and before use, thawed by standing same in an atmospheric pressure environment in a refrigerator at 4°C for 30 min to obtain a suspension.

### Example 7 (Preparation of frozen composition containing nucleic acid-encapsulating lipid nanoparticles)

SS-OP was used as the ionic lipid (1), and DOPC, Chol, and DMG-PEG2000 were used as different lipids. The molar ratio of SS-OP:DOPC:Chol used was 52.5:7.5:40, and 1.5 mol% of DMG-PEG2000 was used with respect to the total of SS-OP, DOPC and Chol. The lipids were mixed such that the total lipids in a lipid ethanol solution were 8.0 mM.

2225 µL of a nucleic acid solution (nucleic acid concentration: 13.3 µg/mL) obtained by diluting FLuc mRNA with an acidic citrate buffer (buffer component concentration: 20 mM, pH: 5.0), and 775 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 12000 µL of a MES buffer (buffer component concentration: 20 mM, pH: 6.0), and the obtained suspension was transferred to an Amicon Ultra 15. Ultrafiltration was performed under centrifugation conditions (25°C, 1000 g, 3 min) to concentrate same to about 2000 µL. The obtained concentrate was diluted to 15 mL with a MES buffer (buffer component concentration: 20 mM, pH: 6.0), and ultrafiltration was performed again under centrifugation conditions (25°C, 1000 g, 3 min) to concentrate same to about 500 µL. The obtained concentrate was transferred to an Amicon Ultra 4, diluted to 4 mL with a MES buffer (buffer component concentration: 20 mM, pH: 6.0), concentrated again to about 100 µL by ultrafiltration under centrifugation conditions (25°C, 1000 g, 3 min), and diluted with a MES buffer (buffer component concentration: 20 mM, pH: 6.0) to obtain a suspension containing nucleic acid-encapsulating lipid nanoparticles (100 µL, nucleic acid concentration: 200 µg/mL) (step b)).

To the obtained suspension was added a sucrose aqueous solution to achieve a final sucrose concentration of 160 mg/mL (step c)). The obtained suspension was placed in an Eppendorf tube by 25 µL, and frozen at -80°C to obtain a frozen composition (ionic lipid (1) content: 0.9 wt%, nucleic acid content: 2.5 µg, N/P ratio: 60, cryoprotective agent (sucrose) content: 14 wt%, water (ice) content: 85 wt%) (step d)).

After freezing, the frozen composition was stored at - 20°C, and before use, thawed by standing same in an atmospheric pressure environment in a refrigerator at 4°C for 30 min to obtain a suspension.

### Example 8 (Preparation method of frozen composition containing nucleic acid-encapsulating lipid nanoparticles)

SS-OP was used as the ionic lipid (1), and DOPC, Chol, and DMG-PEG2000 were used as different lipids. The molar ratio of SS-OP:DOPC:Chol used was 52.5:7.5:40, and 1.5 mol% of DMG-PEG2000 was used with respect to the total of SS-OP, DOPC and Chol. The lipids were mixed such that the total lipids in a lipid ethanol solution were 8.0 mM.

2225 µL of a nucleic acid solution (nucleic acid concentration: 13.3 µg/mL) obtained by diluting FLuc mRNA with an acidic citrate buffer (buffer component concentration: 20 mM, pH: 5.0), and 775 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 12000 µL of a Tris/maleate buffer (buffer component concentration: 25 mM, pH: 6.0), and the obtained suspension was transferred to an Amicon Ultra 15. Ultrafiltration was performed under centrifugation conditions (25°C, 1000 g, 3 min) to concentrate same to about 2000 µL. The obtained concentrate was diluted with a Tris/maleate buffer (buffer component concentration: 20 mM, pH: 6.0) to 15 mL, and ultrafiltration was performed again under centrifugation conditions (25°C, 1000 g, 3 min) to concentrate same to about 500 µL. The obtained concentrate was transferred to an Amicon Ultra 4, diluted with a Tris/maleate buffer (buffer component concentration: 25 mM, pH: 6.0) to 4 mL, ultrafiltrated again under centrifugation conditions (25°C, 1000 g, 3 min) to concentrate to about 100 µL, and diluted with a Tris/maleate buffer (buffer component concentration: 25 mM, pH: 6.0) to obtain a suspension containing nucleic acid-encapsulating lipid nanoparticles (100 µL, nucleic acid concentration: 200 µg/mL) (step b)).

To the obtained suspension was added a sucrose aqueous solution to achieve a final sucrose concentration of 160 mg/mL (step c)). The obtained suspension was placed in an Eppendorf tube by 25 µL, and frozen at -80°C to obtain a frozen composition (ionic lipid (1) content: 0.9 wt%, nucleic acid content: 2.5 µg, N/P ratio: 60, cryoprotective agent (sucrose) content: 14 wt%, water (ice) content: 85 wt%) (step d)).

After freezing, the frozen composition was stored at - 20°C, and before use, thawed by standing same in an atmospheric pressure environment in a refrigerator at 4°C for 30 min to obtain a suspension.

### Example 9 (Preparation of frozen composition containing nucleic acid-encapsulating lipid nanoparticles)

SS-OP was used as the ionic lipid (1), and DOPC, Chol, and DMG-PEG2000 were used as different lipids. The molar ratio of SS-OP:DOPC:Chol:DMG-PEG2000 used was 48.5:7.5:42.5:1.5. The lipids were mixed such that the total lipids in a lipid ethanol solution were 8.0 mM.

1613 µL of a nucleic acid solution (nucleic acid concentration: 13.3 µg/mL) obtained by diluting FLuc mRNA with an acidic citrate buffer (buffer component concentration: 20 mM, pH: 5.0), and 638 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 7000 µL of a Tris/NaCl/sucrose buffer (buffer component concentration: 25 mM, sucrose concentration: 400 mM, pH: 7.4), and the obtained suspension was transferred to an Amicon Ultra 15. Ultrafiltration was performed under centrifugation conditions (25°C, 1000 g, 3 min) to concentrate same to about 2000 µL. The obtained concentrate was diluted to 15 mL with a Tris/NaCl/sucrose buffer (buffer component concentration: 20 mM, pH: 7.4), and ultrafiltration was performed again under centrifugation conditions (25°C, 1000 g, 3 min) to concentrate same to about 500 µL. The obtained concentrate was transferred to an Amicon Ultra 4, diluted to 4 mL with a Tris/NaCl/sucrose buffer (buffer component concentration: 25 mM, sucrose concentration: 137 mg/mL, pH: 7.4), ultrafiltrated again under centrifugation conditions (25°C, 1000 g, 3 min) to concentrate same to about 240 µL, and diluted with a Tris/NaCl/sucrose buffer (buffer component concentration: 25 mM, sucrose concentration: 137 mg/mL, pH: 7.4) to obtain a suspension containing nucleic acid-encapsulating lipid nanoparticles (880 µL, nucleic acid concentration: 200 µg/mL) (step b')).

The obtained suspension was placed in an Eppendorf tube by 35 µL, and frozen at -80°C to obtain a frozen composition (ionic lipid (1) content: 1.9 wt%, nucleic acid content: 7 g, N/P ratio: 60, cryoprotective agent (sucrose) content: 12 wt%, water (ice) content: 84 wt%) (step d)).

After freezing, the frozen composition was stored at - 80°C, and before use, thawed by standing same in an atmospheric pressure environment in a refrigerator at 4°C for 30 min to obtain a suspension.

### Example 10 (Preparation of frozen composition containing nucleic acid-encapsulating lipid nanoparticles)

SS-OP was used as the ionic lipid (1), and DOPC, Chol, and DMG-PEG2000 were used as different lipids. The molar ratio of SS-OP:DOPC:Chol:DMG-PEG2000 used was 48.5:7.5:42.5:1.5. The lipids were mixed such that the total lipids in a lipid ethanol solution were 6.0 mM.

3148 µL of a nucleic acid solution (nucleic acid concentration: 56 µg/mL) obtained by diluting FLuc mRNA with an acidic citrate buffer (buffer component concentration: 20 mM, pH: 5.0), and 1270 µL of the lipid ethanol solution were each measured into syringes. Using NanoAssemblr (registered trademark) ultra-fast nano pharmaceutical production device, nucleic acid-encapsulating lipid nanoparticles were prepared under the conditions of a nucleic acid solution:lipid ethanol solution flow rate ratio of 3:1 and a total flow rate of 12 mL/min (step a)).

To the obtained suspension was added 4000 µL of a Tris/NaCl buffer (buffer component concentration: 50 mM, pH: 7.4), and the obtained suspension was transferred to an Amicon Ultra 15, and ultrafiltrated under centrifugation conditions (25°C, 1000 g, 3 min) to concentrate same to about 1500 µL. The obtained concentrate was diluted to 15 mL with a Tris/NaCl buffer (buffer component concentration: 50 mM, pH: 7.4), and ultrafiltration was performed again under centrifugation conditions (25°C, 1000 g, 3 min) to concentrate same to about 500 µL. The obtained concentrate was transferred to an Amicon Ultra 4, diluted to 4 mL with a Tris/NaCl buffer (buffer component concentration: 50 mM, pH: 7.4), ultrafiltrated again under centrifugation conditions (25°C, 1000 g, 3 min) to concentrate same to about 250 µL, and diluted with a Tris/NaCl buffer (buffer component concentration: 50 mM, pH: 7.4) to obtain a suspension containing nucleic acid-encapsulating lipid nanoparticles (nucleic acid concentration: 200 µg/mL) (step b)).

To the obtained suspension was added a sucrose aqueous solution to achieve a final sucrose concentration of 160 mg/mL (step c)). The obtained suspension was placed in an Eppendorf tube by 60 µL, and frozen at -20°C to obtain a frozen composition (ionic lipid (1) content: 0.5 wt%, nucleic acid content: 88 µg, N/P ratio: 33, cryoprotective agent (sucrose) content: 14 wt%, water (ice) content: 84 wt%) (step d)).

After freezing, the frozen composition was stored at - 20°C, and before use, thawed by standing same in an atmospheric pressure environment at 25°C for 30 min to obtain a suspension.

The particle size, Polydispersity Index (PdI), and mRNA encapsulation rate of the nucleic acid-encapsulating lipid nanoparticles in the suspensions obtained by thawing the frozen compositions of Examples 6 to 10 were analyzed. The results are shown in the line of "after freezing and thawing " in Table 5. Similarly, the particle size, PdI, and mRNA encapsulation rate were analyzed for the nucleic acid-encapsulating lipid nanoparticles in the suspension obtained in step b') or step c) of Examples 6-10 and the nucleic acid-encapsulating lipid nanoparticles in the suspension obtained in Comparative Example 4. The results are shown in the line of "step b')" or "step c)" in Table 5. The particle size and PdI were measured by a dynamic light scattering method using ZetaSizer (registered trademark). The mRNA encapsulation rate (referred to as "encapsulation rate" in the table below) was measured by the Ribogreen (registered trademark) assay. The particle size is expressed in Z-Average size (referred to as "Z-ave" in the Table below).

**[Table 5]**

| | | encapsulation rate (%) | Z-ave (nm) | PdI |
|---|---|---|---|---|
| Example 6 | step c) | 99 | 88 | 0.06 |
| | after freezing and thawing | 90 | 109 | 0.21 |
| Example 7 | step c) | 98 | 86 | 0.05 |
| | after freezing and thawing | 90 | 106 | 0.22 |
| Example 8 | step c) | 98 | 76 | 0.06 |
| | after freezing and thawing | 94 | 96 | 0.20 |
| Example 9 | step b') | 97 | 113 | 0.12 |
| | after freezing and thawing | 83 | 98 | 0.22 |
| Example 10 | step c) | 82 | 78 | 0.07 |
| | after freezing and thawing | 76 | 116 | 0.20 |
| Comparative Example 4 | - | 89 | 99 | 0.18 |

| | | | | |
|---|---|---|---|---|
| thawing temperature in Examples 6 to 9: 4°C thawing temperature in Example 10: 25°C | | | | |

Each of the frozen compositions of Examples 6 to 9 was thawed and further diluted with a Tris-buffered saline (pH: 7.6) to obtain a suspension containing nucleic acid-encapsulating lipid nanoparticles (nucleic acid concentration: 2 µg/mL). In addition, the suspension of Comparative Example 4 was diluted with a Tris-buffered saline (pH: 7.6) to obtain a suspension containing nucleic acid-encapsulating lipid nanoparticles (nucleic acid concentration: 2 µg/mL). The nucleic acid-encapsulating lipid nanoparticles in the obtained suspension were applied to HeLa cells, and the gene expression activity thereof was evaluated. Specifically, HeLa cells were seeded in D-MEM medium (high glucose) (containing L-glutamine, phenol red) (10% by volume FBS, 1% by volume penicillin-streptomycin solution added) at 3×10⁴ cells/600 µL into the wells of a 24-well flat-bottom clear black plate. The obtained suspension containing nucleic acid-encapsulating lipid nanoparticles was added to the aforementioned well such that the nucleic acid concentration in the medium was 0.12 µg/60 µL. Luciferin was added to the aforementioned well such that the concentration in the medium was 0.1 mM, and the amount of luciferase protein introduced was evaluated over time using the amount of luminescence. HeLa cells cultured in the medium containing nucleic acid-encapsulating lipid nanoparticles and luciferin were placed in an incubator-type luminometer ("WSL-1565 Kronos HT" manufactured by ATTO CORPORATION), and the cumulative amount of luminescence was measured every hour. The results are shown in Fig. 3. In Fig. 3, "1.2.E+08" and the like mean "1.2×10⁸" and the like.

As shown in Fig. 3, the nucleic acid-encapsulating lipid nanoparticles in the suspensions obtained by thawing the frozen compositions of Examples 6 to 9 showed gene expression activity equivalent to or greater than that of the nucleic acid-encapsulating lipid nanoparticles in the unfrozen suspension of Comparative Example 4.

### Example 11 (Preparation of frozen composition containing nucleic acid-encapsulating lipid nanoparticles)

SS-OP was used as the ionic lipid (1), and DOPC, Chol, and DMG-PEG2000 were used as different lipids. The molar ratio of SS-OP:DOPC:Chol:DMG-PEG2000 used was 48.5:7.5:42.5:1.5. The lipids were mixed such that the total lipids in a lipid ethanol solution were 8.0 mM.

2400 µL of a nucleic acid solution (nucleic acid concentration: 13.3 µg/mL) obtained by diluting FLuc mRNA with an acidic citrate buffer (buffer component concentration: 20 mM, pH: 5.0), and 900 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 8300 µL of a MES buffer (buffer component concentration: 20 mM, pH: 6.0), and the obtained suspension was transferred to an Amicon Ultra 15. Ultrafiltration was performed under centrifugation conditions (25°C, 1000 g, 3 min) to concentrate same to about 2000 µL. The obtained concentrate was diluted to 15 mL with a MES buffer (buffer component concentration: 20 mM, pH: 6.0), and ultrafiltration was performed again under centrifugation conditions (25°C, 1000 g, 3 min) to concentrate same to about 500 µL. The obtained concentrate was transferred to Amicon Ultra 15, diluted to 4 mL with a MES buffer (buffer component concentration: 20 mM, pH: 6.0), ultrafiltrated again under centrifugation conditions (25°C, 1000 g, 3 min) to concentrate same to about 230 µL, and diluted with a MES buffer (buffer component concentration: 20 mM, pH: 6.0) to obtain a suspension containing nucleic acid-encapsulating lipid nanoparticles (230 µL, nucleic acid concentration: 100 µg/mL) (step b)).

To the obtained suspension was added a sucrose aqueous solution to achieve a final sucrose concentration of 160 mg/mL (step c)). The obtained suspension was placed in an Eppendorf tube by 35 µL, and frozen by cooling at -80°C for 24 hr to obtain a frozen composition (ionic lipid (1) content: 0.5 wt%, nucleic acid content: 1.75 µg, N/P ratio: 60, cryoprotective agent (sucrose) content: 14 wt%, water (ice) content: 85 wt%) (step d)).

After freezing, the frozen composition was stored at - 20°C, and before use, thawed by standing same in an atmospheric pressure environment in a refrigerator at 4°C for 30 min to obtain a suspension.

### Example 12 (Preparation of frozen composition containing nucleic acid-encapsulating lipid nanoparticles)

SS-EC was used as the ionic lipid (1), and DOPE, Chol, and DMG-PEG2000 were used as different lipids. The molar ratio of SS-EC:DOPE:Chol:DMG-PEG2000 used was 59:29.6:9.9:1.5. The lipids were mixed such that the total lipids in a lipid ethanol solution were 8.0 mM.

2400 µL of a nucleic acid solution (nucleic acid concentration: 13.3 µg/mL) obtained by diluting FLuc mRNA with an acidic citrate buffer (buffer component concentration: 20 mM, pH: 5.0), and 900 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 8300 µL of a MES buffer (buffer component concentration: 20 mM, pH: 6.0), and the obtained suspension was transferred to an Amicon Ultra 15. Ultrafiltration was performed under centrifugation conditions (25°C, 1000 g, 3 min) to concentrate same to about 2000 µL.

The obtained concentrate was diluted to 15 mL with a MES buffer (buffer component concentration: 20 mM, pH: 6.0), and ultrafiltration was performed again under centrifugation conditions (25°C, 1000 g, 3 min) to concentrate same to about 500 µL. The obtained concentrate was transferred to Amicon Ultra 15, diluted to 4 mL with a MES buffer (buffer component concentration: 20 mM, pH: 6.0), ultrafiltrated again under centrifugation conditions (25°C, 1000 g, 3 min) to concentrate same to about 230 µL, and diluted with a MES buffer (buffer component concentration: 20 mM, pH: 6.0) to obtain a suspension containing nucleic acid-encapsulating lipid nanoparticles (230 µL, nucleic acid concentration: 100 µg/mL) (step b)).

To the obtained suspension was added a sucrose aqueous solution to achieve a final sucrose concentration of 160 mg/mL (step c)). The obtained suspension was placed in an Eppendorf tube by 35 µL, and frozen by cooling at -80°C for 24 hr to obtain a frozen composition (ionic lipid (1) content: 0.7 wt%, nucleic acid content: 1.75 µg, N/P ratio: 73, cryoprotective agent (sucrose) content: 14 wt%, water (ice) content: 85 wt%) (step d)).

After freezing, the frozen composition was stored at - 20°C, and before use, thawed by standing same in an atmospheric pressure environment in a refrigerator at 4°C for 30 min to obtain a suspension.

### Example 13 (Preparation of frozen composition containing nucleic acid-encapsulating lipid nanoparticles)

SS-OP and SS-EC was used as the ionic lipid (1), and DOPC, Chol, and DMG-PEG2000 were used as different lipids. The molar ratio of SS-OP:SS-EC:DOPC:Chol:DMG-PEG2000 used was 30:18.5:7.5:42.5:1.5. The lipids were mixed such that the total lipids in a lipid ethanol solution were 8.0 mM.

2400 µL of a nucleic acid solution (nucleic acid concentration: 13.3 µg/mL) obtained by diluting FLuc mRNA with an acidic citrate buffer (buffer component concentration: 20 mM, pH: 5.0), and 900 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 8300 µL of a MES buffer (buffer component concentration: 20 mM, pH: 6.0), and the obtained suspension was transferred to an Amicon Ultra 15. Ultrafiltration was performed under centrifugation conditions (25°C, 1000 g, 3 min) to concentrate same to about 2000 µL. The obtained concentrate was diluted to 15 mL with a MES buffer (buffer component concentration: 20 mM, pH: 6.0), and ultrafiltration was performed again under centrifugation conditions (25°C, 1000 g, 3 min) to concentrate same to about 500 µL. The obtained concentrate was transferred to Amicon Ultra 15, diluted to 4 mL with a MES buffer (buffer component concentration: 20 mM, pH: 6.0), ultrafiltrated again under centrifugation conditions (25°C, 1000 g, 3 min) to concentrate same to about 230 µL, and diluted with a MES buffer (buffer component concentration: 20 mM, pH: 6.0) to obtain a suspension containing nucleic acid-encapsulating lipid nanoparticles (230 µL, nucleic acid concentration: 100 µg/mL) (step b)).

To the obtained suspension was added a sucrose aqueous solution to achieve a final sucrose concentration of 160 mg/mL (step c)). The obtained suspension was placed in an Eppendorf tube by 35 µL, and frozen by cooling at -80°C for 24 hr to obtain a frozen composition (ionic lipid (1) content: 0.5 wt%, nucleic acid content: 1.75 µg, N/P ratio: 60, cryoprotective agent (sucrose) content: 14 wt%, water (ice) content: 85 wt%) (step d)).

After freezing, the frozen composition was stored at - 20°C, and before use, thawed by standing same in an atmospheric pressure environment in a refrigerator at 4°C for 30 min to obtain a suspension.

### Example 14 (Preparation of frozen composition containing nucleic acid-encapsulating lipid nanoparticles)

Compound 13 was used as the ionic lipid (1), and DOPC, Chol, and DMG-PEG2000 were used as different lipids. The molar ratio of compound 13:DOPC:Chol:DMG-PEG2000 used was 48.5:7.5:42.5:1.5. The lipids were mixed such that the total lipids in a lipid ethanol solution were 8.0 mM.

2400 µL of a nucleic acid solution (nucleic acid concentration: 13.3 µg/mL) obtained by diluting FLuc mRNA with an acidic citrate buffer (buffer component concentration: 20 mM, pH: 5.0), and 900 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 8300 µL of a MES buffer (buffer component concentration: 20 mM, pH: 6.0), and the obtained suspension was transferred to an Amicon Ultra 15. Ultrafiltration was performed under centrifugation conditions (25°C, 1000 g, 3 min) to concentrate same to about 2000 µL. The obtained concentrate was diluted to 15 mL with a MES buffer (buffer component concentration: 20 mM, pH: 6.0), and ultrafiltration was performed again under centrifugation conditions (25°C, 1000 g, 3 min) to concentrate same to about 500 µL. The obtained concentrate was transferred to Amicon Ultra 15, diluted to 4 mL with a MES buffer (buffer component concentration: 20 mM, pH: 6.0), ultrafiltrated again under centrifugation conditions (25°C, 1000 g, 3 min) to concentrate same to about 230 µL, and diluted with a MES buffer (buffer component concentration: 20 mM, pH: 6.0) to obtain a suspension containing nucleic acid-encapsulating lipid nanoparticles (230 µL, nucleic acid concentration: 100 µg/mL) (step b)).

To the obtained suspension was added a sucrose aqueous solution to achieve a final sucrose concentration of 160 mg/mL (step c)). The obtained suspension was placed in an Eppendorf tube by 35 µL, and frozen by cooling at -80°C for 24 hr to obtain a frozen composition (ionic lipid (1) content: 0.5 wt%, nucleic acid content: 1.75 µg, N/P ratio: 60, cryoprotective agent (sucrose) content: 14 wt%, water (ice) content: 85 wt%) (step d)).

After freezing, the frozen composition was stored at - 20°C, and before use, thawed by standing same in an atmospheric pressure environment in a refrigerator at 4°C for 30 min to obtain a suspension.

The particle size, Polydispersity Index(PdI), and mRNA encapsulation rate were analyzed for the nucleic acid-encapsulating lipid nanoparticles in the suspensions obtained by thawing the frozen compositions of Examples 11 to 14. The particle size and PdI were measured by a dynamic light scattering method using ZetaSizer (registered trademark). The mRNA encapsulation rate (referred to as "encapsulation rate" in the table below) was measured by the Ribogreen (registered trademark) assay. The results are shown in Table 6. The particle size is expressed in Z-Average size (referred to as "Z-ave" in the Table below).

**[Table 6]**

| | encapsulation rate (%) | Z-ave (nm) | PdI |
|---|---|---|---|
| Example 11 | 92 | 111 | 0.16 |
| Example 12 | 99 | 122 | 0.08 |
| Example 13 | 100 | 97 | 0.08 |
| Example 14 | 94 | 108 | 0.12 |

Each of the frozen compositions of Examples 11 to 14 was thawed and further diluted with a Tris-buffered saline (pH: 7.6) to obtain a suspension containing nucleic acid-encapsulating lipid nanoparticles (nucleic acid concentration: 2 µg/mL). The obtained nucleic acid-encapsulating lipid nanoparticles were applied to HeLa cells, and the gene expression activity thereof was evaluated. Specifically, HeLa cells were seeded in D-MEM medium (high glucose) (containing L-glutamine, phenol red) (10% by volume FBS, 1% by volume penicillin-streptomycin solution added) at 3×10⁴ cells/600 µL into the wells of a 24-well flat-bottom clear black plate. The obtained suspension containing nucleic acid-encapsulating lipid nanoparticles was added to the aforementioned well such that the nucleic acid concentration in the medium was 0.12 µg/60 µL. Luciferin was added to the aforementioned well such that the concentration in the medium was 0.1 mM, and the amount of luciferase protein introduced was evaluated over time using the amount of luminescence. HeLa cells cultured in the medium containing nucleic acid-encapsulating lipid nanoparticles and luciferin were placed in an incubator-type luminometer (Kronos), and the cumulative amount of luminescence was measured every hour. The results are shown in Fig. 4. In Fig. 4, "6.00.E+07" and the like mean "6.00×10⁷" and the like.

As shown in Fig. 4, the nucleic acid-encapsulating lipid nanoparticles in the suspensions obtained by thawing the frozen compositions of Examples 11 to 14 all showed gene expression activity. In particular, the nucleic acid-encapsulating lipid nanoparticles of Examples 11 and 14 showed high gene expression activity.

### Example 15 (Preparation of frozen composition containing nucleic acid-encapsulating lipid nanoparticles)

SS-OP was used as the ionic lipid (1), and DOPC, Chol, and DMG-PEG2000 were used as different lipids. The molar ratio of SS-OP:DOPC:Chol:DMG-PEG2000 used was 48.5:7.5:42.5:1.5. The lipids were mixed such that the total lipids in a lipid ethanol solution were 8.0 mM.

11700 µL of a nucleic acid solution (nucleic acid concentration: 13.3 µg/mL) obtained by diluting FLuc mRNA with an acidic citrate buffer (buffer component concentration: 20 mM, pH: 5.0), and 4100 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 11400 µL of a Tris/maleate buffer (buffer component concentration: 25 mM, pH: 6.0), and the obtained suspension was transferred to an Amicon Ultra 15. Ultrafiltration was performed under centrifugation conditions (25°C, 1000 g, 3 min) to concentrate same to about 2000 µL. The obtained concentrate was diluted to 15 mL with a Tris/maleate buffer (buffer component concentration: 25 mM, pH: 6.0), ultrafiltrated again under centrifugation conditions (25°C, 1000 g, 3 min) to concentrate same to about 2000 µL. The obtained concentrate was diluted to 15 mL with a Tris/maleate buffer (buffer component concentration: 25 mM, pH: 6.0), ultrafiltrated again under centrifugation conditions (25°C, 1000 g, 3 min) to concentrate same to about 1000 µL, and diluted with a Tris/maleate buffer (buffer component concentration: 25 mM, pH: 6.0) to obtain a suspension containing nucleic acid-encapsulating lipid nanoparticles (1315 µL, nucleic acid concentration: 8.8 µg/mL) (step b)).

To the obtained suspension was added a sucrose aqueous solution to achieve a final sucrose concentration of 160 mg/mL (step c)). The obtained suspension was placed in a screw tube by 200 µL, and frozen by cooling at -80°C or -20°C for 24 hr to obtain a frozen composition (ionic lipid (1) content: 0.04 wt%, nucleic acid content: 0.88 µg, N/P ratio: 60, cryoprotective agent (sucrose) content: 14 wt%, water (ice) content: 86 wt%) (step d)).

After freezing, the frozen composition was stored at - 20°C, and before use, thawed by standing same in an atmospheric pressure environment in a refrigerator at 4°C for 30 min to obtain a suspension.

### Example 16 (Preparation of frozen composition containing nucleic acid-encapsulating lipid nanoparticles)

SS-OP was used as the ionic lipid (1), and DOPC, Chol, and DMG-PEG2000 were used as different lipids. The molar ratio of SS-OP:DOPC:Chol:DMG-PEG2000 used was 48.5:7.5:42.5:1.5. The lipids were mixed such that the total lipids in a lipid ethanol solution were 8.0 mM.

11700 µL of a nucleic acid solution (nucleic acid concentration: 13.3 µg/mL) obtained by diluting FLuc mRNA with an acidic citrate buffer (buffer component concentration: 20 mM, pH: 5.0), and 4100 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 11400 µL of a Tris/maleate/NaCl buffer (buffer component concentration: 25 mM, pH: 6.0), and the obtained suspension was transferred to an Amicon Ultra 15. Ultrafiltration was performed under centrifugation conditions (25°C, 1000 g, 3 min) to concentrate same to about 2000 µL. The obtained concentrate was diluted to 15 mL with a Tris/maleate/NaCl buffer (buffer component concentration: 50 mM, pH: 6.0), ultrafiltrated again under centrifugation conditions (25°C, 1000 g, 3 min) to concentrate same to about 2000 µL. The obtained concentrate was diluted to 15 mL with a Tris/maleate/NaCl buffer (buffer component concentration: 50 mM, pH: 6.0), ultrafiltrated again under centrifugation conditions (25°C, 1000 g, 3 min) to concentrate same to about 1000 µL, and diluted with a Tris/maleate/NaCl buffer (buffer component concentration: 50 mM, pH: 6.0) to obtain a suspension containing nucleic acid-encapsulating lipid nanoparticles (1315 µL, nucleic acid concentration: 8.8 µg/mL) (step b)).

To the obtained suspension was added a sucrose aqueous solution to achieve a final sucrose concentration of 160 mg/mL (step c)). The obtained suspension was placed in a screw tube by 200 µL, and frozen by cooling at -80°C or -20°C for 24 hr to obtain a frozen composition (ionic lipid (1) content: 0.04 wt%, nucleic acid content: 0.88 µg, N/P ratio: 60, cryoprotective agent (sucrose) content: 14 wt%, water (ice) content: 85 wt%) (step d)).

After freezing, the frozen composition was stored at - 20°C, and before use, thawed by standing same in an atmospheric pressure environment in a refrigerator at 4°C for 30 min to obtain a suspension.

### Comparative Example 5 (Preparation of suspension containing nucleic acid-encapsulating lipid nanoparticles)

SS-OP was used as the ionic lipid (1), and DOPC, Chol, and DMG-PEG2000 were used as different lipids. The molar ratio of SS-OP:DOPC:Chol used was 52.5:7.5:40, and 1.5 mol% of DMG-PEG2000 was used with respect to the total of SS-OP, DOPC and Chol. The lipids were mixed such that the total lipids in a lipid ethanol solution were 4.0 mM.

1986 µL of a nucleic acid solution (nucleic acid concentration: 6.7 µg/mL) obtained by diluting FLuc mRNA with an acidic malic acid buffer (buffer component concentration: 20 mM, pH: 3.0), and 663 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1, the total flow rate of 4 mL/min, and syringe holder temperature: 25°C (step a)).

To the obtained suspension was added 10600 µL of a MES buffer (buffer component concentration: 20 mM, pH: 6.5), and the obtained suspension was transferred to an Amicon Ultra 15, and ultrafiltrated under centrifugation conditions (25°C, 1000 g, 5 min) to concentrate same to about 2000 µL. The obtained concentrate was diluted to 15 mL with PBS (pH: 7.4), and ultrafiltrated again under centrifugation conditions (25°C, 1000 g, 5 min) to concentrate same to about 2000 µL. The following operation was performed twice to concentrate same to 600 µL concentrated to obtain a suspension containing nucleic acid-encapsulating lipid nanoparticles (nucleic acid concentration: 10 µg/mL, N/P ratio: 60) (step b)). After preparation, the suspension was stored at 4°C.

The particle size, Polydispersity Index(PdI), and mRNA encapsulation rate were analyzed for the nucleic acid-encapsulating lipid nanoparticles in the suspensions obtained in Examples 15 and 16, step c). Similarly, one day after the freezing date (hereinafter "Day 1"), 7 days after the freezing date (hereinafter "Day 7"), and 30 days after the freezing date (hereinafter "Day 30"), the particle size, PdI, and mRNA encapsulation rate were analyzed for the nucleic acid-encapsulating lipid nanoparticles in the suspension obtained by thawing the frozen composition obtained in Examples 15 and 16, step d). Similarly, one day after the suspension preparation date (hereinafter "Day 1"), 7 days after the suspension preparation date (hereinafter "Day 7"), and 14 days after the suspension preparation date (hereinafter "Day 14"), the particle size, PdI, and mRNA encapsulation rate were analyzed for the nucleic acid-encapsulating lipid nanoparticles in the suspension of Comparative Example 4. The particle size and PdI were measured by a dynamic light scattering method using ZetaSizer (registered trademark). The mRNA encapsulation rate (referred to as "encapsulation rate" in the table below) was measured by the Ribogreen (registered trademark) assay. The results are shown in Table 7. The particle size is expressed in Z-Average size (referred to as "Z-ave" in the Table below).

**[Table 7]**

| | freezing temperature (°C) | storage temperature (°C) | | encapsulation rate (%) | Z-ave (nm) | PdI |
|---|---|---|---|---|---|---|
| Example 15 | -80 | -20 | step c) | 94 | 117 | 0.15 |
| | | | Day 1 | 94 | 108 | 0.12 |
| | | | Day 7 | 94 | 123 | 0.12 |
| | | | Day 30 | 99 | 122 | 0.12 |
| Example 15 | -20 | -20 | step c) | 94 | 117 | 0.15 |
| | | | Day 1 | 94 | 107 | 0.10 |
| | | | Day 7 | 94 | 122 | 0.13 |
| | | | Day 30 | 98 | 122 | 0.13 |
| Example 16 | -80 | -20 | step c) | 94 | 113 | 0.12 |
| | | | Day 1 | 94 | 109 | 0.11 |
| | | | Day 7 | 94 | 128 | 0.09 |
| | | | Day 30 | 98 | 127 | 0.11 |
| Example 16 | -20 | -20 | step c) | 94 | 113 | 0.12 |
| | | | Day 1 | 95 | 110 | 0.12 |
| | | | Day 7 | 98 | 126 | 0.08 |
| | | | Day 30 | 97 | 125 | 0.11 |
| Comparative Example 5 | - | 4 | Day 1 | 81 | 94 | 0.10 |
| | | | Day 7 | 88 | 95 | 0.08 |
| | | | Day 14 | 85 | 94 | 0.09 |

As shown in Table 7, the frozen compositions of Examples 15 and 16 showed no marked changes in the physical properties of the nucleic acid-encapsulating lipid nanoparticles even after being stored for 30 days and then thawed, thus showing high storage stability.

The frozen compositions of Example 9 (freezing temperature: -80°C), Example 15 (freezing temperature: -20°C or -80°C), and Example 16 (freezing temperature: -20°C or -80°C) were stored at -20°C after freezing as mentioned above. One day after the freezing date (hereinafter "Day 1"), 1 week after the freezing date (hereinafter "1 week"), 2 weeks after the freezing date (hereinafter "2 weeks"), and 1 month after the freezing date (hereinafter "1 month "), the aforementioned frozen composition was thawed and further diluted with a Tris-buffered saline (pH: 7.6) to obtain a suspension containing nucleic acid-encapsulating lipid nanoparticles (nucleic acid concentration: 0.12 µg/60 µL). The obtained nucleic acid-encapsulating nanoparticles were applied to HeLa cell, and the gene expression activity thereof was evaluated.

Similarly, the suspension containing the nucleic acid-encapsulating lipid nanoparticles obtained in Comparative Example 5 was stored at 4°C after preparation as mentioned above. One day after the preparation date (hereinafter "Day 1"), 1 week after the preparation date (hereinafter "1 week"), and 2 weeks after the preparation date (hereinafter "2 weeks"), the aforementioned frozen composition was diluted with PBS (pH: 7.4) to obtain a suspension containing nucleic acid-encapsulating lipid nanoparticles (nucleic acid concentration: 0.12 µg/60 µL). The obtained nucleic acid-encapsulating nanoparticles were applied to HeLa cell, and the gene expression activity thereof was evaluated.

Specifically, HeLa cells were seeded in D-MEM medium (high glucose) (containing L-glutamine, phenol red) (10% by volume FBS, 1% by volume penicillin-streptomycin solution added) at 3×10⁴ cells/600 µL into a 24-well flat-bottom clear black plate. The suspension of the nucleic acid-encapsulating nanoparticles obtained as mentioned above was added to the medium. Luciferin was added to the medium such that the concentration was 0.1 mM, and the amount of luciferase protein introduced was evaluated over time using the amount of luminescence. HeLa cells cultured in the medium containing nucleic acid-encapsulating nanoparticles and luciferin were placed in an incubator-type luminometer (Kronos), and the cumulative amount of luminescence was measured every hour. The results are shown in Fig. 5. The bar graphs of Examples 9, 15 and 16 and Comparative Example 5 in Fig. 5 show the results of 1 day, 1 week, 2 week, and 1 month in this order from the left. In Fig. 5, "1.6.E+08" and the like mean "1.6×10⁸" and the like. In addition, Fig. 5 also shows the freezing temperatures of Examples 9, 15 and 16.

As shown in Fig. 5, the nucleic acid-encapsulating lipid nanoparticles in the frozen compositions of Examples 9, 15, and 16 maintained high gene expression activity even after 1 month of storage, thus showing high storage stability thereof. On the other hand, in Comparative Example where cryopreservation was not performed, a decrease in the gene expression activity of nucleic acid-encapsulating lipid nanoparticles was observed one week after the preparation day, and the activity decreased further after two weeks after the preparation day, thus indicating poor storage stability.

### Example 17 (Preparation of frozen composition containing nucleic acid-encapsulating lipid nanoparticles)

Compound 7 was used as the ionic lipid (1), and POPE, Chol, and DMG-PEG2000 were used as different lipids. The molar ratio of compound 7:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 13.47 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2 (REPLIGEN, MWCO: 8 to 10kD), and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 100 µL to obtain a suspension containing nucleic acid-encapsulating lipid nanoparticles (100 µL, nucleic acid concentration: 101 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.7 wt%, nucleic acid content: 10 µg, N/P ratio: 64, cryoprotective agent (sucrose) content: 7 wt%, water (ice) content: 91 wt%) (step d)).

After freezing, the frozen composition was stored at - 80°C, and before use, thawed by standing same in an atmospheric pressure environment in a refrigerator at 4°C for 30 min to obtain a suspension.

### Example 18 (Preparation of frozen composition containing nucleic acid-encapsulating lipid nanoparticles)

Compound 9 was used as the ionic lipid (1), and POPE, Chol, and DMG-PEG2000 were used as different lipids. The molar ratio of compound 9:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.33 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (200 µL, nucleic acid concentration: 200 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.3 wt%, nucleic acid content: 20 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 19 (Preparation of frozen composition containing nucleic acid-encapsulating lipid nanoparticles)

Compound 10 was used as the ionic lipid (1), and POPE, Chol, and DMG-PEG2000 were used as different lipids. The molar ratio of compound 10:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.33 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (200 µL, nucleic acid concentration: 200 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.3 wt%, nucleic acid content: 20 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 20 (Preparation of frozen composition containing nucleic acid-encapsulating lipid nanoparticles)

compound 11 was used as the ionic lipid (1), and POPE, Chol, and DMG-PEG2000 were used as different lipids. The molar ratio of compound 11:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 13.47 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 500 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 100 µL to obtain a suspension containing nucleic acid-encapsulating lipid nanoparticles (100 µL, nucleic acid concentration: 101 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.6 wt%, nucleic acid content: 10 µg, N/P ratio: 64, cryoprotective agent (sucrose) content: 7 wt%, water (ice) content: 91 wt%) (step d)).

After freezing, the frozen composition was stored at - 80°C, and before use, thawed by standing same in an atmospheric pressure environment in a refrigerator at 4°C for 30 min to obtain a suspension.

### Example 21 (Preparation of frozen composition containing nucleic acid-encapsulating lipid nanoparticles)

Compound 12 was used as the ionic lipid (1), and POPE, Chol, and DMG-PEG2000 were used as different lipids. The molar ratio of compound 12:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.33 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (200 µL, nucleic acid concentration: 200 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.3 wt%, nucleic acid content: 20 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 22 (Preparation of frozen composition containing nucleic acid-encapsulating lipid nanoparticles)

Compound 23 was used as the ionic lipid (1), and POPE, Chol, and DMG-PEG2000 were used as different lipids. The molar ratio of compound 23:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 13.47 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 100 µL to obtain a suspension containing nucleic acid-encapsulating lipid nanoparticles (100 µL, nucleic acid concentration: 101 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.6 wt%, nucleic acid content: 10 µg, N/P ratio: 64, cryoprotective agent (sucrose) content: 7 wt%, water (ice) content: 91 wt%) (step d)).

After freezing, the frozen composition was stored at - 80°C, and before use, thawed by standing same in an atmospheric pressure environment in a refrigerator at 4°C for 30 min to obtain a suspension.

### Example 23 (Preparation of frozen composition containing nucleic acid-encapsulating lipid nanoparticles)

Compound 33 was used as the ionic lipid (1), and POPE, Chol, and DMG-PEG2000 were used as different lipids. The molar ratio of compound 33:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.33 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (200 µL, nucleic acid concentration: 200 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.3 wt%, nucleic acid content: 20 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 24 (Preparation of frozen composition containing nucleic acid-encapsulating lipid nanoparticles)

Compound 34 was used as the ionic lipid (1), and POPE, Chol, and DMG-PEG2000 were used as different lipids. The molar ratio of compound 34:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 13.47 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 100 µL to obtain a suspension containing nucleic acid-encapsulating lipid nanoparticles (100 µL, nucleic acid concentration: 101 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.6 wt%, nucleic acid content: 10 µg, N/P ratio: 64, cryoprotective agent (sucrose) content: 7 wt%, water (ice) content: 91 wt%) (step d)).

After freezing, the frozen composition was stored at - 80°C, and before use, thawed by standing same in an atmospheric pressure environment in a refrigerator at 4°C for 30 min to obtain a suspension.

### Example 25 (Preparation of frozen composition containing nucleic acid-encapsulating lipid nanoparticles)

Compound 37 was used as the ionic lipid (1), and POPE, Chol, and DMG-PEG2000 were used as different lipids. The molar ratio of compound 37:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 13.47 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 100 µL to obtain a suspension containing nucleic acid-encapsulating lipid nanoparticles (100 µL, nucleic acid concentration: 101 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.6 wt%, nucleic acid content: 10 µg, N/P ratio: 64, cryoprotective agent (sucrose) content: 7 wt%, water (ice) content: 91 wt%) (step d)).

After freezing, the frozen composition was stored at - 80°C, and before use, thawed by standing same in an atmospheric pressure environment in a refrigerator at 4°C for 30 min to obtain a suspension.

### Example 26 (Preparation of frozen composition containing nucleic acid-encapsulating lipid nanoparticles)

Compound 58 was used as the ionic lipid (1), and POPE, Chol, and DMG-PEG2000 were used as different lipids. The molar ratio of compound 58:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 13.47 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 100 µL to obtain a suspension containing nucleic acid-encapsulating lipid nanoparticles (100 µL, nucleic acid concentration: 101 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.6 wt%, nucleic acid content: 10 µg, N/P ratio: 64, cryoprotective agent (sucrose) content: 7 wt%, water (ice) content: 91 wt%) (step d)).

After freezing, the frozen composition was stored at - 80°C, and before use, thawed by standing same in an atmospheric pressure environment in a refrigerator at 4°C for 30 min to obtain a suspension.

### Example 27 (Preparation of frozen composition containing nucleic acid-encapsulating lipid nanoparticles)

Compound 155 was used as the ionic lipid (1), and POPE, Chol, and DMG-PEG2000 were used as different lipids. The molar ratio of compound 155:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 13.47 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 100 µL to obtain a suspension containing nucleic acid-encapsulating lipid nanoparticles (100 µL, nucleic acid concentration: 101 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.7 wt%, nucleic acid content: 10 µg, N/P ratio: 64, cryoprotective agent (sucrose) content: 7 wt%, water (ice) content: 91 wt%) (step d)).

After freezing, the frozen composition was stored at - 80°C, and before use, thawed by standing same in an atmospheric pressure environment in a refrigerator at 4°C for 30 min to obtain a suspension.

### Example 28 (Preparation of frozen composition containing nucleic acid-encapsulating lipid nanoparticles)

Compound 159 was used as the ionic lipid (1), and POPE, Chol, and DMG-PEG2000 were used as different lipids. The molar ratio of compound 159:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.33 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (200 µL, nucleic acid concentration: 200 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.3 wt%, nucleic acid content: 20 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 29 (Preparation of frozen composition containing nucleic acid-encapsulating lipid nanoparticles)

Compound 161 was used as the ionic lipid (1), and POPE, Chol, and DMG-PEG2000 were used as different lipids. The molar ratio of compound 161:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.33 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (200 µL, nucleic acid concentration: 200 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.3 wt%, nucleic acid content: 20 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 30 (Preparation of frozen composition containing nucleic acid-encapsulating lipid nanoparticles)

Compound 162 was used as the ionic lipid (1), and POPE, Chol, and DMG-PEG2000 were used as different lipids. The molar ratio of compound 162:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 13.47 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 100 µL to obtain a suspension containing nucleic acid-encapsulating lipid nanoparticles (100 µL, nucleic acid concentration: 101 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.6 wt%, nucleic acid content: 10 µg, N/P ratio: 64, cryoprotective agent (sucrose) content: 7 wt%, water (ice) content: 91 wt%) (step d)).

After freezing, the frozen composition was stored at - 80°C, and before use, thawed by standing same in an atmospheric pressure environment in a refrigerator at 4°C for 30 min to obtain a suspension.

### Example 31 (Preparation of frozen composition containing nucleic acid-encapsulating lipid nanoparticles)

Compound 163 was used as the ionic lipid (1), and POPE, Chol, and DMG-PEG2000 were used as different lipids. The molar ratio of compound 163:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.33 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (200 µL, nucleic acid concentration: 200 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.3 wt%, nucleic acid content: 20 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 32 (Preparation of frozen composition containing nucleic acid-encapsulating lipid nanoparticles)

Compound 164 was used as the ionic lipid (1), and POPE, Chol, and DMG-PEG2000 were used as different lipids. The molar ratio of compound 164:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.33 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (200 µL, nucleic acid concentration: 200 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.3 wt%, nucleic acid content: 20 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 33 (Preparation of frozen composition containing nucleic acid-encapsulating lipid nanoparticles)

Compound 165 was used as the ionic lipid (1), and POPE, Chol, and DMG-PEG2000 were used as different lipids. The molar ratio of compound 165:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.33 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (200 µL, nucleic acid concentration: 200 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.3 wt%, nucleic acid content: 20 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 34 (Preparation of frozen composition containing nucleic acid-encapsulating lipid nanoparticles)

Compound 166 was used as the ionic lipid (1), and POPE, Chol, and DMG-PEG2000 were used as different lipids. The molar ratio of compound 166:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.33 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (200 µL, nucleic acid concentration: 200 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.3 wt%, nucleic acid content: 20 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 35 (Preparation of frozen composition containing nucleic acid-encapsulating lipid nanoparticles)

Compound 167 was used as the ionic lipid (1), and POPE, Chol, and DMG-PEG2000 were used as different lipids. The molar ratio of compound 167:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.33 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (200 µL, nucleic acid concentration: 200 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.3 wt%, nucleic acid content: 20 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 36 (Preparation of frozen composition containing nucleic acid-encapsulating lipid nanoparticles)

Compound 168 was used as the ionic lipid (1), and POPE, Chol, and DMG-PEG2000 were used as different lipids. The molar ratio of compound 168:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.33 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (200 µL, nucleic acid concentration: 200 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.3 wt%, nucleic acid content: 20 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 37 (Preparation of frozen composition containing nucleic acid-encapsulating lipid nanoparticles)

Compound 169 was used as the ionic lipid (1), and POPE, Chol, and DMG-PEG2000 were used as different lipids. The molar ratio of compound 169:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.33 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (200 µL, nucleic acid concentration: 200 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.3 wt%, nucleic acid content: 20 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 38 (Preparation of frozen composition containing nucleic acid-encapsulating lipid nanoparticles)

Compound 198 was used as the ionic lipid (1), and POPE, Chol, and DMG-PEG2000 were used as different lipids. The molar ratio of compound 198:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 13.47 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 100 µL to obtain a suspension containing nucleic acid-encapsulating lipid nanoparticles (100 µL, nucleic acid concentration: 101 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.6 wt%, nucleic acid content: 10 µg, N/P ratio: 64, cryoprotective agent (sucrose) content: 7 wt%, water (ice) content: 91 wt%) (step d)).

After freezing, the frozen composition was stored at - 80°C, and before use, thawed by standing same in an atmospheric pressure environment in a refrigerator at 4°C for 30 min to obtain a suspension.

### Example 39 (Preparation of frozen composition containing nucleic acid-encapsulating lipid nanoparticles)

Compound 197 was used as the ionic lipid (1), and POPE, Chol, and DMG-PEG2000 were used as different lipids. The molar ratio of compound 167:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.33 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (200 µL, nucleic acid concentration: 200 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.4 wt%, nucleic acid content: 20 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 40 (Preparation of frozen composition containing nucleic acid-encapsulating lipid nanoparticles)

Compound 196 was used as the ionic lipid (1), and POPE, Chol, and DMG-PEG2000 were used as different lipids. The molar ratio of compound 196:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 13.47 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 100 µL to obtain a suspension containing nucleic acid-encapsulating lipid nanoparticles (100 µL, nucleic acid concentration: 101 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.6 wt%, nucleic acid content: 10 µg, N/P ratio: 64, cryoprotective agent (sucrose) content: 7 wt%, water (ice) content: 91 wt%) (step d)).

After freezing, the frozen composition was stored at - 80°C, and before use, thawed by standing same in an atmospheric pressure environment in a refrigerator at 4°C for 30 min to obtain a suspension.

### Comparative Example 6 (Preparation of suspension containing nucleic acid-encapsulating lipid nanoparticles)

MC3 was used as the ionic lipid, and DSPC, Chol, and DMG-PEG2000 were further used as different lipids. The molar ratio of MC3:DSPC:Chol:DMG-PEG2000 used was 50:10:38.5:1.5. The lipids were mixed such that the total lipids in a lipid ethanol solution were 3.0 mM.

955 µL of a nucleic acid solution (nucleic acid concentration: 27.4 µg/mL) obtained by diluting FLuc mRNA with an acidic citrate buffer (buffer component concentration: 20 mM, pH: 5.0), and 318 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 1000 µL of a Tris-buffered saline (buffer component concentration: 50 mM, pH: 7.6), and the obtained suspension was transferred to an Amicon Ultra 4 and ultrafiltrated under centrifugation conditions (25°C, 1000 g, 3 min) to concentrate same to about 500 µL. Thereafter, it was diluted with 3500 µL of a Tris-buffered saline (buffer component concentration: 50 mM, pH: 7.6). The following operation was performed twice to concentrate same to 400 µL to obtain a suspension containing nucleic acid-encapsulating lipid nanoparticles (nucleic acid concentration: 40 µg/mL, N/P ratio: 6) (step b)).

Polydispersity Index (PdI) and mRNA encapsulation rate were analyzed for the nucleic acid-encapsulating lipid nanoparticles in the suspensions obtained in Examples 18 to 26 and 28 to 39, step c). The results are shown in the line of "step c)" in Table 8 and Table 9. Similarly, the particle size, PdI, and mRNA encapsulation rate were analyzed for the nucleic acid-encapsulating lipid nanoparticles in the suspensions obtained by thawing the frozen compositions of Examples 18 to 26 and 28 to 39. The results are shown in the line of "after freezing and thawing " in Table 8 and Table 9. The particle size and PdI were measured by a dynamic light scattering method using ZetaSizer (registered trademark). The mRNA encapsulation rate (referred to as "encapsulation rate" in the table below) was measured by the Ribogreen (registered trademark) assay. The particle size is expressed in Z-Average size (referred to as "Z-ave" in the Table below).

**[Table 8]**

| | | encapsulation rate (%) | Z-ave (nm) | PdI |
|---|---|---|---|---|
| Example 18 | step c) | 93 | 80 | 0.08 |
| | after freezing and thawing | 91 | 83 | 0.13 |
| Example 19 | step c) | 89 | 75 | 0.14 |
| | after freezing and thawing | 89 | 78 | 0.13 |
| Example 20 | step c) | 96 | 78 | 0.07 |
| | after freezing and thawing | 96 | 81 | 0.10 |
| Example 21 | step c) | 92 | 77 | 0.13 |
| | after freezing and thawing | 92 | 81 | 0.11 |
| Example 22 | step c) | 100 | 104 | 0.05 |
| | after freezing and thawing | 94 | 134 | 0.01 |
| Example 23 | step c) | 84 | 83 | 0.11 |
| | after freezing and thawing | 84 | 89 | 0.12 |
| Example 24 | step c) | 95 | 77 | 0.07 |
| | after freezing and thawing | 95 | 89 | 0.06 |
| Example 25 | step c) | 99 | 95 | 0.04 |
| | after freezing and thawing | 97 | 102 | 0.04 |
| Example 26 | step c) | 100 | 103 | 0.04 |
| | after freezing and thawing | 99 | 127 | 0.03 |

**[Table 9]**

| | | encapsulation rate (%) | Z-ave (nm) | PdI |
|---|---|---|---|---|
| Example 28 | step c) | 92 | 79 | 0.14 |
| | after freezing and thawing | 92 | 95 | 0.12 |
| Example 29 | step c) | 92 | 88 | 0.14 |
| | after freezing and thawing | 90 | 95 | 0.12 |
| Example 30 | step c) | 97 | 78 | 0.08 |
| | after freezing and thawing | 97 | 84 | 0.09 |
| Example 31 | step c) | 88 | 80 | 0.10 |
| | after freezing and thawing | 88 | 89 | 0.13 |
| Example 32 | step c) | 90 | 75 | 0.14 |
| | after freezing and thawing | 87 | 78 | 0.12 |
| Example 33 | step c) | 88 | 77 | 0.14 |
| | after freezing and thawing | 88 | 89 | 0.12 |
| Example 34 | step c) | 89 | 73 | 0.13 |
| | after freezing and thawing | 84 | 77 | 0.12 |
| Example 35 | step c) | 91 | 75 | 0.15 |
| | after freezing and thawing | 91 | 93 | 0.10 |
| Example 36 | step c) | 90 | 72 | 0.10 |
| | after freezing and thawing | 86 | 77 | 0.10 |
| Example 37 | step c) | 87 | 76 | 0.12 |
| | after freezing and thawing | 87 | 99 | 0.08 |
| Example 38 | step c) | 99 | 81 | 0.05 |
| | after freezing and thawing | 97 | 102 | 0.11 |
| Example 39 | step c) | 90 | 102 | 0.02 |
| | after freezing and thawing | 89 | 99 | 0.05 |

From Tables 8 and 9, it was shown that the physical properties of the nucleic acid-encapsulating lipid nanoparticles in the frozen compositions of Examples 18 to 26 and 28 to 39 did not change even after freezing and thawing.

Each of the frozen compositions of Examples 17, 20, 22, 24, 25, 26, 27, 29, 30, 38, and 40 was thawed and further diluted with a Tris-buffered saline (pH: 7.6) to obtain a suspension containing nucleic acid-encapsulating lipid nanoparticles (nucleic acid concentration: 1 µg/mL). In addition, the suspension of Comparative Example 6 was diluted with a Tris-buffered saline (pH: 7.6) to obtain a suspension containing nucleic acid-encapsulating lipid nanoparticles (nucleic acid concentration: 1 µg/mL). The nucleic acid-encapsulating lipid nanoparticles in the obtained suspension were applied to Hep G2 cell, and the gene expression activity thereof was evaluated. Specifically, Hep G2 cells were seeded in D-MEM medium (high glucose) (containing L-glutamine, phenol red) (10% by volume FBS, 1% by volume penicillin-streptomycin solution added) at 3×10⁴ cells/600 µL into the wells of a 24-well flat-bottom clear black plate. The obtained suspension containing nucleic acid-encapsulating lipid nanoparticles was added to the aforementioned well such that the nucleic acid concentration in the medium was 0.03 µg/30 µL. Luciferin was added to the aforementioned well such that the concentration in the medium was 0.1 mM, and the amount of luciferase protein introduced was evaluated over time using the amount of luminescence. Hep G2 cells cultured in the medium containing nucleic acid-encapsulating nanoparticles and luciferin were placed in an incubator-type luminometer (Kronos), and the cumulative amount of luminescence was measured every hour. The results are shown in Fig. 6. In Fig. 6, "2.0.E+08" and the like mean "2.0×10⁸" and the like.

Each of the frozen compositions of Examples 18, 19, 21, 23, 28, 31, 32, 34, and 36 was thawed and further diluted with a Tris-buffered saline (pH: 7.6) to obtain a suspension containing nucleic acid-encapsulating lipid nanoparticles (nucleic acid concentration: 1 µg/mL). In addition, the suspension of Comparative Example 6 was diluted with a Tris-buffered saline (pH: 7.6) to obtain a suspension containing nucleic acid-encapsulating lipid nanoparticles (nucleic acid concentration: 1 µg/mL). The nucleic acid-encapsulating lipid nanoparticles in the obtained suspension were applied to HeLa cells, and the gene expression activity thereof was evaluated. Specifically, HeLa cells were seeded in D-MEM medium (high glucose) (containing L-glutamine, phenol red) (10% by volume FBS, 1% by volume penicillin-streptomycin solution added) at 3×10⁴ cells/600 µL into the wells of a 24-well flat-bottom clear black plate. The obtained suspension containing nucleic acid-encapsulating lipid nanoparticles was added to the aforementioned well such that the nucleic acid concentration in the medium was 0.03 µg/30 µL. Luciferin was added to the aforementioned well such that the concentration in the medium was 0.1 mM, and the amount of luciferase protein introduced was evaluated over time using the amount of luminescence. Hep G2 cells cultured in the medium containing nucleic acid-encapsulating nanoparticles and luciferin were placed in an incubator-type luminometer (Kronos), and the cumulative amount of luminescence was measured every hour. The results are shown in Fig. 7. In Fig. 7, "2.5.E+08" and the like mean "2.5×10⁸" and the like.

As shown in Figs. 6 and 7, the nucleic acid-encapsulating lipid nanoparticles in the suspension obtained by thawing the frozen composition of any of Examples 17 to 32, 34, 36, 38, and 40 showed high gene expression activity equivalent to or greater than that of the nucleic acid-encapsulating lipid nanoparticles in the unfrozen suspension of Comparative Example 6, even after freezing and thawing.

Each of the frozen compositions of Examples 18 to 21, 23 to 25, 28 to 37, and 39 was thawed and further diluted with a Tris-buffered saline (pH: 7.6) to obtain a suspension containing nucleic acid-encapsulating lipid nanoparticles (nucleic acid concentration: 1 µg/mL). In addition, the suspension of Comparative Example 6 was diluted with a Tris-buffered saline (pH: 7.6) to obtain a suspension containing nucleic acid-encapsulating lipid nanoparticles (nucleic acid concentration: 1 µg/mL). The obtained nucleic acid-encapsulating lipid nanoparticles were applied to Hep G2 cell, and the cytotoxicity thereof was evaluated. Specifically, Hep G2 cells were seeded in D-MEM medium (high glucose) (containing L-glutamine, phenol red) (10% by volume FBS, 1% by volume penicillin-streptomycin solution added) at 5×10³ cells/100 µL into the wells of a 96-well flat-bottom clear black plate. The obtained suspension containing nucleic acid-encapsulating lipid nanoparticles was added to the aforementioned well such that the lipid concentration in the medium was 1 mmol/100 µL. After incubation for 12 hr, fluorescence of dead cells was measured using CytoTox-Fluor (registered trademark) Cytotoxicity Assay. The results thereof are shown in Fig. 8. In Fig. 8, "3.0E+05" and the like mean "3.0×10⁵" and the like.

As shown in Fig. 8, the nucleic acid-encapsulating lipid nanoparticles in the suspensions obtained by thawing the frozen compositions of Examples 18 to 21, 23 to 25, 28 to 37, and 39 showed lower amount of fluorescence, which indicates the number of dead cells, compared to those of Comparative Example 6, which represents the conventional technology. From these results, it is clear that the nucleic acid-encapsulating lipid nanoparticles of Examples 18 to 21, 23 to 25, 28 to 37, and 39 (N/P ratio: 16 to 64) have lower toxicity compared to Comparative Example 6 (N/P ratio: 6), even though having a high N/P ratio.

### Example 41 (Preparation of frozen composition containing nucleic acid-encapsulating lipid nanoparticles)

SS-OP was used as the ionic lipid (1), and POPE, Chol, and DMG-PEG2000 were used as different lipids. The molar ratio of SS-OP:POPE:Chol:DMG-PEG2000 used was 49:15:34:2. The lipids were mixed such that the total lipids in a lipid ethanol solution were 8.0 mM.

2475 µL of a nucleic acid solution (nucleic acid concentration: 53.9 µg/mL) obtained by diluting FLuc mRNA with an acidic citrate buffer (buffer component concentration: 20 mM, pH: 5.0), and 825 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Slide-A-Lyzer G3 Dialysis Cassettes (manufactured by Thermo Scientific, MWCO: 3.5kD), and dialyzed in a beaker containing 600 mL of a Tris/maleate/sucrose buffer (buffer component concentration: 50 mM, pH: 6.0, sucrose concentration: 80 mg/mL) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (200 µL, nucleic acid concentration: 606.38 µg/mL) (step b')).

The obtained suspension was placed in a screw tube by 100 µL, and frozen by cooling at -80°C for 24 hr to obtain a frozen composition (ionic lipid (1) content: 1.6 wt%, nucleic acid content: 61 µg, N/P ratio: 16, cryoprotective agent (sucrose) content: 7 wt%, water (ice) content: 90 wt%) (step d)).

After freezing, the frozen composition was stored at - 80°C, and before use, thawed by standing same in an atmospheric pressure environment in a refrigerator at 4°C for 30 min to obtain a suspension.

### Example 42 (Preparation of frozen composition containing nucleic acid-encapsulating lipid nanoparticles)

SS-OP was used as the ionic lipid (1), and POPE, Chol, and DMG-PEG2000 were used as different lipids. The molar ratio of SS-OP:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 71.87 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Slide-A-Lyzer G3 Dialysis Cassettes, and dialyzed in a beaker containing 600 mL of a Tris/maleate/sucrose buffer (buffer component concentration: 50 mM, pH: 6.0, sucrose concentration: 80 mg/mL) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (500 µL, nucleic acid concentration: 107.8 µg/mL) (step b')).

The obtained suspension was placed in a screw tube by 200 µL, and frozen by cooling at -80°C for 24 hr to obtain a frozen composition (ionic lipid (1) content: 0.2 wt%, nucleic acid content: 22 µg, N/P ratio: 12, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 43 (Preparation of frozen composition containing nucleic acid-encapsulating lipid nanoparticles)

SS-OP was used as the ionic lipid (1), and POPE, Chol, and DMG-PEG2000 were used as different lipids. The molar ratio of SS-OP:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 107.8 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Slide-A-Lyzer G3 Dialysis Cassettes, and dialyzed in a beaker containing 600 mL of a Tris/maleate/sucrose buffer (buffer component concentration: 50 mM, pH: 6.0, sucrose concentration: 80 mg/mL) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (500 µL, nucleic acid concentration: 101.1 µg/mL) (step b')).

The obtained suspension was placed in a screw tube by 200 µL, and frozen by cooling at -80°C for 24 hr to obtain a frozen composition (ionic lipid (1) content: 0.1 wt%, nucleic acid content: 20 µg, N/P ratio: 8, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 44 (Preparation of frozen composition containing nucleic acid-encapsulating lipid nanoparticles)

SS-OP was used as the ionic lipid (1), and DOPG, DEPC, Chol, and DMG-PEG2000 were used as different lipids. The molar ratio of SS-OP:DOPG:DEPC:Chol:DMG-PEG2000 used was 57.2:6.5:6.5:28.8:1. The lipids were mixed such that the total lipids in a lipid ethanol solution were 8.0 mM.

1725 µL of a nucleic acid solution (nucleic acid concentration: 62.92 µg/mL) obtained by diluting MS2 mRNA (manufactured by Roche), which is a bacteriophage-derived RNA, with an acidic citrate buffer (buffer component concentration: 20 mM, pH: 5.0), and 575 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Slide-A-Lyzer G3 Dialysis Cassettes, and dialyzed in a beaker containing 600 mL of a Tris/maleate/sucrose buffer (buffer component concentration: 50 mM, pH: 6.0, sucrose concentration: 80 mg/mL) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min), and concentrated to about 157 µL to obtain a suspension containing nucleic acid-encapsulating lipid nanoparticles (500 µL, nucleic acid concentration: 601.1 µg/mL) (step b')).

The obtained suspension was placed in a screw tube by 157 µL, and frozen by cooling at -80°C for 24 hr to obtain a frozen composition (ionic lipid (1) content: 1.4 wt%, nucleic acid content: 94 µg, N/P ratio: 16, cryoprotective agent (sucrose) content: 7 wt%, water (ice) content: 90 wt%) (step d)).

After freezing, the frozen composition was stored at - 80°C, and before use, thawed by standing same in an atmospheric pressure environment in a refrigerator at 4°C for 30 min to obtain a suspension.

### Example 45 (Preparation of frozen composition containing nucleic acid-encapsulating lipid nanoparticles)

SS-OP was used as the ionic lipid (1), and POPE, Chol, and DMG-PEG2000 were used as different lipids. The molar ratio of SS-OP:POPE:Chol:DMG-PEG2000 used was 49:15:34:2. The lipids were mixed such that the total lipids in a lipid ethanol solution were 8.0 mM.

9938 µL of a nucleic acid solution (nucleic acid concentration: 53.33 µg/mL) obtained by diluting ribonucleic acid, which is a yeast-derived total RNA, with an acidic citrate buffer (buffer component concentration: 20 mM, pH: 5.0), and 3313 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)). The step a was performed 7 times, and the suspensions were combined into one.

The obtained suspension was diluted with 70 mL of a Tris/maleate buffer (buffer component concentration: 50 mM, pH: 6.0) and then further diluted with 560 mL of a Tris/maleate buffer (buffer component concentration: 50 mM, pH: 6.0). The diluted suspension was concentrated 10-fold using KrosFlo (registered trademark) KR2i TFF System (manufactured by REPLIGEN), and then subjected to tangential flow filtration using diafiltration until seven times the volume of the suspension permeated through. Thereafter, the suspension was further concentrated 10-fold using KrosFlo (registered trademark) KR2i TFF System to obtain a suspension containing nucleic acid-encapsulating lipid nanoparticles (7000 µL, nucleic acid concentration: 400 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.5 wt%, nucleic acid content: 40 µg, N/P ratio: 16, cryoprotective agent (sucrose) content: 8.0 wt%, water (ice) content: 91 wt%) (step d)).

After freezing, the frozen composition was stored at - 80°C, and before use, thawed by standing same in an atmospheric pressure environment in a refrigerator at 4°C for 30 min to obtain a suspension.

### Example 46 (Preparation of frozen composition containing nucleic acid-encapsulating lipid nanoparticles)

SS-OP was used as the ionic lipid (1), and POPE, Chol, and DMG-PEG2000 were used as different lipids. The molar ratio of SS-OP:POPE:Chol:DMG-PEG2000 used was 49:15:34:2. The lipids were mixed such that the total lipids in a lipid ethanol solution were 8.0 mM.

2475 µL of a nucleic acid solution (nucleic acid concentration: 53.9 µg/mL) obtained by diluting FLuc siRNA with an acidic citrate buffer (buffer component concentration: 20 mM, pH: 5.0), and 825 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Slide-A-Lyzer G3 Dialysis Cassettes, and dialyzed in a beaker containing 600 mL of a Tris/maleate/sucrose buffer (buffer component concentration: 50 mM, pH: 6.0, sucrose concentration: 80 mg/mL) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (200 µL, nucleic acid concentration: 606.38 µg/mL) (step b')).

The obtained suspension was placed in a screw tube by 100 µL, and frozen by cooling at -80°C for 24 hr to obtain a frozen composition (ionic lipid (1) content: 1.6 wt%, nucleic acid content: 61 µg, N/P ratio: 16, cryoprotective agent (sucrose) content: 7 wt%, water (ice) content: 90 wt%) (step d)).

After freezing, the frozen composition was stored at - 80°C, and before use, thawed by standing same in an atmospheric pressure environment in a refrigerator at 4°C for 30 min to obtain a suspension.

The particle size, Polydispersity Index(PdI), and mRNA encapsulation rate of nucleic acid-encapsulating lipid nanoparticles in the suspensions obtained in Examples 41 to 46, step c) were analyzed. The results are shown in the line of "step c)" in Table 10. Similarly, the particle size, PdI, and mRNA encapsulation rate were analyzed for the nucleic acid-encapsulating lipid nanoparticles in the suspensions obtained by thawing the frozen compositions of Examples 41 to 44 and 46. The results are shown in the line of "after freezing and thawing" in Table 10. Similarly, one day after the freezing date (hereinafter referred to as "Day 1"), and 14 days after the freezing date (hereinafter referred to as "Day 14"), the particle size, PdI, and mRNA encapsulation rate were analyzed for the nucleic acid-encapsulating lipid nanoparticles in the suspension obtained by thawing the frozen composition obtained in Example 45, step d). The results are shown in the line of "Day 1" or "Day 14" in Table 10. The particle size and PdI were measured by a dynamic light scattering method using ZetaSizer (registered trademark). The mRNA encapsulation rate (referred to as "encapsulation rate" in the table below) was measured by the Ribogreen (registered trademark) assay. The particle size is expressed in Z-Average size (referred to as "Z-ave" in the Table below).

**[Table 10]**

| | | encapsulation rate (%) | Z-ave (nm) | PdI |
|---|---|---|---|---|
| Example 41 | step c) | 93 | 72 | 0.11 |
| | after freezing and thawing | 85 | 79 | 0.18 |
| Example 42 | step c) | 91 | 70 | 0.19 |
| | after freezing and thawing | 90 | 68 | 0.12 |
| Example 43 | step c) | 89 | 66 | 0.16 |
| | after freezing and thawing | 89 | 69 | 0.15 |
| Example 44 | step c) | 88 | 104 | 0.09 |
| | after freezing and thawing | 92 | 104 | 0.07 |
| Example 45 | step c) | 83 | 75 | 0.19 |
| | Day 1 | 80 | 75 | 0.21 |
| | Day 14 | 80 | 77 | 0.19 |
| Example 46 | step c) | 90 | 74 | 0.09 |
| | after freezing and thawing | 89 | 77 | 0.11 |

As shown in Table 10, the frozen compositions of Examples 41 to 46 showed no marked changes in the physical properties of the nucleic acid-encapsulating lipid nanoparticles even after freezing and thawing. In particular, the frozen composition of Example 45 showed no marked changes in the physical properties of the nucleic acid-encapsulating lipid nanoparticles even after 14 days, by which superior storage stability thereof was shown. Therefrom it was shown that even when the N/P ratio was changed within the scope of the present invention, when two types of phospholipids were used, when tangential flow filtration was used in the buffer exchange step, and when siRNA was used as the nucleic acid, no marked changes in the physical properties of the nucleic acid-encapsulating lipid nanoparticles were observed even after freezing and thawing.

Each of the frozen compositions of Examples 41 to 43 was thawed and further diluted with a Tris-buffered saline (pH: 7.6) to obtain a suspension containing nucleic acid-encapsulating lipid nanoparticles (nucleic acid concentration: 1 µg/mL). The nucleic acid-encapsulating lipid nanoparticles in the obtained suspension were applied to Hep G2 cell, and the gene expression activity thereof was evaluated. Specifically, Hep G2 cells were seeded in D-MEM medium (high glucose) (containing L-glutamine, phenol red) (10% by volume FBS, 1% by volume penicillin-streptomycin solution added) at 3×10⁴ cells/600 µL into the wells of a 24-well flat-bottom clear black plate. The obtained suspension containing nucleic acid-encapsulating lipid nanoparticles was added to the aforementioned well such that the nucleic acid concentration in the medium was 0.03 µg/30 µL. Luciferin was added to the aforementioned well such that the concentration in the medium was 0.1 mM, and the amount of luciferase protein introduced was evaluated over time using the amount of luminescence. Hep G2 cells cultured in the medium containing nucleic acid-encapsulating nanoparticles and luciferin were placed in an incubator-type luminometer (Kronos), and the cumulative amount of luminescence was measured every hour. The results are shown in Fig. 9. In Fig. 9, "5.0.E+07" and the like mean "5.0×10⁷" and the like.

Similar to Fig. 1, Fig. 9 also shows that the gene expression activity of the nucleic acid-encapsulating lipid nanoparticles in the frozen composition of the present invention increases as the N/P ratio increases.

### Example 47 (Preparation of frozen composition containing nucleic acid-encapsulating lipid nanoparticles)

SS-OP was used as the ionic lipid (1), and DOPE, Chol, and DMG-PEG2000 were used as different lipids. The molar ratio of SS-OP:DOPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.9 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)). The step a was performed 4 times, and the suspensions were combined into one.

The obtained suspension was transferred to Slide-A-Lyzer G3 Dialysis Cassettes, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 100 µL to obtain a suspension containing nucleic acid-encapsulating lipid nanoparticles (100 µL, nucleic acid concentration: 100 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 190 µL, and frozen by cooling at -80°C for 24 hr to obtain a frozen composition (ionic lipid (1) content: 0.1 wt%, nucleic acid content: 10 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 48 (Preparation of frozen composition containing nucleic acid-encapsulating lipid nanoparticles)

SS-OP was used as the ionic lipid (1), and POPE, Chol, and DMG-PEG2000 were used as different lipids. The molar ratio of SS-OP:POPE:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.9 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)). The step a was performed 4 times, and the suspensions were combined into one.

The obtained suspension was transferred to Slide-A-Lyzer G3 Dialysis Cassettes, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 100 µL to obtain a suspension containing nucleic acid-encapsulating lipid nanoparticles (100 µL, nucleic acid concentration: 100 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 190 µL, and frozen by cooling at -80°C for 24 hr to obtain a frozen composition (ionic lipid (1) content: 0.1 wt%, nucleic acid content: 10 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 49 (Preparation of frozen composition containing nucleic acid-encapsulating lipid nanoparticles)

SS-OP was used as the ionic lipid (1), and DSPC, Chol, and DMG-PEG2000 were used as different lipids. The molar ratio of SS-OP:DSPC:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.9 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)). The step a was performed 4 times, and the suspensions were combined into one.

The obtained suspension was transferred to Slide-A-Lyzer G3 Dialysis Cassettes, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 100 µL to obtain a suspension containing nucleic acid-encapsulating lipid nanoparticles (100 µL, nucleic acid concentration: 100 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 190 µL, and frozen by cooling at -80°C for 24 hr to obtain a frozen composition (ionic lipid (1) content: 0.1 wt%, nucleic acid content: 10 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 50 (Preparation of frozen composition containing nucleic acid-encapsulating lipid nanoparticles)

SS-OP was used as the ionic lipid (1), and POPC, Chol, and DMG-PEG2000 were used as different lipids. The molar ratio of SS-OP:POPC:Chol:DMG-PEG2000 used was 49:15:34:2. The lipids were mixed such that the total lipids in a lipid ethanol solution were 8.0 mM.

975 µL of a nucleic acid solution (nucleic acid concentration: 53.9 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)). The step a was performed 4 times, and the suspensions were combined into one.

The obtained suspension was transferred to Slide-A-Lyzer G3 Dialysis Cassettes, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 100 µL to obtain a suspension containing nucleic acid-encapsulating lipid nanoparticles (100 µL, nucleic acid concentration: 100 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 190 µL, and frozen by cooling at -80°C for 24 hr to obtain a frozen composition (ionic lipid (1) content: 0.1 wt%, nucleic acid content: 10 µg, N/P ratio: 16, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

The particle size, Polydispersity Index (PdI), and mRNA encapsulation rate were analyzed for the nucleic acid-encapsulating lipid nanoparticles in the suspensions obtained in Examples 47 to 50, step c). Similarly, one day after the freezing date (hereinafter referred to as "Day 1"), 7 days after the freezing date (hereinafter referred to as "Day 7"), 14 days after the freezing date (hereinafter referred to as "Day 14"), and 28 days after the freezing date (hereinafter referred to as "Day 28"), the particle size, PdI, and mRNA encapsulation rate were analyzed for the nucleic acid-encapsulating lipid nanoparticles in the suspensions obtained by thawing the frozen compositions obtained in Examples 47 to 50, step d). The particle size and PdI were measured by a dynamic light scattering method using ZetaSizer (registered trademark). The mRNA encapsulation rate (referred to as "encapsulation rate" in the table below) was measured by the Ribogreen (registered trademark) assay. The results are shown in Table 11. The particle size is expressed in Z-Average size (referred to as "Z-ave" in the Table below).

**[Table 11]**

| | | encapsulation rate (%) | Z-ave (nm) | PdI |
|---|---|---|---|---|
| Example 47 | step c) | 88 | 88 | 0.16 |
| | Day 1 | 96 | 89 | 0.14 |
| | Day 7 | 96 | 89 | 0.15 |
| | Day 14 | 94 | 85 | 0.15 |
| | Day 28 | 95 | 87 | 0.14 |
| Example 48 | step c) | 86 | 85 | 0.13 |
| | Day 1 | 98 | 86 | 0.16 |
| | Day 7 | 95 | 88 | 0.14 |
| | Day 14 | 95 | 86 | 0.19 |
| | Day 28 | 93 | 87 | 0.15 |
| Example 49 | step c) | 96 | 64 | 0.18 |
| | Day 1 | 98 | 68 | 0.17 |
| | Day 7 | 97 | 66 | 0.16 |
| | Day 14 | 96 | 66 | 0.18 |
| | Day 28 | 96 | 67 | 0.18 |
| Example 50 | step c) | 92 | 64 | 0.17 |
| | Day 1 | 97 | 68 | 0.17 |
| | Day 7 | 98 | 69 | 0.19 |
| | Day 14 | 94 | 68 | 0.16 |
| | Day 28 | 95 | 67 | 0.18 |

The nucleic acid-encapsulating lipid nanoparticles in the suspensions obtained by thawing each frozen composition of Example 47 to 50 maintained equivalent particle physical properties even after 28 days of storage, thus showing the high storage stability thereof.

Each of the frozen compositions of Examples 47 to 50 was thawed and further diluted with a Tris-buffered saline (pH: 7.6) to obtain a suspension containing nucleic acid-encapsulating lipid nanoparticles (nucleic acid concentration: 1 µg/mL). The nucleic acid-encapsulating lipid nanoparticles in the obtained suspension were applied to Hep G2 cell, and the gene expression activity thereof was evaluated. Specifically, Hep G2 cells were seeded in D-MEM medium (high glucose) (containing L-glutamine, phenol red) (10% by volume FBS, 1% by volume penicillin-streptomycin solution added) at 3×10⁴ cells/600 µL into the wells of a 24-well flat-bottom clear black plate. The obtained suspension containing nucleic acid-encapsulating lipid nanoparticles was added to the aforementioned well such that the nucleic acid concentration in the medium was 0.03 µg/30 µL. Luciferin was added to the aforementioned well such that the concentration in the medium was 0.1 mM, and the amount of luciferase protein introduced was evaluated over time using the amount of luminescence. Hep G2 cells cultured in the medium containing nucleic acid-encapsulating nanoparticles and luciferin were placed in an incubator-type luminometer (Kronos), and the cumulative amount of luminescence was measured every hour. The results are shown in Figs. 10 to 13. In Figs. 10 to 13, "1.2.E+08" and the like mean "1.2×10⁸" and the like.

As shown in Figs. 10 to 13, the nucleic acid-encapsulating lipid nanoparticles in the suspension obtained by thawing each frozen compositions of Examples 47 to 50 all showed high gene expression activity even after thawing. This indicates that similar effects can be achieved even when using various phospholipids within the scope of the present invention.

The frozen composition of Example 49 was stored at -80°C after freezing as mentioned above. One day after the freezing date ("Day 1" in Fig. 14), 7 days after the freezing date ("Day 7⁷ in Fig. 14), 14 days after the freezing date ("Day 14" in Fig. 14), and 30 days after the freezing date ("Day 30" in Fig. 14), the aforementioned frozen composition was thawed and further diluted with a Tris-buffered saline (pH: 7.6) to obtain a suspension containing nucleic acid-encapsulating lipid nanoparticles (nucleic acid concentration: 0.03 µg/30 µL). The obtained nucleic acid-encapsulating nanoparticles were applied to Hep G2 cell, and the gene expression activity thereof was evaluated.

Specifically, Hep G2 cells were seeded in D-MEM medium (high glucose) (containing L-glutamine, phenol red) (10% by volume FBS, 1% by volume penicillin-streptomycin solution added) at 3×10⁴ cells/600 µL into a 24-well flat-bottom clear black plate. The suspension of the nucleic acid-encapsulating nanoparticles obtained as mentioned above was added to the medium. Luciferin was added to the medium such that the concentration was 0.1 mM, and the amount of luciferase protein introduced was evaluated over time using the amount of luminescence. Hep G2 cells cultured in the medium containing nucleic acid-encapsulating nanoparticles and luciferin were placed in an incubator-type luminometer (Kronos), and the cumulative amount of luminescence was measured every hour. The results are shown in Fig. 14. The bar graph of Example 49 in Fig. 14 shows the results of 1 day, 7 days, 14 days, and 30 days later in this order from the left. In Fig. 14, "3.5E+07" and the like mean "3.5×10⁷" and the like.

As shown in Fig. 14, the nucleic acid-encapsulating lipid nanoparticles in the frozen composition of Example 49 maintained high gene expression activity even after 30 days of storage, thus showing high gene expression activity.

### Example 51 (Preparation of frozen composition containing nucleic acid-encapsulating lipid nanoparticles)

SS-OP was used as the ionic lipid, and POPE, Chol, and DMG-PEG2000 were used as different lipids. The molar ratio of SS-OP:POPE:Chol:DMG-PEG2000 used was 49:5:45:1. The lipids were mixed such that the total lipids in a lipid ethanol solution were 8.0 mM.

975 µL of a nucleic acid solution (nucleic acid concentration: 107.80 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, ultrafiltrated under centrifugation conditions (25°C, 1000 g, 3 min), and concentrated to about 1000 µL to obtain a suspension containing nucleic acid-encapsulating lipid nanoparticles (1000 µL, nucleic acid concentration: 108 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.1 wt%, nucleic acid content: 11 µg, N/P ratio: 8, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 52 (Preparation of frozen composition containing nucleic acid-encapsulating lipid nanoparticles)

SS-OP was used as the ionic lipid, and POPE, Chol, and DMG-PEG2000 were used as different lipids. The molar ratio of SS-OP:POPE:Chol:DMG-PEG2000 used was 30:15:53:2. The lipids were mixed such that the total lipids in a lipid ethanol solution were 8.0 mM.

975 µL of a nucleic acid solution (nucleic acid concentration: 44.0 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, ultrafiltrated under centrifugation conditions (25°C, 1000 g, 3 min), and concentrated to about 330 µL to obtain a suspension containing nucleic acid-encapsulating lipid nanoparticles (330 µL, nucleic acid concentration: 100 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.1 wt%, nucleic acid content: 10 µg, N/P ratio: 12, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 53 (Preparation of frozen composition containing nucleic acid-encapsulating lipid nanoparticles)

SS-OP was used as the ionic lipid, and POPE, Chol, and DMG-PEG2000 were used as different lipids. The molar ratio of SS-OP:POPE:Chol:DMG-PEG2000 used was 30:30:37:3. The lipids were mixed such that the total lipids in a lipid ethanol solution were 8.0 mM.

975 µL of a nucleic acid solution (nucleic acid concentration: 29.33 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, ultrafiltrated under centrifugation conditions (25°C, 1000 g, 3 min), and concentrated to about 220 µL to obtain a suspension containing nucleic acid-encapsulating lipid nanoparticles (220 µL, nucleic acid concentration: 100 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.1 wt%, nucleic acid content: 10 µg, N/P ratio: 18, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 54 (Preparation of frozen composition containing nucleic acid-encapsulating lipid nanoparticles)

SS-OP was used as the ionic lipid, and POPE, Chol, and DMG-PEG2000 were used as different lipids. The molar ratio of SS-OP:POPE:Chol:DMG-PEG2000 used was 45:5:48:2. The lipids were mixed such that the total lipids in a lipid ethanol solution were 8.0 mM.

975 µL of a nucleic acid solution (nucleic acid concentration: 44.0 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, ultrafiltrated under centrifugation conditions (25°C, 1000 g, 3 min), and concentrated to about 330 µL to obtain a suspension containing nucleic acid-encapsulating lipid nanoparticles (330 µL, nucleic acid concentration: 100 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.1 wt%, nucleic acid content: 10 µg, N/P ratio: 18, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 55 (Preparation of frozen composition containing nucleic acid-encapsulating lipid nanoparticles)

SS-OP was used as the ionic lipid, and POPE, Chol, and DMG-PEG2000 were used as different lipids. The molar ratio of SS-OP:POPE:Chol:DMG-PEG2000 used was 45:15:37:3. The lipids were mixed such that the total lipids in a lipid ethanol solution were 8.0 mM.

975 µL of a nucleic acid solution (nucleic acid concentration: 99.0 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 990 µL to obtain a suspension containing nucleic acid-encapsulating lipid nanoparticles (990 µL, nucleic acid concentration: 100 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.1 wt%, nucleic acid content: 10 µg, N/P ratio: 8, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 56 (Preparation of frozen composition containing nucleic acid-encapsulating lipid nanoparticles)

SS-OP was used as the ionic lipid, and POPE, Chol, and DMG-PEG2000 were used as different lipids. The molar ratio of SS-OP:POPE:Chol:DMG-PEG2000 used was 40:30:29:1. The lipids were mixed such that the total lipids in a lipid ethanol solution were 8.0 mM.

975 µL of a nucleic acid solution (nucleic acid concentration: 58.67 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 440 µL to obtain a suspension containing nucleic acid-encapsulating lipid nanoparticles (440 µL, nucleic acid concentration: 100 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.1 wt%, nucleic acid content: 10 µg, N/P ratio: 12, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 57 (Preparation of frozen composition containing nucleic acid-encapsulating lipid nanoparticles)

SS-OP was used as the ionic lipid, and POPE, Chol, and DMG-PEG2000 were used as different lipids. The molar ratio of SS-OP:POPE:Chol:DMG-PEG2000 used was 60:5:32:3. The lipids were mixed such that the total lipids in a lipid ethanol solution were 8.0 mM.

975 µL of a nucleic acid solution (nucleic acid concentration: 88.0 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 660 µL to obtain a suspension containing nucleic acid-encapsulating lipid nanoparticles (660 µL, nucleic acid concentration: 100 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.1 wt%, nucleic acid content: 10 µg, N/P ratio: 12, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Example 58 (Preparation of frozen composition containing nucleic acid-encapsulating lipid nanoparticles)

SS-OP was used as the ionic lipid, and POPE, Chol, and DMG-PEG2000 were used as different lipids. The molar ratio of SS-OP:POPE:Chol:DMG-PEG2000 used was 60:10:29:1. The lipids were mixed such that the total lipids in a lipid ethanol solution were 8.0 mM.

975 µL of a nucleic acid solution (nucleic acid concentration: 58.67 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Spectra/Por Float-A-Lyzer G2, and dialyzed in a beaker containing 400 mL of a Tris/maleate buffer (buffer component concentration: 50 mM, pH: 6.0) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 440 µL to obtain a suspension containing nucleic acid-encapsulating lipid nanoparticles (440 µL, nucleic acid concentration: 100 µg/mL) (step b)).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 200 µL, and frozen by cooling at -80°C for 24 hr to obtain a frozen composition (ionic lipid (1) content: 0.1 wt%, nucleic acid content: 10 µg, N/P ratio: 18, cryoprotective agent (sucrose) content: 7 wt%, water (ice) content: 92 wt%) (step d)).

After freezing, the frozen composition was stored at - 80°C, and before use, thawed by standing same in an atmospheric pressure environment in a refrigerator at 4°C for 30 min to obtain a suspension.

### Comparative Example 7 (Preparation of suspension containing nucleic acid-encapsulating lipid nanoparticles)

SS-OP was used as the ionic lipid, and POPE, Chol, and DMG-PEG2000 were used as different lipids. The molar ratio of SS-OP:POPE:Chol:DMG-PEG2000 used was 49:5:55:1. The lipids were mixed such that the total lipids in a lipid ethanol solution were 3.0 mM.

975 µL of a nucleic acid solution (nucleic acid concentration: 53.9 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 1000 µL of a Tris-buffered saline (buffer component concentration: 50 mM, pH: 7.6), and the obtained suspension was transferred to an Amicon Ultra 4, and ultrafiltrated under centrifugation conditions (25°C, 1000 g, 3 min) to concentrate same to about 500 µL. Thereafter, it was diluted with 3500 µL of a Tris-buffered saline (buffer component concentration: 50 mM, pH: 7.6). The following operation was performed twice to concentrate same to 880 µL to obtain a suspension containing nucleic acid-encapsulating lipid nanoparticles (nucleic acid concentration: 51 µg/mL, N/P ratio: 6) (step b)).

### Comparative Example 8 (Preparation of suspension containing nucleic acid-encapsulating lipid nanoparticles)

SS-OP was used as the ionic lipid, and POPE, Chol, and DMG-PEG2000 were used as different lipids. The molar ratio of SS-OP:POPE:Chol:DMG-PEG2000 used was 30:15:53:2. The lipids were mixed such that the total lipids in a lipid ethanol solution were 3.0 mM.

975 µL of a nucleic acid solution (nucleic acid concentration: 16.5 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 1000 µL of a Tris-buffered saline (buffer component concentration: 50 mM, pH: 7.6), and the obtained suspension was transferred to an Amicon Ultra 4, and ultrafiltrated under centrifugation conditions (25°C, 1000 g, 3 min) to concentrate same to about 500 µL. Thereafter, it was diluted with 3500 µL of a Tris-buffered saline (buffer component concentration: 50 mM, pH: 7.6). The following operation was performed twice to concentrate same to 250 µL to obtain a suspension containing nucleic acid-encapsulating lipid nanoparticles (nucleic acid concentration: 50 µg/mL, N/P ratio: 12) (step b)).

### Comparative Example 9 (Preparation of suspension containing nucleic acid-encapsulating lipid nanoparticles)

SS-OP was used as the ionic lipid, and POPE, Chol, and DMG-PEG2000 were used as different lipids. The molar ratio of SS-OP:POPE:Chol:DMG-PEG2000 used was 30:30:37:3. The lipids were mixed such that the total lipids in a lipid ethanol solution were 3.0 mM.

975 µL of a nucleic acid solution (nucleic acid concentration: 11.0 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 1000 µL of a Tris-buffered saline (buffer component concentration: 50 mM, pH: 7.6), and the obtained suspension was transferred to an Amicon Ultra 4, and ultrafiltrated under centrifugation conditions (25°C, 1000 g, 3 min) to concentrate same to about 500 µL. Thereafter, it was diluted with 3500 µL of a Tris-buffered saline (buffer component concentration: 50 mM, pH: 7.6). The following operation was performed twice to concentrate same to 165 µL to obtain a suspension containing nucleic acid-encapsulating lipid nanoparticles (nucleic acid concentration: 50 µg/mL, N/P ratio: 18) (step b)).

### Comparative Example 10 (Preparation of suspension containing nucleic acid-encapsulating lipid nanoparticles)

SS-OP was used as the ionic lipid, and POPE, Chol, and DMG-PEG2000 were used as different lipids. The molar ratio of SS-OP:POPE:Chol:DMG-PEG2000 used was 45:5:48:2. The lipids were mixed such that the total lipids in a lipid ethanol solution were 3.0 mM.

975 µL of a nucleic acid solution (nucleic acid concentration: 16.5 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 1000 µL of a Tris-buffered saline (buffer component concentration: 50 mM, pH: 7.6), and the obtained suspension was transferred to an Amicon Ultra 4, and ultrafiltrated under centrifugation conditions (25°C, 1000 g, 3 min) to concentrate same to about 500 µL. Thereafter, it was diluted with 3500 µL of a Tris-buffered saline (buffer component concentration: 50 mM, pH: 7.6). The following operation was performed twice to concentrate same to 250 µL to obtain a suspension containing nucleic acid-encapsulating lipid nanoparticles (nucleic acid concentration: 50 µg/mL, N/P ratio: 18) (step b)).

### Comparative Example 11 (Preparation of suspension containing nucleic acid-encapsulating lipid nanoparticles)

SS-OP was used as the ionic lipid, and POPE, Chol, and DMG-PEG2000 were used as different lipids. The molar ratio of SS-OP:POPE:Chol:DMG-PEG2000 used was 45:15:37:3. The lipids were mixed such that the total lipids in a lipid ethanol solution were 3.0 mM.

975 µL of a nucleic acid solution (nucleic acid concentration: 49.5 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 1000 µL of a Tris-buffered saline (buffer component concentration: 50 mM, pH: 7.6), and the obtained suspension was transferred to an Amicon Ultra 4, and ultrafiltrated under centrifugation conditions (25°C, 1000 g, 3 min) to concentrate same to about 500 µL. Thereafter, it was diluted with 3500 µL of a Tris-buffered saline (buffer component concentration: 50 mM, pH: 7.6). The following operation was performed twice to concentrate same to 750 µL to obtain a suspension containing nucleic acid-encapsulating lipid nanoparticles (nucleic acid concentration: 50 µg/mL, N/P ratio: 6) (step b)).

### Comparative Example 12 (Preparation of suspension containing nucleic acid-encapsulating lipid nanoparticles)

SS-OP was used as the ionic lipid, and POPE, Chol, and DMG-PEG2000 were used as different lipids. The molar ratio of SS-OP:POPE:Chol:DMG-PEG2000 used was 40:30:29:1. The lipids were mixed such that the total lipids in a lipid ethanol solution were 3.0 mM.

975 µL of a nucleic acid solution (nucleic acid concentration: 22.0 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 1000 µL of a Tris-buffered saline (buffer component concentration: 50 mM, pH: 7.6), and the obtained suspension was transferred to an Amicon Ultra 4, and ultrafiltrated under centrifugation conditions (25°C, 1000 g, 3 min) to concentrate same to about 500 µL. Thereafter, it was diluted with 3500 µL of a Tris-buffered saline (buffer component concentration: 50 mM, pH: 7.6). The following operation was performed twice to concentrate same to 430 µL to obtain a suspension containing nucleic acid-encapsulating lipid nanoparticles (nucleic acid concentration: 50 µg/mL, N/P ratio: 12) (step b)).

### Comparative Example 13 (Preparation of suspension containing nucleic acid-encapsulating lipid nanoparticles)

SS-OP was used as the ionic lipid, and POPE, Chol, and DMG-PEG2000 were used as different lipids. The molar ratio of SS-OP:POPE:Chol:DMG-PEG2000 used was 60:5:32:3. The lipids were mixed such that the total lipids in a lipid ethanol solution were 3.0 mM.

975 µL of a nucleic acid solution (nucleic acid concentration: 33.0 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 1000 µL of a Tris-buffered saline (buffer component concentration: 50 mM, pH: 7.6), and the obtained suspension was transferred to an Amicon Ultra 4, and ultrafiltrated under centrifugation conditions (25°C, 1000 g, 3 min) to concentrate same to about 500 µL. Thereafter, it was diluted with 3500 µL of a Tris-buffered saline (buffer component concentration: 50 mM, pH: 7.6). The following operation was performed twice to concentrate same to 500 µL to obtain a suspension containing nucleic acid-encapsulating lipid nanoparticles (nucleic acid concentration: 50 µg/mL, N/P ratio: 12) (step b)).

### Comparative Example 14 (Preparation of suspension containing nucleic acid-encapsulating lipid nanoparticles)

SS-OP was used as the ionic lipid, and POPE, Chol, and DMG-PEG2000 were used as different lipids. The molar ratio of SS-OP:POPE:Chol:DMG-PEG2000 used was 60:10:29:1. The lipids were mixed such that the total lipids in a lipid ethanol solution were 3.0 mM.

975 µL of a nucleic acid solution (nucleic acid concentration: 22.0 µg/mL) obtained by diluting FLuc mRNA with an acidic citrate buffer (buffer component concentration: 20 mM, pH: 5.0), and 325 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

To the obtained suspension was added 1000 µL of a Tris-buffered saline (buffer component concentration: 50 mM, pH: 7.6), and the obtained suspension was transferred to an Amicon Ultra 4, and ultrafiltrated under centrifugation conditions (25°C, 1000 g, 3 min) to concentrate same to about 500 µL. Thereafter, it was diluted with 3500 µL of a Tris-buffered saline (buffer component concentration: 50 mM, pH: 7.6). The following operation was performed twice to concentrate same to 330 µL to obtain a suspension containing nucleic acid-encapsulating lipid nanoparticles (nucleic acid concentration: 50 µg/mL, N/P ratio: 18) (step b)).

The particle size, Polydispersity Index (PdI), and mRNA encapsulation rate were analyzed for the nucleic acid-encapsulating lipid nanoparticles in the suspensions obtained in Examples 51 to 58, step c). The results are shown in the line of "step c)" in Table 12. Similarly, the particle size, PdI, and mRNA encapsulation rate were analyzed for the nucleic acid-encapsulating lipid nanoparticles in the suspensions obtained by thawing the frozen compositions of Examples 51 to 58, step d). The results are shown in the line of "after freezing and thawing " in Table 12. Similarly, the particle size, PdI, and mRNA encapsulation rate were analyzed for the nucleic acid-encapsulating lipid nanoparticles in the suspensions obtained in Comparative Examples 7 to 14. The particle size and PdI were measured by a dynamic light scattering method using ZetaSizer (registered trademark). The mRNA encapsulation rate (referred to as "encapsulation rate" in the table below) was measured by the Ribogreen (registered trademark) assay. The particle size is expressed in Z-Average size (referred to as "Z-ave" in the Table below).

**[Table 12]**

| | | encapsulation rate (%) | Z-ave (nm) | PdI |
|---|---|---|---|---|
| Example 51 | step c) | 93 | 79 | 0.14 |
| | after freezing and thawing | 87 | 83 | 0.16 |
| Example 52 | step c) | 90 | 75 | 0.17 |
| | after freezing and thawing | 86 | 82 | 0.19 |
| Example 53 | step c) | 90 | 71 | 0.15 |
| | after freezing and thawing | 80 | 75 | 0.16 |
| Example 54 | step c) | 88 | 80 | 0.12 |
| | after freezing and thawing | 87 | 81 | 0.16 |
| Example 55 | step c) | 88 | 70 | 0.09 |
| | after freezing and thawing | 84 | 79 | 0.12 |
| Example 56 | step c) | 84 | 90 | 0.13 |
| | after freezing and thawing | 85 | 89 | 0.18 |
| Example 57 | step c) | 90 | 70 | 0.1 |
| | after freezing and thawing | 87 | 78 | 0.13 |
| Example 58 | step c) | 95 | 85 | 0.14 |
| | after freezing and thawing | 90 | 85 | 0.17 |
| Comparative Example 7 | - | 92 | 81 | 0.16 |
| Comparative Example 8 | - | 91 | 73 | 0.18 |
| Comparative Example 9 | - | 95 | 70 | 0.18 |
| Comparative Example 10 | - | 86 | 79 | 0.15 |
| Comparative Example 11 | - | 90 | 72 | 0.12 |
| Comparative Example 12 | - | 88 | 87 | 0.15 |
| Comparative Example 13 | - | 86 | 74 | 0.11 |
| Comparative Example 14 | - | 93 | 85 | 0.17 |

As shown in Table 12, the nucleic acid-encapsulating lipid nanoparticles in the suspensions obtained by thawing the frozen compositions of Examples 54 to 58 all exhibited particle physical properties similar to those of the nucleic acid-encapsulating lipid nanoparticles in the suspensions of Comparative Examples 7 to 14, which corresponded to those of Examples 54 to 58, but were not frozen.

Each of the frozen compositions of Examples 52 and 54 was thawed and further diluted with a Tris-buffered saline (pH: 7.6) to obtain a suspension containing nucleic acid-encapsulating lipid nanoparticles (nucleic acid concentration: 1 µg/mL). In addition, each suspension of Comparative Examples 8 and 10 was diluted with a Tris-buffered saline (pH: 7.6) to obtain a suspension containing nucleic acid-encapsulating lipid nanoparticles (nucleic acid concentration: 1 µg/mL). The obtained nucleic acid-encapsulating lipid nanoparticles were applied to Hep G2 cell, and the gene expression activity thereof was evaluated. Specifically, Hep G2 cells were seeded in D-MEM medium (high glucose) (containing L-glutamine, phenol red) (10% by volume FBS, 1% by volume penicillin-streptomycin solution added) at 3×10⁴ cells/600 µL into the wells of a 24-well flat-bottom clear black plate. The obtained suspension containing nucleic acid-encapsulating lipid nanoparticles was added to the aforementioned well such that the nucleic acid concentration in the medium was 0.03 µg/30 µL. Luciferin was added to the aforementioned well such that the concentration in the medium was 0.1 mM, and the amount of luciferase protein introduced was evaluated over time using the amount of luminescence. Hep G2 cells cultured in the medium containing nucleic acid-encapsulating nanoparticles and luciferin were placed in an incubator-type luminometer (Kronos), and the cumulative amount of luminescence was measured every hour.

The gene expression activity of nucleic acid-encapsulating lipid nanoparticles in the suspension obtained in Example 52, step c), the gene expression activity of the nucleic acid-encapsulating lipid nanoparticles in the suspension obtained by thawing the frozen composition, and the gene expression activity of nucleic acid-encapsulating lipid nanoparticles in the suspension of Comparative Example 8 are shown in Fig. 15. In Fig. 15, the relative values with the gene expression activity (luciferase activity) of Comparative Example 8 as 1.

The gene expression activity of nucleic acid-encapsulating lipid nanoparticles in the suspension obtained in Example 54, step c), the gene expression activity of the nucleic acid-encapsulating lipid nanoparticles in the suspension obtained by thawing the frozen composition, and the gene expression activity of nucleic acid-encapsulating lipid nanoparticles in the suspension of Comparative Example 10 are shown in Fig. 16. In Fig. 16, the relative values with the gene expression activity (luciferase activity) of Comparative Example 10 as 1.

As shown in Figs. 15 and 16, the nucleic acid-encapsulating lipid nanoparticles in the suspensions obtained by thawing the frozen compositions of Examples 52 and 54 all exhibited gene expression activity equivalent to that of the nucleic acid-encapsulating lipid nanoparticles in the suspensions of Comparative Examples 8 and 10, which corresponded to those of Examples 52 and 54, but were not frozen. Therefrom it was shown that similar effects can be achieved even when frozen compositions are prepared with various lipid compositions or N/P ratios within the scope of the present invention.

### Example 59 (Preparation of frozen composition containing nucleic acid-encapsulating lipid nanoparticles)

SS-OP was used as the ionic lipid (1), and POPE, Chol, and DMG-PEG2000 were used as different lipids. The molar ratio of SS-OP:POPE:Chol:DMG-PEG2000 used was 49:15:34:2. The lipids were mixed such that the total lipids in a lipid ethanol solution were 8.0 mM.

2475 µL of a nucleic acid solution (nucleic acid concentration: 53.9 µg/mL) obtained by diluting FLuc mRNA with an acidic citrate buffer (buffer component concentration: 20 mM, pH: 5.0), and 825 µL of the lipid ethanol solution were each measured into syringes. Using NanoAssemblr (registered trademark) ultra-fast nano pharmaceutical production device, a suspension containing nucleic acid-encapsulating lipid nanoparticles was prepared under the conditions of a nucleic acid solution:lipid ethanol solution flow rate ratio of 3:1 and a total flow rate of 12 mL/min (step a)).

The obtained suspension was transferred to Slide-A-Lyzer G3 Dialysis Cassettes, and dialyzed in a beaker containing 600 mL of a Tris/maleate/sucrose buffer (buffer component concentration: 50 mM, pH: 6.0, sucrose concentration: 10 mg/mL) in a chamber at 4°C for 3 hr. After 3 hr, the buffer was exchanged with a new one, and dialysis was performed again in the chamber at 4°C for 3 hr. Thereafter, the buffer was exchanged again with a new one, and dialysis was performed in the chamber at 4°C for 16 hr. The obtained suspension was transferred to an Amicon Ultra 4, and ultrafiltrated under centrifugation conditions (25°C, 1000 g, 3 min) to concentrate same to about 200 µL to obtain a suspension containing nucleic acid-encapsulating lipid nanoparticles (200 µL, nucleic acid concentration: 606.38 µg/mL) (step b')).

To the obtained suspension was added a Tris/maleate/sucrose buffer (buffer component concentration: 50 mM, pH: 6.0, sucrose concentration: 160 mg/mL) such that the final concentration of sucrose was 80 mg/mL (step c)). The obtained suspension was placed in a screw tube by 100 µL, and frozen by cooling at -80°C for 24 hr to obtain a frozen composition (ionic lipid (1) content: 1.6 wt%, nucleic acid content: 61 µg, N/P ratio: 16, cryoprotective agent (sucrose) content: 7 wt%, water (ice) content: 90 wt%) (step d)).

After freezing, the frozen composition was stored at - 80°C, and before use, thawed by standing same in an atmospheric pressure environment in a refrigerator at 4°C for 30 min to obtain a suspension.

The particle size, Polydispersity Index (PdI), and mRNA encapsulation rate were analyzed for the nucleic acid-encapsulating lipid nanoparticles in the suspension obtained in Example 59, step c). The results are shown in the line of "step c)" in Table 13. Similarly, the particle size, PdI, and mRNA encapsulation rate were analyzed for the nucleic acid-encapsulating lipid nanoparticles in the suspension obtained by thawing the frozen composition obtained in Example 59, step d). The results are shown in the line of "after freezing and thawing " in Table 13. The mRNA encapsulation rate (referred to as "encapsulation rate" in the table below) was measured by the Ribogreen (registered trademark) assay. The particle size is expressed in Z-Average size (referred to as "Z-ave" in the Table below).

**[Table 13]**

| | | encapsulation rate (%) | Z-ave (nm) | PdI |
|---|---|---|---|---|
| Example 59 | step c) | 95 | 73 | 0.13 |
| | after freezing and thawing | 92 | 73 | 0.09 |

As shown in Table 13, the frozen composition of Example 59, produced by a method including step a), step b'), step c), and step d), showed no marked changes in the physical properties of the nucleic acid-encapsulating lipid nanoparticles even after freezing and thawing.

### [Industrial Applicability]

The frozen composition containing nucleic acid-encapsulating lipid nanoparticles of the present invention is useful for nucleic acid medicament, gene therapy, and biochemical experiment.

This application is based on a patent application No. 2024-003329 filed in Japan, the contents of which are encompassed in full herein.

## Claims

1. A frozen composition comprising nucleic acid-encapsulating lipid nanoparticles, wherein
the nucleic acid-encapsulating lipid nanoparticles comprise an ionic lipid represented by the formula (1): 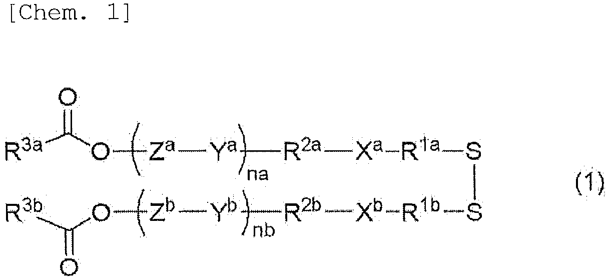 wherein, in the formula (1), R^{1a} and R^{1b} are each independently an alkylene group having 1 to 6 carbon atoms,
X^{a} and X^{b} are each independently an acyclic alkyl tertiary amino group having 1 to 6 carbon atoms and 1 tertiary amino group, or a cyclic alkylene tertiary amino group having 2 to 5 carbon atoms and 1 to 2 tertiary amino groups,
R^{2a} and R^{2b} are each independently an alkylene group having 1 to 8 carbon atoms or an oxydialkylene group having 2 to 8 carbon atoms,
Y^{a} and Y^{b} are each independently an ester bond, an amide bond, a carbamate bond, an ether bond, or a urea bond,
Z^{a} and Z^{b} are each independently a divalent group derived from an aromatic compound having 3 to 16 carbon atoms and at least one aromatic ring, and optionally having a hetero atom,
na and nb are each independently 0 or 1, and
R^{3a} and R^{3b} are each independently
(i) a monovalent group having 10 to 50 carbon atoms, one carbonyl group, and at least one unsaturated bond selected from the group consisting of an olefinic carbon-carbon double bond and a carbon-carbon triple bond (excluding a monovalent group containing a residue of a liposoluble vitamin having a hydroxy group and a residue of a sterol derivative having a hydroxy group),
(ii) a monovalent group having 10 to 50 carbon atoms and at least two carbonyl groups (excluding a monovalent group containing a residue of a liposoluble vitamin having a hydroxy group and a residue of a sterol derivative having a hydroxy group),
(iii) a monovalent group represented by the formula (2):
*-R⁴-X¹-R⁵ (2)
wherein, in the formula (2), * is a bonding position,
R⁴ is an alkylene group having 1 to 10 carbon atoms,
X¹ is a carbamate bond, a carbonate bond, or an amide bond, and
R⁵ is an alkyl group having 1 to 25 carbon atoms, and R⁵ is optionally substituted by a substituent selected from the group consisting of a halogen atom and a hydroxy group,
(iv) a monovalent group represented by the formula (3):
*-R⁶-CO-O-R⁷ (3)
wherein, in the formula (3), * is a bonding position,
R⁶ is an alkylene group having 1 to 10 carbon atoms, and
R⁷ is an alkyl group having 1 to 25 carbon atoms and substituted by at least one halogen atom,
(v) a monovalent group represented by the formula (4): 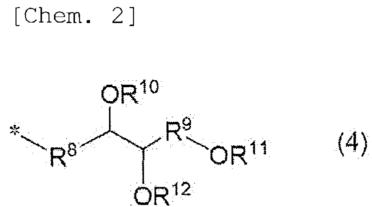 wherein, in the formula (4), * is a bonding position,
R⁸ and R⁹ are each independently an alkylene group having 1 to 10 carbon atoms, an alkenediyl group having 2 to 10 carbon atoms, or an alkynediyl group having 2 to 10 carbon atoms,
R¹⁰ to R¹² are each independently a hydrogen atom, a benzyl group, or a *-Si(R¹³)(R¹⁴)(R¹⁵) group (wherein * is a bonding position, and R¹³ to R¹⁵ are each independently an alkyl group having 1 to 4 carbon atoms or a phenyl group),
(vi) a monovalent group represented by the formula (5): 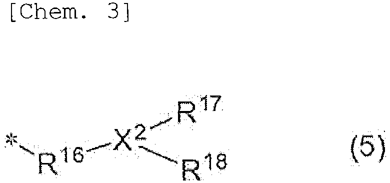
wherein, in the formula (5), * is a bonding position,
X² is a nitrogen atom or a trivalent group represented by the formula (6): 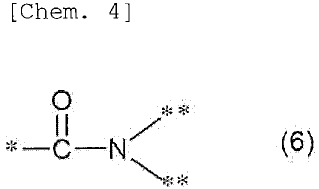
wherein, in the formula (6), * is a bonding position with R¹⁶, and
** is a bonding position with R¹⁷ or R¹⁸,
when X² is a nitrogen atom, R¹⁶ is an alkylene group having 1 to 10 carbon atoms, an alkenediyl group having 2 to 10 carbon atoms, or an alkynediyl group having 2 to 10 carbon atoms, and R¹⁶ is optionally substituted by a substituent selected from the group consisting of a halogen atom and a hydroxy group,
when X² is a trivalent group represented by the formula (6), R¹⁶ is an alkylene group having 1 to 10 carbon atoms, and R¹⁶ is optionally substituted by a substituent selected from the group consisting of a halogen atom and a hydroxy group,
when X² is a nitrogen atom, R¹⁷ and R¹⁸ are each independently an alkyl group having 1 to 10 carbon atoms, an alkenyl group having 2 to 10 carbon atoms, or an alkynyl group having 2 to 10 carbon atoms, and R¹⁷ and R¹⁸ are each independently optionally substituted by a substituent selected from the group consisting of a halogen atom and a hydroxy group, and
when X² is a trivalent group represented by the formula (6), R¹⁷ and R¹⁸ are each independently an alkyl group having 1 to 10 carbon atoms, and R¹⁷ and R¹⁸ are each independently optionally substituted by a substituent selected from the group consisting of a halogen atom and a hydroxy group,
(vii) a monovalent group represented by the formula (7): 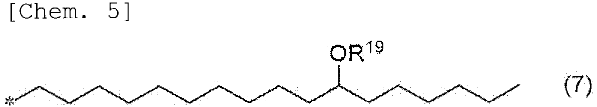 wherein, in the formula (7), * is a bonding position, and
R¹⁹ is a hydrogen atom, a benzyl group, or a *-Si (R¹³)(R¹⁴)(R¹⁵) group (wherein * is a bonding position, and R¹³ to R¹⁵ are each independently an alkyl group having 1 to 4 carbon atoms or a phenyl group), or a *-CO-R²⁰ group (wherein * is a bonding position, and R²⁰ is an alkyl group having 1 to 9 carbon atoms),
(viii) a monovalent group represented by the formula (8): 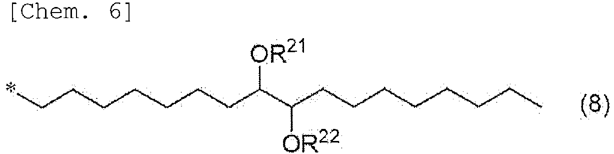 wherein, in the formula (8), * is a bonding position, and
R²¹ and R²² are each independently a hydrogen atom, a benzyl group, or a *-Si (R¹³)(R¹⁴)(R¹⁵) group (wherein * is a bonding position, and R¹³ to R¹⁵ are each independently an alkyl group having 1 to 4 carbon atoms or a phenyl group),
(ix) a monovalent group represented by the formula (9): 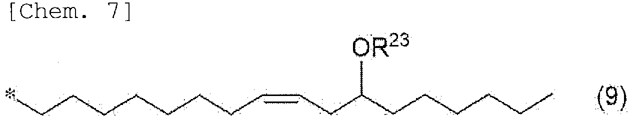 wherein, in the formula (9), * is a bonding position, and
R²³ is a hydrogen atom, a benzyl group, or a *-Si(R¹³)(R¹⁴)(R¹⁵) group (wherein * is a bonding position, and R¹³ to R¹⁵ are each independently an alkyl group having 1 to 4 carbon atoms or a phenyl group),
(x) a monovalent group represented by the formula (10): 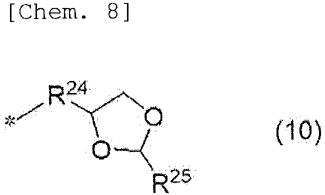 wherein, in the formula (10), * is a bonding position,
R²⁴ is an alkylene group having 1 to 10 carbon atoms, an alkenediyl group having 2 to 10 carbon atoms, or an alkynediyl group having 2 to 10 carbon atoms, and
R²⁵ is an alkyl group having 1 to 30 carbon atoms, an alkenyl group having 2 to 30 carbon atoms, or an alkynyl group having 2 to 30 carbon atoms,
(xi) a monovalent group represented by the formula (11): 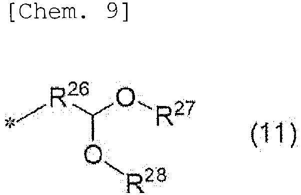
wherein, in the formula (11), * is a bonding position, and
R²⁶ is an alkylene group having 1 to 10 carbon atoms, an alkenediyl group having 2 to 10 carbon atoms, or an alkynediyl group having 2 to 10 carbon atoms, and
R²⁷ and R²⁸ are each independently an alkyl group having 1 to 10 carbon atoms, an alkenyl group having 2 to 10 carbon atoms, or an alkynyl group having 2 to 10 carbon atoms, or
(xii) a monovalent group represented by the formula (12): 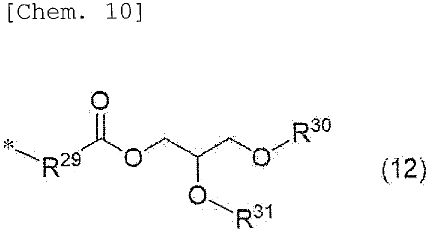 wherein, in the formula (12), * is a bonding position,
R²⁹ is an alkylene group having 1 to 10 carbon atoms, and
R³⁰ and R³¹ are each independently an alkyl group having 1 to 10 carbon atoms,
(xiii) a monovalent group which is an alkyl group having 1 to 30 carbon atoms, an alkenyl group having 2 to 20 carbon atoms, or an alkynyl group having 2 to 20 carbon atoms, in which one ethylene group in the alkyl group is optionally replaced by one ester bond, or
(xiv) an R^{3c}-CO-(CH₂)ₚ- group (wherein R^{3c} is a residue of a liposoluble vitamin having a hydroxy group or a residue of a sterol derivative having a hydroxy group, and p is an integer of 1 to 8), and
R^{3a} and R^{3b} may be the same or different, and
a molar ratio of amino groups of the ionic lipid represented by the formula (1)/phosphate groups of the nucleic acid is 7 or more and 250 or less.

2. The frozen composition according to claim 1, wherein R^{3a} and R^{3b} are each independently
(i) a monovalent group having 10 to 50 carbon atoms, one carbonyl group, and at least one unsaturated bond selected from the group consisting of an olefinic carbon-carbon double bond and a carbon-carbon triple bond (excluding a monovalent group containing a residue of a liposoluble vitamin having a hydroxy group and a residue of a sterol derivative having a hydroxy group),
(ii) a monovalent group having 10 to 50 carbon atoms and at least two carbonyl groups (excluding a monovalent group containing a residue of a liposoluble vitamin having a hydroxy group and a residue of a sterol derivative having a hydroxy group),
(iii) a monovalent group represented by the formula (2),
(vii) a monovalent group represented by the formula (7),
(ix) a monovalent group represented by the formula (9),
(xiii) a monovalent group which is an alkyl group having 1 to 30 carbon atoms, an alkenyl group having 2 to 20 carbon atoms, or an alkynyl group having 2 to 20 carbon atoms, in which one ethylene group in the alkyl group is optionally replaced by one ester bond, or
(xiv) an R^{3c}-Co-(CH₂)ₚ- group (wherein R^{3c} is a residue of a liposoluble vitamin having a hydroxy group or a residue of a sterol derivative having a hydroxy group, and p is an integer of 1 to 8).

3. The frozen composition according to claim 1, wherein the nucleic acid is RNA or DNA.

4. The frozen composition according to claim 1, wherein the nucleic acid is mRNA.

5. The frozen composition according to claim 1, wherein nucleic acid-encapsulating lipid nanoparticles in a suspension obtained by thawing the frozen composition have a particle size of 10 nm or more and 200 nm or less.

6. The frozen composition according to claim 1, wherein nucleic acid-encapsulating lipid nanoparticles in a suspension obtained by thawing the frozen composition have a PdI of 0.01 or more and 0.30 or less.

7. The frozen composition according to claim 1, wherein nucleic acid-encapsulating lipid nanoparticles in a suspension obtained by thawing the frozen composition have a nucleic acid encapsulation rate of 75% or more and 100% or less.

8. A method for producing a frozen composition comprising nucleic acid-encapsulating lipid nanoparticles, wherein
the nucleic acid-encapsulating lipid nanoparticles comprise an ionic lipid represented by the formula (1): 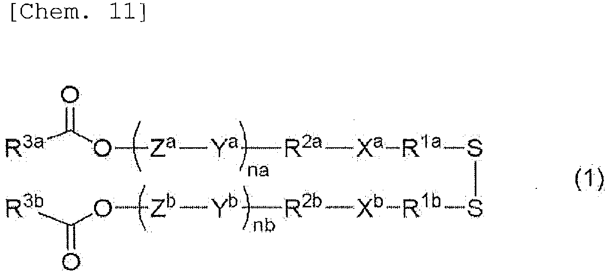 wherein, in the formula (1), R^{1a} and R^{1b} are each independently an alkylene group having 1 to 6 carbon atoms,
X^{a} and X^{b} are each independently an acyclic alkyl tertiary amino group having 1 to 6 carbon atoms and 1 tertiary amino group, or a cyclic alkylene tertiary amino group having 2 to 5 carbon atoms and 1 to 2 tertiary amino groups,
R^{2a} and R^{2b} are each independently an alkylene group having 1 to 8 carbon atoms or an oxydialkylene group having 2 to 8 carbon atoms,
Y^{a} and Y^{b} are each independently an ester bond, an amide bond, a carbamate bond, an ether bond, or a urea bond,
Z^{a} and Z^{b} are each independently a divalent group derived from an aromatic compound having 3 to 16 carbon atoms and at least one aromatic ring, and optionally having a hetero atom,
na and nb are each independently 0 or 1, and
R^{3a} and R^{3b} are each independently
(i) a monovalent group having 10 to 50 carbon atoms, one carbonyl group, and at least one unsaturated bond selected from the group consisting of an olefinic carbon-carbon double bond and a carbon-carbon triple bond (excluding a monovalent group containing a residue of a liposoluble vitamin having a hydroxy group and a residue of a sterol derivative having a hydroxy group),
(ii) a monovalent group having 10 to 50 carbon atoms and at least two carbonyl groups (excluding a monovalent group containing a residue of a liposoluble vitamin having a hydroxy group and a residue of a sterol derivative having a hydroxy group),
(iii) a monovalent group represented by the formula (2):
*-R⁴-X¹-R⁵ (2)
wherein, in the formula (2), * is a bonding position,
R⁴ is an alkylene group having 1 to 10 carbon atoms,
X¹ is a carbamate bond, a carbonate bond, or an amide bond, and
R⁵ is an alkyl group having 1 to 25 carbon atoms, and R⁵ is optionally substituted by a substituent selected from the group consisting of a halogen atom and a hydroxy group,
(iv) a monovalent group represented by the formula (3):
*-R⁶-CO-O-R⁷ (3)
wherein, in the formula (3), * is a bonding position,
R⁶ is an alkylene group having 1 to 10 carbon atoms, and
R⁷ is an alkyl group having 1 to 25 carbon atoms and substituted by at least one halogen atom,
(v) a monovalent group represented by the formula (4): 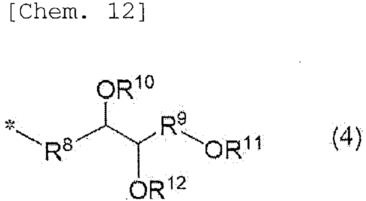 wherein, in the formula (4), * is a bonding position,
R⁸ and R⁹ are each independently an alkylene group having 1 to 10 carbon atoms, an alkenediyl group having 2 to 10 carbon atoms, or an alkynediyl group having 2 to 10 carbon atoms,
R¹⁰ to R¹² are each independently a hydrogen atom, a benzyl group, or a *-Si(R¹³)(R¹⁴)(R¹⁵) group (wherein * is a bonding position, and R¹³ to R¹⁵ are each independently an alkyl group having 1 to 4 carbon atoms or a phenyl group),
(vi) a monovalent group represented by the formula (5): 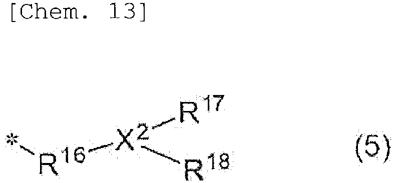
wherein, in the formula (5), * is a bonding position,
X² is a nitrogen atom or a trivalent group represented by the formula (6): 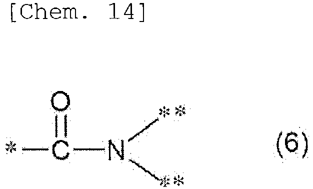
wherein, in the formula (6), * is a bonding position with R¹⁶, and
** is a bonding position with R¹⁷ or R¹⁸,
when X² is a nitrogen atom, R¹⁶ is an alkylene group having 1 to 10 carbon atoms, an alkenediyl group having 2 to 10 carbon atoms, or an alkynediyl group having 2 to 10 carbon atoms, and R¹⁶ is optionally substituted by a substituent selected from the group consisting of a halogen atom and a hydroxy group,
when X² is a trivalent group represented by the formula (6), R¹⁶ is an alkylene group having 1 to 10 carbon atoms, and R¹⁶ is optionally substituted by a substituent selected from the group consisting of a halogen atom and a hydroxy group,
when X² is a nitrogen atom, R¹⁷ and R¹⁸ are each independently an alkyl group having 1 to 10 carbon atoms, an alkenyl group having 2 to 10 carbon atoms, or an alkynyl group having 2 to 10 carbon atoms, and R¹⁷ and R¹⁸ are each independently optionally substituted by a substituent selected from the group consisting of a halogen atom and a hydroxy group, and
when X² is a trivalent group represented by the formula (6), R¹⁷ and R¹⁸ are each independently an alkyl group having 1 to 10 carbon atoms, and R¹⁷ and R¹⁸ are each independently optionally substituted by a substituent selected from the group consisting of a halogen atom and a hydroxy group,
(vii) a monovalent group represented by the formula (7): 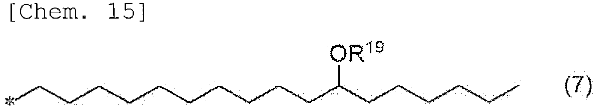 wherein, in the formula (7), * is a bonding position, and
R¹⁹ is a hydrogen atom, a benzyl group, or a *-Si(R¹³)(R¹⁴)(R¹⁵) group (wherein * is a bonding position, and R¹³ to R¹⁵ are each independently an alkyl group having 1 to 4 carbon atoms or a phenyl group), or a *-CO-R²⁰ group (wherein * is a bonding position, and R²⁰ is an alkyl group having 1 to 9 carbon atoms),
(viii) a monovalent group represented by the formula (8): 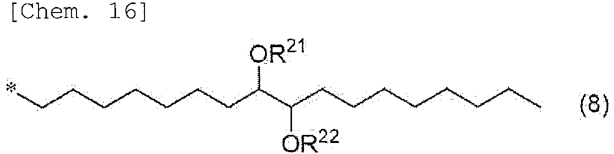 wherein, in the formula (8), * is a bonding position, and
R²¹ and R²² are each independently a hydrogen atom, a benzyl group, or a *-Si(R¹³)(R¹⁴)(R¹⁵) group (wherein * is a bonding position, and R¹³ to R¹⁵ are each independently an alkyl group having 1 to 4 carbon atoms or a phenyl group),
(ix) a monovalent group represented by the formula (9): 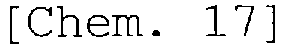 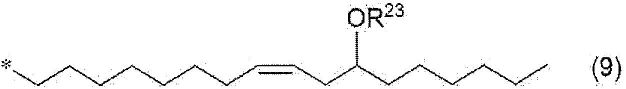 wherein, in the formula (9), * is a bonding position, and
R²³ is a hydrogen atom, a benzyl group, or a *-Si(R¹³)(R¹⁴)(R¹⁵) group (wherein * is a bonding position, and R¹³ to R¹⁵ are each independently an alkyl group having 1 to 4 carbon atoms or a phenyl group),
(x) a monovalent group represented by the formula (10): 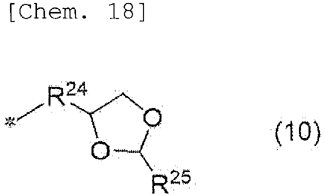 wherein, in the formula (10), * is a bonding position,
R²⁴ is an alkylene group having 1 to 10 carbon atoms, an alkenediyl group having 2 to 10 carbon atoms, or an alkynediyl group having 2 to 10 carbon atoms, and
R²⁵ is an alkyl group having 1 to 30 carbon atoms, an alkenyl group having 2 to 30 carbon atoms, or an alkynyl group having 2 to 30 carbon atoms,
(xi) a monovalent group represented by the formula (11): 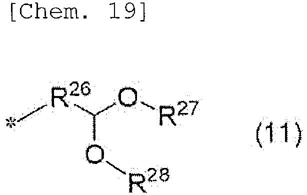
wherein, in the formula (11), * is a bonding position, and
R²⁶ is an alkylene group having 1 to 10 carbon atoms, an alkenediyl group having 2 to 10 carbon atoms, or an alkynediyl group having 2 to 10 carbon atoms, and
R²⁷ and R²⁸ are each independently an alkyl group having 1 to 10 carbon atoms, an alkenyl group having 2 to 10 carbon atoms, or an alkynyl group having 2 to 10 carbon atoms, or
(xii) a monovalent group represented by the formula (12): 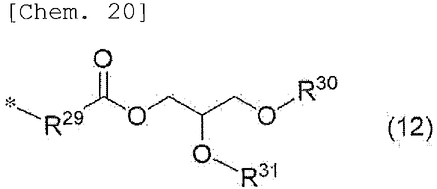 wherein, in the formula (12), * is a bonding position,
R²⁹ is an alkylene group having 1 to 10 carbon atoms, and
R³⁰ and R³¹ are each independently an alkyl group having 1 to 10 carbon atoms,
(xiii) a monovalent group which is an alkyl group having 1 to 30 carbon atoms, an alkenyl group having 2 to 20 carbon atoms, or an alkynyl group having 2 to 20 carbon atoms, in which one ethylene group in the alkyl group is optionally replaced by one ester bond, or
(xiv) an R^{3c}-CO-(CH₂)ₚ- group (wherein R^{3c} is a residue of a liposoluble vitamin having a hydroxy group or a residue of a sterol derivative having a hydroxy group, and p is an integer of 1 to 8), and
R^{3a} and R^{3b} may be the same or different, and
a molar ratio of amino groups of the ionic lipid represented by the formula (1)/phosphate groups of the nucleic acid is 7 or more and 250 or less, and
the method comprises
the following step a), step b), step c), and step d),
the following step a), step b'), and step d), or
the following step a), step b'), step c), and step d):
a) a step of mixing an alcohol solution containing the ionic lipid represented by the formula (1), a sterol, and a PEG lipid, and a nucleic acid solution containing an acidic buffer with a pH of 1.0 to 6.5 as a solvent to prepare a suspension containing nucleic acid-encapsulating lipid nanoparticles,
b) a step of exchanging a dispersion medium of the suspension obtained in step a) with a buffer with a pH of 4.5 to 8.0, which is other than the acidic buffer used in step a), to obtain a suspension containing nucleic acid-encapsulating lipid nanoparticles,
b') a step of exchanging a dispersion medium of the suspension obtained in step a) with a buffer with a pH of 4.5 to 8.0, which is other than the acidic buffer used in step a), and containing a cryoprotective agent to obtain a suspension containing nucleic acid-encapsulating lipid nanoparticles and the cryoprotective agent,
c) a step of mixing the suspension obtained in step b) or step b') and a cryoprotective agent to obtain a suspension containing nucleic acid-encapsulating lipid nanoparticles and the cryoprotective agent,
d) a step of freezing the suspension obtained in step b') or step c) at -10°C or less.

9. The method according to claim 8, wherein the method comprises step a), step b), step c), and step d), or step a), step b'), and step d), and wherein in step c), the suspension obtained in step b) and a cryoprotective agent is mixed to obtain a suspension containing nucleic acid-encapsulating lipid nanoparticles and the cryoprotective agent.

10. The method according to claim 8, wherein a ratio of (a particle size of nucleic acid-encapsulating lipid nanoparticles in a suspension obtained by thawing the frozen composition obtained in step d))/(a particle size of the nucleic acid-encapsulating lipid nanoparticles in the suspension obtained in step b') or step c)) is 0.8 or more and 1.5 or less.

11. The method according to claim 8, wherein nucleic acid-encapsulating lipid nanoparticles in a suspension obtained by thawing the frozen composition obtained in step d) have a PdI of 0.01 or more and 0.25 or less.

12. The method according to claim 8, wherein a ratio of (a nucleic acid encapsulation rate of nucleic acid-encapsulating lipid nanoparticles in a suspension obtained by thawing the frozen composition obtained in step d))/(a nucleic acid encapsulation rate of the nucleic acid-encapsulating lipid nanoparticles in the suspension obtained in step b') or step c)) is 0.8 or more and 1.0 or less.

13. A method for producing a pharmaceutical product composition, comprising the method according to any one of claims 8 to 12.

14. A method for introducing a nucleic acid in a suspension into a cell, comprising a step of contacting in vitro the cell with a suspension obtained by thawing the frozen composition according to any one of claims 1 to 7 or a frozen composition produced by the method according to any one of claims 8 to 12, or a suspension obtained by exchanging a dispersion medium of the suspension with a different dispersion medium.

15. A method for introducing a nucleic acid in a suspension into a biological target cell of a living organism, comprising a step of administering, to the living organism, a suspension obtained by thawing the frozen composition according to any one of claims 1 to 7 or a frozen composition produced by the method according to any one of claims 8 to 12, or a suspension obtained by exchanging a dispersion medium of the suspension with a different dispersion medium.
